# EUROPEAN PATENT SPECIFICATION

(11) **EP 0 914 165 B1**
(45) Date of publication and mention of the grant of the patent: **18.10.2006**
(21) Application number: 97937019.4
(22) Date of filing: 23.07.1997
(51) Int. Cl.: A61K 31/57, A61P 15/00

(54) **STEROIDS AS NEUROCHEMICAL STIMULATORS OF THE VNO TO ALLEVIATE SYMPTOMS OF PMS**
STEROIDE ALS NEUROCHEMISCHE STIMULATOREN DES NASENRAUMES ZUR LINDERUNG DER PRÄMENSTRUALEN SYMPTOME
STEROIDES UTILISES EN QUALITE DE STIMULATEURS NEUROCHIMIQUES DE L'ORGANE VOMERO-NASAL ET PERMETTANT DE SOULAGER LES SYMPTOMES PREMENSTRUEL

(30) Priority: 23.07.1996 US 686092; 04.10.1996 US 725862
(43) Date of publication of application: 12.05.1999
(73) Proprietor: Pherin Pharmaceuticals, Inc., Menlo Park, CA 94025-3444 (US)
(72) Inventor: JENNINGS-WHITE, Clive, L., Salt Lake City, UT 84109 (US); BERLINER, David, L., Atherton, CA 94027 (US); ADAMS, Nathan, W., Salt Lake City, UT 84123 (US); BLOCH-MONTI, Luis, Salt Lake City, UT 84108 (US)
(74) Representative: Barz, Peter
(86) International application number: PCT/US1997/013035
(87) International publication number: WO 1998/003207

(56) References cited:
- WO-A-94/28903
- WO-A-94/28904
- WO-A-96/04296
- WO-A-96/10031
- WO-A-96/10032
- WO-A-96/40727
- WO-A-97/46574
- US-A- 5 208 227

## Description

### Technical Field

This invention relates generally to methods for alleviating symptoms of PMS.

### Description of the Related Art

The present invention relates to certain steroids, and methods of using these steroids as human vomeropherins in order to alter hypothalamic function, thereby affecting certain consequent behavior and physiology, e.g., the reduction of anxiety. Ohloff, G. et al. (Helv. Chim. Acta (1983) 66:192-217) have shown that several steroids (androstenes) have an odor which varies with different isomeric, diastereomeric, and enantiomeric forms. Some members of this group have been reported to act as a pheromone in some mammalian species - for instance, 5α-androst-16-en-3-one and 5α-androst-16-en-3α-ol in pigs (Melrose, D.R., et al., Br. vet. J. (1971) 127:497-502). These 16-androstenes produced by the boar induce mating behavior in estrus sows (Claus, et al., Experimentia (1979) 35:1674-1675).

Some studies have noted that, in some species, various characteristics of certain 16-androstenes (including 5α-Androst-16-en-3α-ol and 5α-Androst-16-en-3-one), such as concentration, metabolism, and localization, are sexually dimorphic (Brooksbank et al., J. Endocr. (1972) 52: 239-251; Claus, et al., J. Endocr. (1976) 68:483-484; Kwan, et al., Med. Sci. Res. (1987) 15:1443-1444). For instance, 5α-Androst-16-en-3α-ol and 5α-Androst-16-en-3-one, as well as Androsta-4,16-dien-3-one, have been found at different concentrations in the peripheral blood, saliva and axillary secretions of men and of women (Kwan, T.K., et al., Med. Sci. Res. (1987) 15:1443-1444), and their function as a human pheromone, to the extent of affecting choice and judgment, has been suggested (Id.; see also Gower, et al., "The Significance of Odorous Steroids in Axillary Odour", In, Perfumery, pp. 68-72, Van Toller and Dodds, Eds., Chapman and Hall, 1988); Kirk-Smith, D.A., et al., Res. Comm. Psychol. Psychiat. Behav. (1978) 3:379). Androstenol (5α-androst-16-en-3α-ol) has been claimed to exhibit a pheromone-like activity in a commercial men's cologne and women's perfume (Andron^{™} for men and Andron^{™} for women by Jövan). Japanese Kokai No. 2295916, refers to perfume compositions containing androstenol and/or its analogues. Androstadien-3β-ol (and perhaps the 3α-ol) has also been identified in human axillary secretion (Gower, et al., supra, at 57-60). On the other hand, there is little agreement in the literature as to whether or not any putative pheromone actually plays any role in the sexual or reproductive behavior of mammals, particularly of humans. See: Beauchamp, G.K., et al., "The Pheromone Concept in Mammalian Chemical Communication: A Critique", In: Mammalian Olfaction, Reproductive Processes and Behavior, Doty, R.L., Ed., Academic Press, 1976). See also: Gower, et al., supra at 68-73.

The pheromone properties of some estrene steroids for some mammalian species has been described. Michael, R.P. et al., Nature (1968) 218:746 refers to Estrogens (particularly Estradiol) as a pheromonal attractant of male rhesus monkeys. Parrot, R.F., Hormones and Behavior (1976) 7:207-215, reports Estradiol benzoate injection induces mating behavior in ovariectomized rats; and the role of the blood level of Estradiol in make sexual response (Phoenix, C.H., Physiol. and Behavior (1976) 16305-310) and female sexual response (Phoenix, C.H., Hormones and Behavior (1977) 8:356-362) in Rhesus monkeys has been described. On the other hand, there is little agreement in the literature as to whether or not pheromones as such play any role in the reproductive behavior and interpersonal communication of mammals (Beuchamp, G.K., et al., The Pheromone Concept in Mammalian Chemical Communication: A Critique', In: Mammalian Olfaction, Reproductive Processes, and Behavior, Doty R.L., Ed., Academic Press, 1976).

The subject invention concerns the non-systemic administration to the vomeronasal organ (VNO) of certain steroids to alleviate symptoms of PMS and anxiety, as well as for treatment of elevated body temperature and paroxistic tachycardia (high pulse rate). Administration provides for contacting neurochemical receptors in the VNO (also known as "Jacobson's organ"), with one or more steroid(s) or with compositions containing the steroid(s). This organ is accessed through the nostrils of most higher animals - from snakes to humans, and has been associated, inter alia, with pheromone reception in certain species (see generally Muller-Schwarze & Silverstein, Chemical Signals, Plenum Press, New York (1980)). The axons of the neuroepithelia of the vomeronasal organ, located supra palatinal, form the vomeronasal nerve and have direct synaptic connection to the accessory olfactory bulb and indirect input from there to the cortico-medial amygdaloid basal forebrain and hypothalamic nuclei of the brain. The distal axons of terminalis nerve neurons may also serve as neurochemical receptors in the VNO. Stensaas, L.J., et al., J. Steroid Biochem. and Molec. Biol. (1991) 39:553. This nerve has direct synaptic connection with the hypothalamus.

Johnson, A. et al. (J. Otolaryngology (1985) 14:71-79) report evidence for the presence of the vomeronasal organ in most adult humans, but conclude that the organ is probably non-functional. Contravening results which suggest that the VNO is a functional chemosensory receptor are reported by Stensaas, L., et al., supra; and by Moran, D.T., et al., Garcia-Velasco, J. and M. Mondragon; Monti-Bloch, L. and B. Grosser all in J. Steroid Biochem. and Molec. Biol. (1991) 39.

This invention relates to the unexpected discovery that, when administered into the VNO of human subjects, certain neurochemical ligands, particularly steroids, and related compounds, specifically bind to chemoreceptors of certain nasal neuroepithelial cells and this binding generates a series of neurophysiological responses resulting in alleviating symptoms of PMS and anxiety. An effect via the VNO on the hypothalamus can affect the function of the autonomic nervous system and a variety of behavioral-or physiological phenomena which include, but are not limited to the following: anxiety, premenstrual stress, fear, aggression, hunger, blood pressure, and other behavioral and physiological functions normally regulated by the hypothalamus. See Otto Appenzeller, The Autonomic Nervous System. An Introduction of basic and clinical concepts (1990); Korner, P.I. Central nervous control of autonomic cardiovascular function, and Levy, N.M. and Martin, P.J. Neural control of the heart, both in Handbook of Physiology; Section 2; Cardiovascular System - the heart, Vol. I, Washington DC, 1979, American Physiological Society; Fishman, A.P., et al. editors, Handbook of Physiology. Section 3: Respiratory System. Vol. II. Control of breaching, Bethesda MD, 1986. American Physiological Society.

Patients diagnosed with premenstrual dysphoric disorder (PMDD, commonly referred as premenstrual syndrome or PMS), show negative changes in mood together with physical symptoms, during the luteal phase of the menstrual cycle (see Table I). The symptoms are severe enough to cause physical or emotional distress, and they remit shortly after the onset of menstruation. However, they are not unique or diagnostic for this disease. Several surveys show that 30% of women have classical premenstrual dysphoric disorder symptoms, 2% to 10% being severely affected (Woods et al., Am. J. Pub. Health, 72:1257-62, 1982; Van Keep et al., "The Premenstrual Syndrome - An Epidemiologic and Statistical Exercise", Van Keep, P.A. and Utian, W.H., Eds., The Premenstrual Syndrome, MTP Press Ltd., Lancaster, England, 1981; and Andersch et al., J. Psychomsom. Obstet. Gyn. 5:39-46, 1986). It is also reported that PMDD is not a disease of just older women since teenagers and young women have symptomatology as frequently as older women (Rivera-Tovar et al., Am. J. Psychiatry, 147:1634-43, 1990).

Many patients that seek treatment for PMDD may be found to suffer from a generalized psychiatric disorder. However, the main feature distinguishing PMDD from chronic psychiatric disorders is the presence of at least one asymptomatic week in the follicular phase of every menstrual cycle. A list of the common symptoms of this syndrome is presented in Table I. Most women report a variety of emotional, physical and behavioral changes associated with the luteal phase of the menstrual cycle. But only those women that report cyclic luteal phase symptoms that remit during menstruation, and that significantly affect their level of psychological or occupational functioning have PMDD.

**Table I.**

| | | |
|---|---|---|
| Table I. Common PMDD symptoms | | |

| Emotional | Physical | Behavioral |
|---|---|---|
| Anxiety | Headache | Food cravings |
| Irritability | Migraines | Increased appetite |
| Labile moods | Breast tenderness | Increased alcohol intake |
| Depression | Swelling of extremities | Decreased motivation |
| Anger | Bloatedness | Decreased efficiency |
| Sadness | Fatigue | Avoid activities |
| Crying easily | Abdominal cramps | Staying at home |
| Nervous tension | Aches and pain | Sleep changes |
| overly sensitive | Weight gain | Changes in libido |
| | Skin problems | Reduced cognitive function |
| | Hot flashes | Social isolation |
| | Gastrointestinal | Poor concentration |
| | Dizziness | Forgetfulness |
| | Palpitation | |

In recent studies it was found that picogram quantities of some vomeropherins locally delivered in vapor form to the VNO of normal women (20 to 60 years old), produce significant behavioral and autonomic changes. Monti-Bloch et al., J. Ster. Biochem. Molec. Biol., 39 (4B):573-582 (1991); Monti-Bloch et al., Psychoneuroendocrinology, 19:673-686 (1994). Subjects show mood changes characterized by decreased negativity. There is a clear decrease in negative aspect and negative character, and the patients feel relaxed. These changes correlate with increased parasympathetic tone in all individuals (mild bradypnea and bradycardia, small but significant increased body temperature [0-7°C + 0.5], change in skin conductance, and also increased alpha-cortical activity). Also, noticeable is the latency of these effects that correlates well with the latency of polysynaptic reflexes. The conclusion is that those vomeropherins stimulated receptors in the vomeronasal organ that in turn influenced the hypothalamus via the vomeronasal-terminalis system.

The present invention also provides attenuation of symptoms of general anxiety, which are characterized by the Hamilton-A standard.

### Summary of the Invention

The method of the invention has the following advantages: 1) administration directly to the chemoreceptors in the nasal passage and the vomeronasal organ, without pills or needles - i.e., non-invasively; 2) a mode of drug action through the invention system and not through the circulatory system - thus brain function can be affected without consideration of the bloodbrain barrier; 3) a direct means of affecting the hypothalamus - there is only one synaptic function between pheromone receptors and the hypothalamus; and, 4) providing a highly specific drug effect, thereby greatly reducing the potential for undesirable side-effects - this because sensory nerves are addressed to a specific location in the brain.

Additional objects, advantages and novel features of the invention will be set forth in part in the description which follows, and in part will become apparent to those skilled in the art upon examination of the following, or may be learned by practice of the invention.

Compositions concain steroids with the formula: 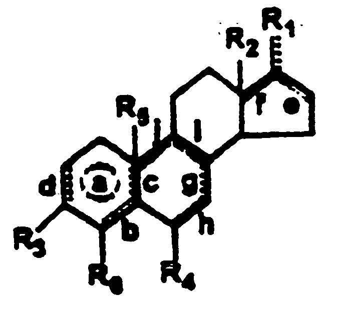 wherein R₁ is selected from the group consisting essentially of one or two hydrogen atoms, methyl, methylene, and one or two halo atoms; R₂ is absent or is selected from the group consisting essentially of hydrogen and methyl; R₃ is selected from the group consisting essentially of oxo, hydroxy, lower alkoxy, lower acyloxy, benzoyl, cypionyl, glucuronide and sulfonyl; R₄ is selected from the group consisting essentially of hydrogen, hydroxy, lower alkoxy, lower acyloxy, oxo and halo; R₅ is absent or is selected from the group consisting essentially of hydrogen, hydroxy, lower alkoxy and lower acyloxy; R₅ is a hydrogen or a halo; and "a" represents optional aromatic unsaturation of ring A of said steroid, or "b", "c", and "d" are each optional double bonds; "e", "f", "g", "h", "i" and "j" are each optional double bonds; and "e" forms an expoxy ring with C₁₆ and C₁₇. In this embodiment, the steroid is preferably administered in the form of a pharmaceutical composition containing one or more pharmaceutically acceptable carriers.

A preferred subset of the third class of steroids are those in which "a" is present and "g", "h" or "i" are optional double bonds. Another preferred class contains "b", "c" or "j" as a double bond. Yet another class contains "c" and "d" as double bonds. Still another class contains R₂ as methyl and "e" as a double bond.

The term lower alkyl, lower alkoxy, etc., encompasses carbon chains of 1 to 6 carbon atoms, preferably 1 to 4 carbon atoms. Halo includes I, Br, F and Cl.

A class of novel steroids is also provided which have oxygen atoms attached to the D-ring. Such steroids have the formula: 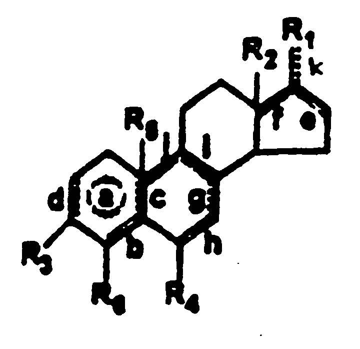 wherein R₁ is selected from the group consisting essentially of one or two hydrogen atoms, alkyl of 1 to 10 carbon atoms; R₂ is absent or is selected from the group consisting essentially of hydrogen, methyl and oxygen to form an epoxy ring with C₁₇; R₃ is selected from the group consisting essentially of oxo, hydroxy, lower alkoxy, lower acyloxy, benzoyl, cypionyl, glucuronide and sulfonyl; R₄ is selected from the group consisting essentially of hydrogen, hydroxy, lower alkoxy, lower acyloxy and halo; R₅ is absent or is selected from the group consisting essentially of hydrogen, hydroxy, lower alkoxy and lower acyloxy; R₆ is a hydrogen or a halo; and "a" represents optional aromatic unsaturation of ring A of said steroid, or "b", "c", and "d" are each optional double bonds; "g", "h", "i" and "j" are each optional double bonds; and "e" forms an epoxy ring with C₁₆ and C₁₇.

Other objects of this invention are achieved by providing a method of altering hypothalamic function and/or autonomic function in an individual. A ligand for a chemoreceptor displayed on the surface of a nasal neuroepithelial cell is provided wherein the cell is a part of tissue other than olfactory epithelia; and, the ligand is administered within a nasal passage of the individual such that the ligand binds specifically to the chemoreceptor, resulting in an alteration of hypothalamic function of the individual.

All embodiments of this application relate to and include the functional equivalents of the steroid structures disclosed in these embodiments and to those modified steroids which demonstrate said functional equivalence, whether or not the modified steroids are explicitly disclosed.

### Brief Description of the Drawings

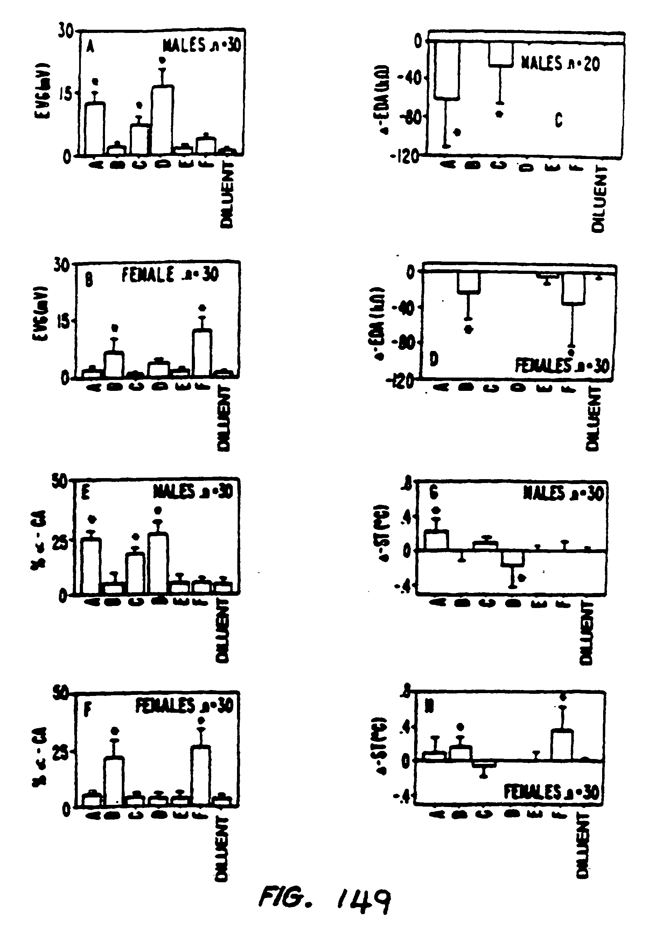

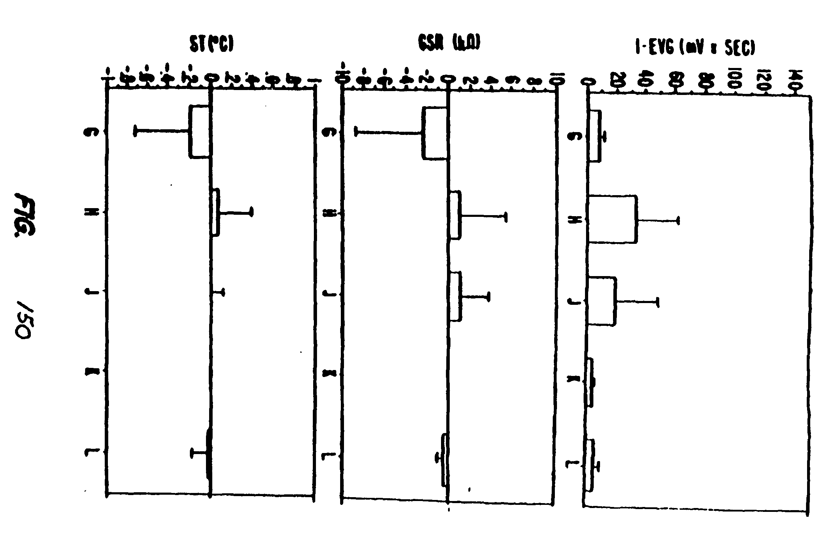

FIG. 1 is the data on the integrated EVG, GSR and ST for compound A1-P1 in males as tested according to Examples 16 and 17.

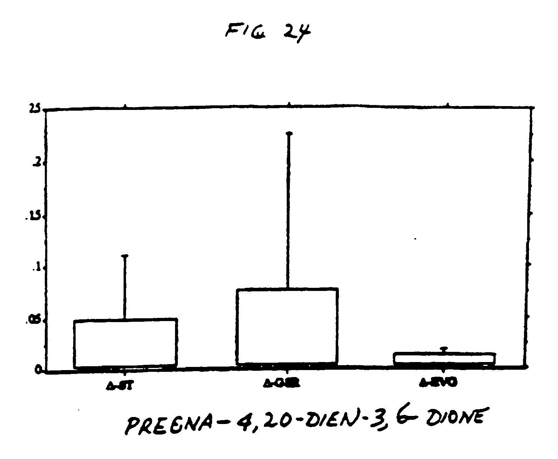

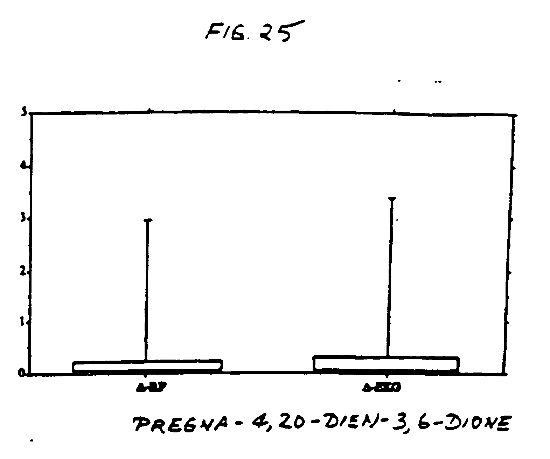

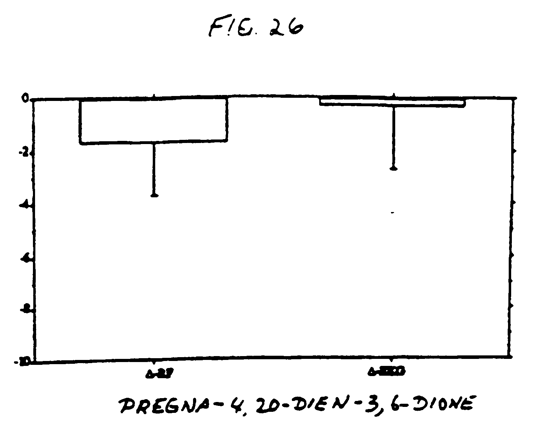

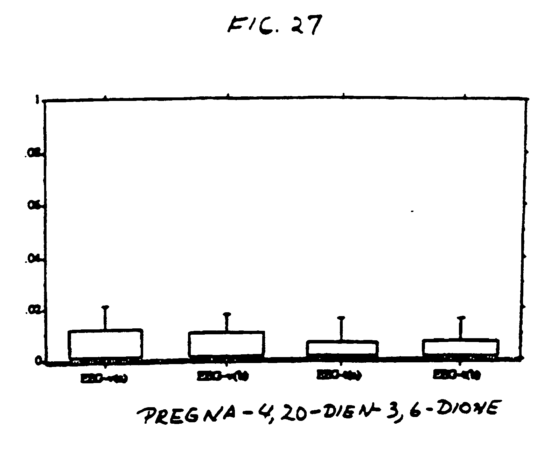

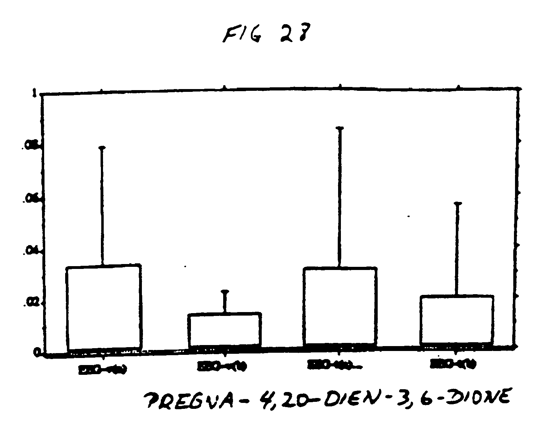

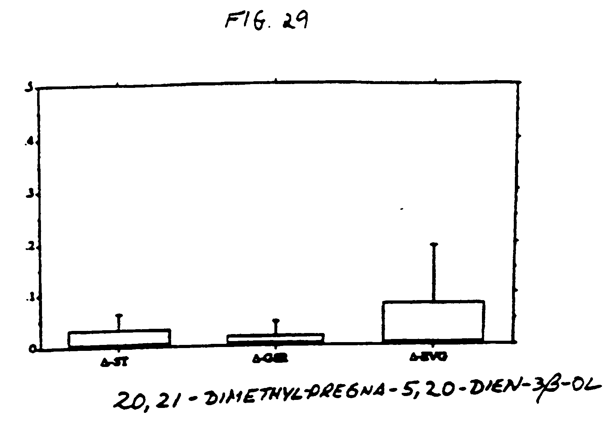

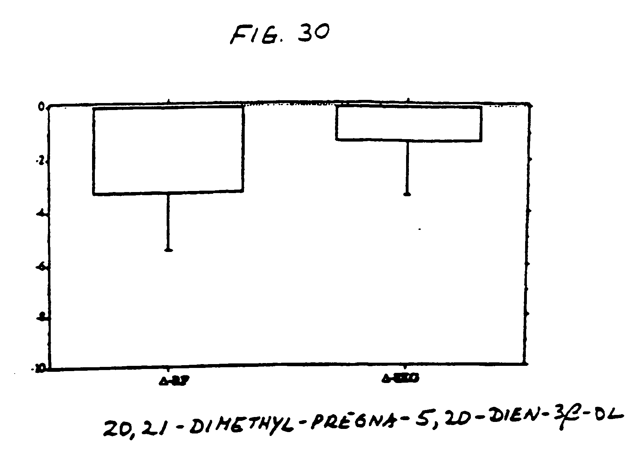

FIG. 2 is the data for the integrated EVG for compounds A1-P1, A2-P1, A4-P1, A3-P1, A1-P4, A2-P4 in females.

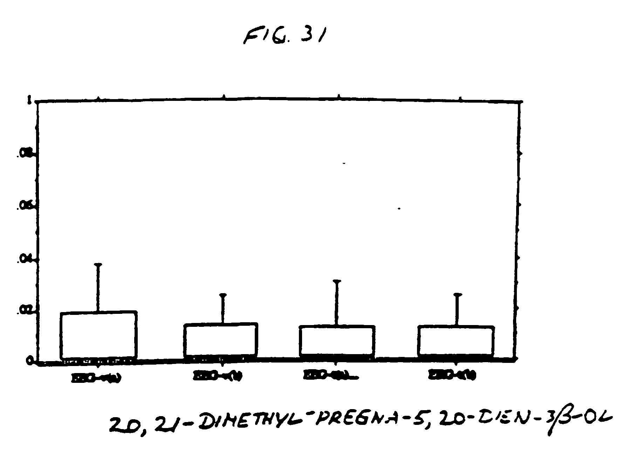

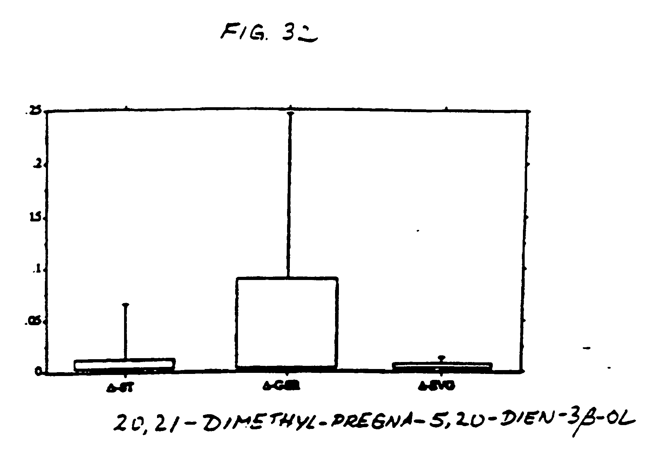

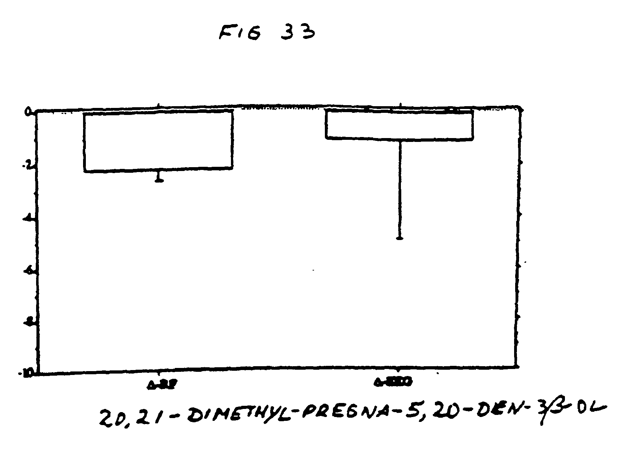

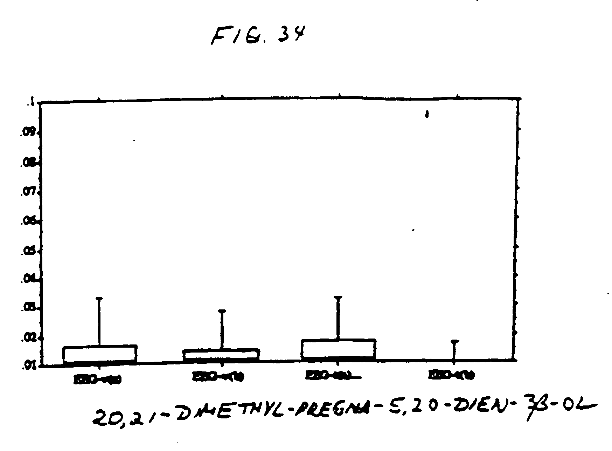

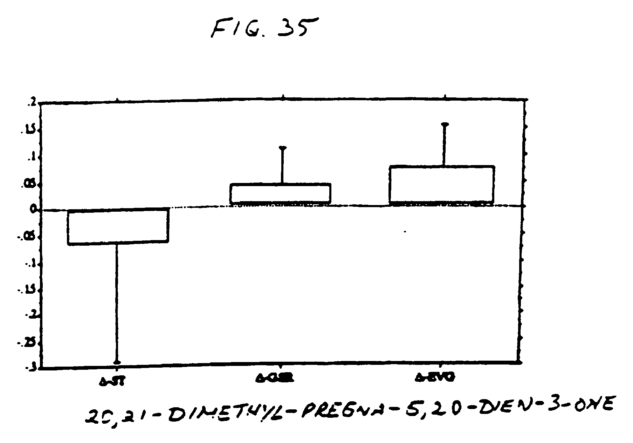

FIG. 3 is the data for the ST measurements of compounds A1-P1, A2-P1, A4-P1, A3-P1, A1-P4, A2-P4 in females.

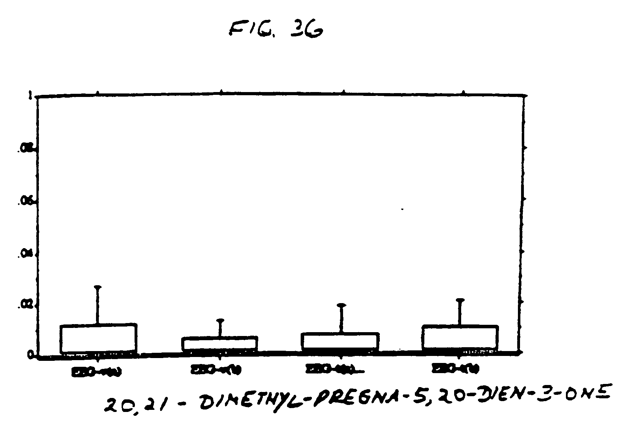

FIG. 4 is the data for the GSR measurements in females of compounds A1-P1, A2-P1, A4-P1, A3-P1, A1-P4, A2-P4.
FIG. 5 is the data for ST, GSR and EVG measurements in females of compound A1-P3.
FIG. 6 is the data for RF and EKG measurements in females of compound A1-P3.
FIG. 7 is the data for EEG measurements in females of the compound A1-P3.
FIG. 8 is the data for the ST, GSR and EVG measurements in males of compound A1-P3.
FIG. 9 is the data for the RF and EKG measurements in males of compound A1-P3.
FIG. 10 is the data for the EEG measurements in males of compound A1-P3.
FIGS. 11 and 12 show the data of the ST, GSR and EVG measurements in males and females, respectively, for compound A2-P3.
FIGS. 13 and 14 show the data of the EEG measurements in males and females, respectively, for compound A2-P3.
FIGS. 15 and 16 show the data for the RF and EKG measurements in males and females, respectively, for compound A2-P3.
FIGS. 17 and 18 show the data of the ST, GSR and EVG measurements in males and females, respectively, for compound A8-P1.
FIGS. 19 and 20 show the data of the RF and EKG measurements in males and females, respectively, for compound A8-P1.
FIGS- 21 and 22 show the data for EEG measurements in males and females, respectively, for compound A8-P1.
FIGS. 23 and 24 show the data for ST, GSR and EVG measurements in males and females, respectively, for compounds A6-P1.
FIGS. 25 and 26 show the data for RF and EKG measurements in males and females, respectively, for compound A6-P1.

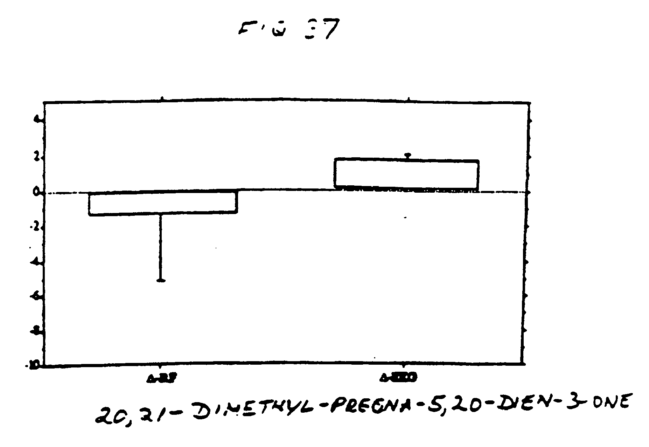

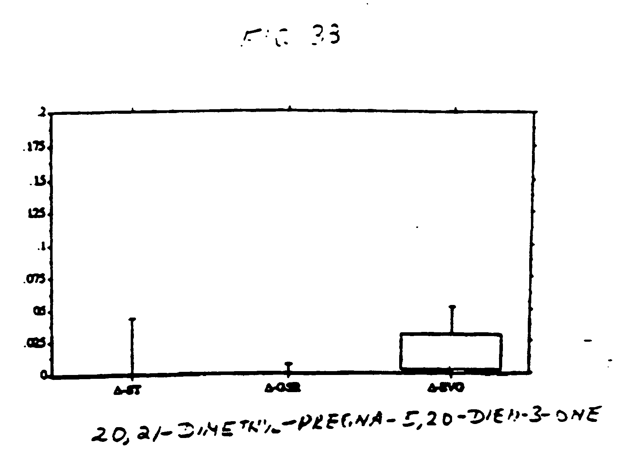

FIGS. 27 and 28 show the data for EEG measurements in males and females, respectively, for compound A6-P1.

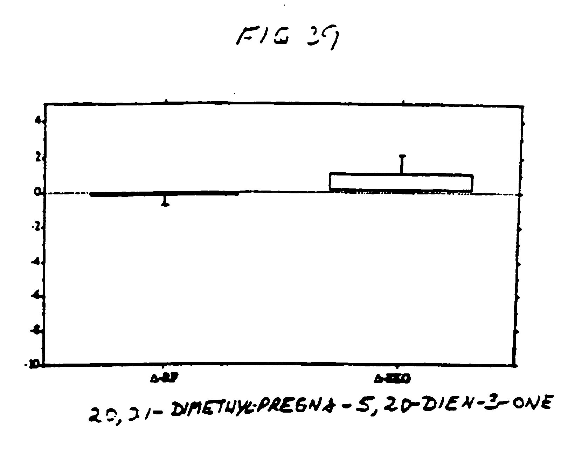

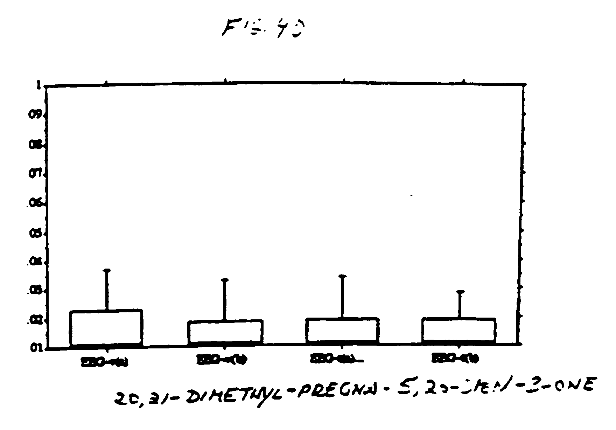

FIGS. 29, 30 and 31 show the data for ST, GSR, EVG, RF EKG and EEG measurements in males of 20,21-dimethylpregna-5,20-dien-3β-ol.

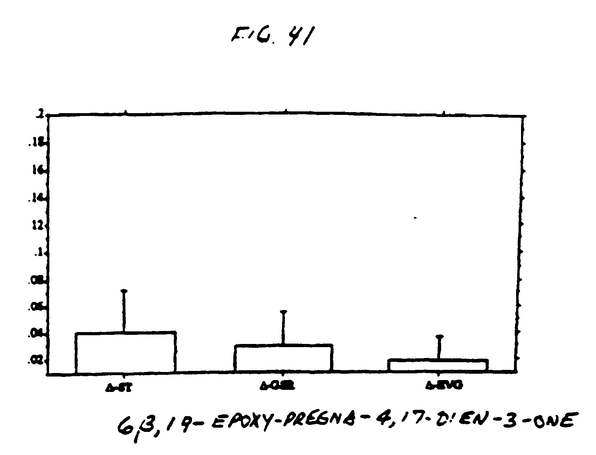

FIGS. 32, 33 and 34 show the data for the ST, GSR, EVG, RF KEG and EEG measurements in females of 20,21-dimethylpregna-5,20-dien-30-ol.

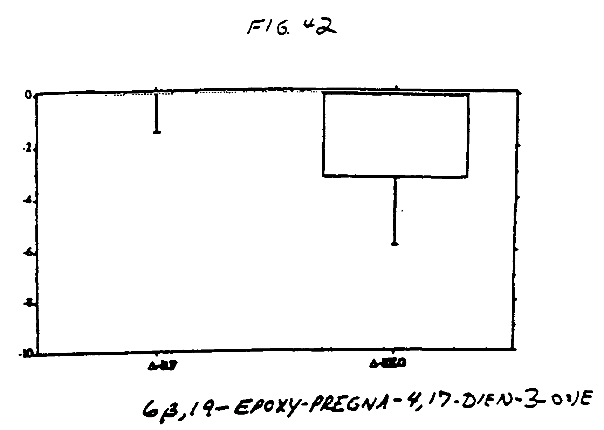

FIGS. 35, 36 and 37 show the data for the ST, GSR, EVG, RF KEG and EEG measurements in males of 20,21-dimethylpregna-5,20-dien-3-one.

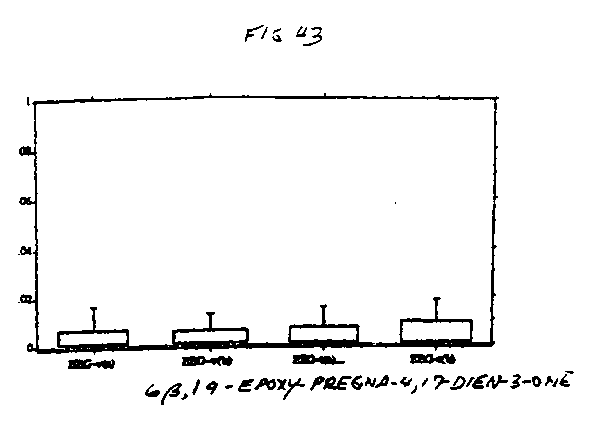

FIGS. 38, 39 and 40 show the ST, GSR, EVG, RF EKG and EEG measurements in females of 20,21-dimethylpregna-5,20-dien-3-one.

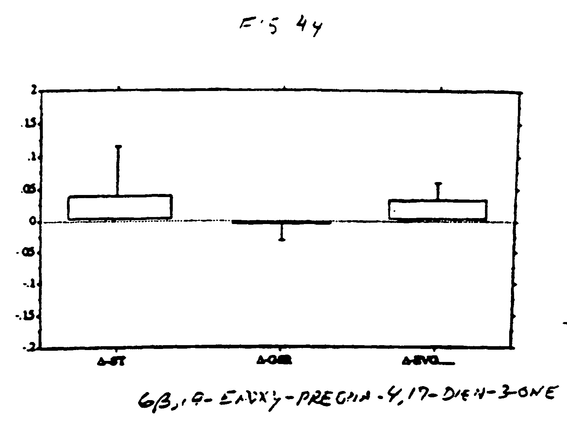

FIGS. 41, 42 and 43 show the ST, GSR, EVG, RF EKG and EEG measurements in males of compound A14-P2.

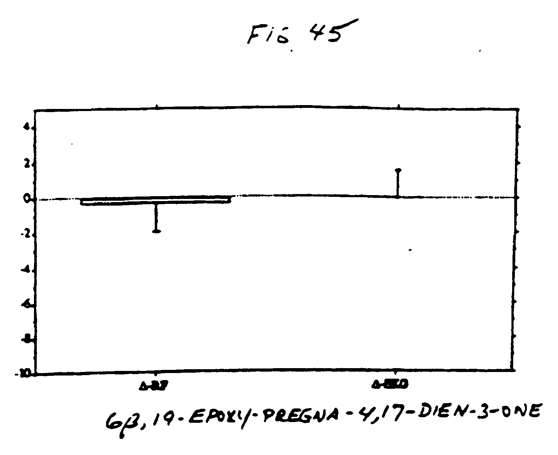

FIGS. 44, 45 and 46 show the ST, GSR, EVG, RF, EKG and EEG measurements in females of compound A14-P2.

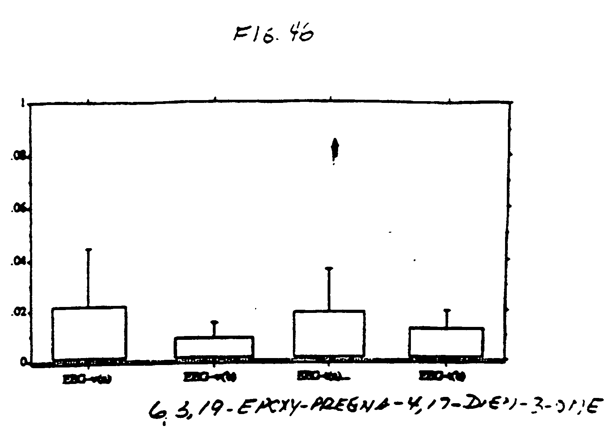

FIGS. 47, 48 and 49 show the ST, GSR, EVG, RF, EKG and EEG measurements in males of compound A7-P2.

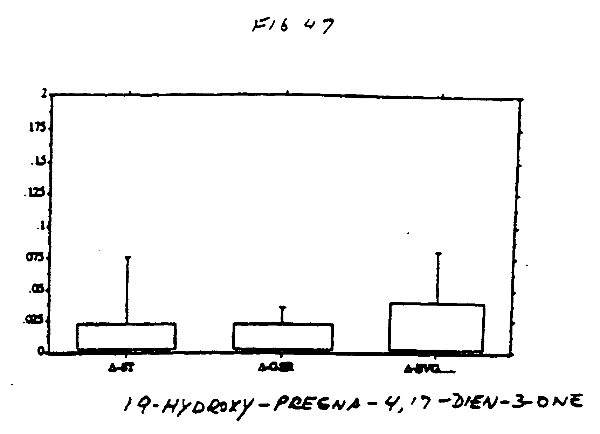

FIGS. 50, 51 and 52 show the ST, GSR, EVG, RF, EKG and EEG measurements in females of compound A7-P2.

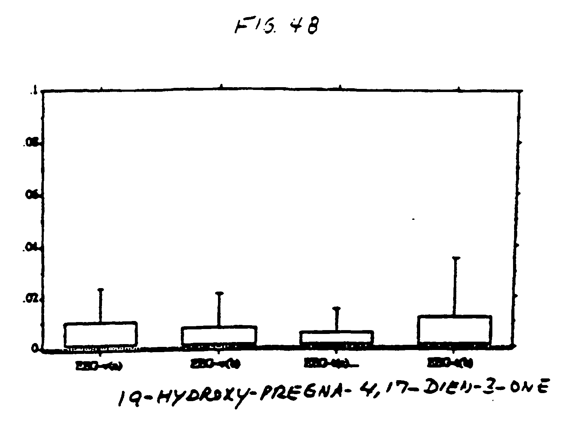

FIGS. 53 and 54 show the ST, GSR, EVG, EEG measurements in males of compound All-P1.
FIGS. 55 shows the data of EEG measurements in males of compound A13-P1.

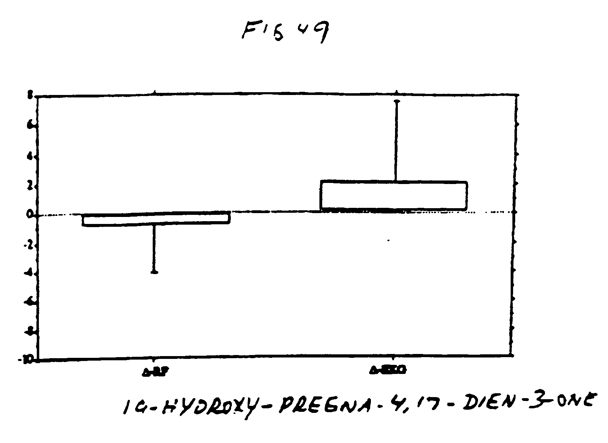

FIGS. 56, 57 and 58 show the data of measurements of ST, GSR, EVG, RF, EKG and EEG in females of compound A13-P1.

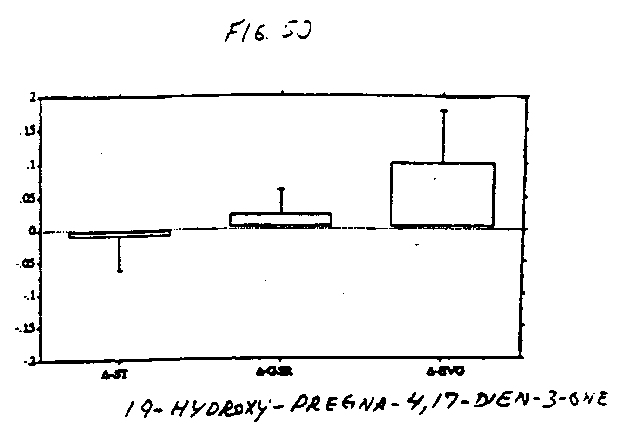

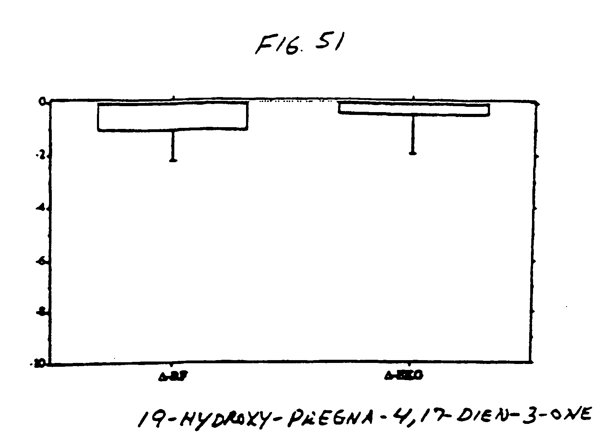

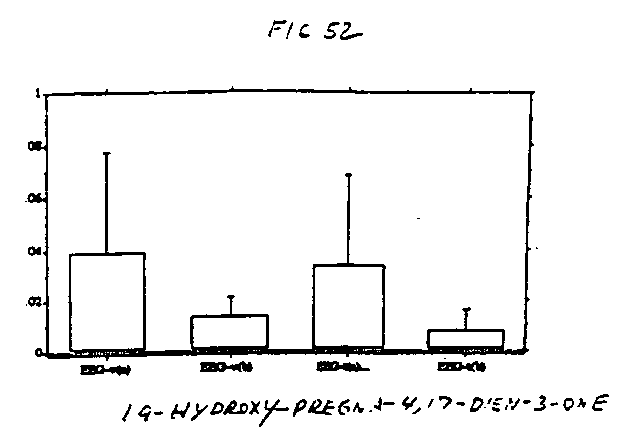

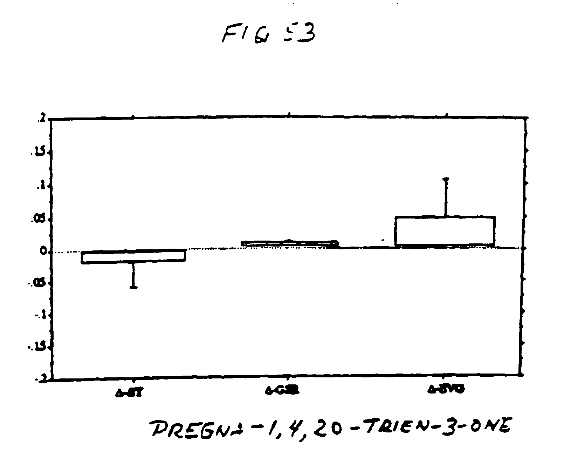

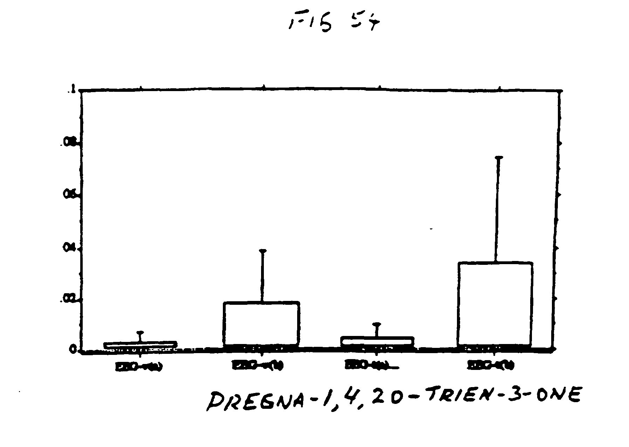

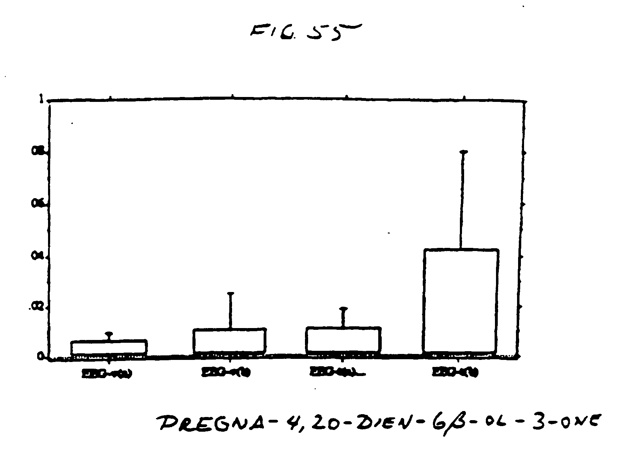

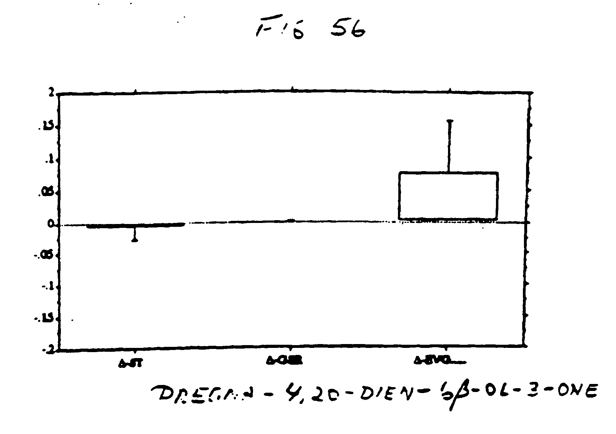

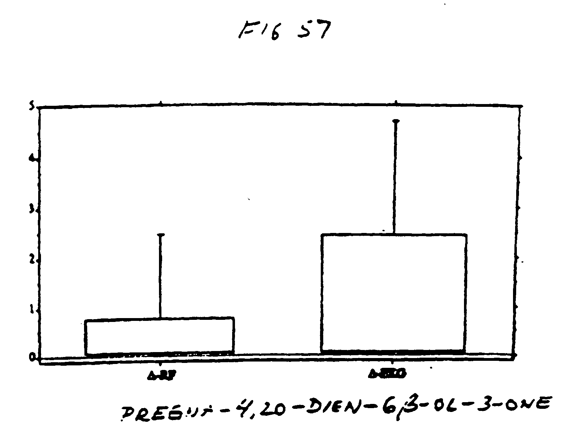

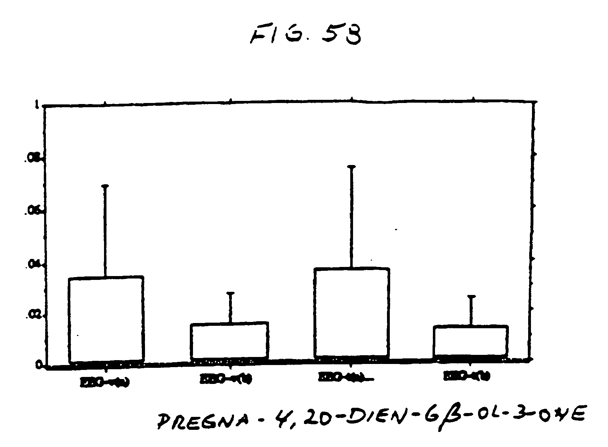

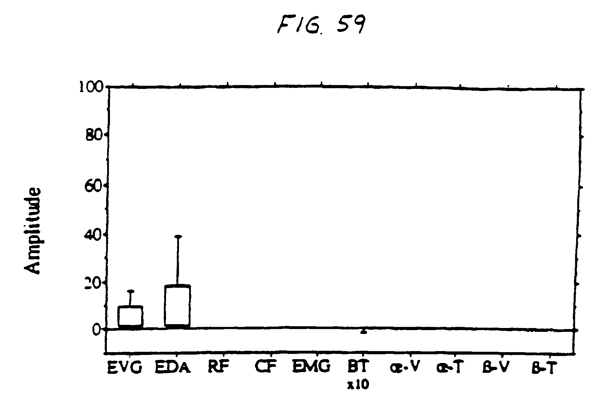

FIGS. 59 shows EVG, EDA and BT data of measurements in women of compound A3/P1.

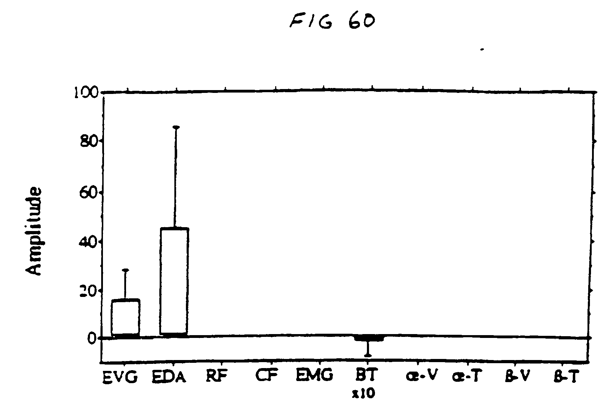

FIG. 60 shows EVG, EDA and BT data of measurements in women of compound A4/P1.

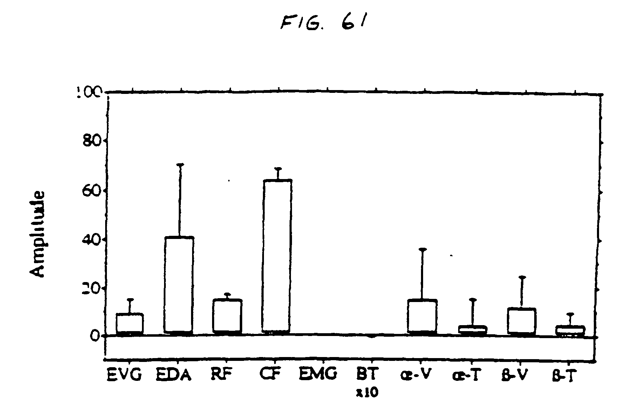

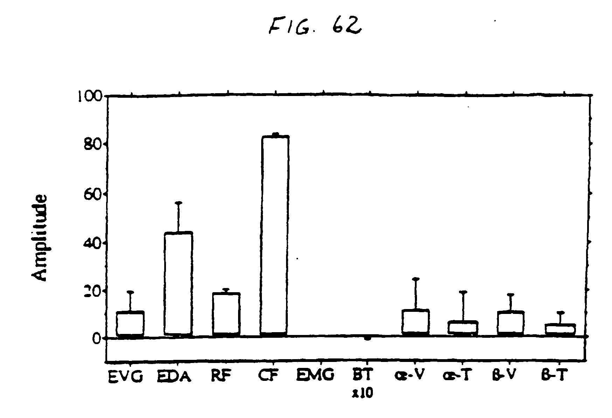

FIGS. 61 and 62 show data of measurements in men and women, respectively, of compound A8/P1.

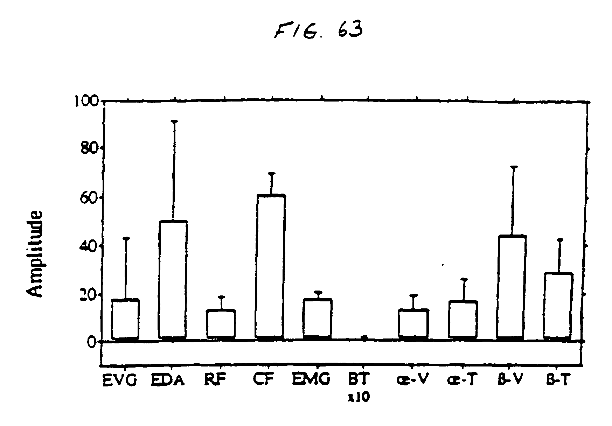

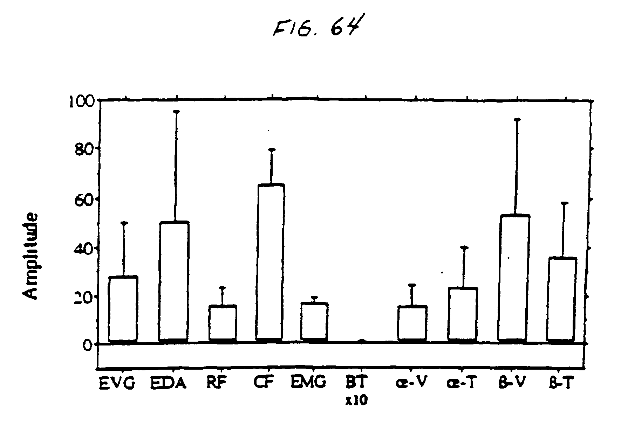

FIGS. 63 and 64 show data of measurements in men and women, respectively, of compound A13/P8.

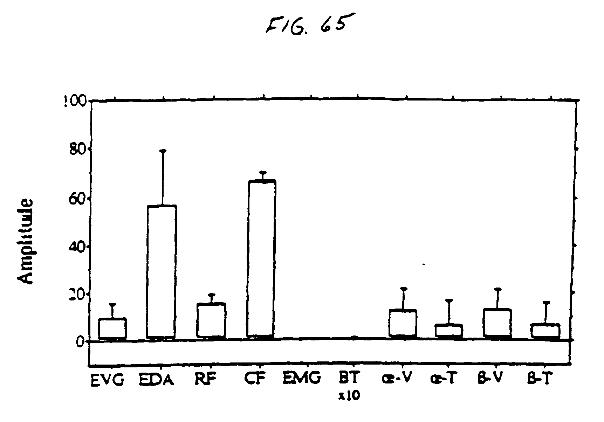

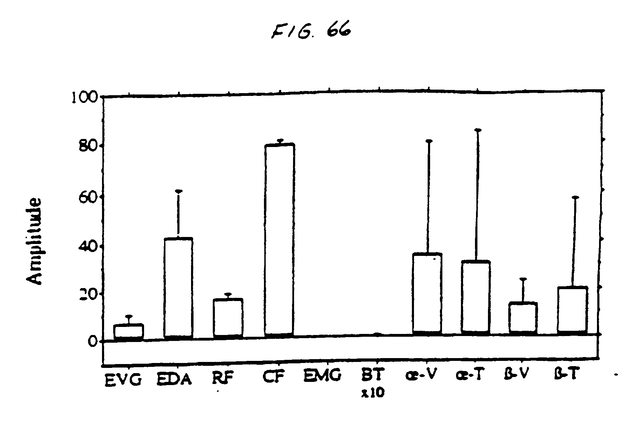

FIGS. 65 and 66 show data of measurements in men and women, respectively, of compound A6/P1.

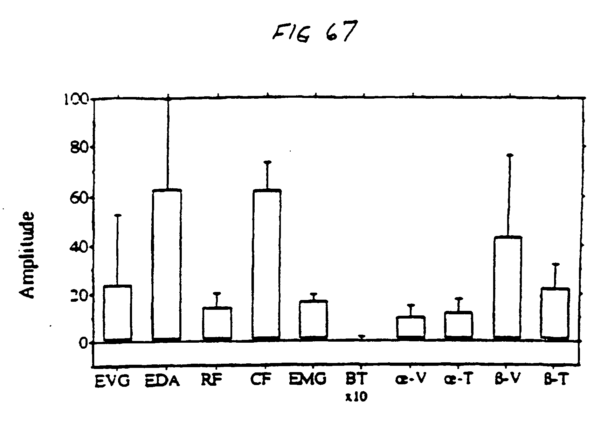

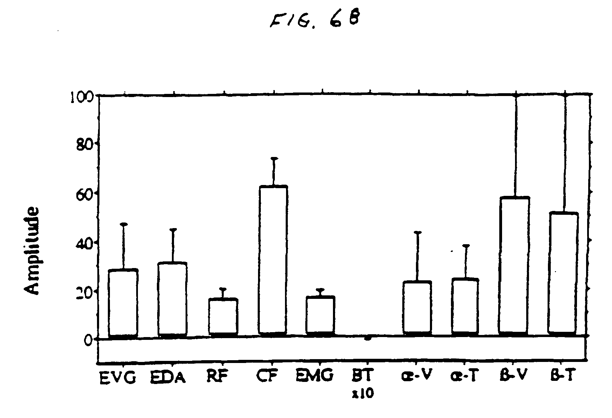

FIGS. 67 and 68 show data of measurements in men and women, respectively, of the 20-methyl derivative of compound A6/P1.

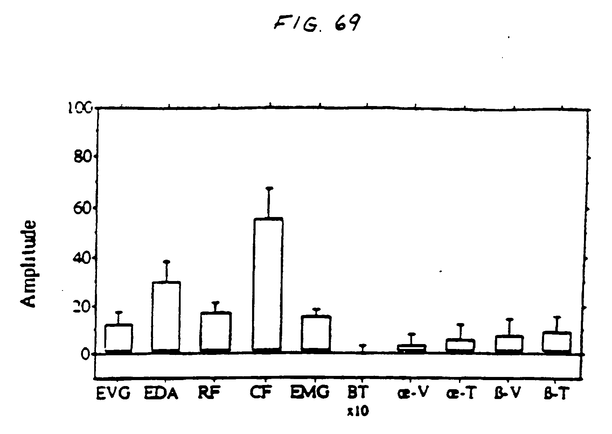

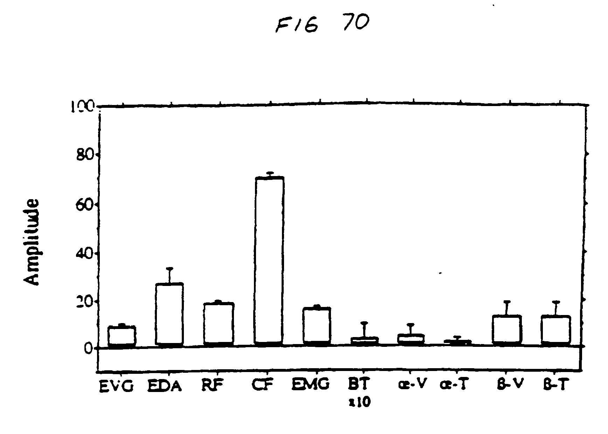

FIGS. 69 and 70 show data of measurements in men and women, respectively, of the 20,21-dimethyl derivative of compound A1/P1.

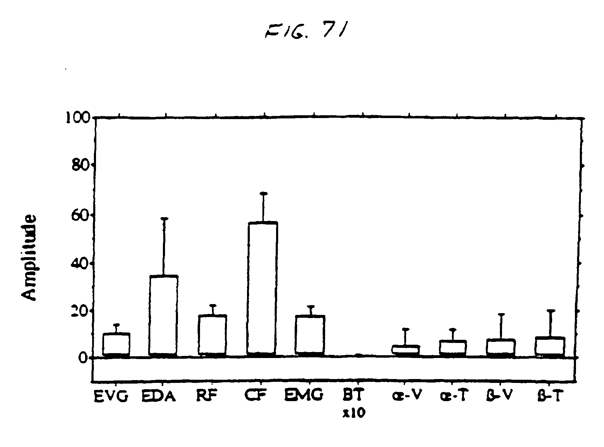

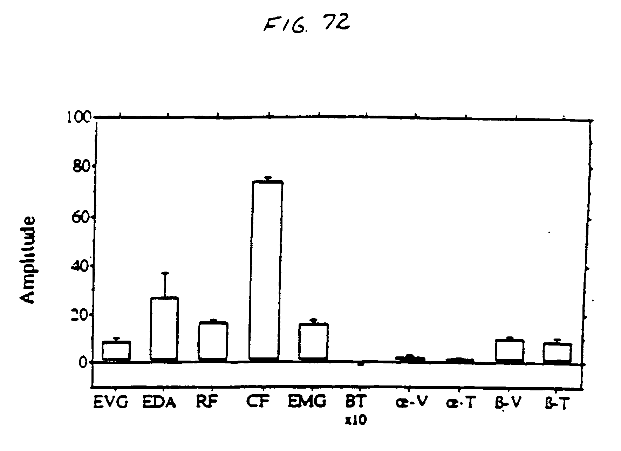

FIGS. 71 and 72 show data of measurements in men and women, respectively, of the 20,21-dimethyl derivative of compound A6/P1.

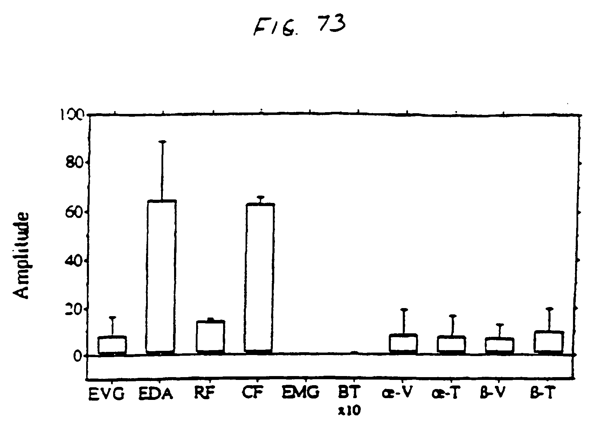

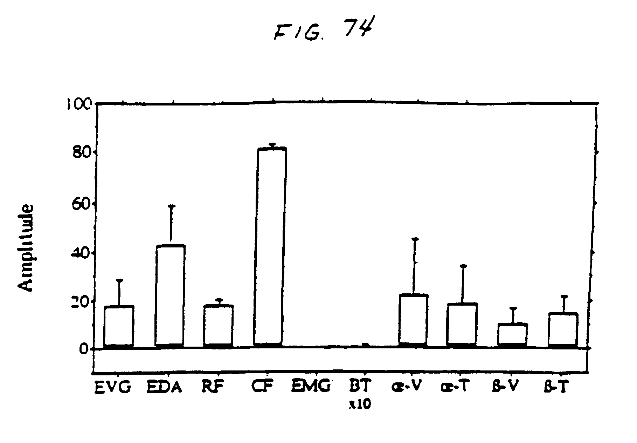

FIGS. 73 and 74 show the data of measurements in men and women, respectively, of compound A14/P2.

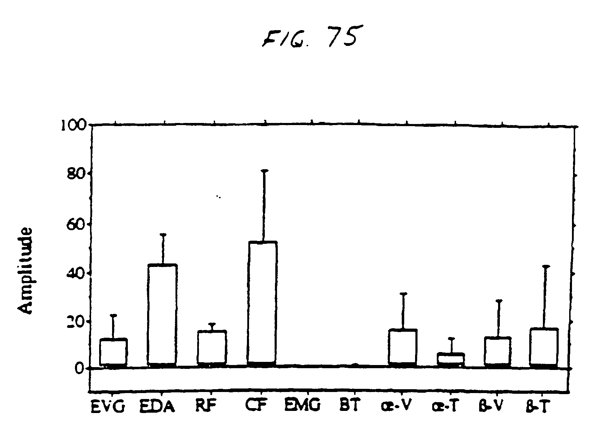

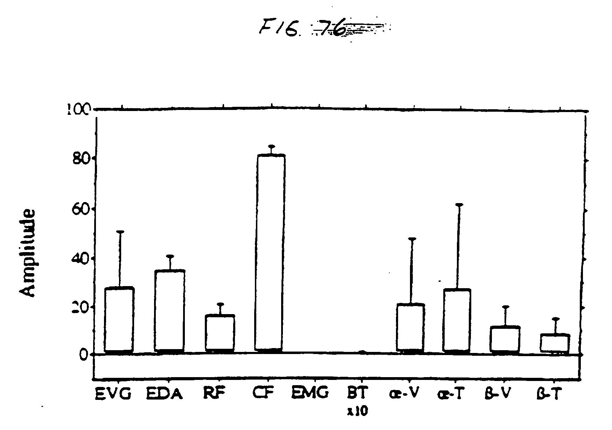

FIGS. 75 and 76 show the data of measurements in men and women, respectively, of compound A12/P1.

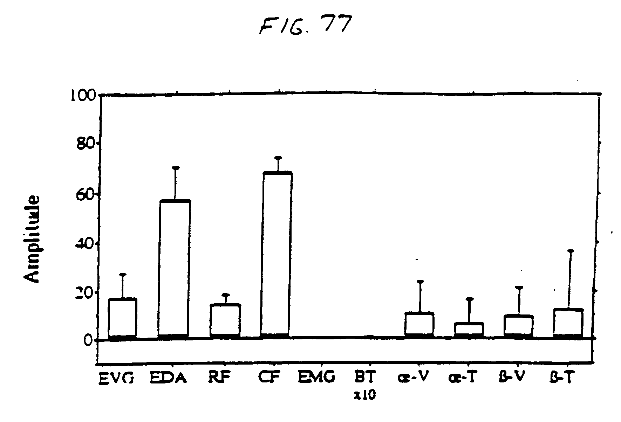

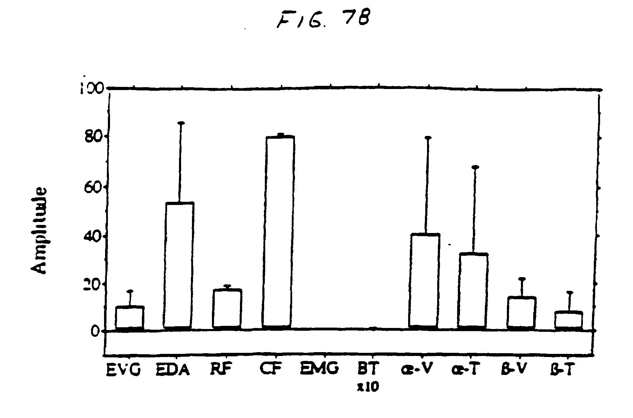

FIGS. 77 and 78 show the data of measurements in men and women, respectively, of compound A7/P2.

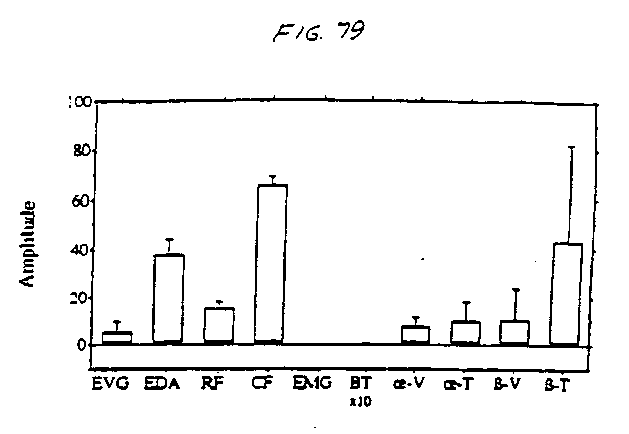

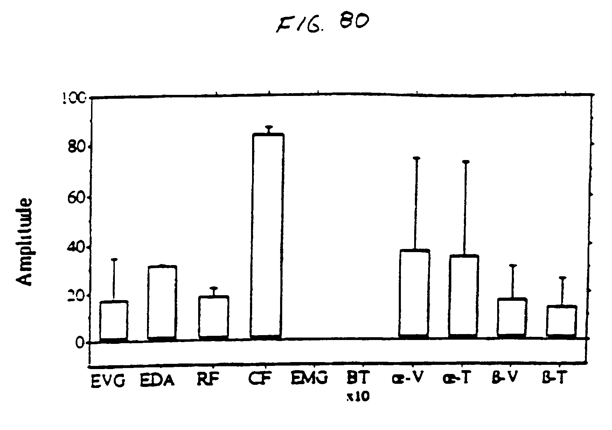

FIGS. 79 and 80 show the data of measurements in men and women, respectively, of compound A13/P1.

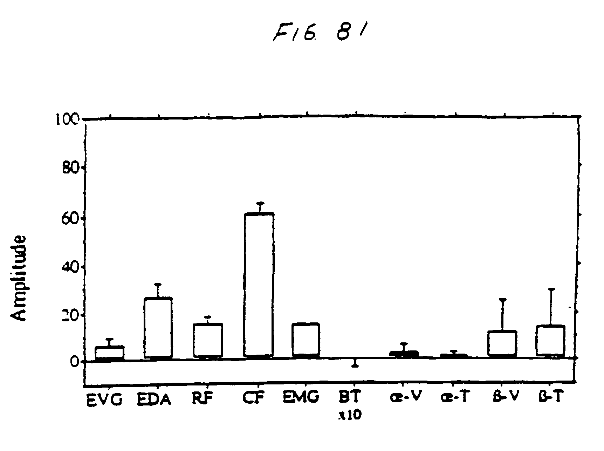

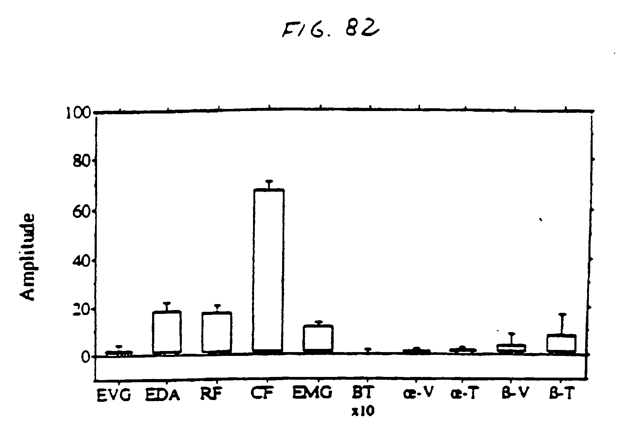

FIGS. 81 and 82 show the data of measurements in men and women, respectively, of compound A2/P7.

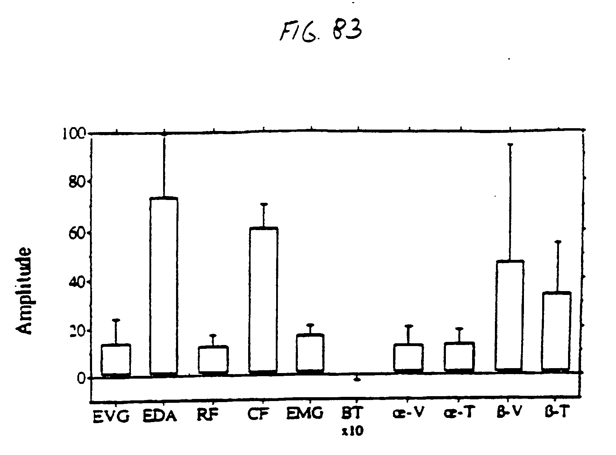

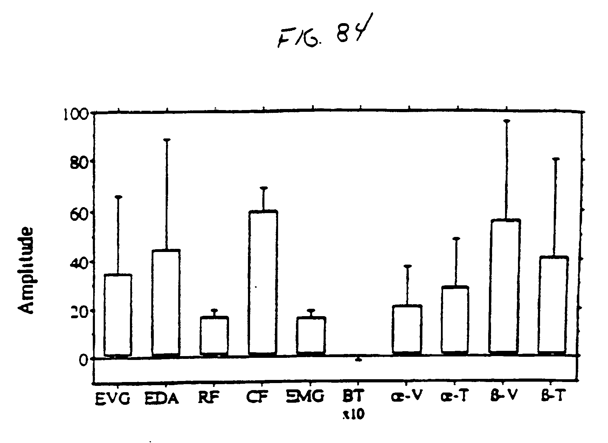

FIGS. 83 and 84 show the data of measurements in men and women, respectively, of compound A3/P5.

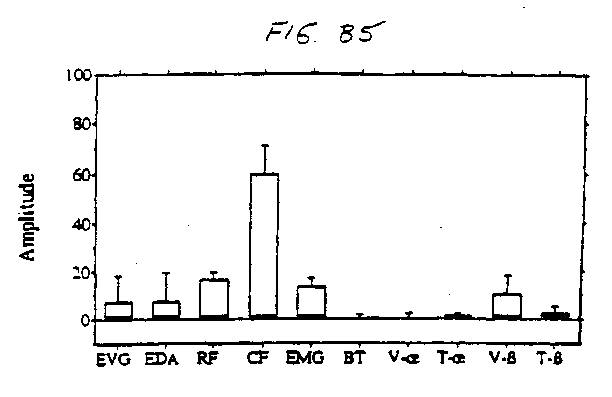

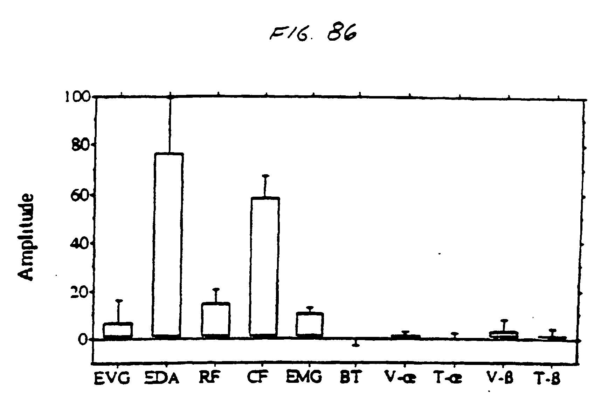

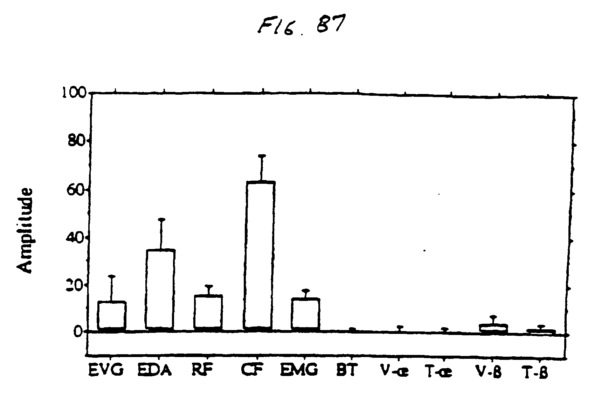

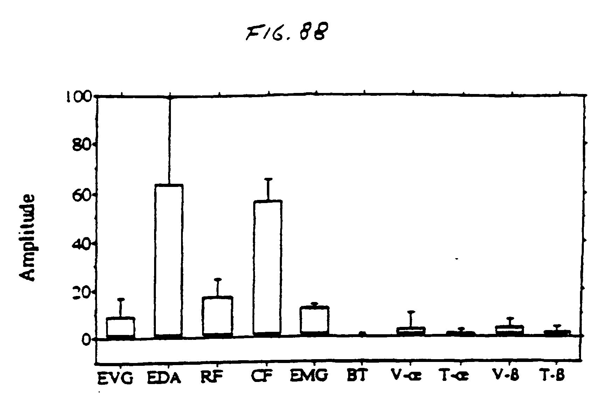

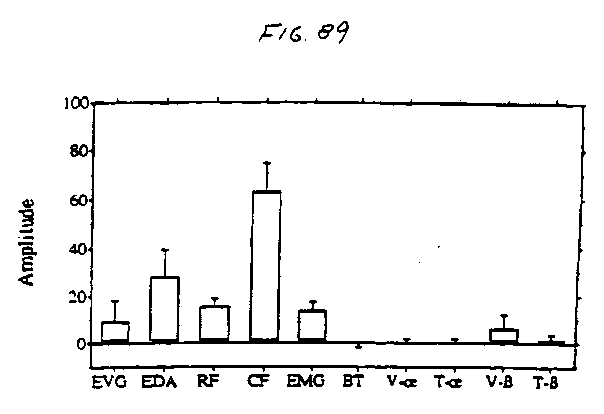

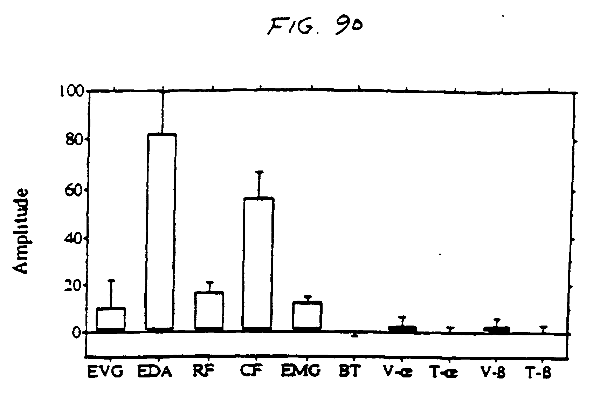

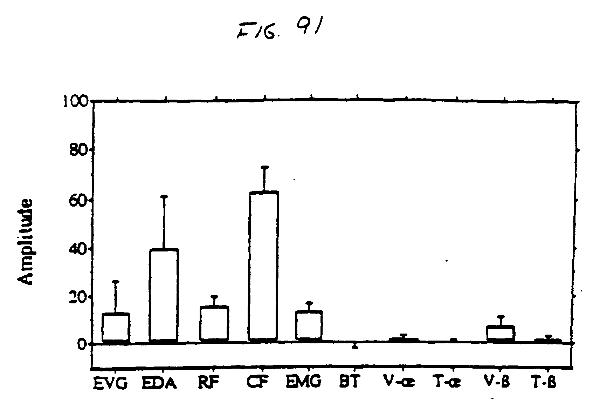

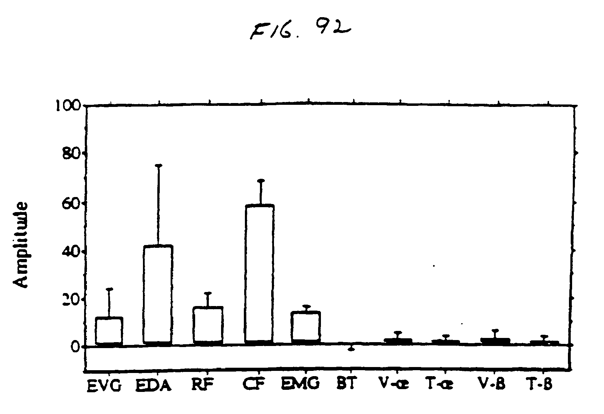

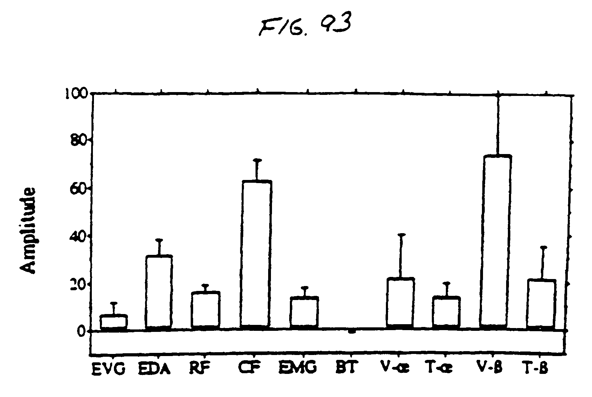

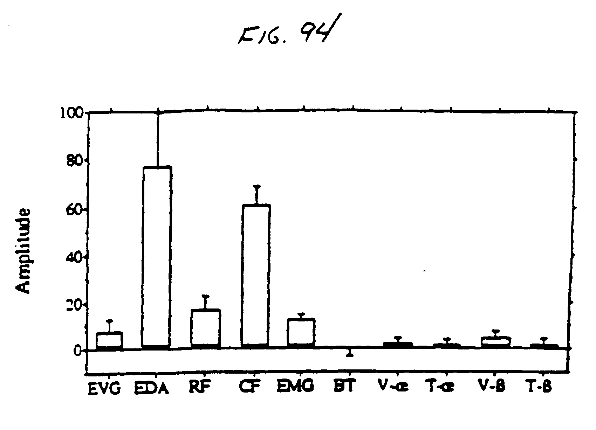

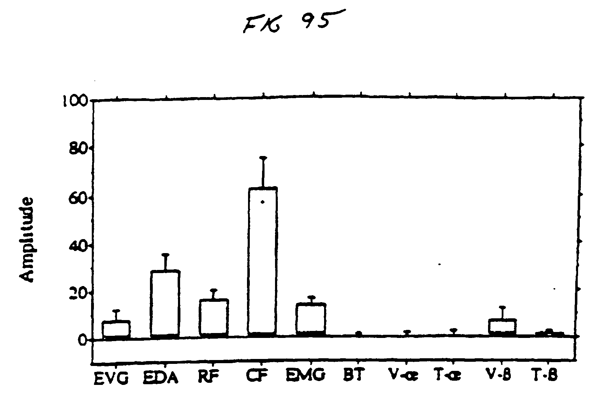

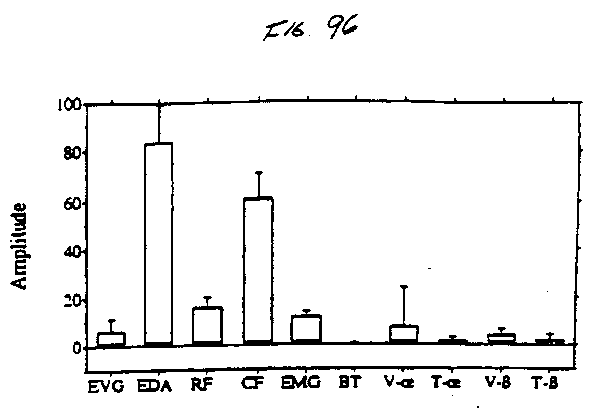

FIGS. 85-96 refer to the cholanes on Chart II. FIGS. 85 and 86 show the data of measurements in men and women, respectively, of compound A8/C1.
FIGS. 87 and 88 show the data of measurements in men and women, respectively, of compound A2/C1.
FIGS. 89 and 90 show the data of measurements in men and women, respectively, of the acetate of compound A2/C1.
FIGS. 91 and 92 show the data of measurements in men and women, respectively, of compound A1/C1.
FIGS. 93 and 94 show the data of measurements in men and women, respectively, of compound A3/C1.
FIGS. 95 and 96 show the data of measurements in men and women, respectively, of compound A13/C1.
FIG. 97 and FIG. 98 show the EVG and vomeronasal nerve discharge frequency, respectively, of steroid E2/P4 and control, in female rats.

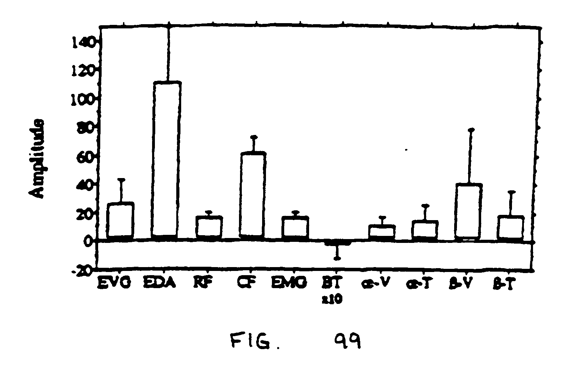

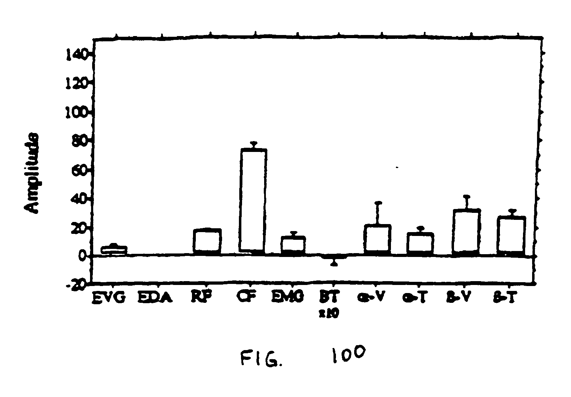

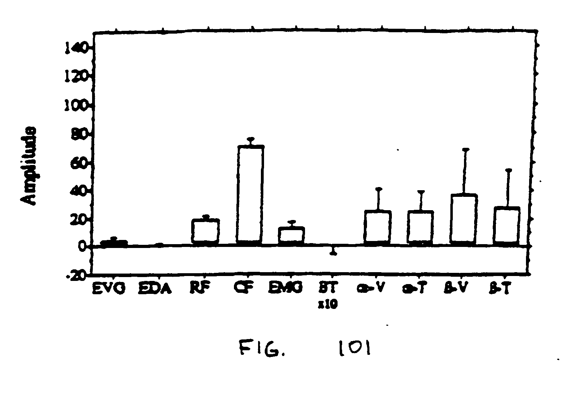

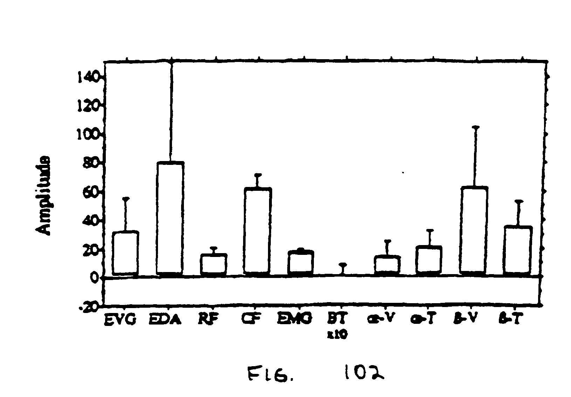

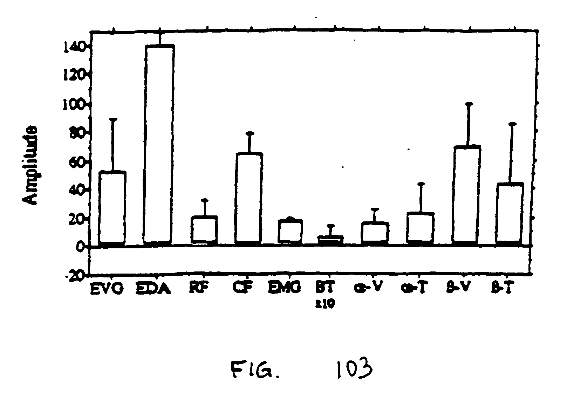

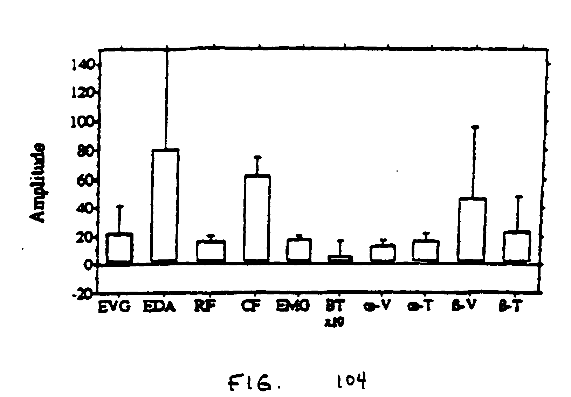

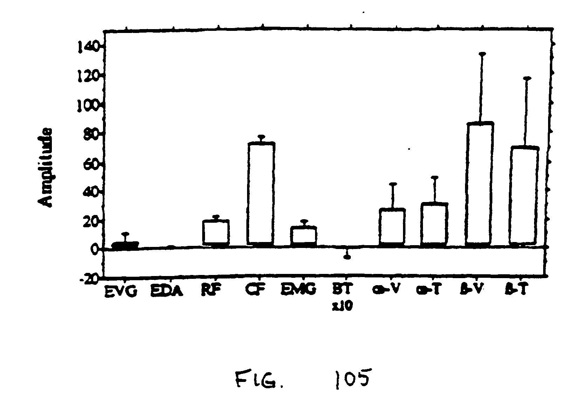

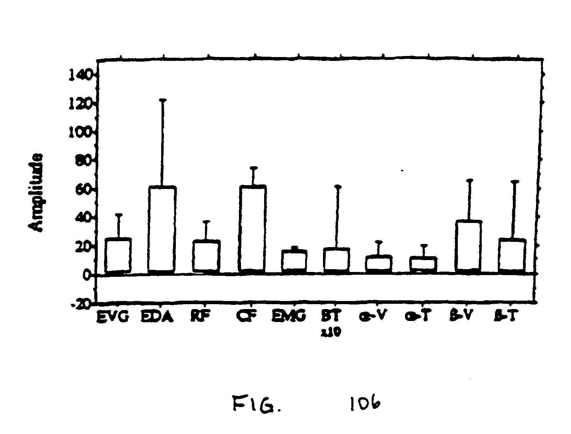

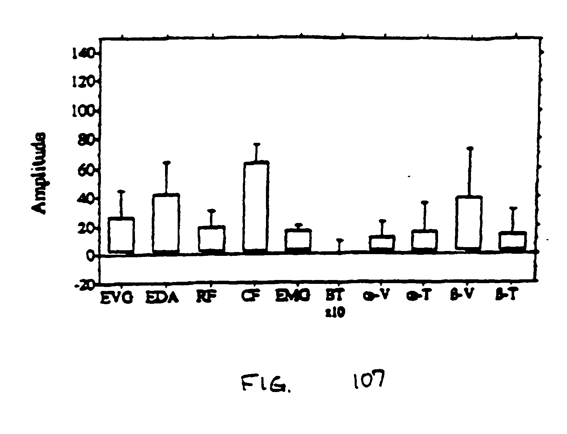

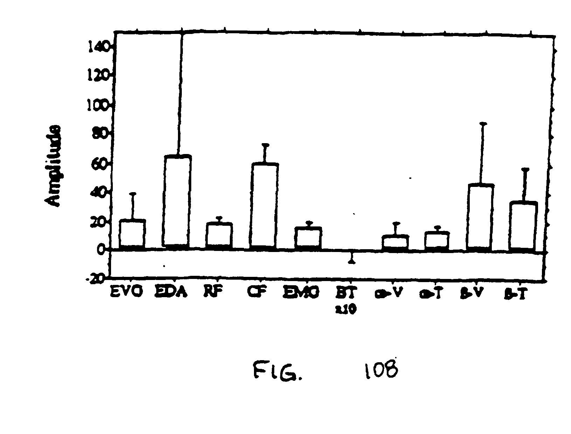

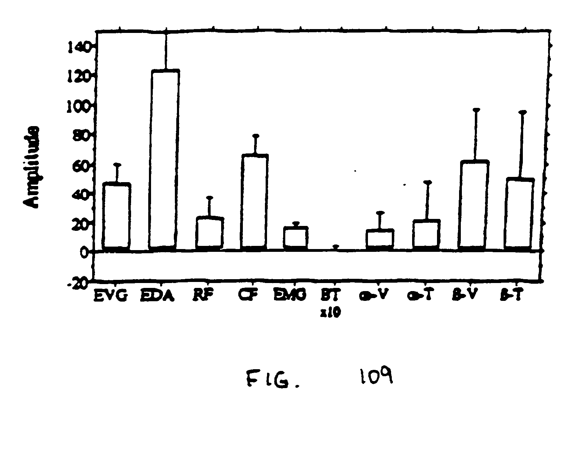

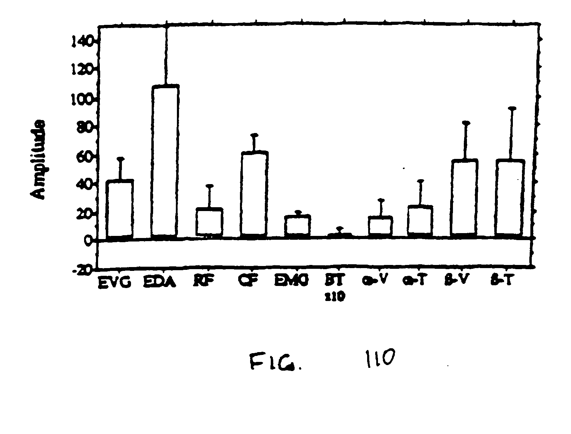

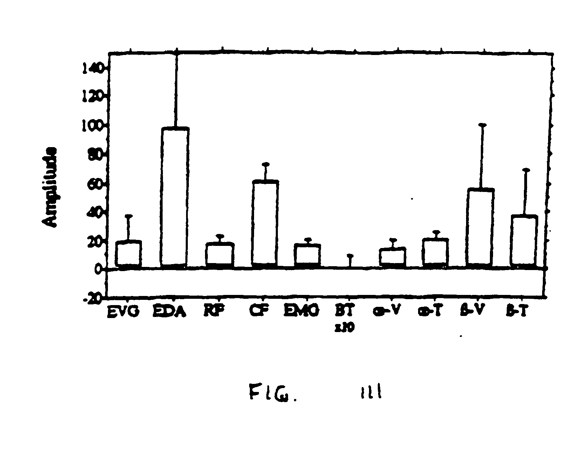

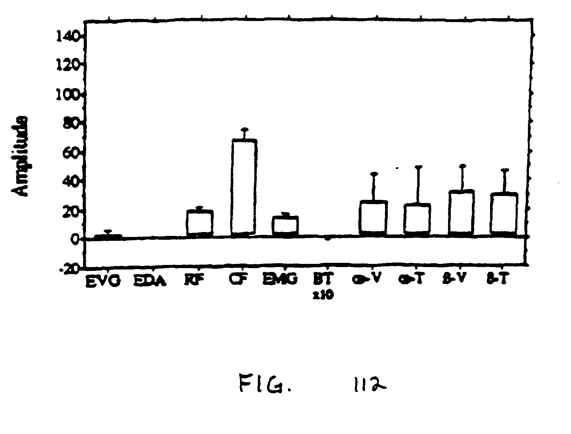

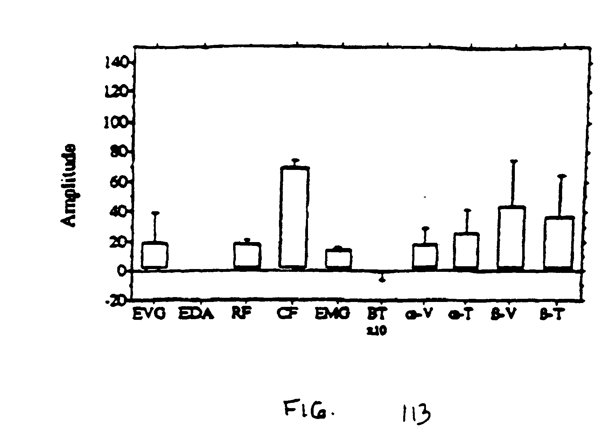

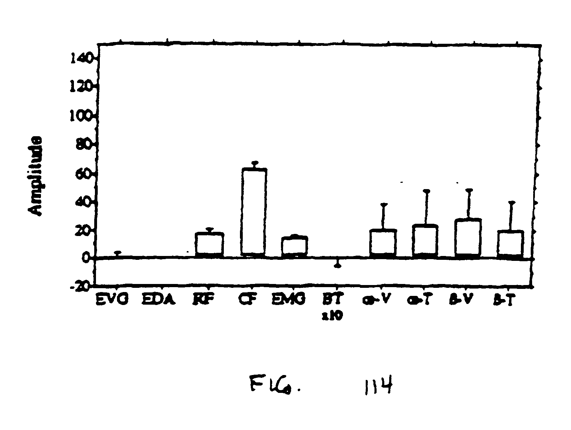

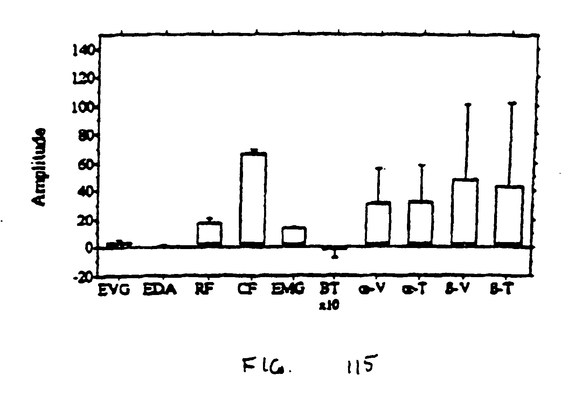

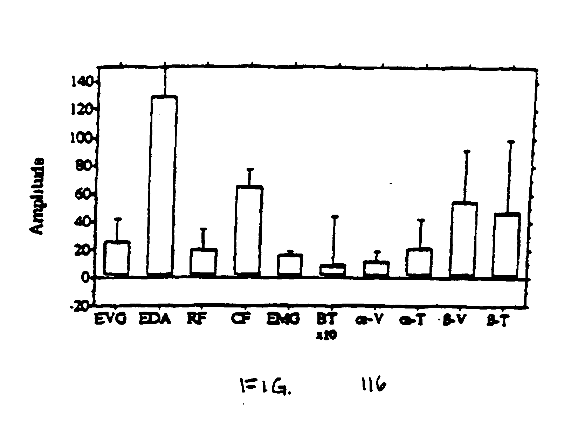

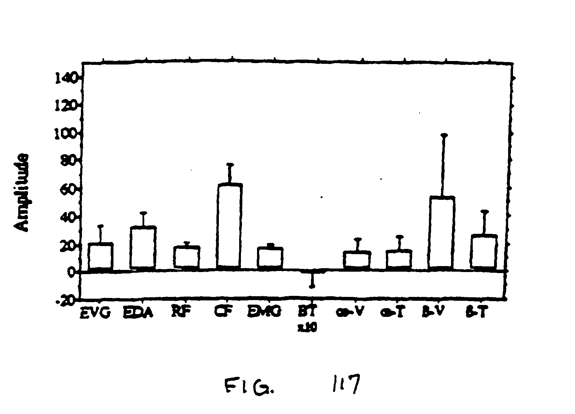

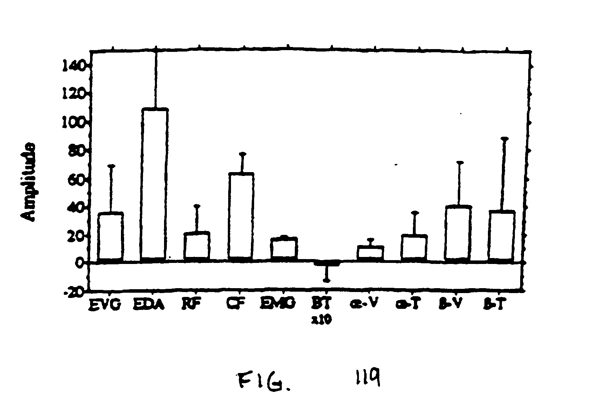

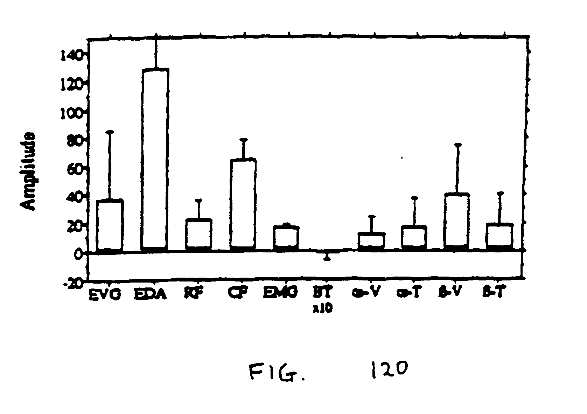

FIGS. 99 through 120 show the EVG, EDA, RF, CF, EMG, BT and EEG (alpha-V, alpha-T, beta-V and beta-T) data of administration of designated 19-nor-steroids in the VNO women.

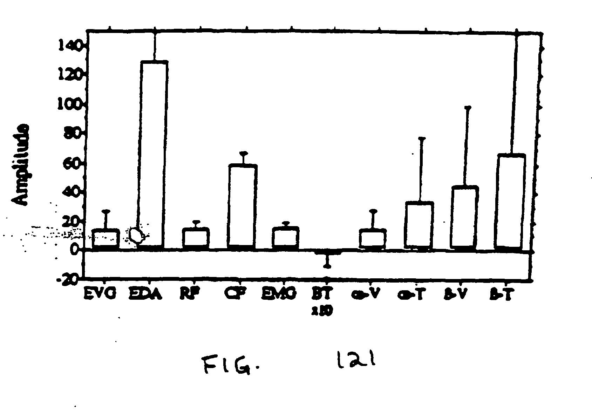

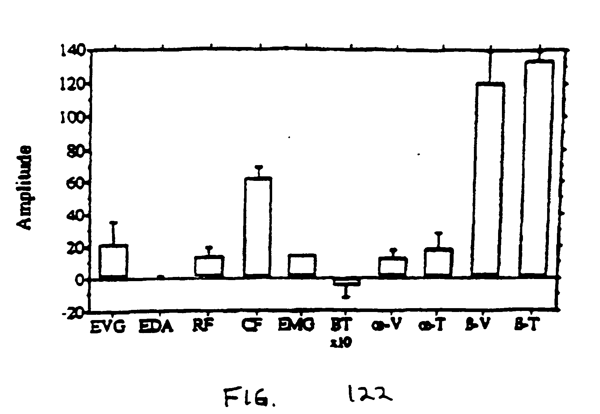

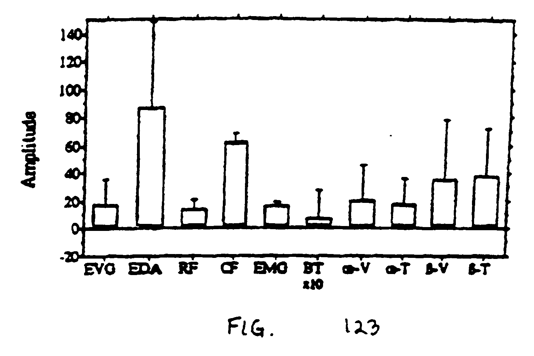

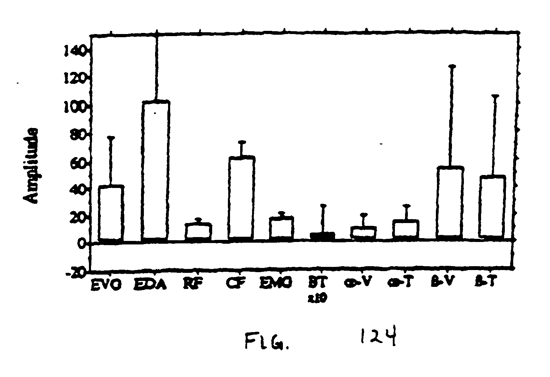

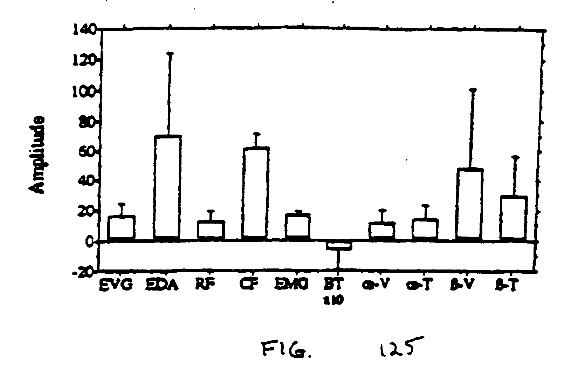

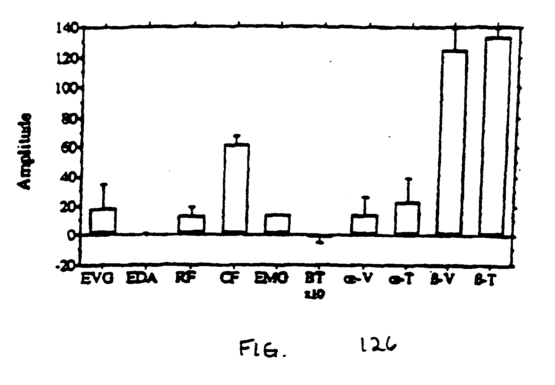

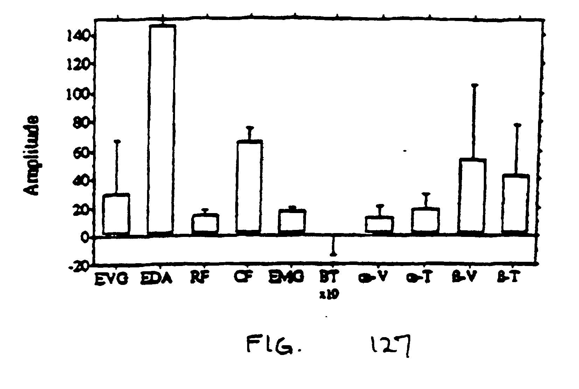

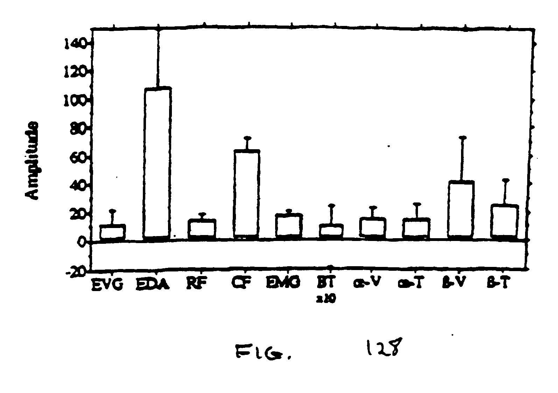

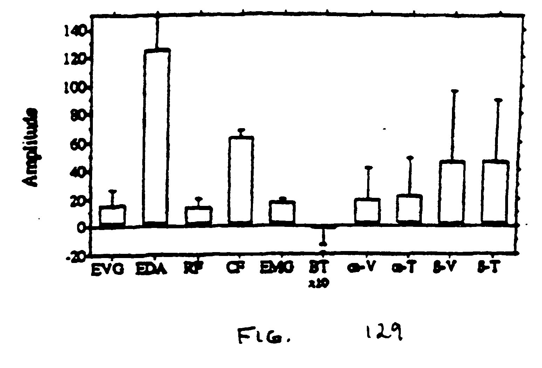

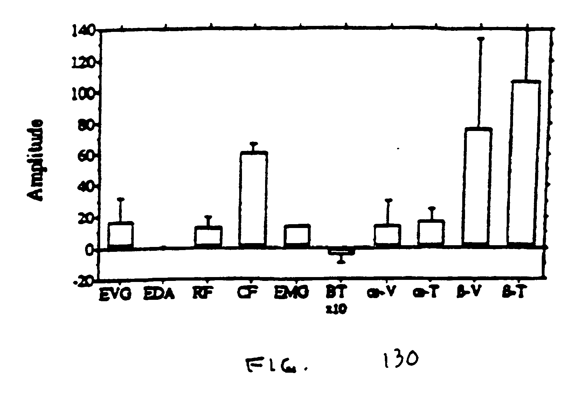

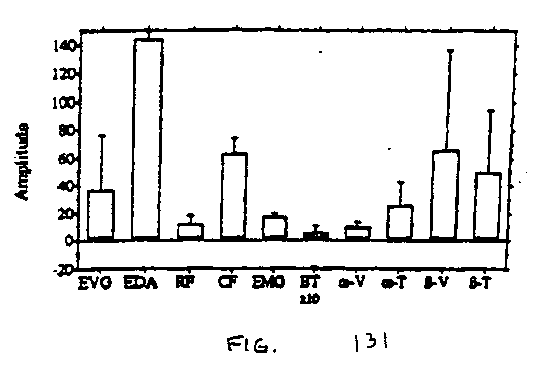

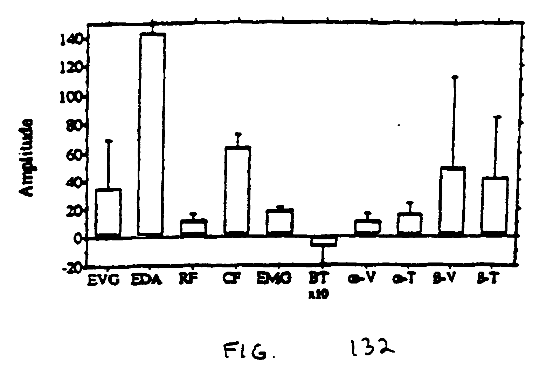

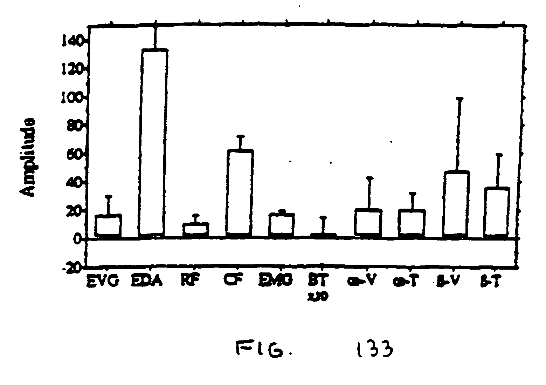

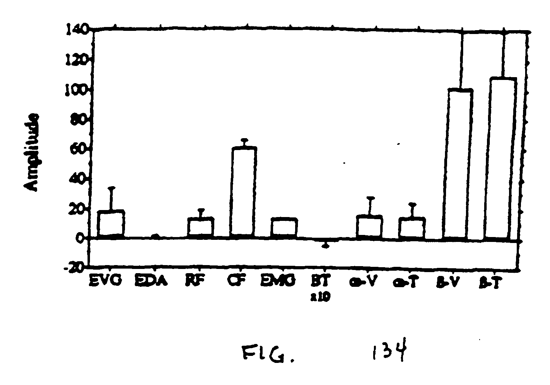

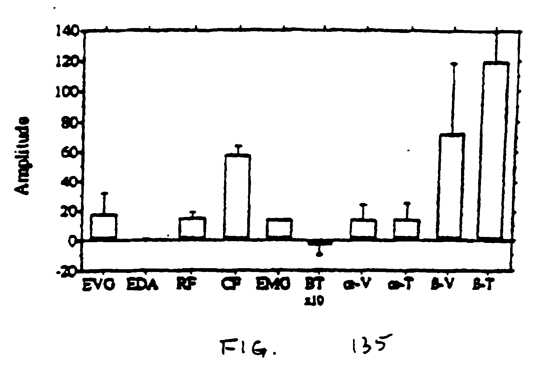

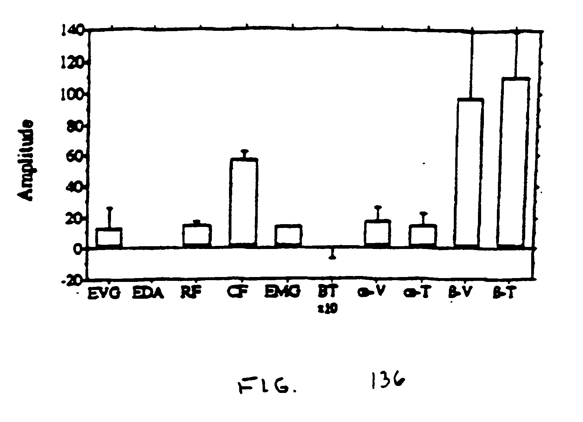

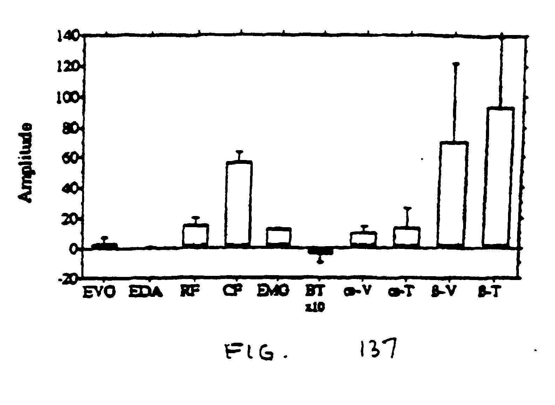

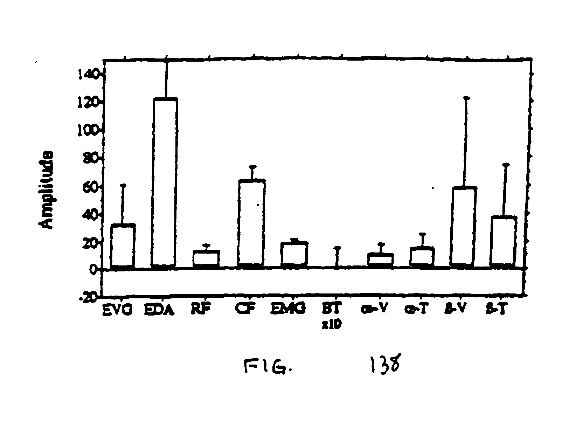

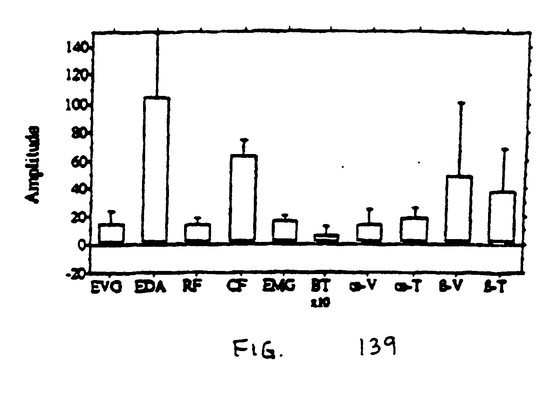

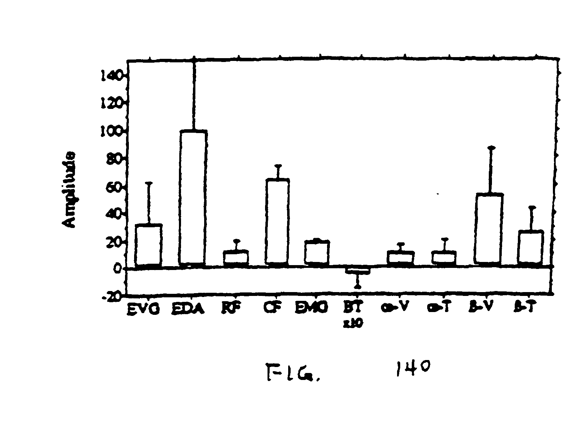

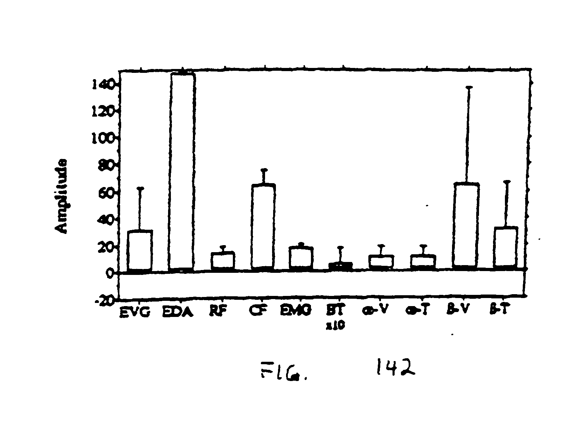

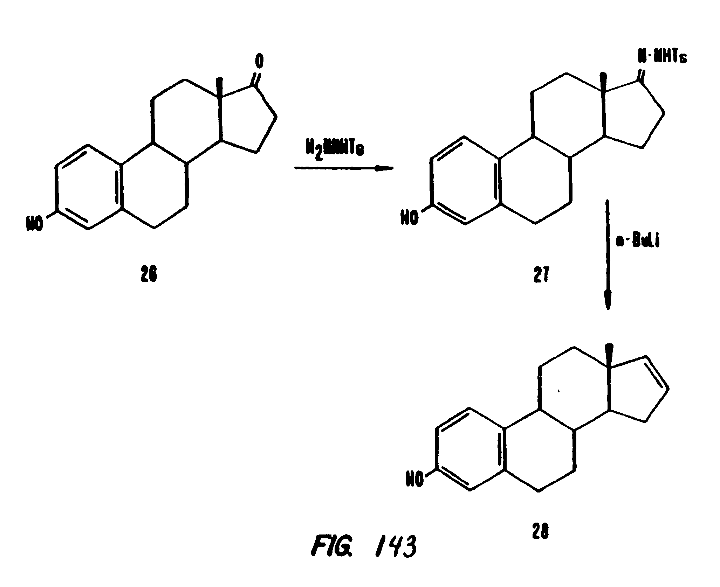

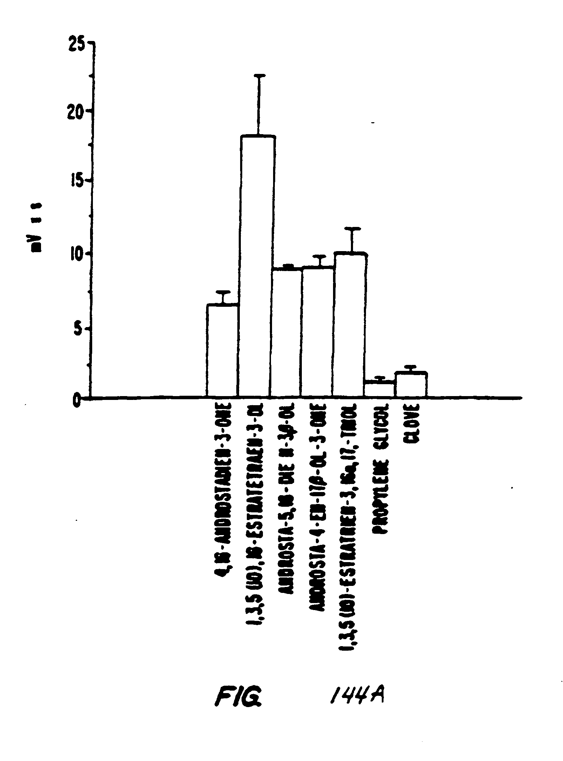

FIGS. 121 through 142 show the EVG, EDA, RF, CF, EMG, BT and EEG data of administration of designated 19-nor-steroids in the VNO of men.

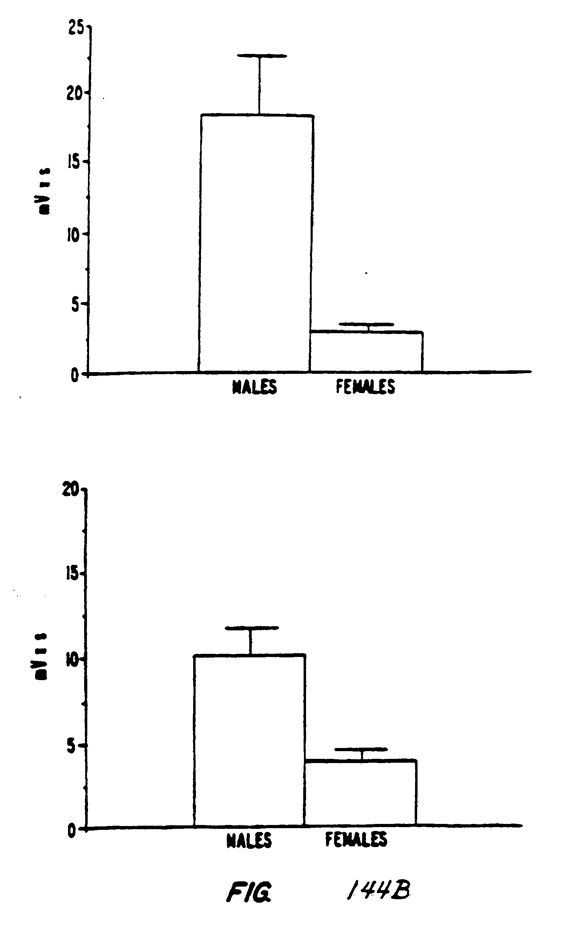

FIG. 143 illustrates the synthesis of 1, 3, 5 (10), 16-Estratetraen-3-ol.

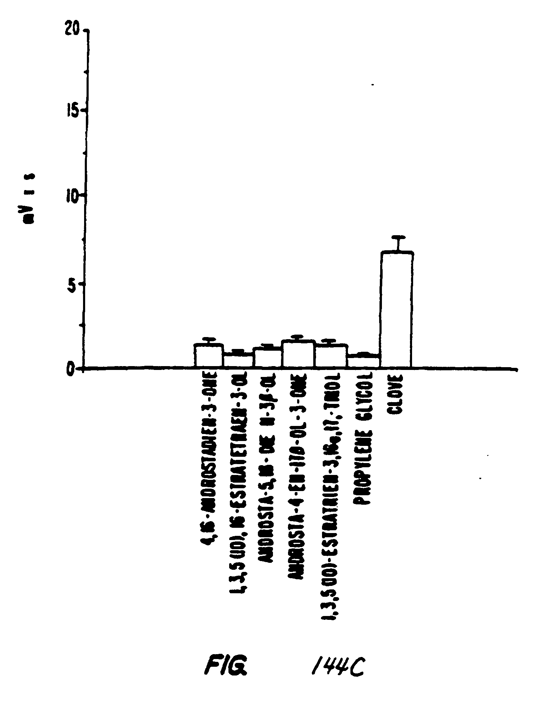

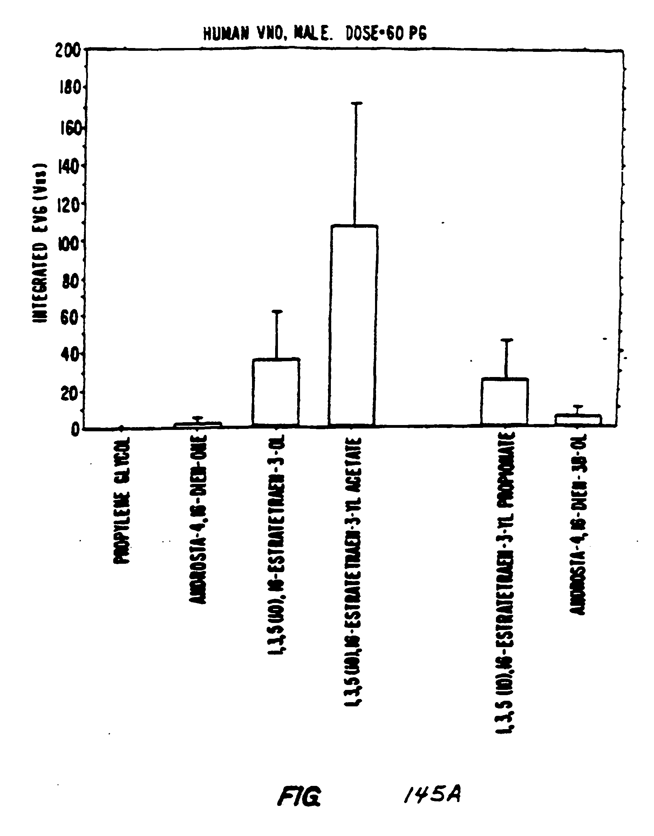

FIGS. 144A, 144B and 144C are graphic representations of the electrophysiological effect on receptor potential of the localized administration of particular steroids to the vomeronasal organ of male subjects (FIG. 144A) and to the olfactory epithelium (FIG. 144C). FIG. 144B is a graphic comparison of the effect of an Estrene on the VNO receptor potential of male and female subjects.

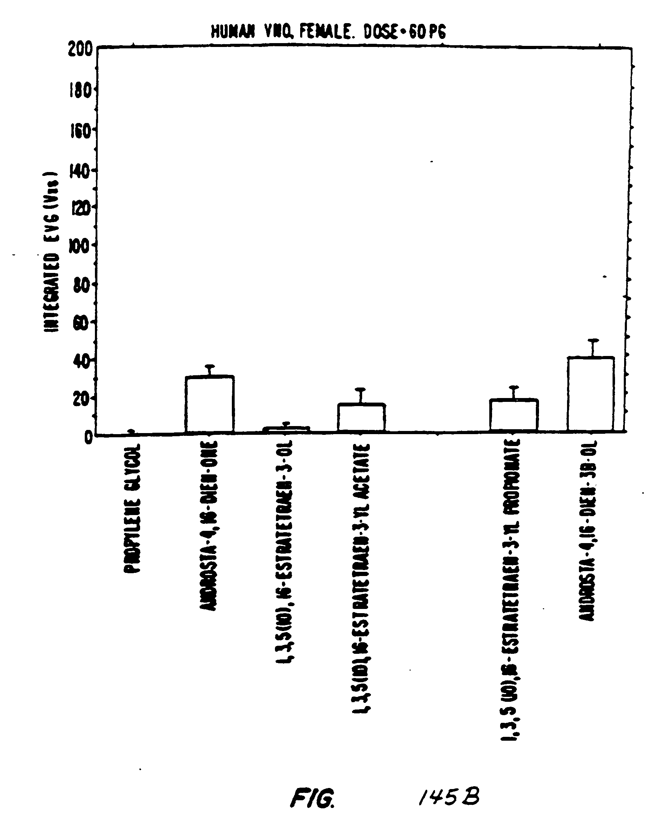

FIG. 145 is a graphic representation of the electrophysiological effect of the localized administration of particular steroids to the vomeronasal organ of male (145A) and female (145B) subjects.

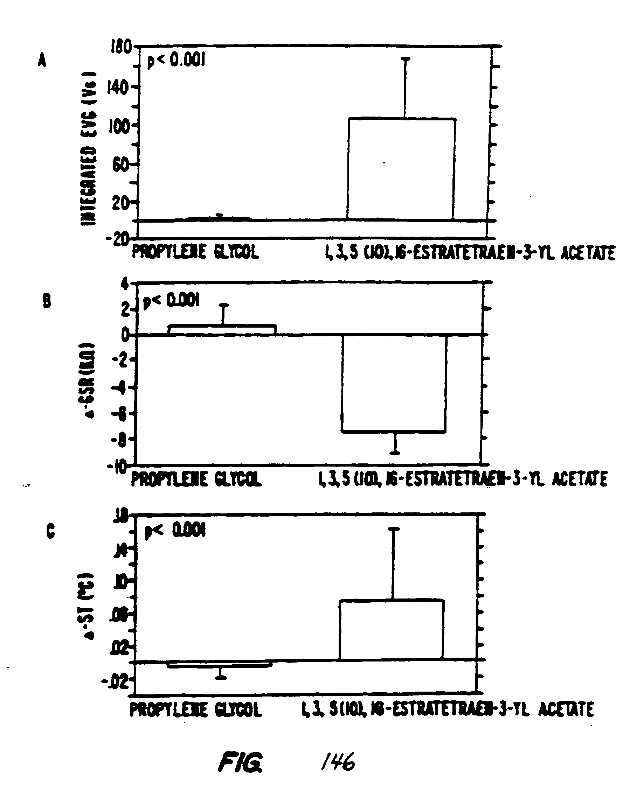

FIG. 146 depicts various autonomic responses of male subjects to 1,3,5(10), 16-Estratetraen-3-yl acetate. A = receptor potential of the vomeronasal neuroepithelium; B = change in galvanic skin response (K-ohms); C = change in skin temperature (degrees C.).

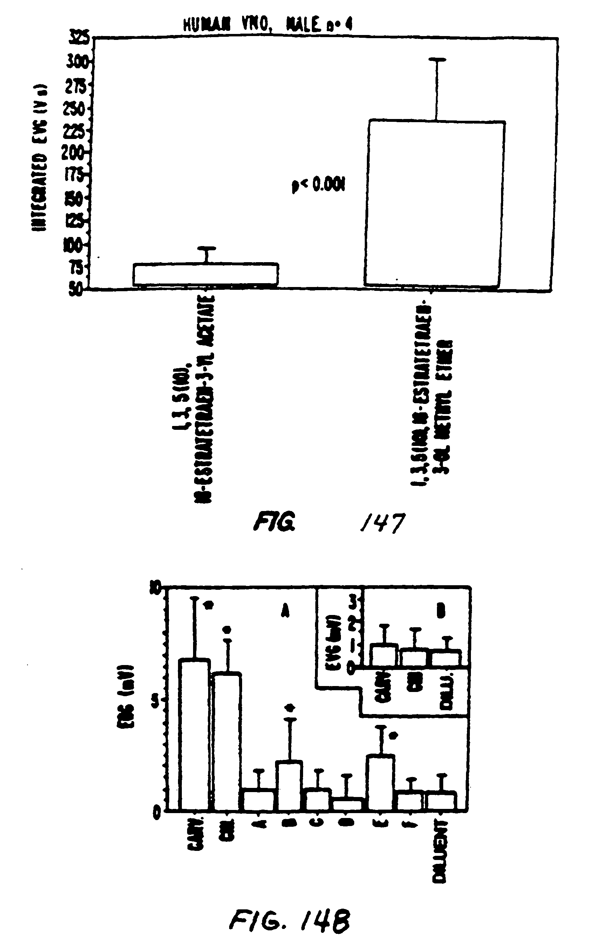

FIG. 147 depicts comparative changes in potential of the VNO after exposure to the methyl 5 ether and the acetate of 1,3,5(10),16-Estratetraen-3-ol.
FIG. 148 depicts sexual dimorphism in local and autonomic responses to the stimulation of the VNO with vomeropherins. Various vomeropherins (200 10 fmoles) and the diluent control were administered to 30 male and 30 female subjects (ages 20 to 45) as described. Bars indicate the mean response of the population.
FIGS. 148A and 148B: EVG responses were measured as 15 described in male (A) and female (B) subjects.
FIGS. 148C and 148D: Electrodermal activity was measured as described. Changes (measured in *x*Ω) in response due to delivery of vomeropherins to the VNO of each subject are shown in male (C) and female (D) subjects.
FIGS. 148E and 148F: Alpha-cortical activity was measured as described. Changes in response due to delivery of vomeropherins to the VNO of male (E) and female (F) subjects.
FIGS. 148G and 148H: Skin temperature (ST) was measured as described. Changes in response due to delivery of vomeropherins to the VNO of each subject are shown in male (G) and female (H) subjects.
   A = 1, 3, 5(10),16-Estratetraen-3-yl acetate
   B = Androsta-4,16-dien-3-one
   C = 1,3,5(10),16-Estratetraen-3-ol
   D = 3-Methoxy-Estra-1,3,5(10),16-tetraene
   E = Androsta-4,16-dien-3α-ol
   F = Androsta-4,16-dien-3β-ol
FIG. 149 depicts electro-olfactograms of male and female subjects induced by stimulation of the OE with olfactants and vomeropherins A: 400 fmoles of the olfactants 1-carvone and cineole as well as 200 fmoles of the vomeropherins A, B, C, D and F; and the 10 stereoisomer E were applied separately as one second pulses to the OE of 20 subjects (both male and female) and each EOG response was recorded as described. The olfactants as well as E and B produced significant (p<0.01) local response. B: 400 fmoles of the olfactants 1-carvone and cineole do not induce a significant EVG response when delivered to the VNO of male and female subjects.

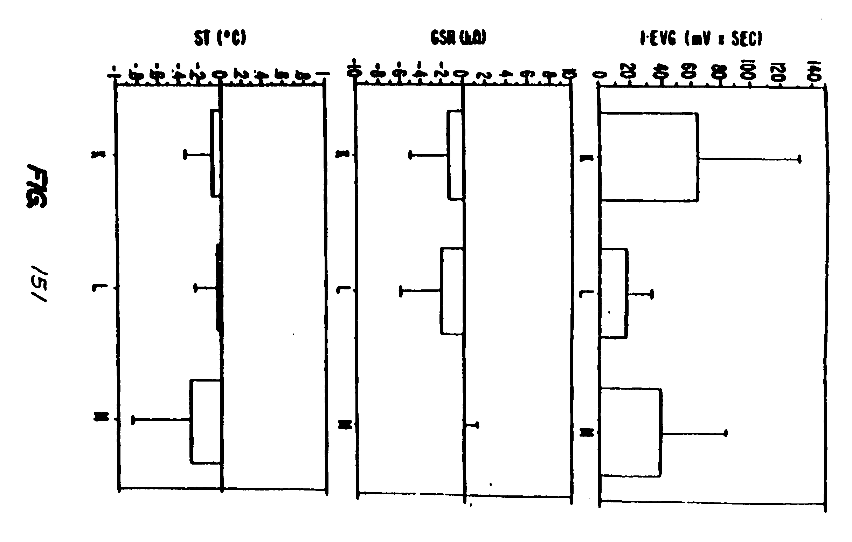

FIG. 150 depicts the electrophysiological effect of the following vomeropherins on the vomeronasal organ of 20 female subjects:
   G = Androst-4-en-3-one
   H = Androsta-4,16-dien-3,6-dione
   J = 10,17-Dimethylgona-4,13(17)-dien-3-one
   K = 1,3,5(10),16-Estratetraen-3-ol-methyl ether
   L = 1,3,5(10),16-Estratetraen-3-yl-propionate

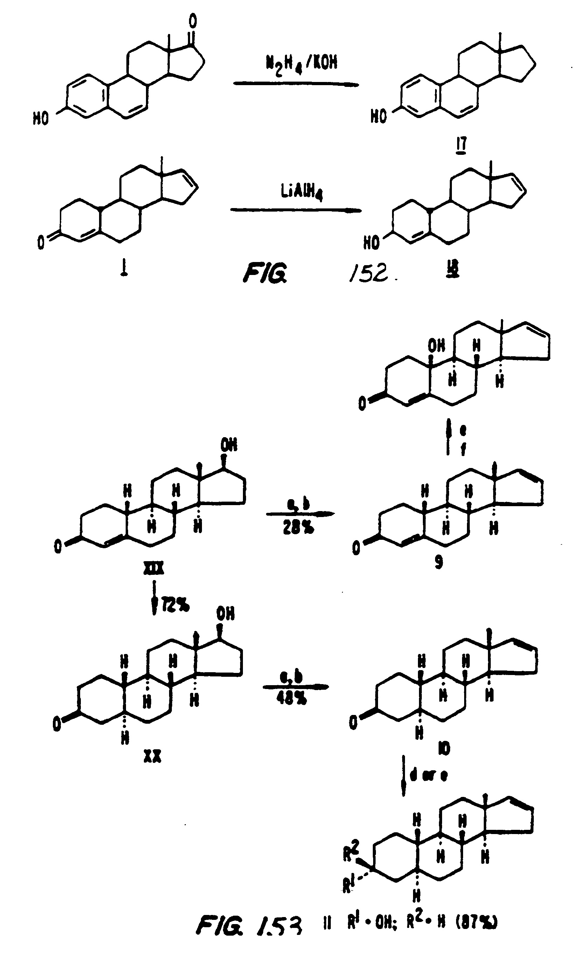

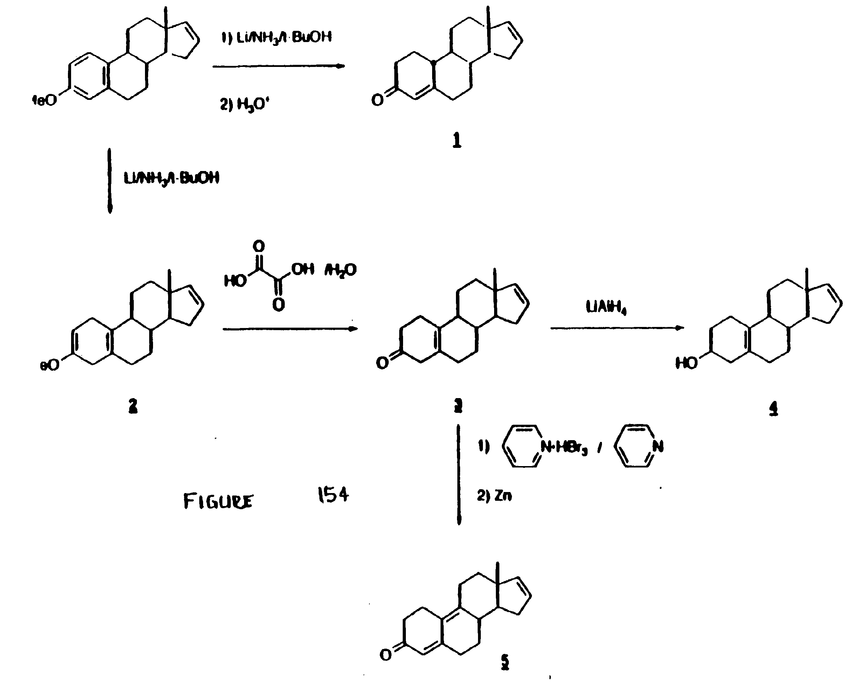

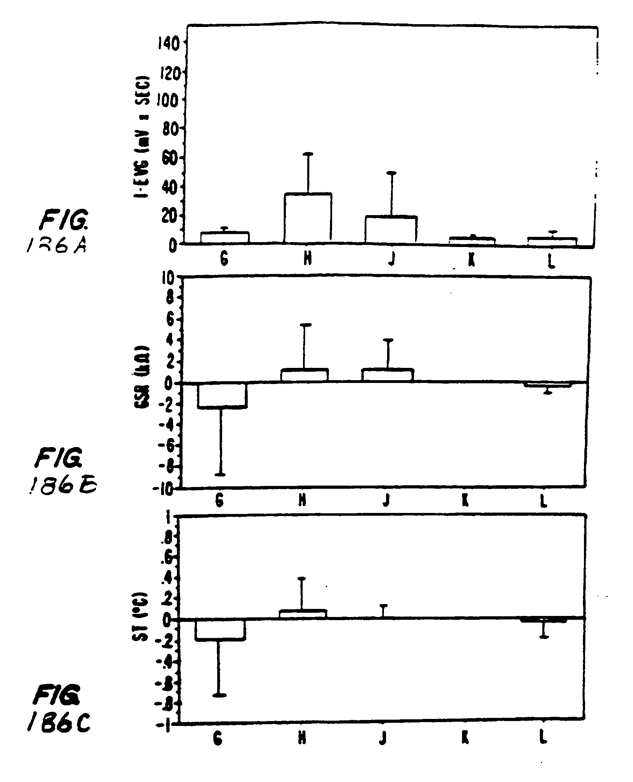

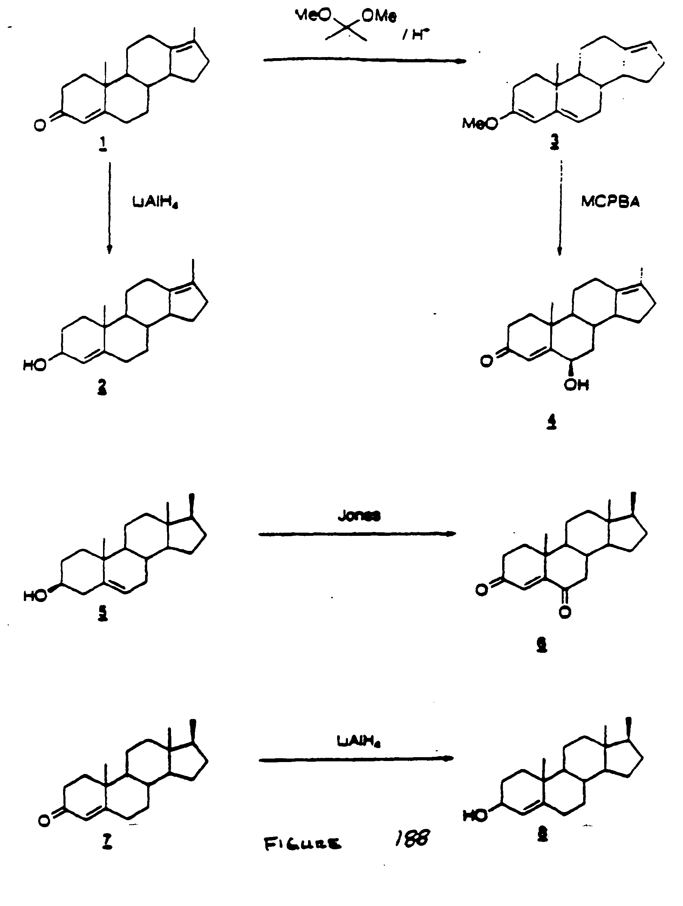

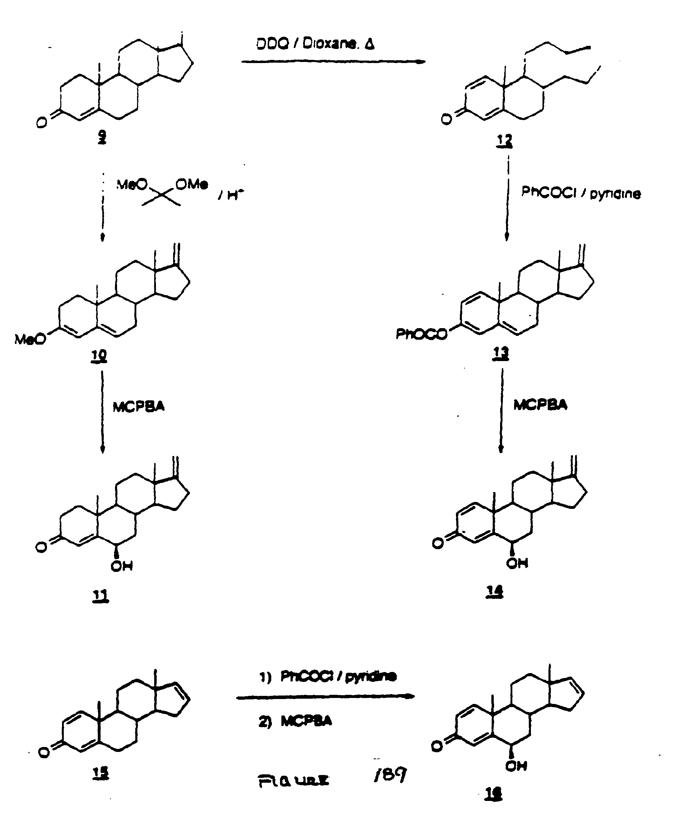

   EVG = Electro-vomeronasogram
   GSR = Galvanic Skin Response
      = Electrodermal Activity (EDA)
   ST = Skin Temperature
FIG. 151 depicts the electrophysiological effect of vomeropherins on the vomeronasal organ of 20 male subjects.
   M = 1,3,5(10)-Estratrien-3-ol
FIG. 152 depicts the synthesis of Estra-1,3,5(10),6-tetraen-3-ol and Estra-4,16-dien-3-ol.

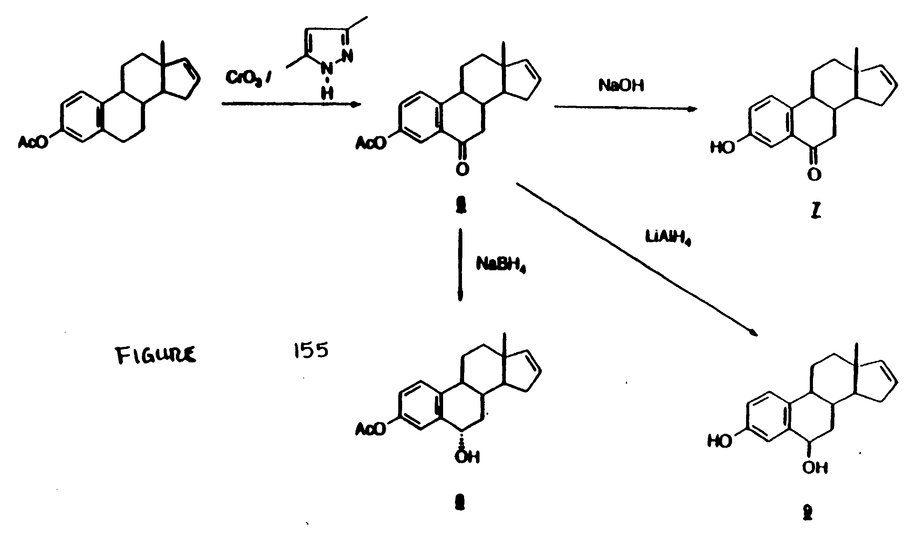

FIG. 153 depicts the synthesis of compounds described in Examples 63 through 66.

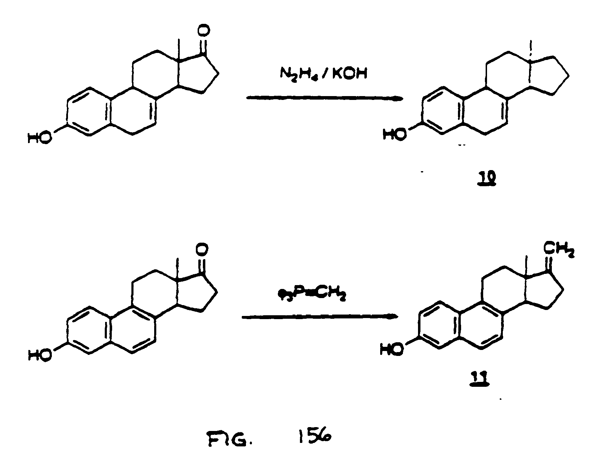

FIG. 154 illustrates the steps of synthesis described in Examples 67 through 71.

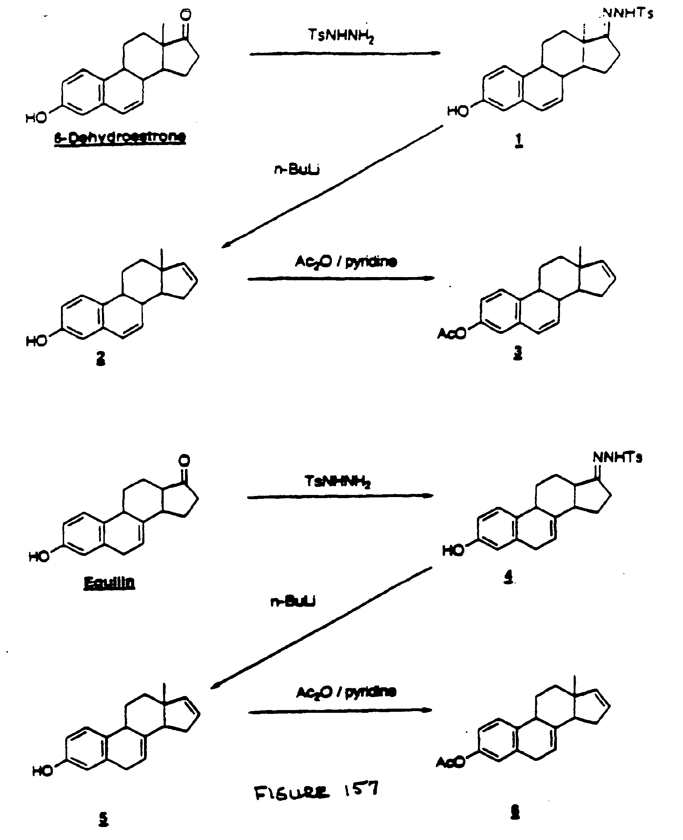

FIG. 155 illustrates the steps of synthesis described in Examples 72 through 75.

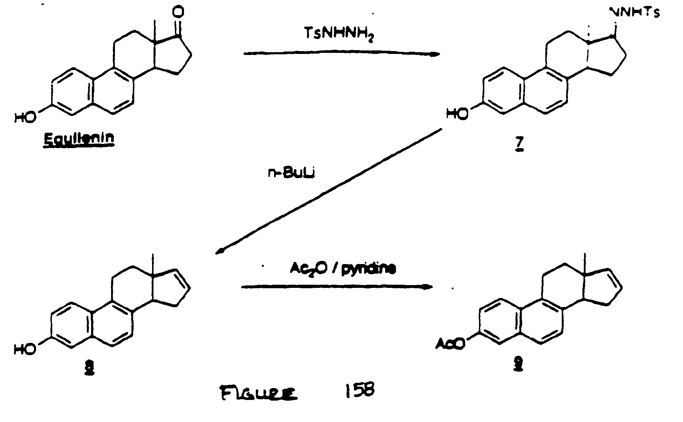

FIG. 156 illustrates the steps of synthesis described in Examples 76 through 77.

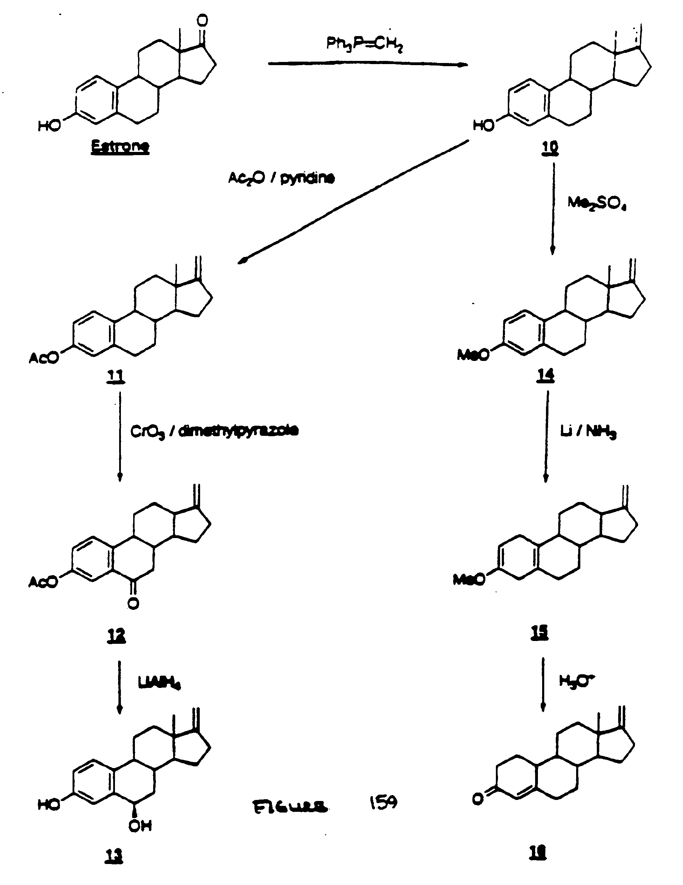

FIG. 157 illustrates the steps of synthesis described in Examples 78 through 83.

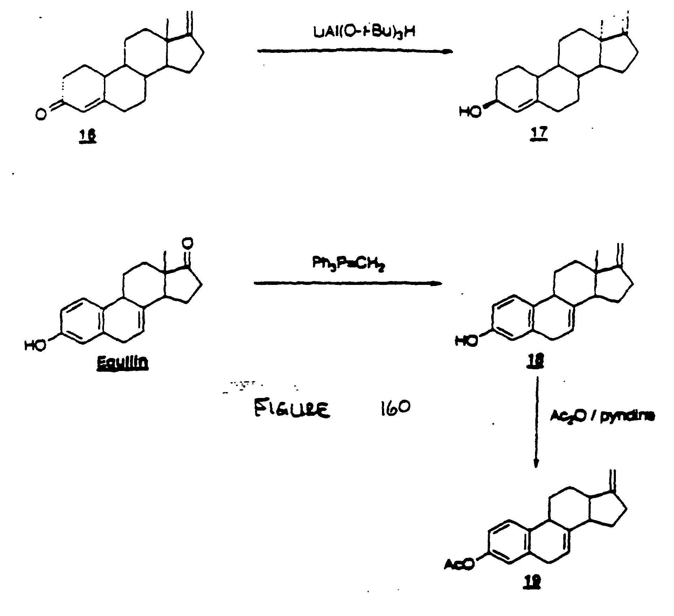

FIG. 158 illustrates the steps of synthesis described in Examples 84 through 86.

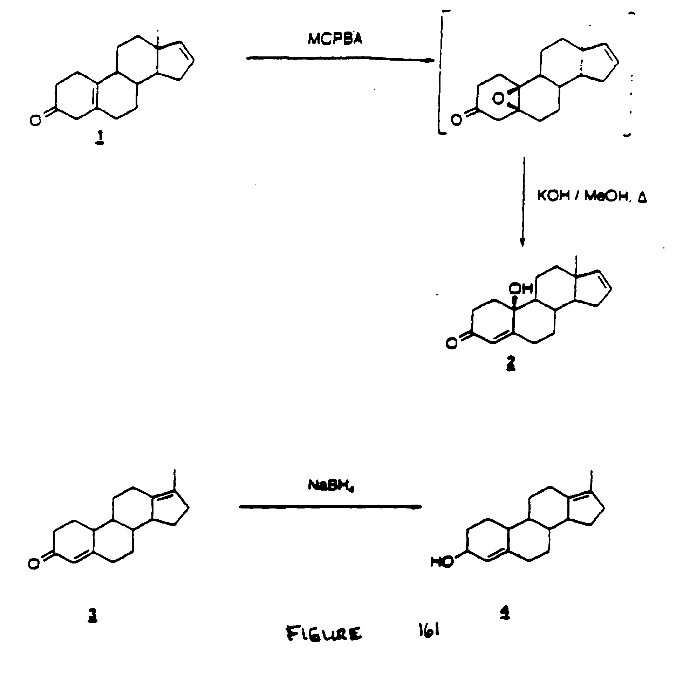

FIG. 159 illustrates the steps of synthesis described in Examples 87 through 93.

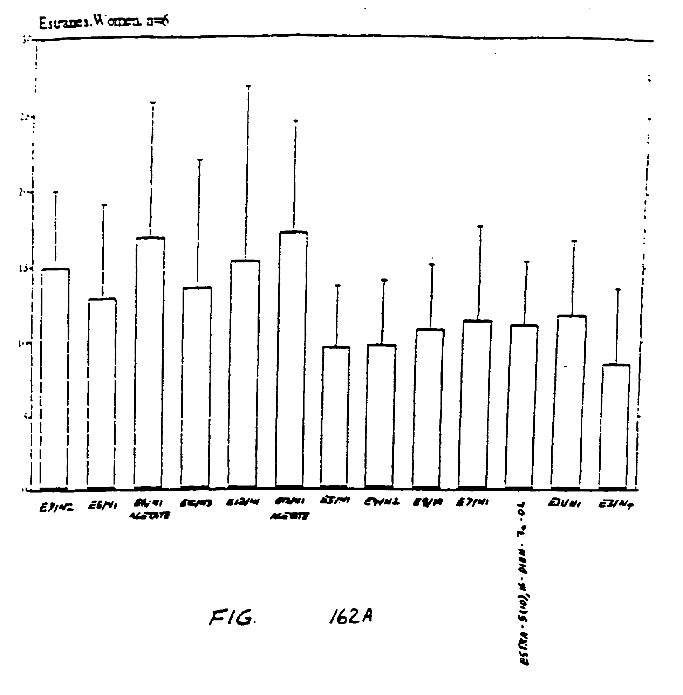

FIG. 160 illustrates the steps of synthesis described in Examples 94 through 96.

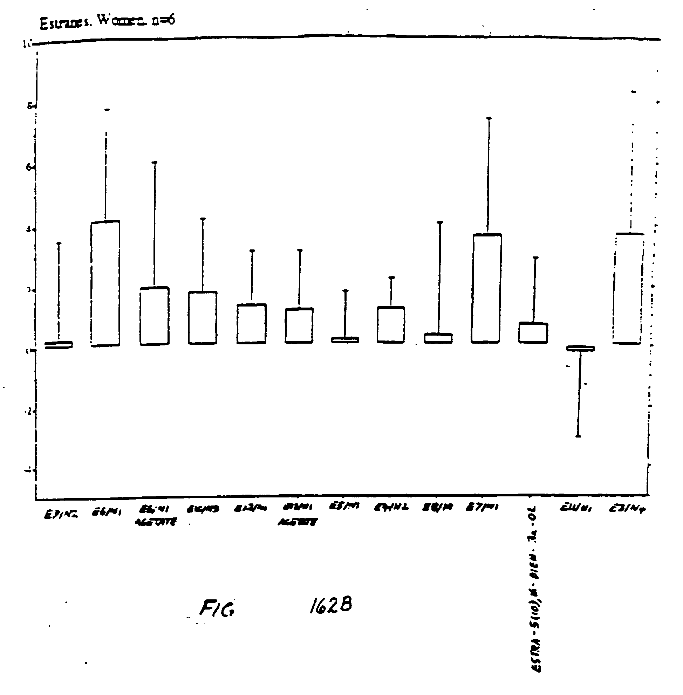

FIG. 161 illustrates the steps of synthesis described in Examples 97 through 98.

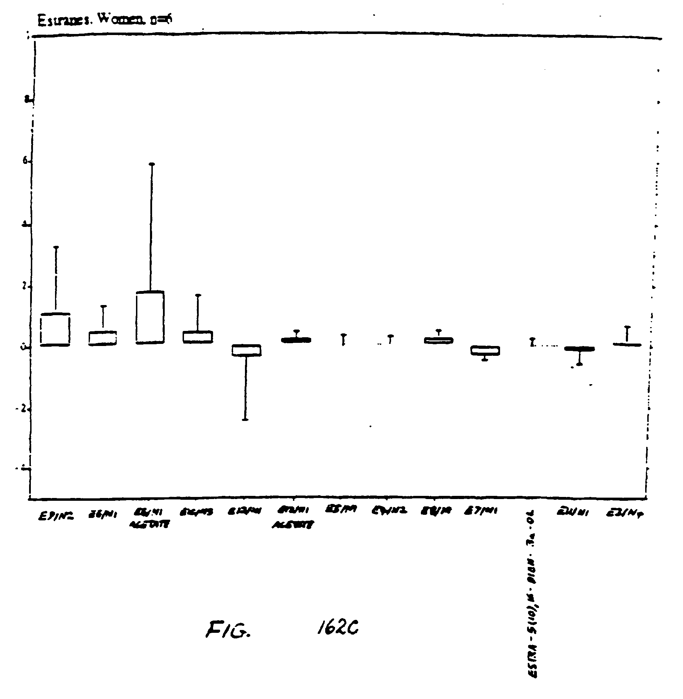

FIGS. 162A, 162B and 162C illustrate the EVG, GSR and ST data on women, respectively, for 13 estranes on Chart 1.

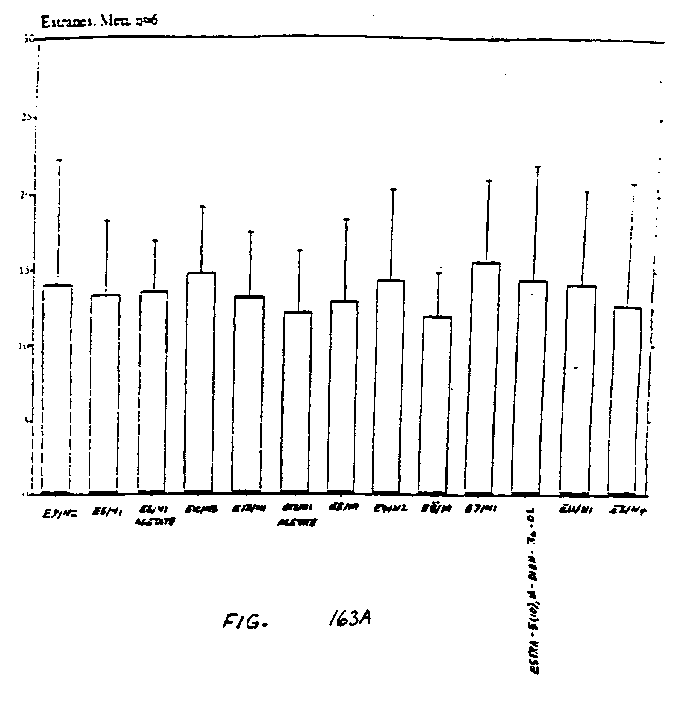

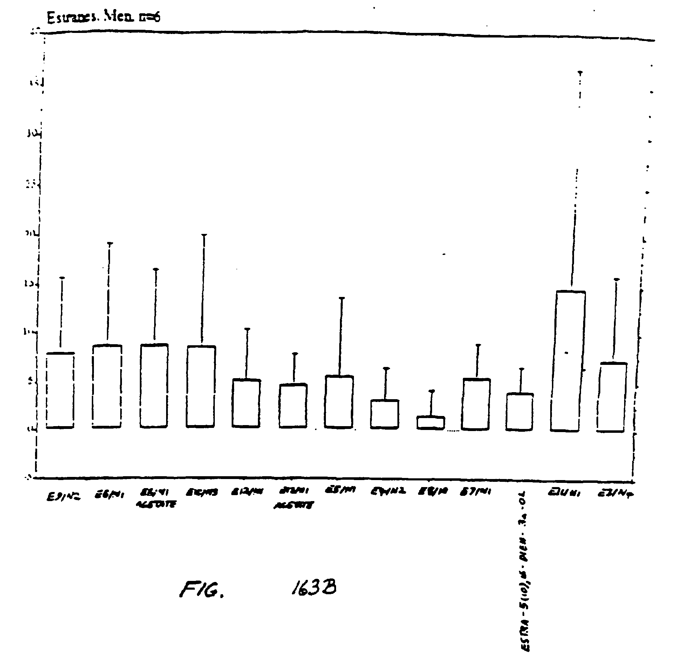

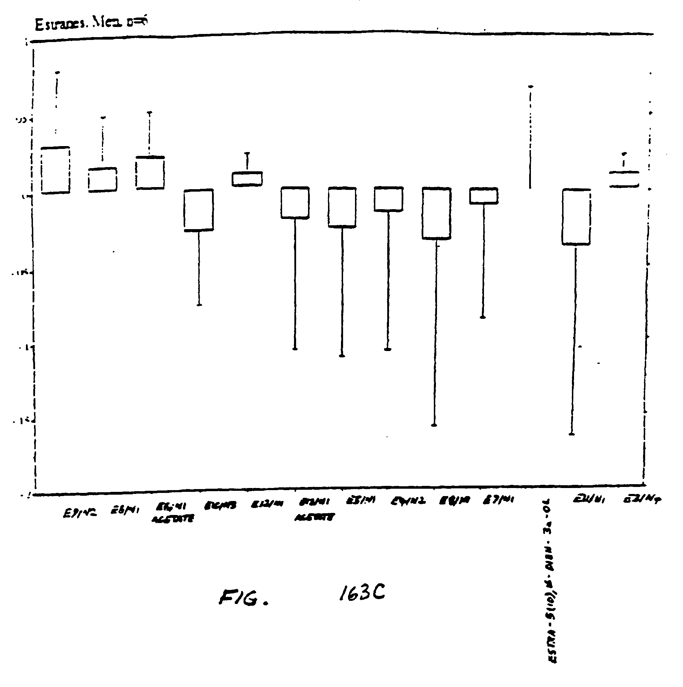

FIGS. 163A, 163B and 163C illustrate the EVG, GSR and ST data on men, respectively, for 13 estranes on Chart 1.

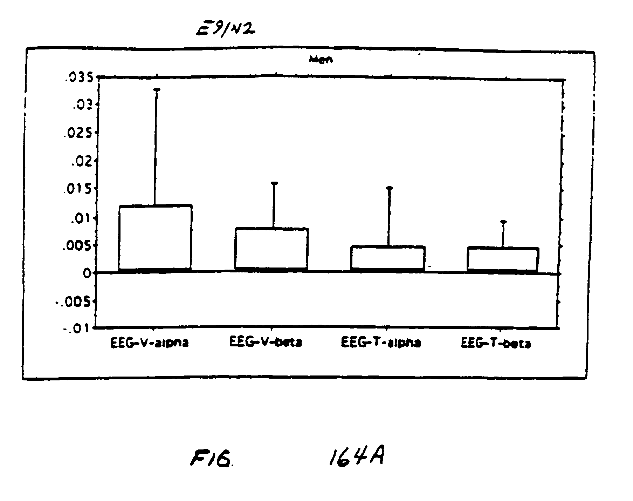

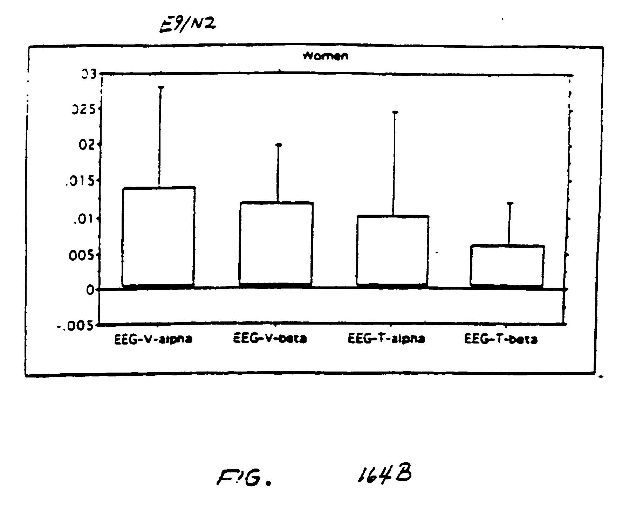

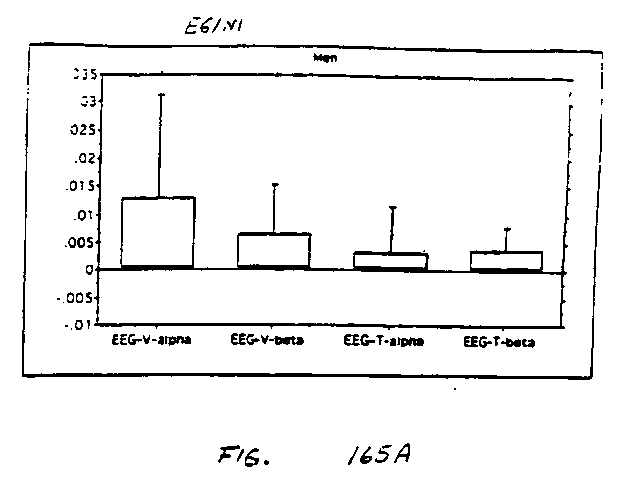

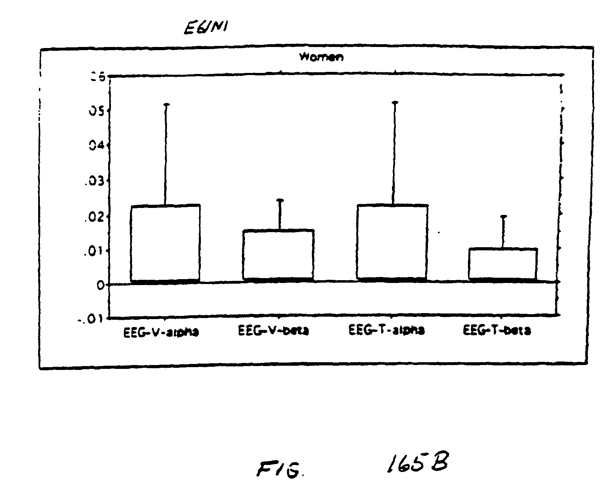

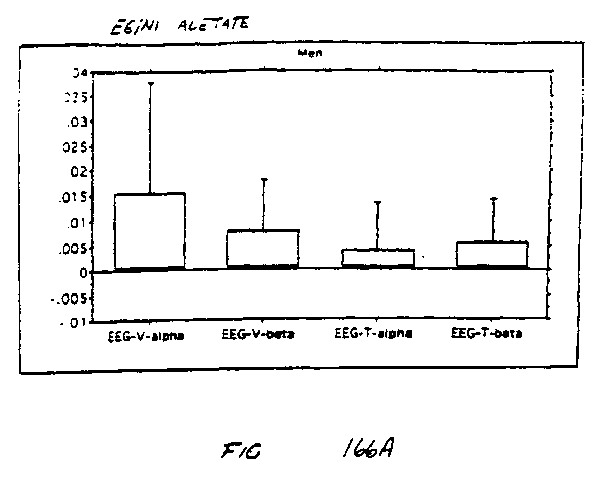

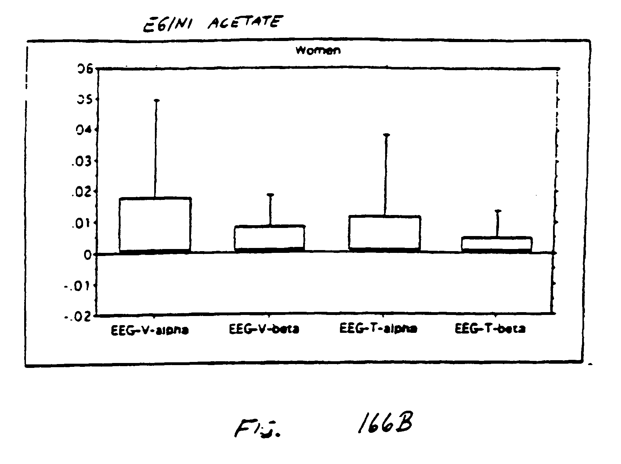

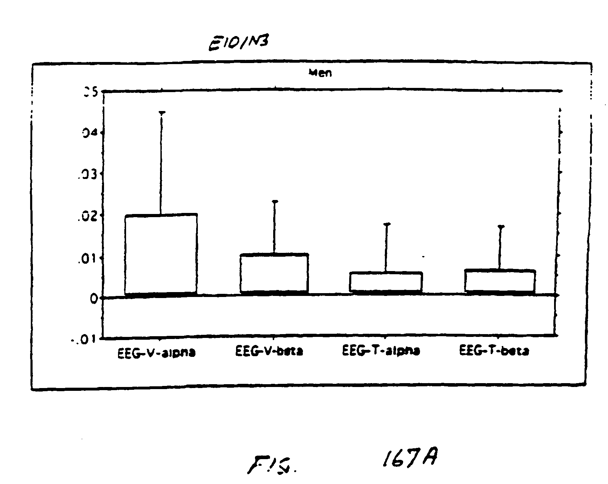

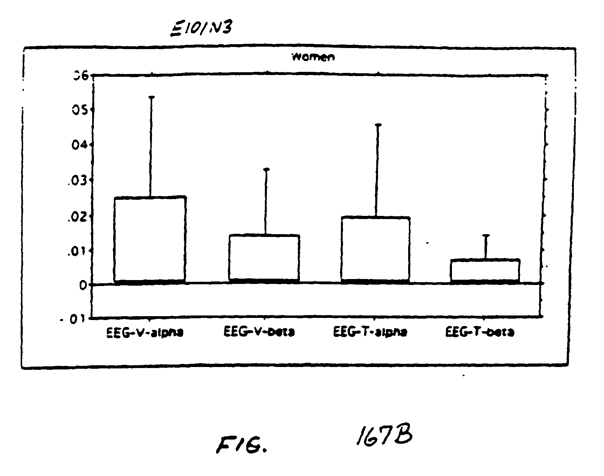

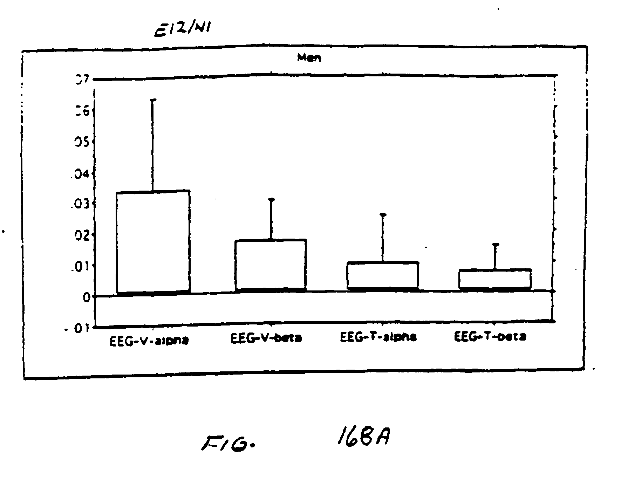

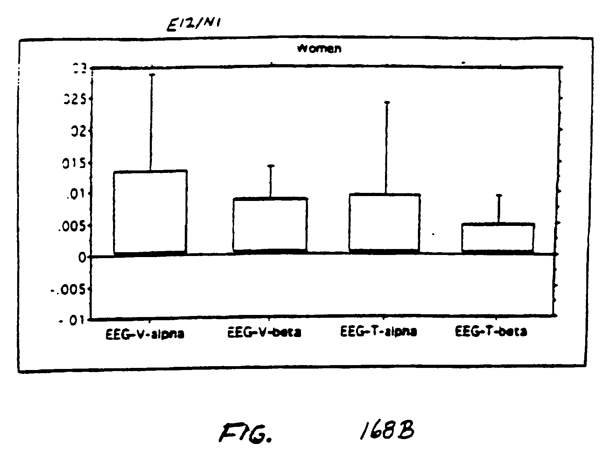

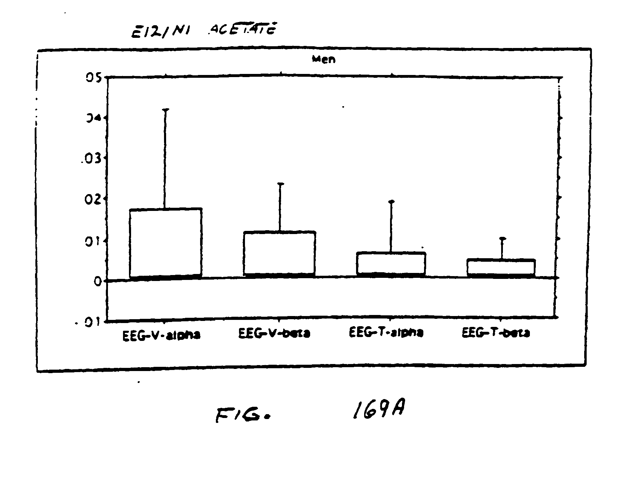

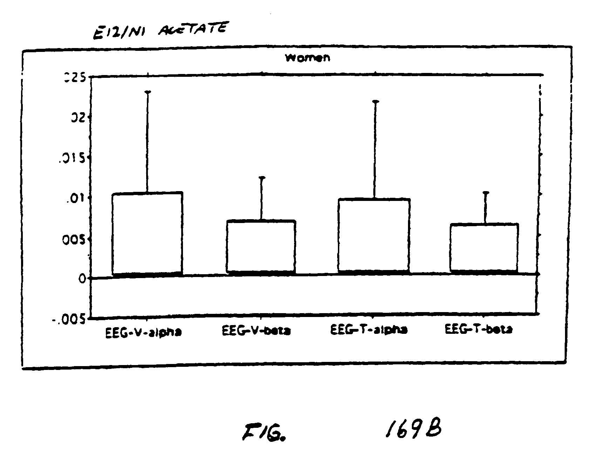

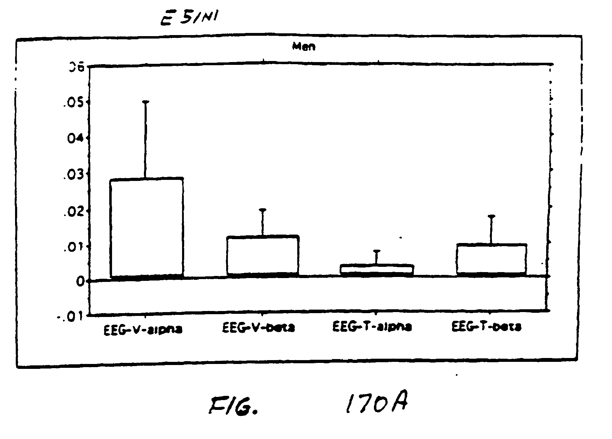

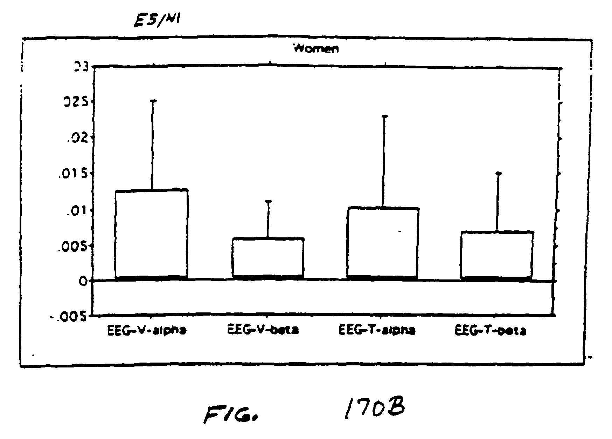

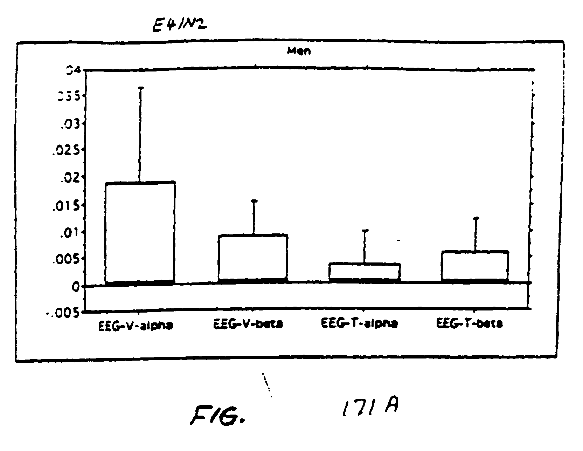

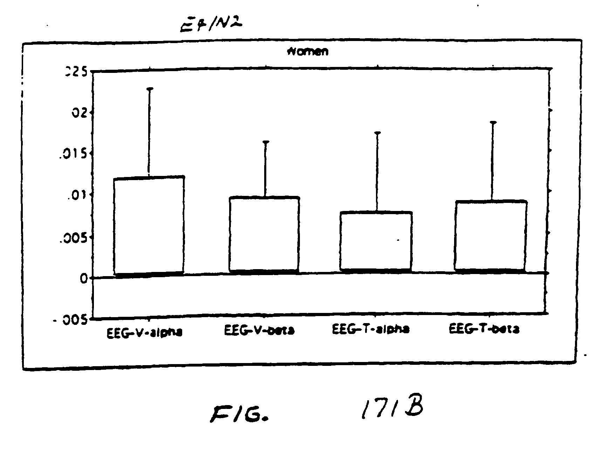

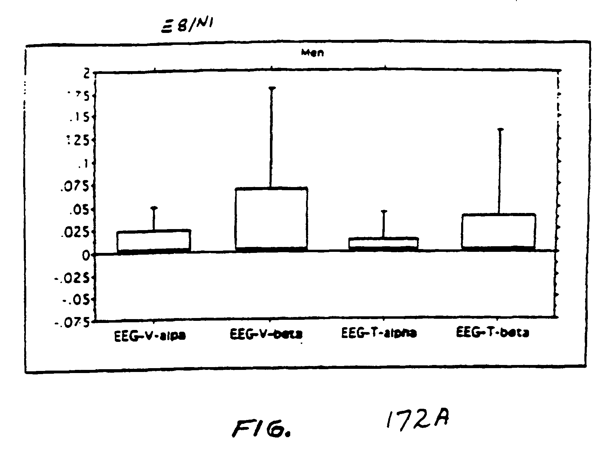

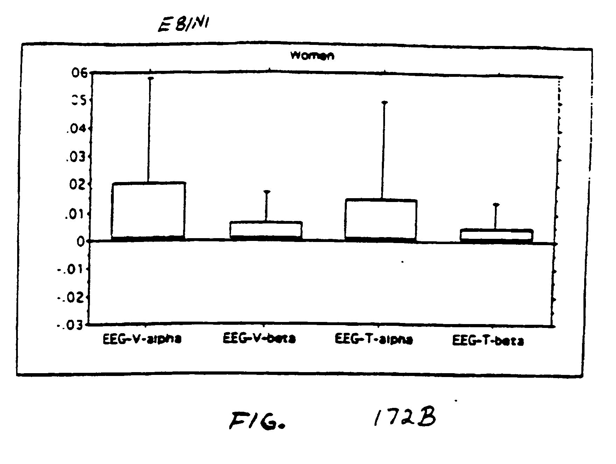

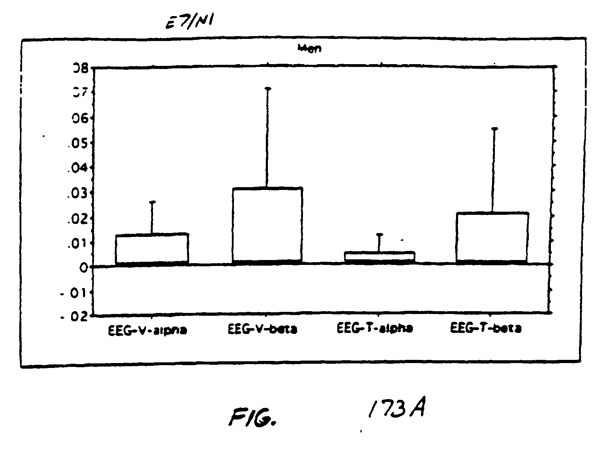

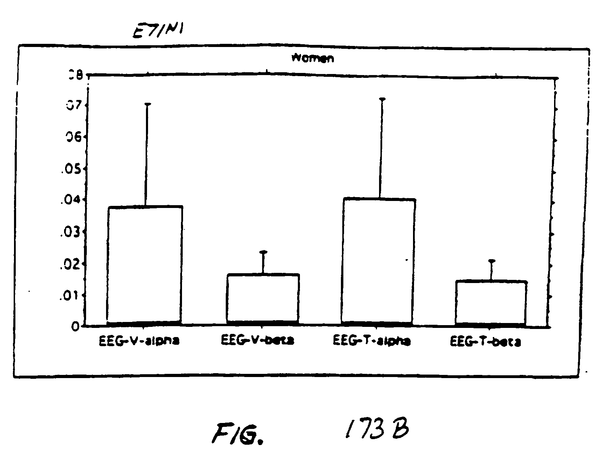

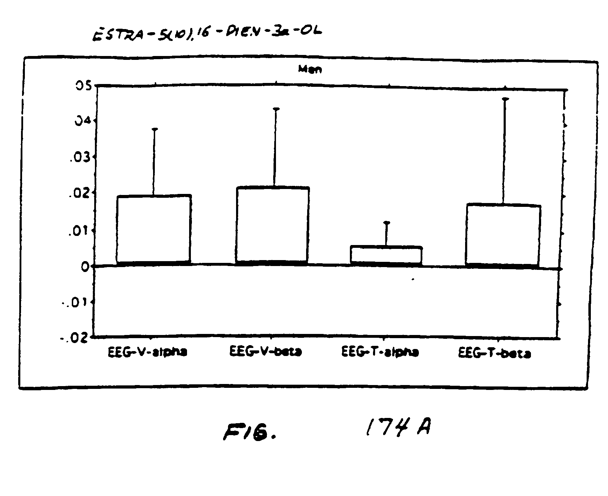

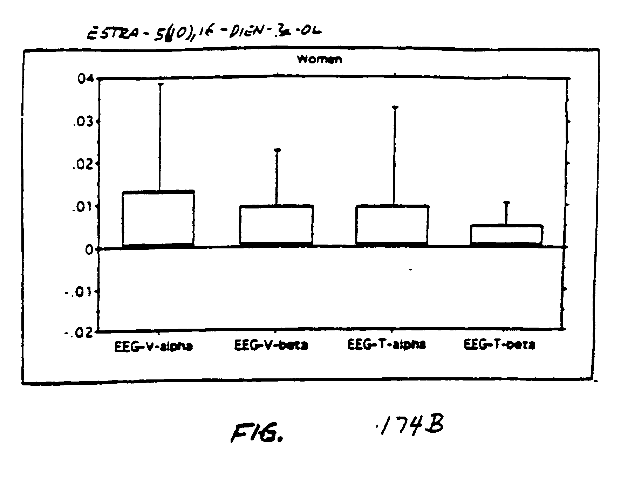

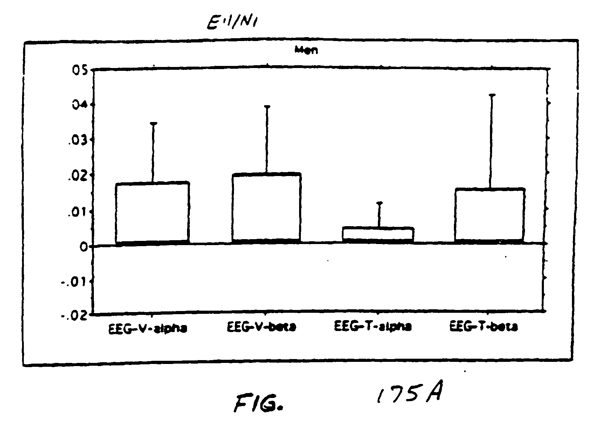

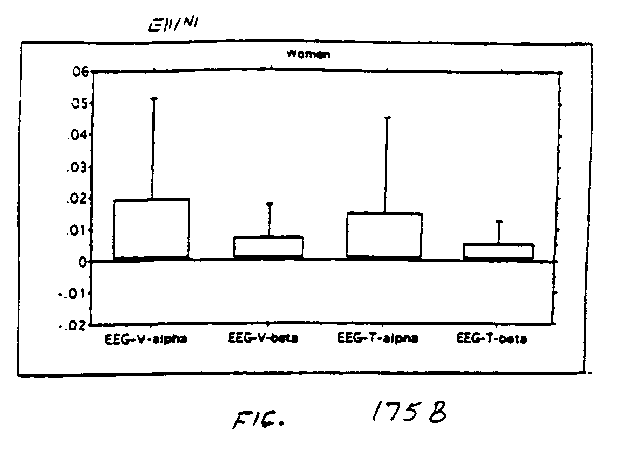

FIGS. 164A and 164B through 176A and 176B illustrate the EEG data on men (A) and women (B) for the 13 estranes, respectively, identified in FIGS. 163A-163C.

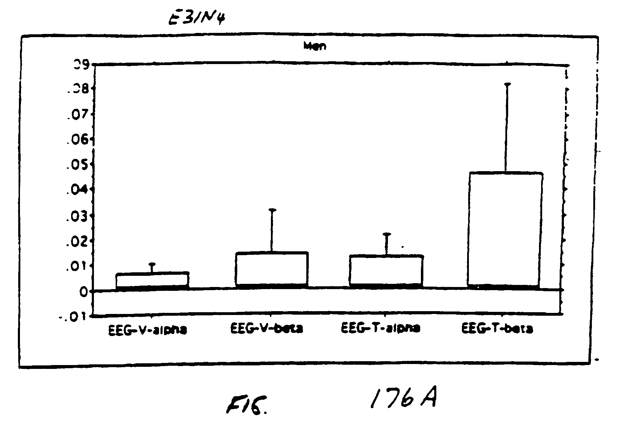

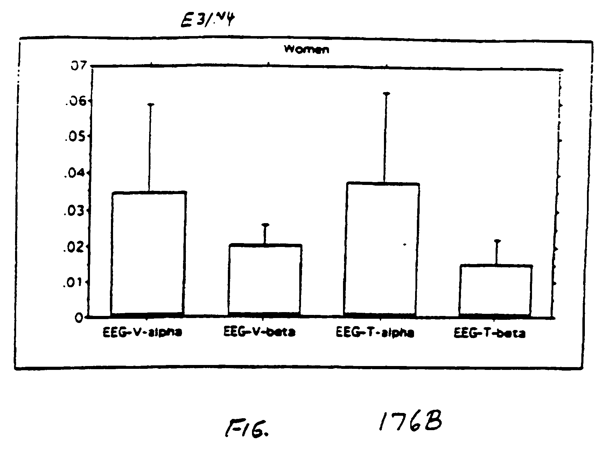

FIG. 177 illustrates the synthesis of Androsta-4,16-dien-3-one, Androsta-4,16-dien-3α-ol, and Androsta-4,16-dien-3β-ol.

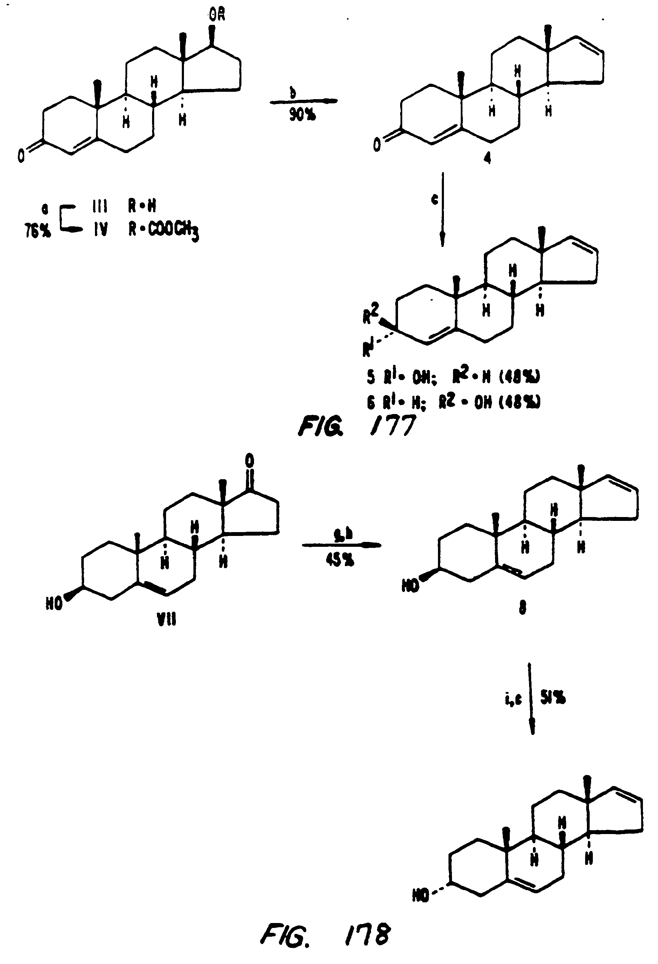

FIG. 178 illustrates the synthesis of Androsta-5,16-dien-3α-ol and Androsta-5,16-dien-3β-ol.

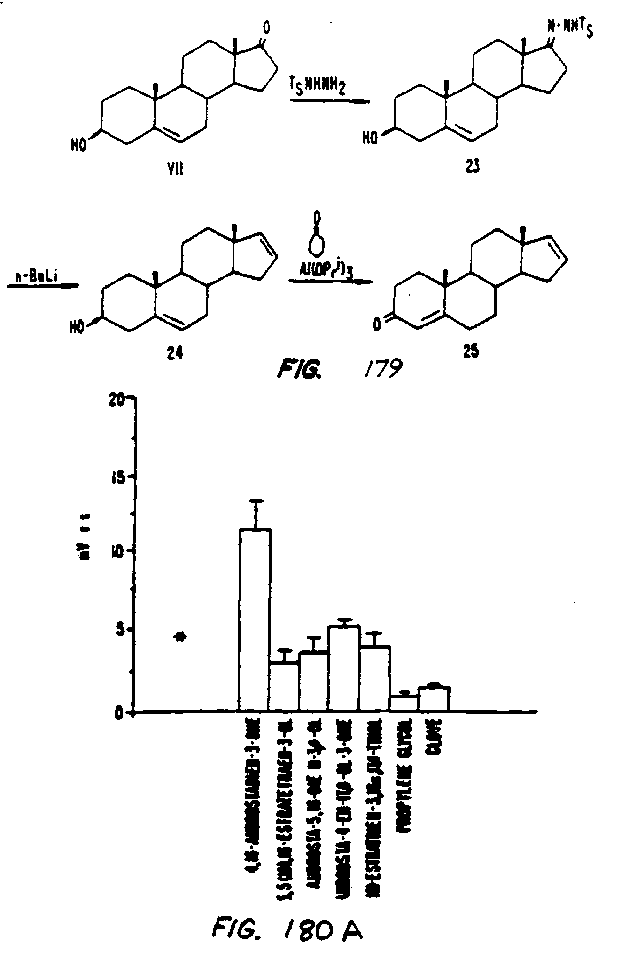

FIG. 179 illustrates an alternate synthesis of Androsta-4, 16-dien-3-one.
FIG. 180 is a graphic representation of the electrophysiological effect on receptor potential of the localized administration of particular steroids to the vomeronasal organ of female subjects (180A) and to the olfactory epithelium (180C). FIG. 180B is a graphic comparison of the effect of an Androstane on the VNO receptor potential of male and female subjects.

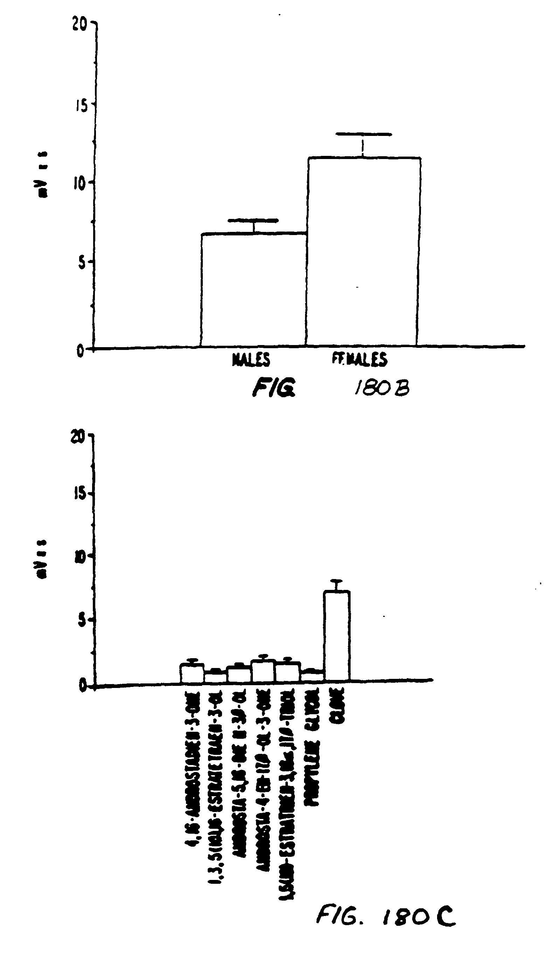

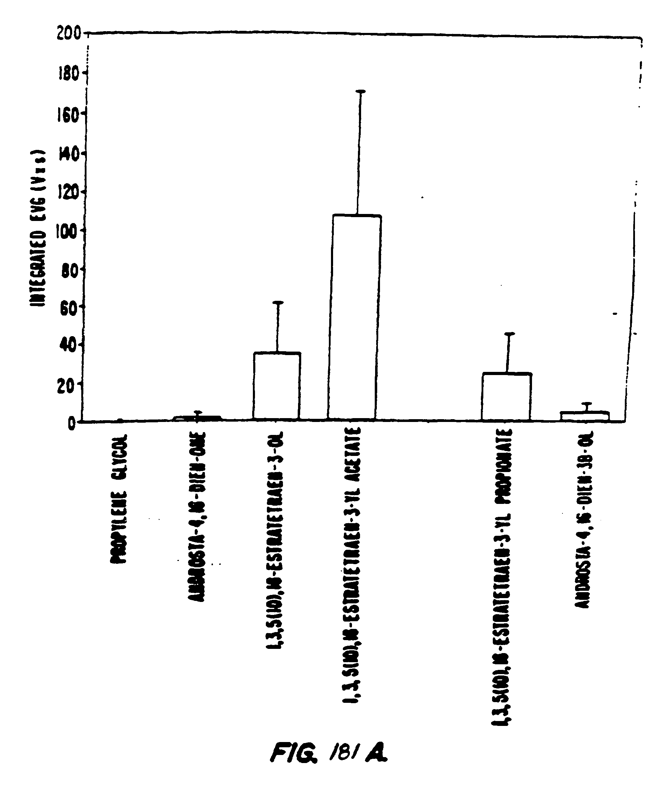

FIG. 181 is a graphic representation of the electrophysiological effect of the localized administration of particular steroids to the vomeronasal organ of male (182A) and female (182B) subjects.

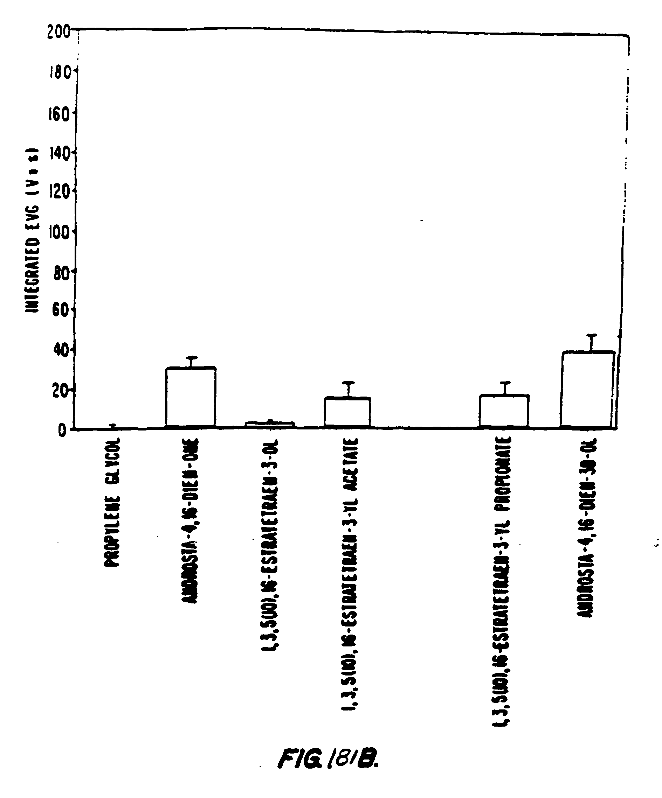

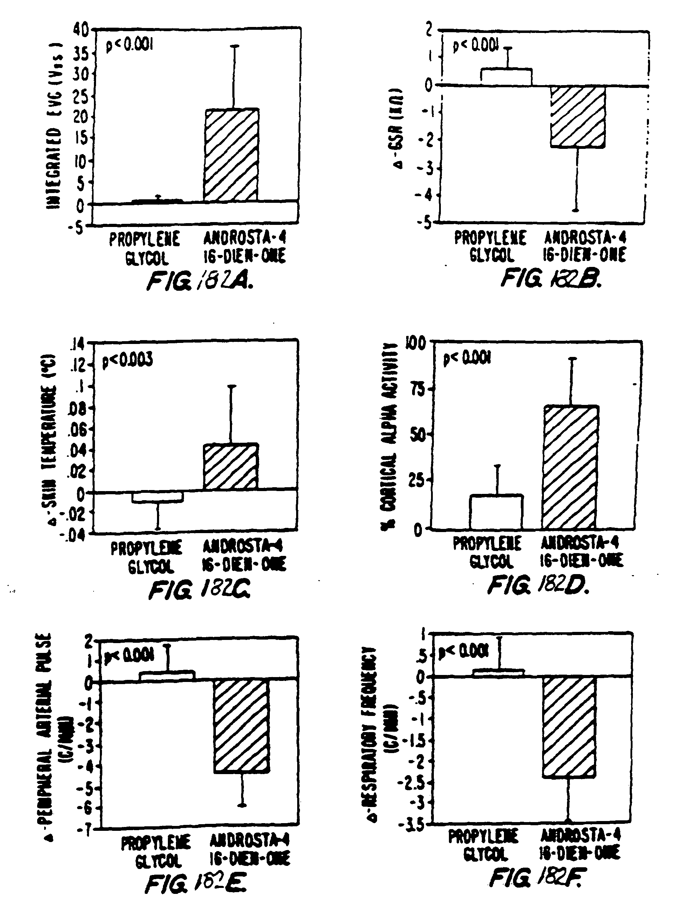

FIG. 182A through 182F depicts various autonomic responses of female subjects to an Androstane. A = receptor potential of the vomeronasal neuroepithelium; B = change in cortical alpha activity of an electroencephalogram (%); C = change in galvanic skin response (K-ohms); D = change in peripheral arterial pulse (counts/min.); E = change in skin temperature (degrees C.); and, F = change in respiratory frequency (counts/min.).

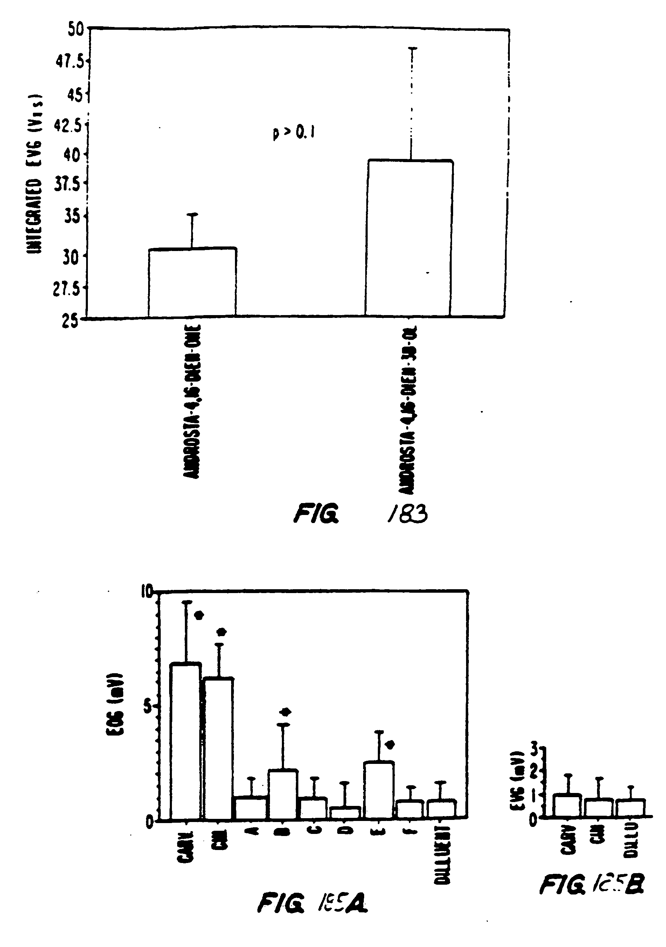

FIG. 183 depicts changes in receptor potential of the VNO after exposure of 5 females to two different Androstanes.

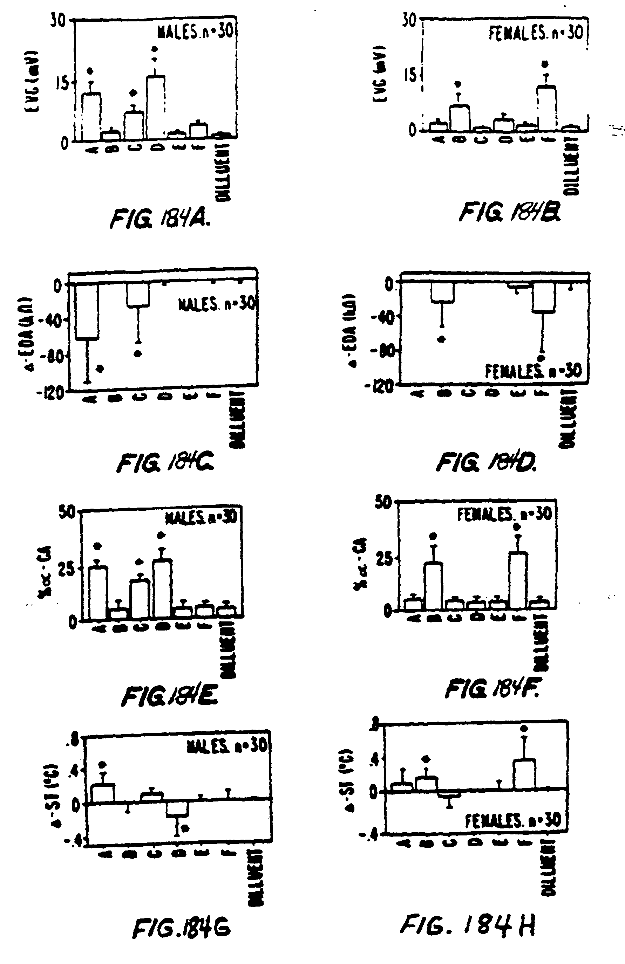

FIG. 184 depicts sexual dimorphism in local and autonomic responses to the stimulation of the VNO with vomeropherins. Various vomeropherins (200 fmoles) and the diluent control were administered to 30 male and 30 female subjects (ages 20 to 45) as described. Bars indicate the mean response of the population.
FIGS. 184A and 184B: EVG responses were measured as described in male (A) and female (B) subjects.
FIGS. 184C and 784D: Electrodermal activity was measured as described. Changes (measured in *x*Ω) in response due to delivery of vomeropherins to the VNO of each subject are shown in male (C) and female (D) subjects.
FIGS. 184E and 184F: Alpha-cortical activity was measured as described. Changes in response due to delivery of vomeropherins to the VNO of male (E) and female (F) subjects.
FIGS. 184G and 184H: Skin temperature (ST) was measured as described. Changes in response due to delivery of vomeropherins to the VNO of each subject are shown in male (G) and female (H) subjects.
   The compounds in the graphs are:
   A = 1, 3, 5(10),16-Estratetraen-3-yl acetate
   B = Androsta-4,16-dien-3-one
   C = 1,3,5(10),16-Estratetraen-3-ol
   D = 3-Methoxy-Estra-1,3,5(10),16-tetraene
   E = Androsta-4,16-dien-3a-ol
   F = Androsta- 4,16-dien-3β-ol
FIG. 185 depicts electro-olfactograms of male and female subjects induced by stimulation of the OE with olfactants and vomeropherins. FIG. 185A: 400 fmoles of the olfactants 1-carvone and cineole as well as 200 fmoles of the vomeropherins A, B, C, D and F; and the stereoisomer E were applied separately as one second pulses to the OE of 20 subjects (both male and female) and each EOG response was recorded as described. The olfactants as well as E and B produced significant (p<0.01) local response. FIG. 185B: 400 fmoles of the olfactants 1-carvone and cineole do not induce a significant EVG response when delivered to the VNO of male and female subjects.

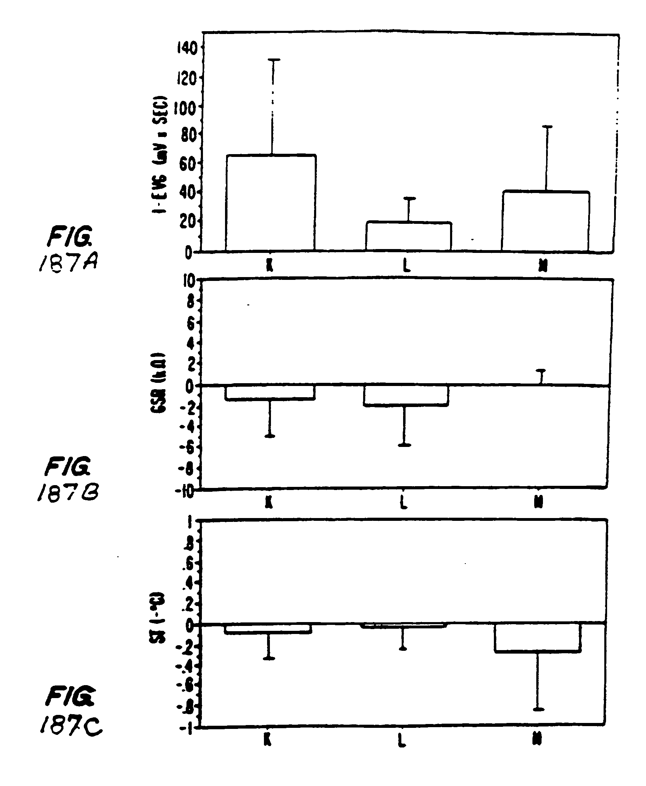

FIG. 186 depicts the electrophysiological effect of the following vomeropherins on the vomeronasal organ of 20 female subjects:
   G = Androst-4-en-3-one
   H = Androsta-4,16-diene-3,6-dione
   J = 10,17-Dimethylgona-4,13 (17)-dien-3-one
   K = 1,3,5 (10),16-Estratetraen-3-ol-methyl ether
   L = 1,3,5 (10),16-Estratetraen-3-yl-propionate
   EVG = Electro-vomeronasogram
   GSR = Galvanic Skin Response
      = Electrodermal Activity, EDA
   ST = Skin Temperature
FIG. 187 depicts the electrophysiological effect of vomeropherins on the vomeronasal organ of 20 male subjects.
   M = 1,3,5(10)-Estratrien-3-ol
FIG. 188 depicts the steps of synthesis for Examples 108 through 112.

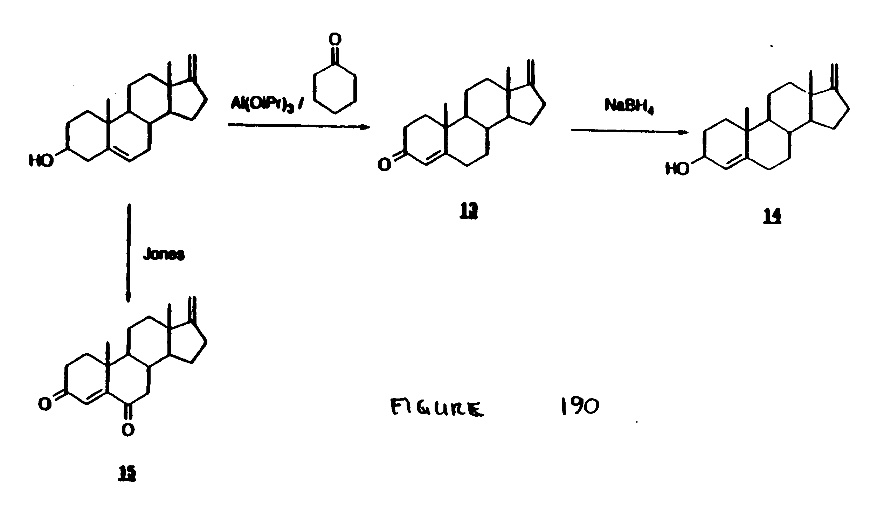

FIG. 189 illustrates the steps of synthesis for Examples 113 through 118.

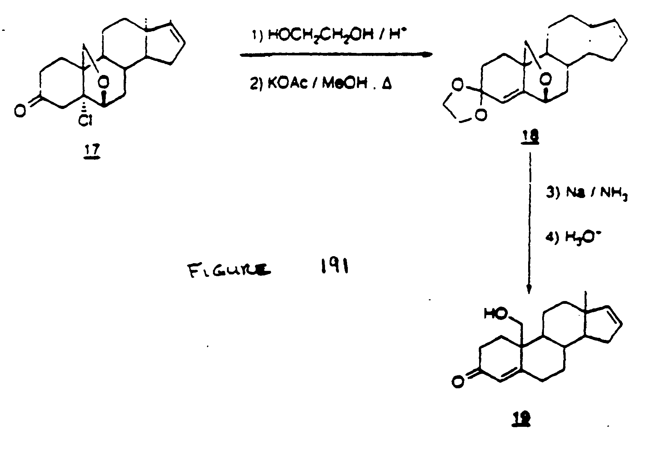

FIG. 190 illustrates the steps of synthesis for Examples 120 through 121.

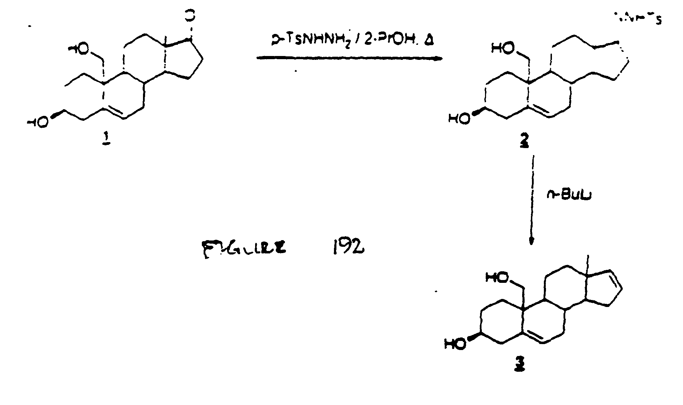

FIG. 191 illustrates the steps of synthesis described in Examples 123 through 124.
FIG. 192 illustrates the steps of synthesis for Examples 125 through 126.

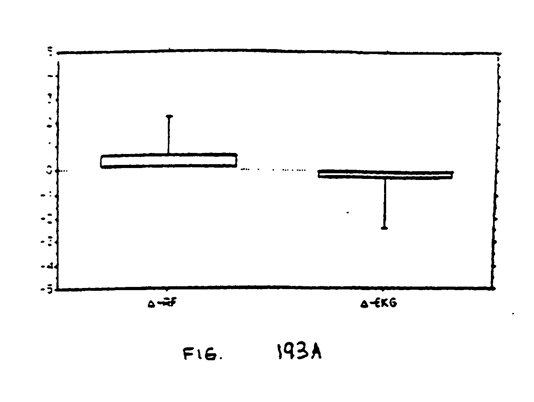

FIG. 193A shows the respiratory frequency and EKG data in females for tests of androsta-5,16-diene-3β,19-diol in the VNO.

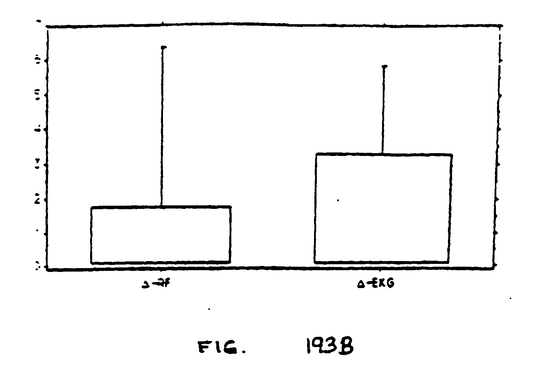

FIG. 193B shows the respiratory frequency and EKG data in females for tests of androsta-5,16-diene-3β,19-diol in the VNO.

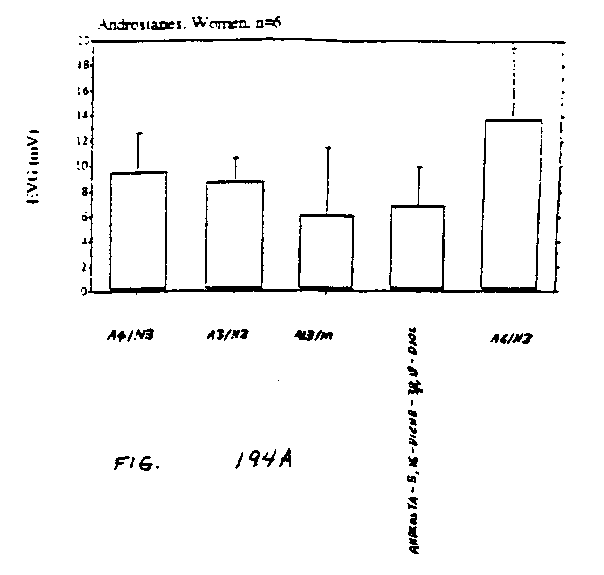

FIG. 194A, 194B and 194C show the EVG, GSR, and ST data in women for four androstanes on the chart and androsta-5,16-diene-3β,19-diol.

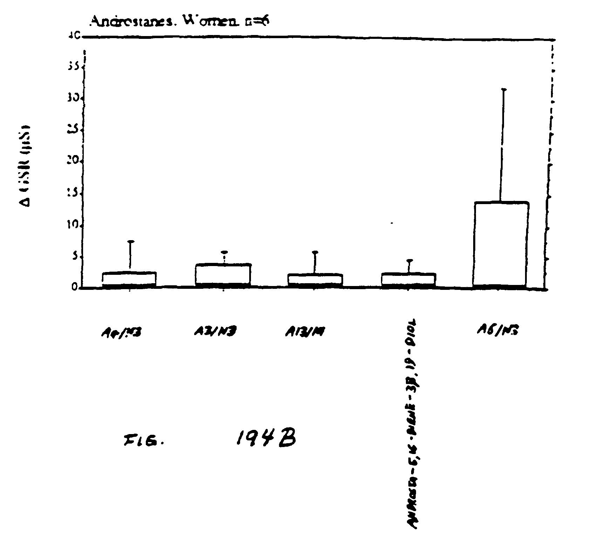

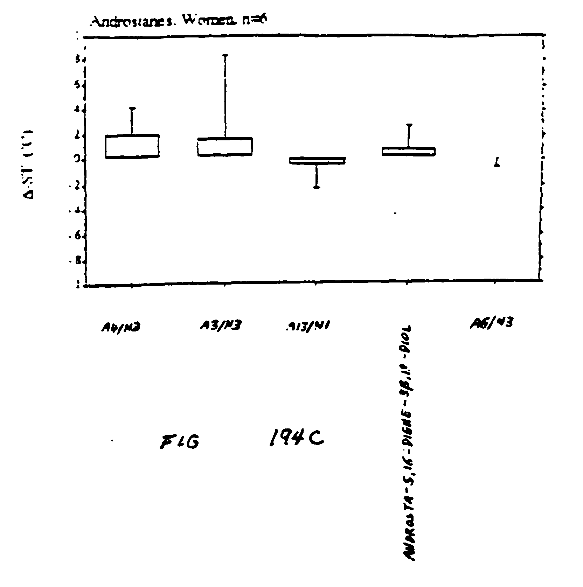

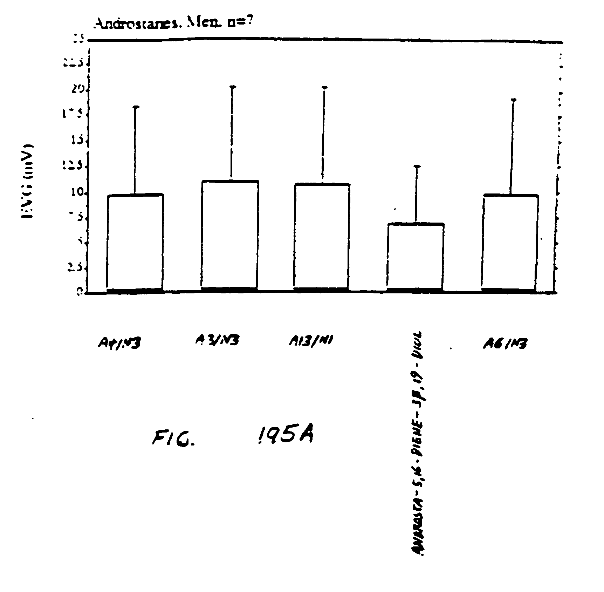

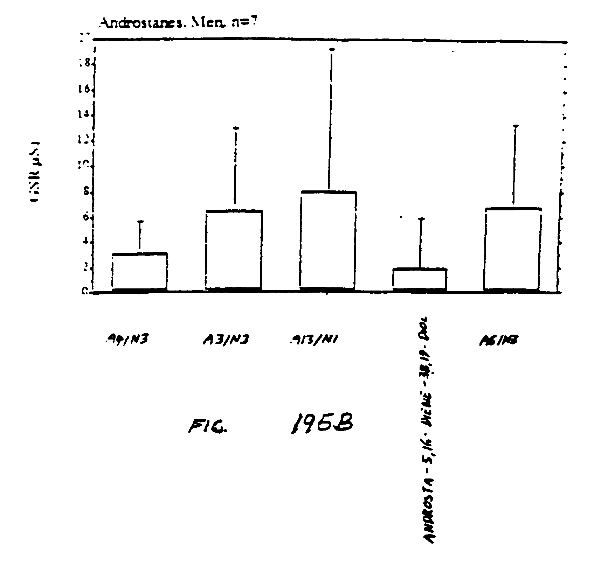

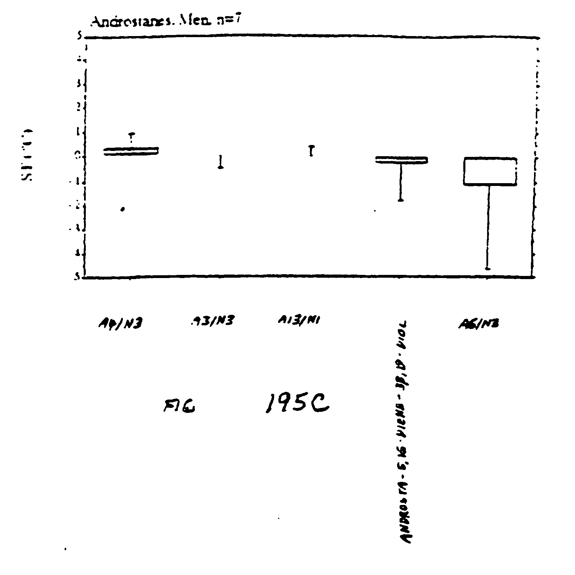

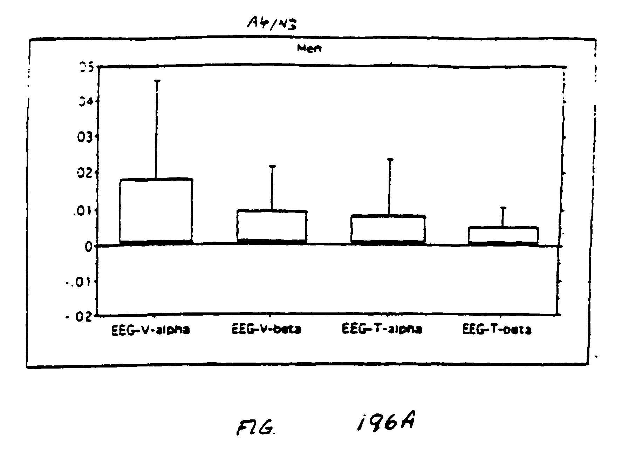

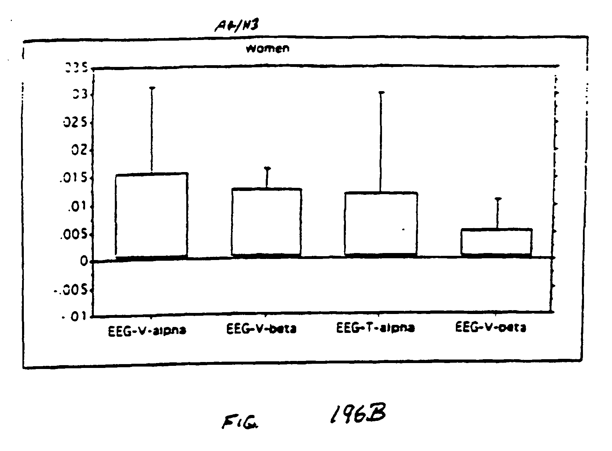

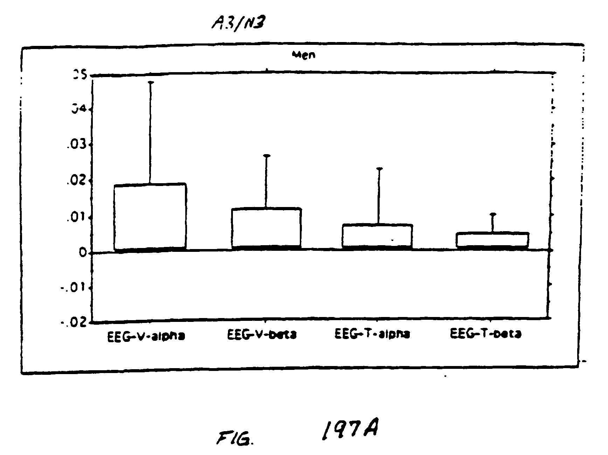

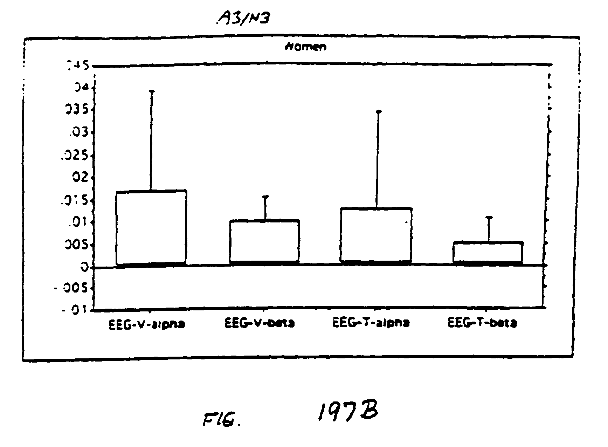

FIG. 195A, 195B and 195C show the EVG, GSR and ST data in men for the five androstanes identified in FIG. 194.

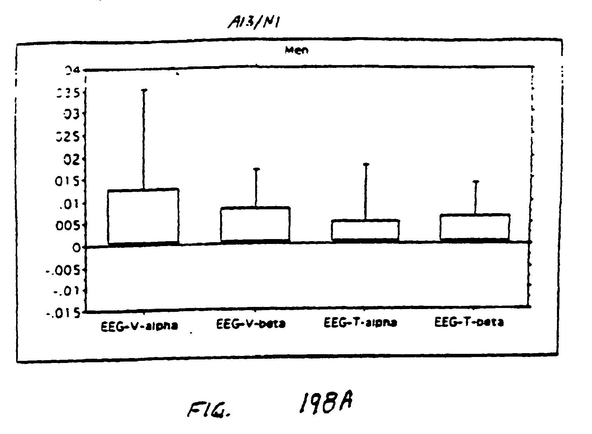

FIG. 196A and 196B show the EEG data in men and women for androstane A4/N3.

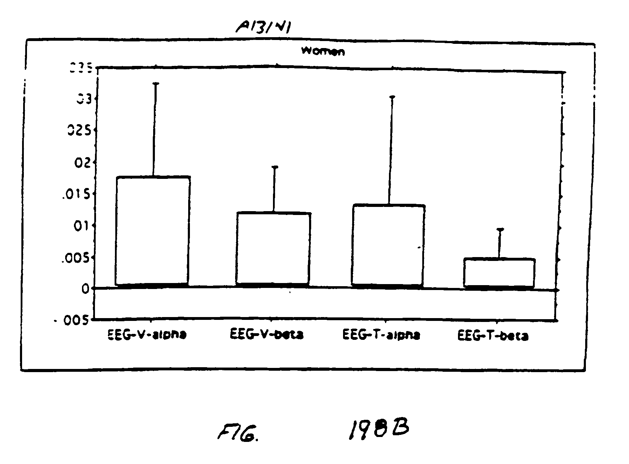

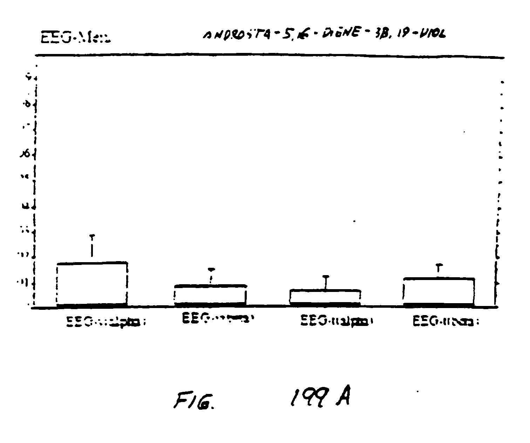

FIGS. 197A and 197B show the EEG data in men and women for androstane A3/N3.

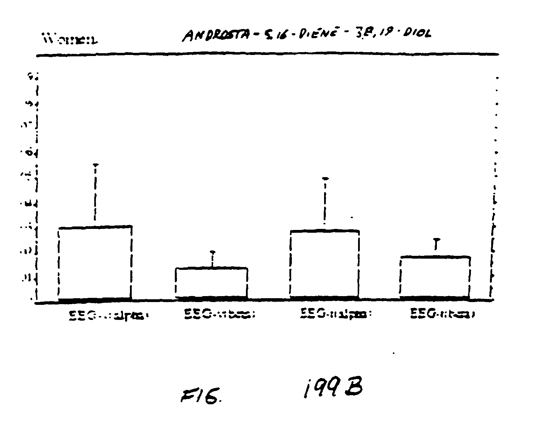

FIGS. 198A and 198B show the EEG data in men and women for androstane A13/N1.

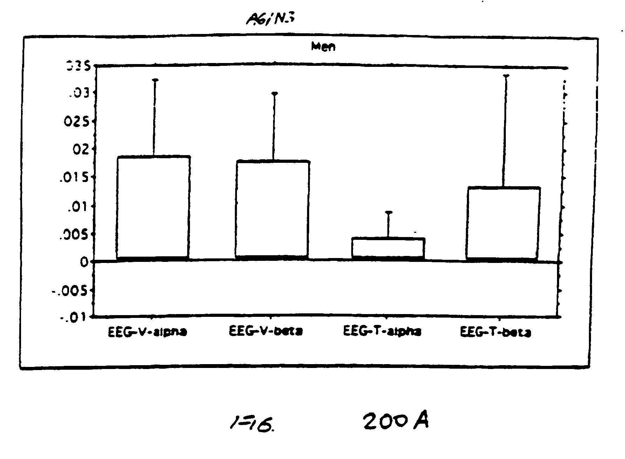

FIGS. 199A and 199B show the EEG data in men and women for androst-5,16-dien-3β,19-diol.

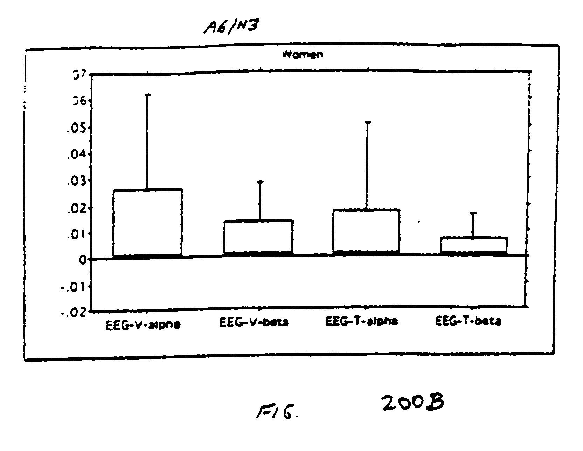

FIGS. 200A and 200B show the EEG data in men and women for androstane A6/N3.

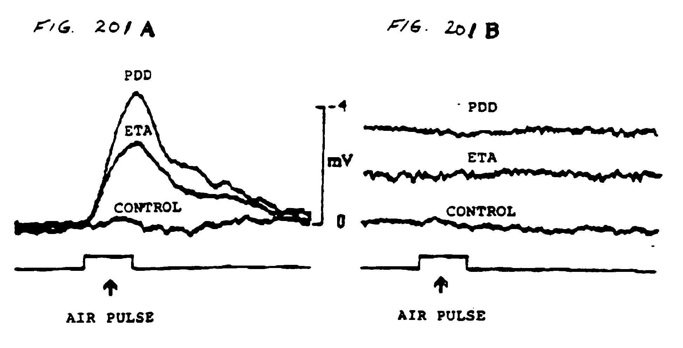

FIGS. 201A and 201B are traces of EVGs in the VNO of a male subject, tested with two vomeropherins (FIG. 201A), and electrograms from the nasal respiratory mucosa (FIG. 201B) using the same vomeropherins.

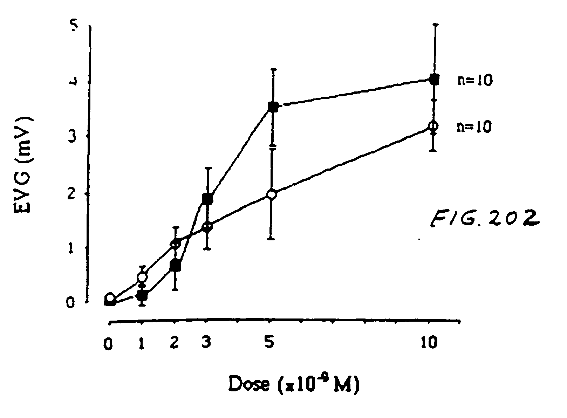

FIG. 202 shows the dose dependent effect of two vomeropherins in male subjects.

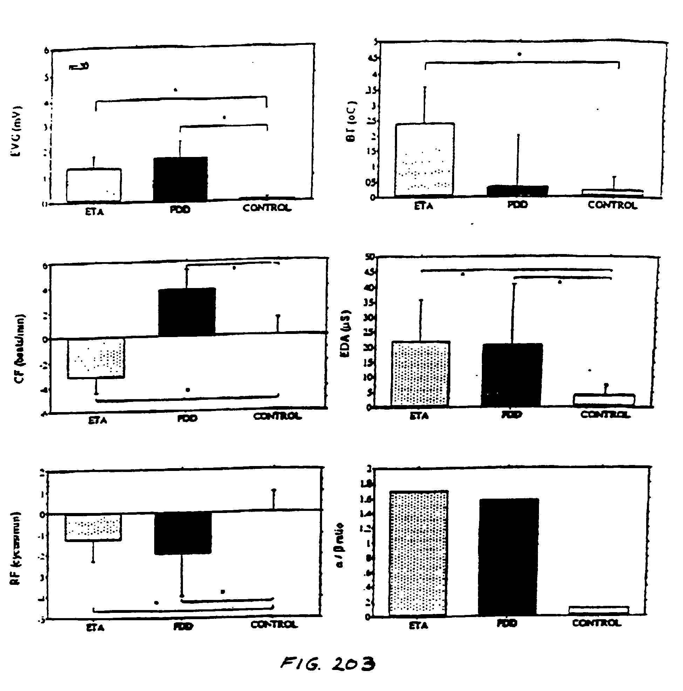

FIG. 203 shows the central nervous system reflex response of two vomeropherins.

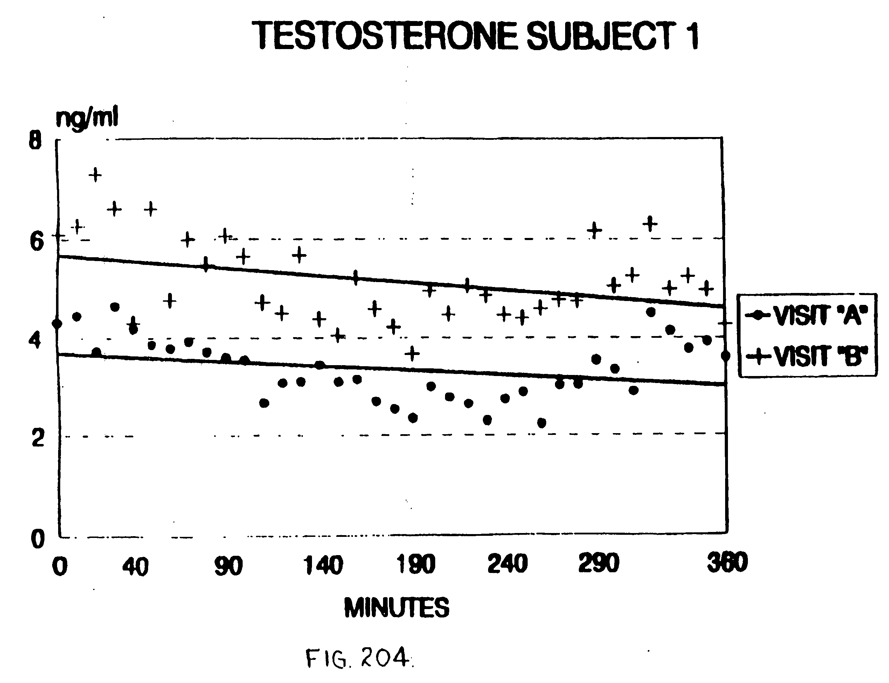

FIG. 204 shows the difference in testosterone levels in a subject administered in one visit a placebo (B curve) to the VNO, and on a second visit (A curve), the compound pregna-4,20 dien-3,6-dione.

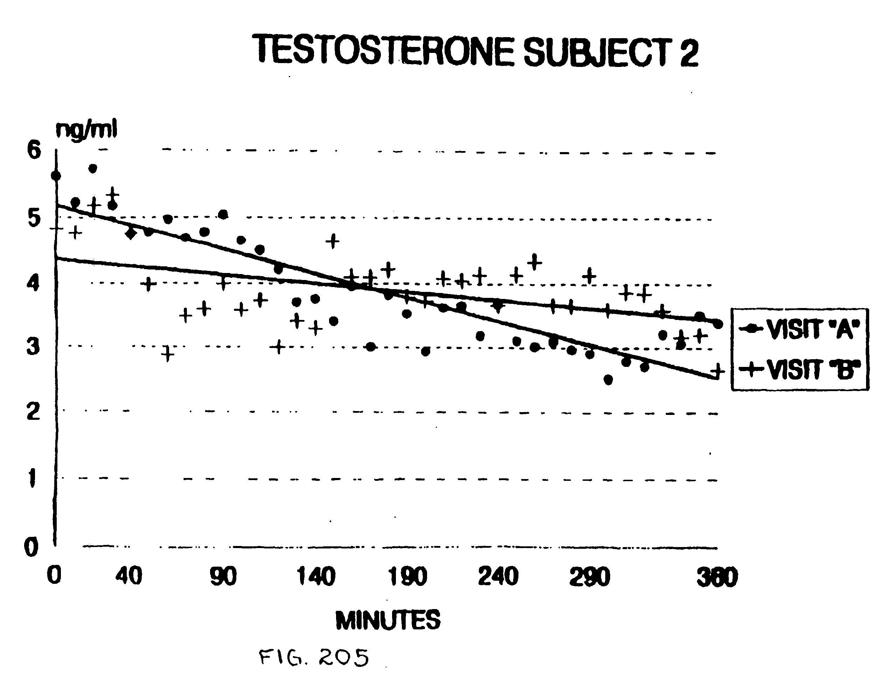

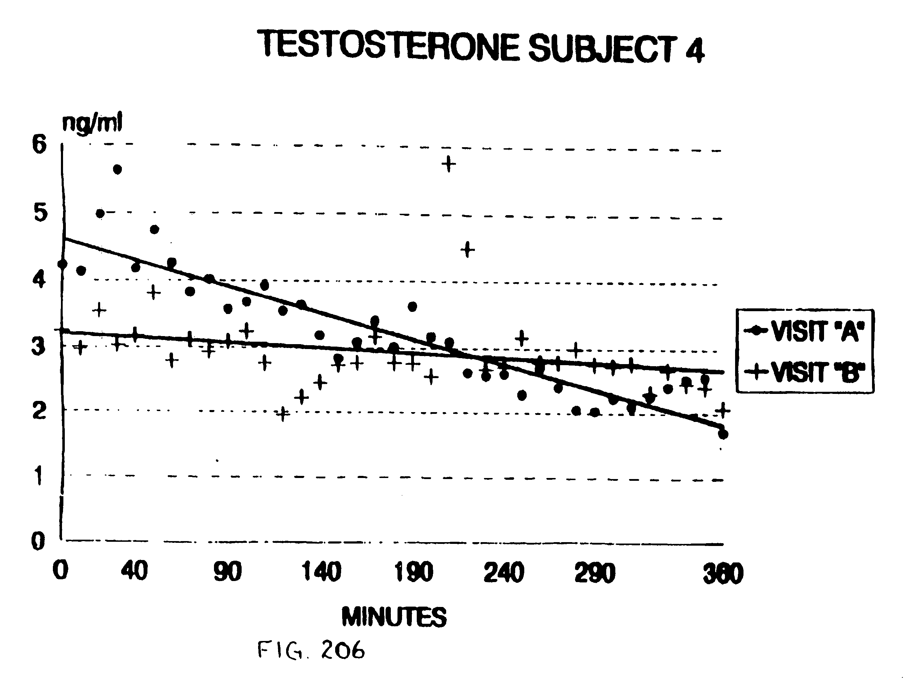

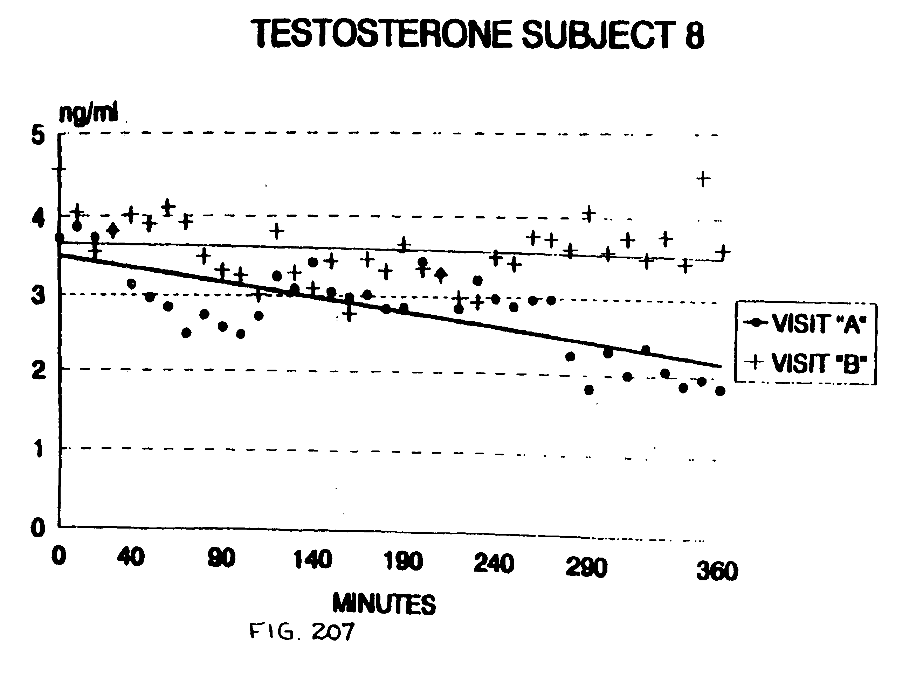

FIGs. 205, 206 and 207 show the data for the testosterone tests on three additional subjects given a placebo, in case B, and pregna -4,20-dien-3,6-dione in case A.

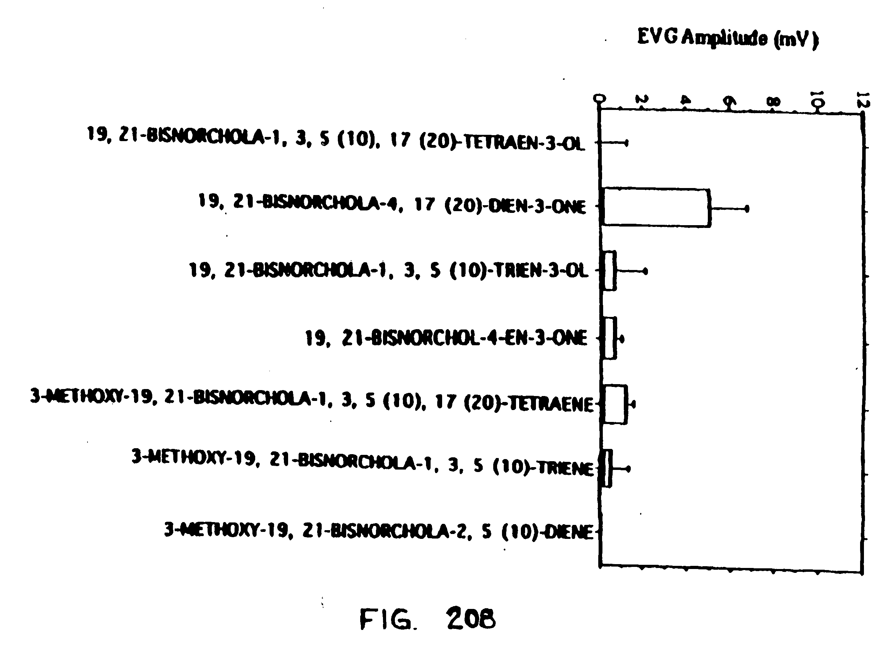

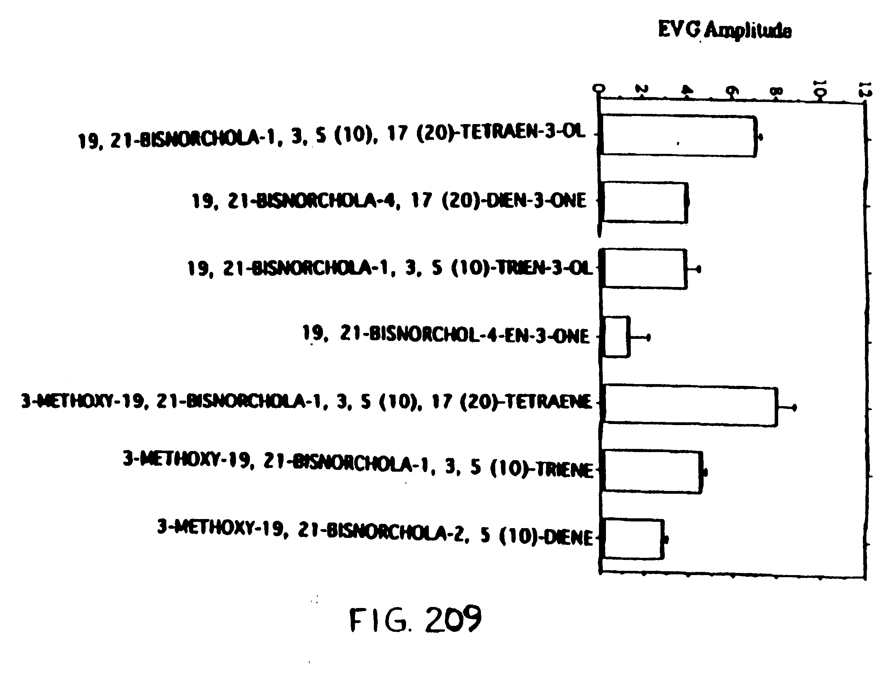

FIG. 208 and FIG. 209 show the amplitudes of steroids E2/NC2, E1/NC2, E2/NC3, E1/NC3, methylated E2/NC2, methylated E2/NC3 and E8/NC3 in Chart VI, in human male and female VNO's, respectively.

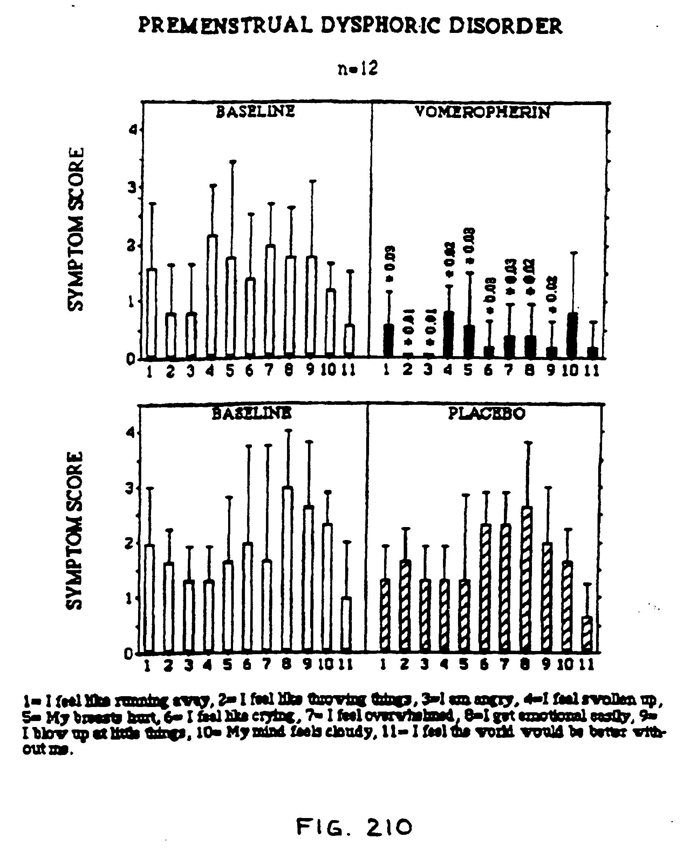

FIG. 210 shows the results on eleven PMDD symptoms in women administered a placebo or estra-4,16-dien-10β-ol-3-one.

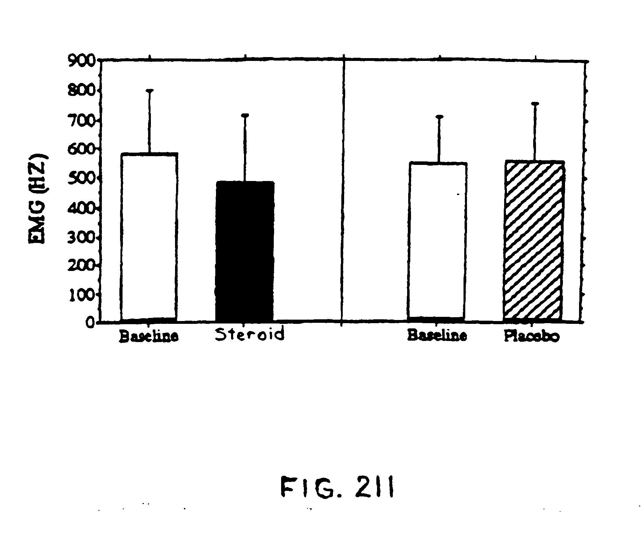

FIG. 211 shows the electromyogram results in women administered a placebo or estra-4,16-dien-10β-ol-3-one.

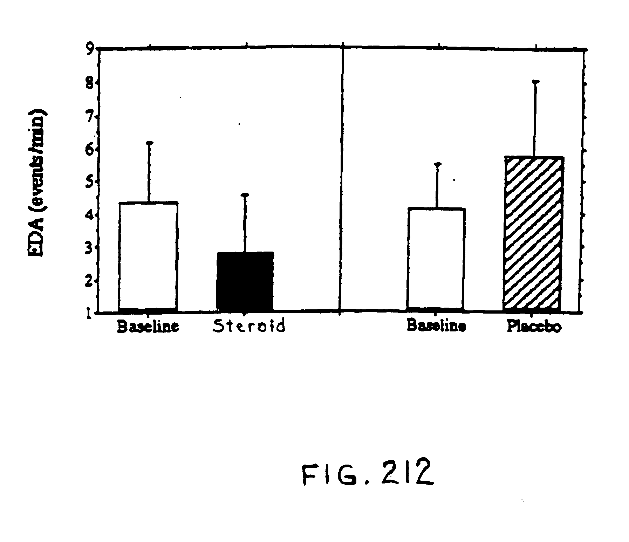

FIG. 212 shows the frequency of electrodermal activity events in women administered on placebo or estra-4,16-dien-10β-ol-3-one.

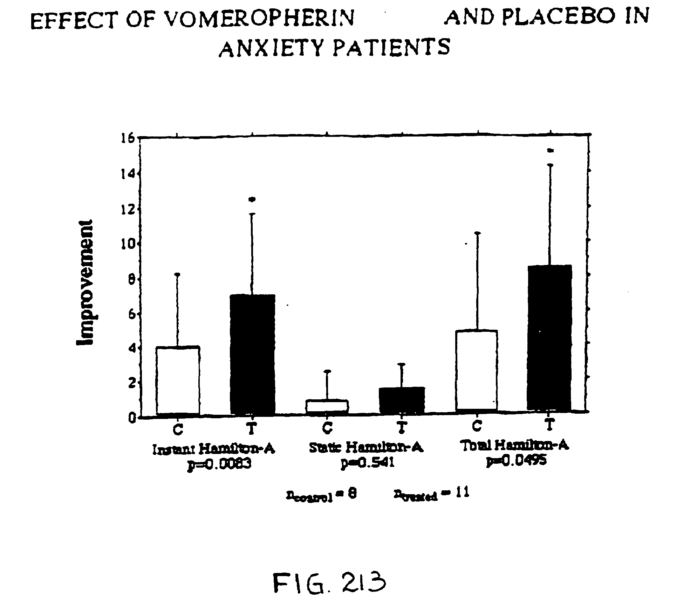

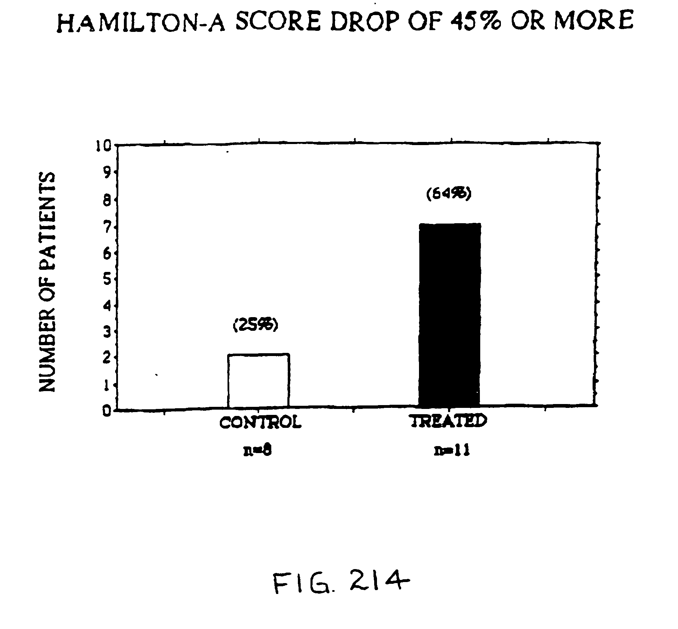

FIG. 213 and 214 show the results of Hamilton-A anxiety tests for treatment to the VNO of anxious patients with androsta-4,16-dien-3β-ol.

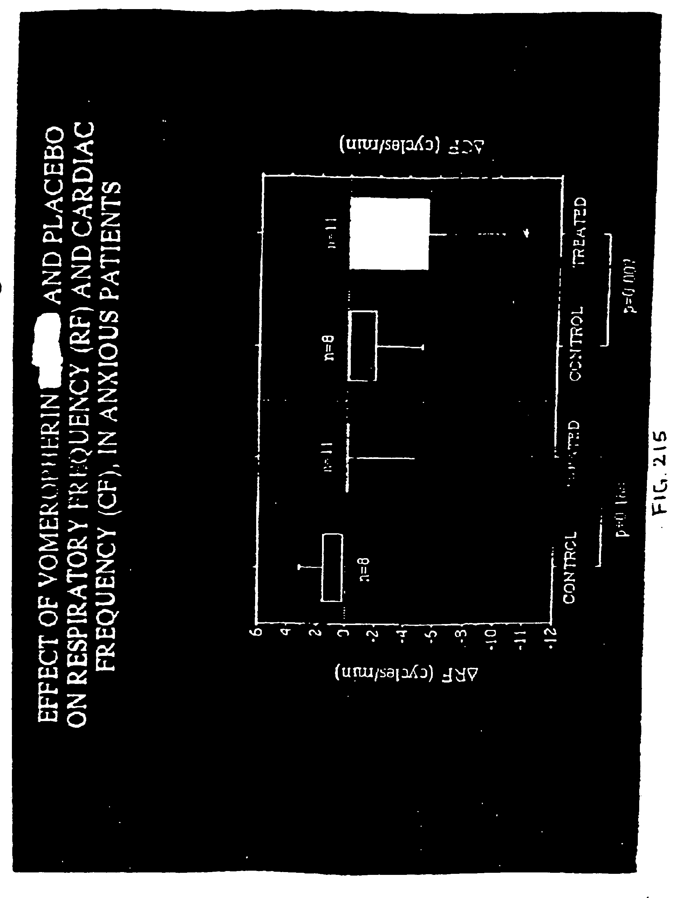

FIG. 215 shows the results on effect on respiratory frequency and cardiac frequency for treatment to the VNO of anxious patients with androsta-4,16-dien-3β-ol.
FIG. 216 shows the results on parasympathetic tone for treatment to the VNO of anxious patients with androsta-4,16-dien-3β-ol.

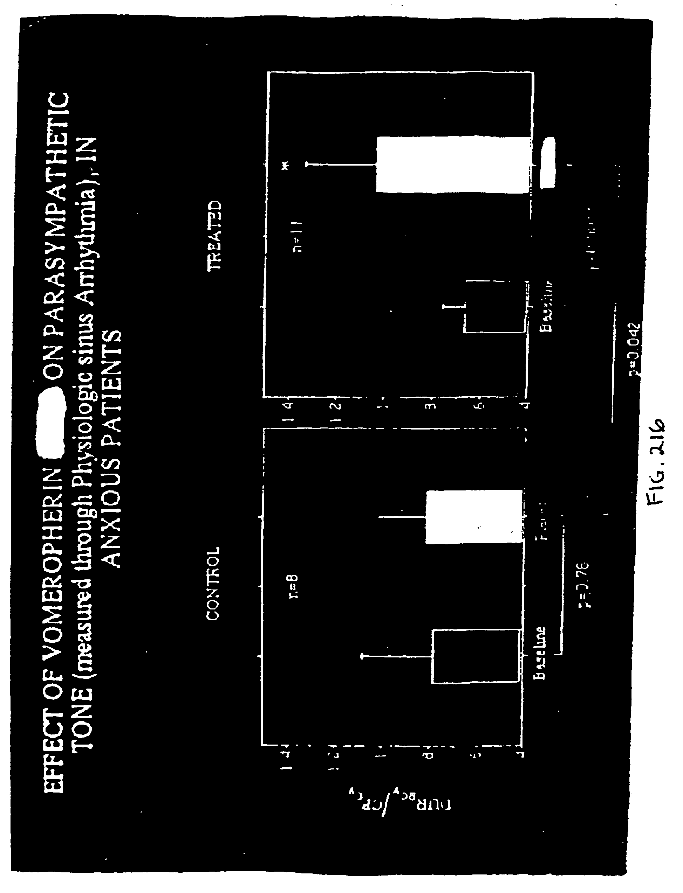

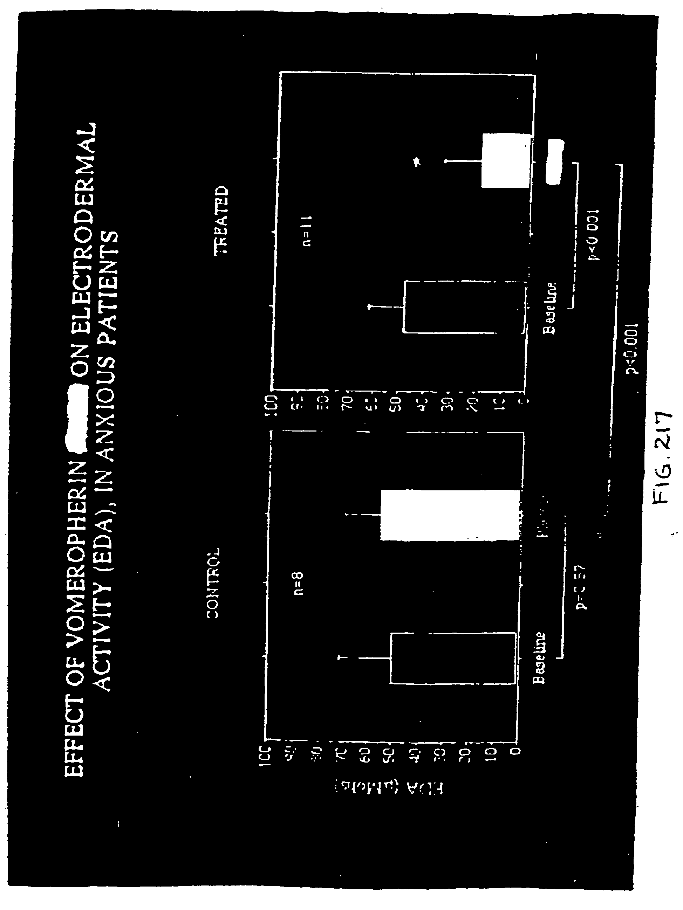

FIG. 217 shows the results on electrodermal activity for treatment to the VNO of anxious patients with androsta-4,16-dien-3β-ol.

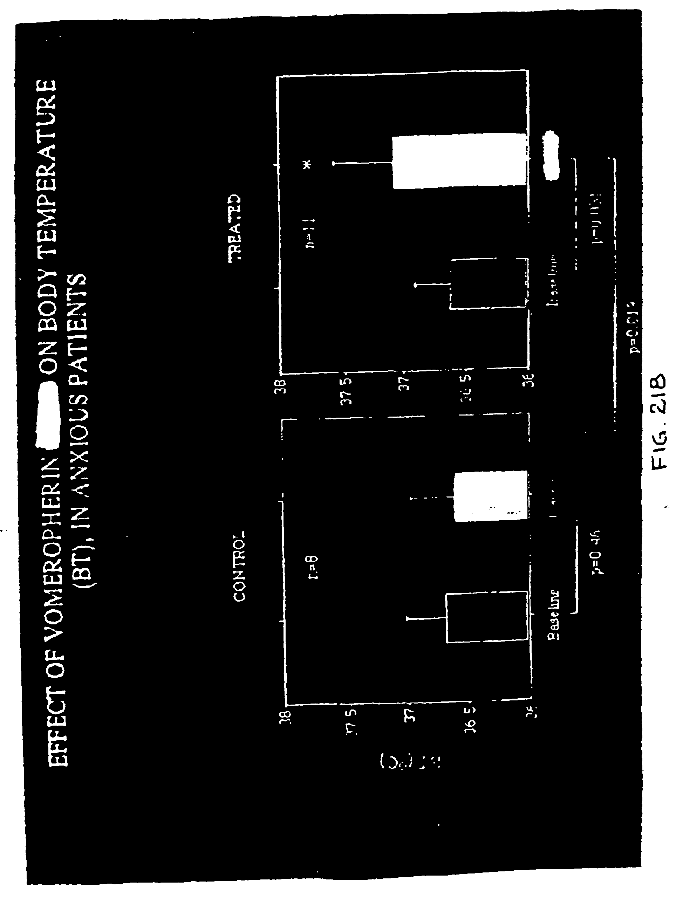

FIG. 218 shows the results on change in body temperature for treatment to the VNO of anxious patients with androsta-4,16-dien-3β-ol.

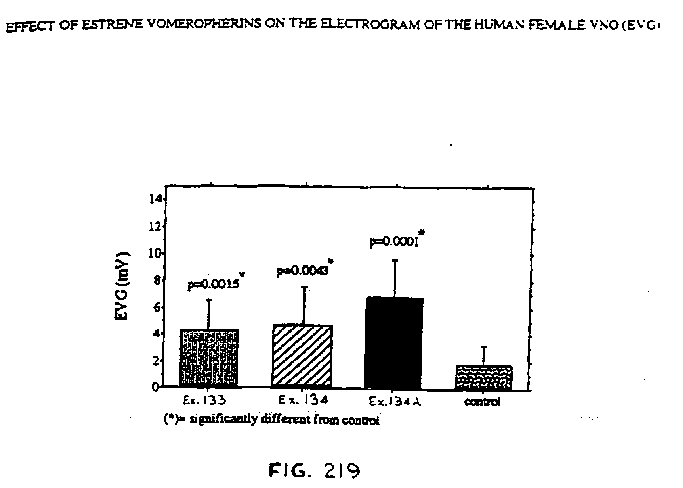

FIG. 219 shows the results of EVG tests on women administered the compounds of Examples 133, 134 and 134A.

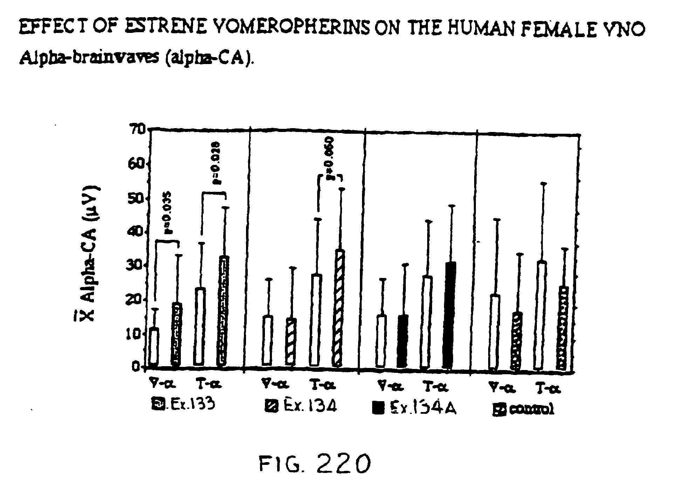

FIG. 220 shows the results of alpha brainwave testing on women administered the compounds of Examples 133, 134 and 134A.

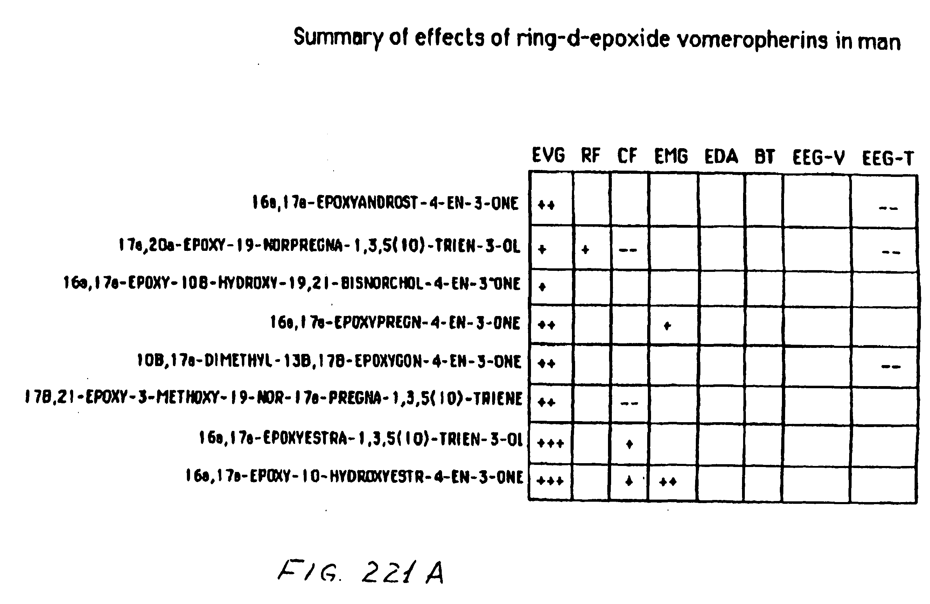

FIG. 221A is a summary of electrophysical effects due to stimulation of the VNO in male subjects by eight steroids having epoxy groups on ring D.

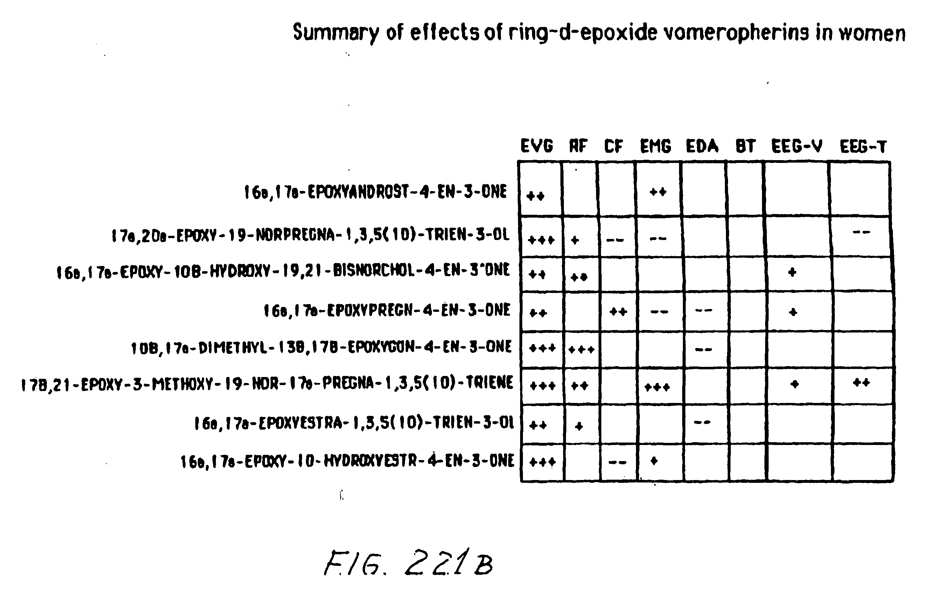

FIG. 221B is a summary of electrophysical effects due to stimulation of the VNO in female subjects by eight steroids having epoxy groups on ring D.

### Detailed Description of the Invention

### I. Definitions

An "affect" is a transient feeling state. Typical negative affects are feelings of nervousness, tenseness, shame, anxiousness, irritability, anger, rage and the like. "Moods" are longer lasting feeling states such as guilt, sadness, hopelessness, worthlessness, remorsefulness, misery, unhappiness and the like. "Character traits" are more permanent aspects of an individual's personality. Typical negative character traits are sensitivity, regretfulness, blameworthiness, stubbornness, resentfulness, bitterness, timidness, laziness and the like.

The vomeropherins according to the present invention may have use for stimulating, through contact with the VNO, of one or more of the hormonal, behavioral and autonomic functions of the hypothalamus. Due to the predominant role played by the hypothalamus in a wide variety of internal body functions and the neural connection between the VNO and the hypothalamus, the vomeropherins according to the present invention are in a position to stimulate such functions as endocrine output control, for example, the control of the pituitary output of vasopressin and oxytocin as well as a number of other peptides. Vasopressin is an anti-diuretic hormone because of its action within the kidney to enhance water uptake and to concentrate the urine. In addition, it has an action within the body to regulate blood pressure through its action on arterial smooth muscle and an action on metabolism through its enhancement of glycogen conversion to glucose in the liver. Oxytocin, receptors of which are found on uterine smooth muscle and on mammary smooth muscle, can cause milk letdown via contraction of the mammary smooth muscle and cause uterine contractions during birth. The hypothalamus also controls release of hormones from the anterior pituitary gland such as ACTH, prolactin, LH (Luteinizing Hormone), GH (Growth Hormone), TSH (Thyroid Stimulating Hormone), FSH (Follicle Stimulating Hormone) and beta-endorphin. Thus, for example, the ability to control LH secretion may lead to control of fertility, in females, or testosterone production in males. Testosterone production may be utilized for treatment of conditions such as low libido in males or for treatment of muscle wasting diseases or conditions, such as aging. Testosterone reduction may be utilized for treatment of conditions such as prostate cancer.

Control of behavior hypothalamic functions is also feasible by use of the vomeropherins according to the present invention. It is known that the hypothalamus controls such behavioral outputs as fear, rage, pleasure and circadian rhythms which regulate sleep and wakefulness. Other functions controlled by the hypothalamus include appetite, thirst, sympathetic functions such as, flight and fight, and functions such as cardiovascular control, thermoregulation and visceral functions such as control of the gut muscle and acid secretion for digestion. Thus, while there is a multitude of sensory inputs into the hypothalamus from various parts of the anatomy it is believed that the vomeropherins of the present invention provide, for the first time, a way of stimulating through the nasal cavity by inhalation to contact the epithelial cells in the VNO, a method of stimulating functions of the hypothalamus discussed above.

"Pregnane steroids" are aliphatic polycyclic hydrocarbons characterized by a four-ring steroidal structure with a methylation at the 10- and 13-positions and ethylation (including unsaturated groups) at the 17-position. A pregnane is a subset of pregnanes commonly understood to mean that the compound has at least one double bond. Furthermore, all derivatives which have the structural characteristics described above are also referred to generically as pregnane steroids.

A "cholane steroid" is an aliphatic polycyclic hydrocarbon characterized by a four-ring steroid structure, with methylation at the 10- and 13-positions and a 2-pentyl group (including unsaturated groups) at the 17-position.

A "chemoreceptor" is a receptor molecule displayed on the surface of a "chemosensory" neuroepithelial cell which binds in a stereospecific fashion to a particular ligand or ligands. This specific binding initiates a signal transduction which initiates an afferent nerve impulse. Chemoreceptores are found, inter alia, in taste buds, olfactory epithelium and vomeronasal tissue.

"Pregnene steroids", as the term is used herein, are aliphatic polycyclic hydrocarbons wich a four-ring steroidal structure, at least one double bond in the A-ring, methylation at the 10-position and 13-position, ethylation (including unsaturated groups) at the 17-position and an oxo, hydroxyl or hydroxyl derivative such as alkoxy, ester, benzoate, cypionate, sulfate or glucuronide, at the 3-position. Derivatives which contain these structural characteristics are also referred to generically as pregnene steroids.

The following structure shows the four-ring steroidal structure common to steroids. For illustrative purposes, a side chain on the D-ring is shown for pregnane. In describing the location of groups and substituents, the following numbering system will be employed: 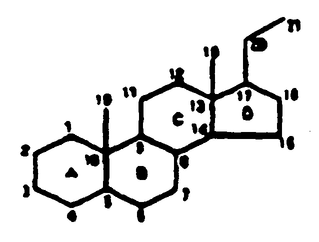 "Sexually dimorphic" refers to a difference in the effect of, or response to, a pharmaceutical agent between males and females of the same species.

An "effective amount" of a drug is a range of quantity and/or concentration which brings about a desired physiological and/or psychological effect when administered to an individual in need of the drug. In the present case, a needy individual is one with a physiological or behavioral trait which is normally regulated by the hypothalamus and wherein it is desirable to affect the function of the hypothalamus or the trait. The effective amount of a given drug may vary depending upon the function to be affected, the desired effect, route of administration, and the like. For example, when the steroid is administered as a solution applied to the facial skin of a subject an effective concentration is from 1 microgram/ml to 100 µg/ml, preferably 10 to 50 µg/ml and most preferably 20 to 30 µg/ml. When the steroid is introduced directly into the VNO an effective amount is about 1 picogram to about 1 nanogram, more preferably about 10 picograms to about 50 picograms. When the steroid is administered to the nasal passage, by ointment, cream or aerosol, or the like, an effective amount is about 100 pg to about 100 micrograms, preferably about 1 ng to about 10 micrograms. It follows that some drugs may be effective when administered by some routes, but not effective when administered by other routes.

The "hypothalamus" is the portion of the diencephalon comprising the ventral wall of the third ventricle below the hypothalamic sulcus and including structures forming the ventricle floor, including the optic chiasma, tuber cinereum, infundibulum, and mammillary bodies. The hypothalamus regulates the autonomic nervous system and controls several physiological and behavioral functions such as the so-called fight and flight responses, sexual motivation, water balance, sugar and fat metabolism, hunger, regulation of body temperature, endocrine secretions, and others. The hypothalamus is also the source of vasopressin which regulates blood pressure, and oxytocin which induces parturition and milk release. All hypothalamic functions are potentially modulatable by the vomeropherin therapy described herein.

A "ligand", as used herein, is a molecule which acts as a chemical signal by specifically binding to a receptor molecule displayed on the surface of a receptor cell, thereby initiating a signal transduction across the cell surface. Binding of ligands to chemosensory receptors can be measured. Chemosensory tissue, such as vomeronasal neuroepithelium or olfactory neuroepithelium, contains a multiplicity of neuroreceptors cells, each displaying at least one cell surface receptor. Many of the receptor molecules have identical ligand specificity. Therefore, when the tissue is exposed to a ligand for which it has specificity (for example a exposure of the VNO to a vomeropherin) a summated change in cell surface receptor potential can be measured.

As used herein, "lower alkyl" means a braced or unbranched saturated hydrocarbon chain of 1 to 4 carbons, such as, for example, methyl, ethyl, n-propyl, i-butyl and the like. "Alkoxy" as used herein is used in its conventional sense to mean the group -OR wherein R is alkyl as herein defined.

A "pheromone" is a substance that provides chemical means of communication between members of the same species through secretion and peripheral chemoreception. In mammals pheromones are usually detected by receptors in the vomeronasal organ of the nose. Commonly, pheromones effect development, reproduction and related behaviors. A "vomeropherin" is a more general term which includes pheromones and describes a substance from any source which functions as a chemosensory messenger, binds to a specific vomeronasal neuroepithelial receptor, and induces a physiological or behavioral effect. The physiologic effect of a "vomeropherin" is mediated through the vomeronasal organ.

A picogram (pg) is equal to .001 nanograms (ng). A ng is equal to .001 micrograms (µg). A µg is equal to .001 mg.

### II. Modes for Carrying Out the Invention

### A. Steroids useful in the Invention

The invention is directed to a group of certain steroids.

Syntheses are described herein for the following compounds as designated on the chart:

### C. Synthetic Methods

### 1. Preparation of 3-, 6-, 19-, 20- and 21- position derivatives.

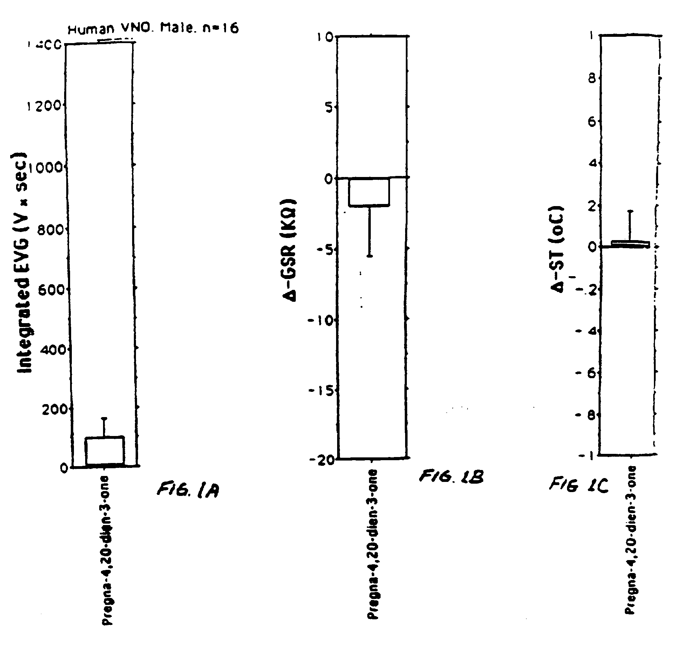

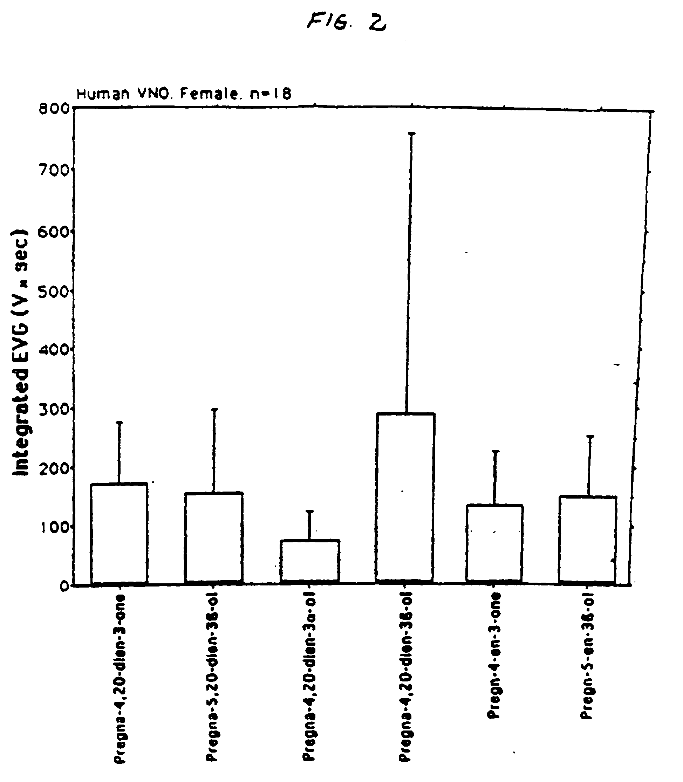

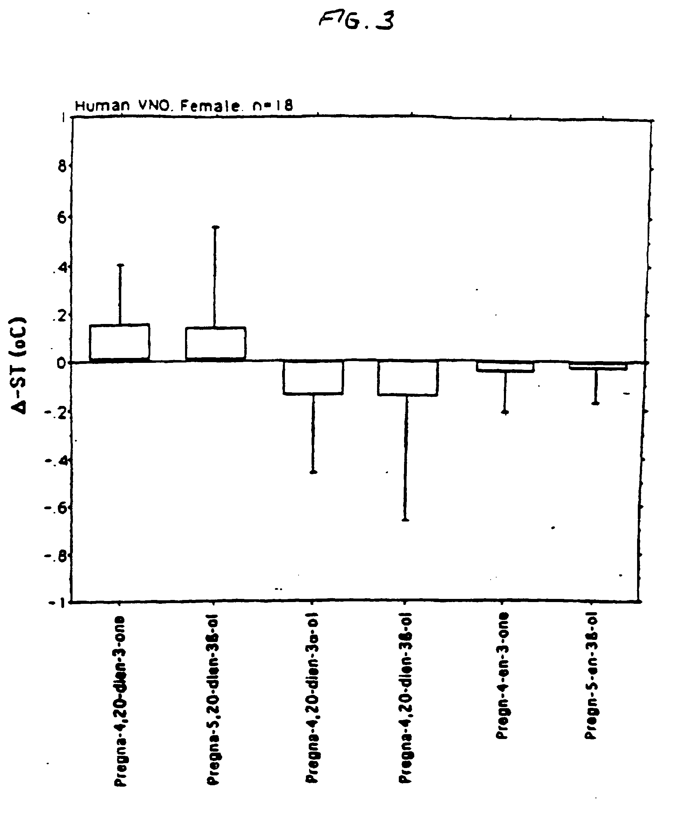

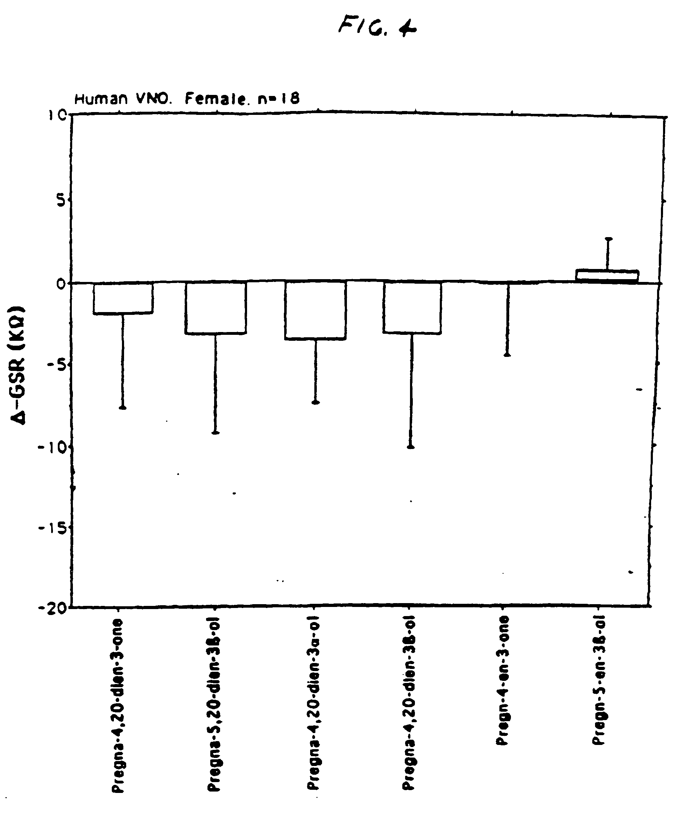

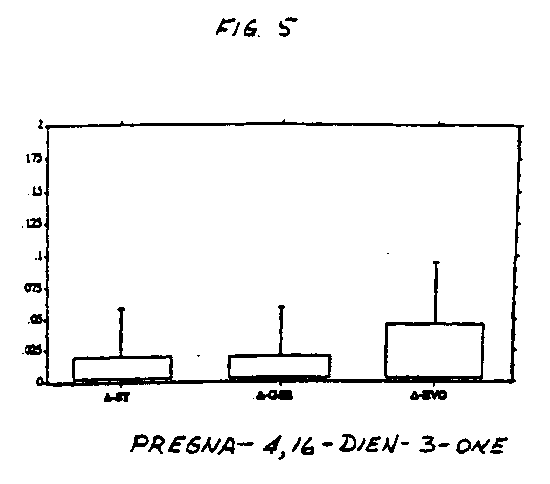

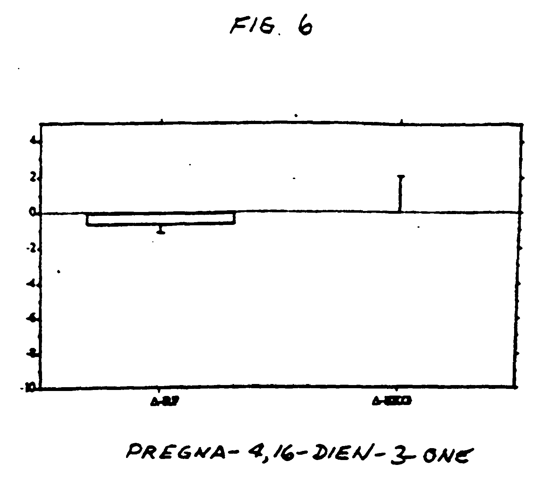

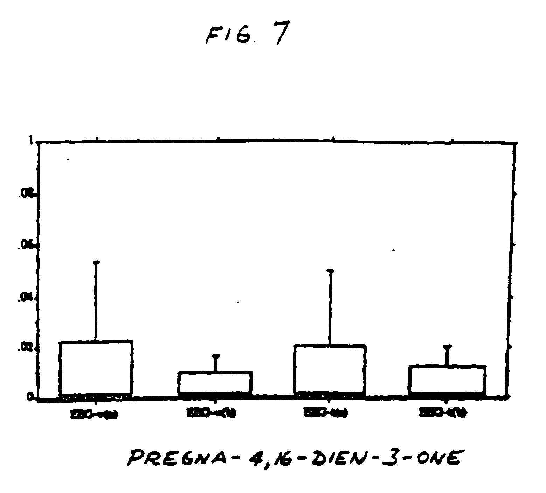

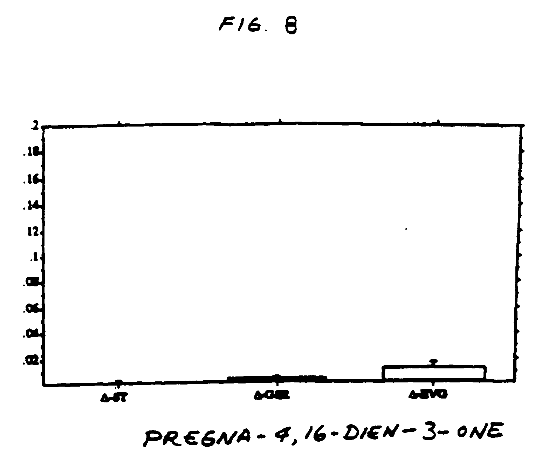

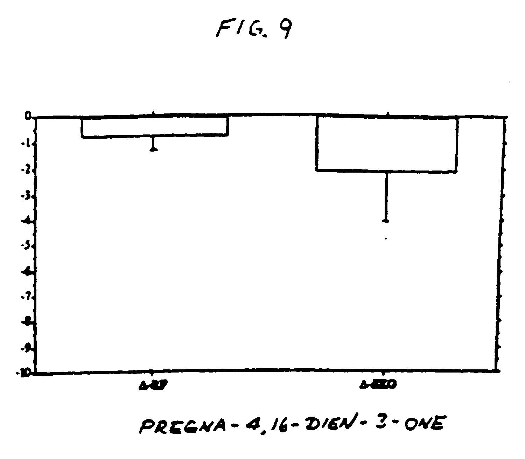

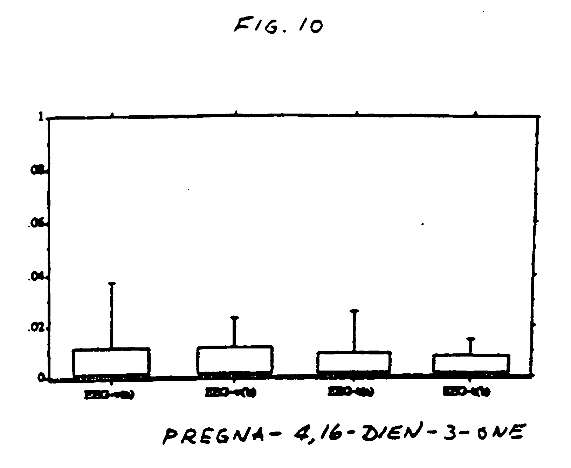

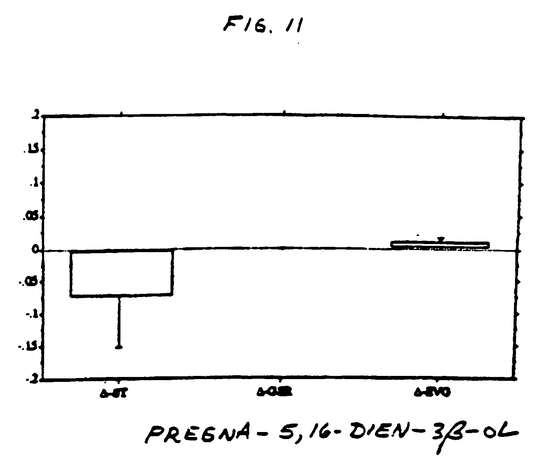

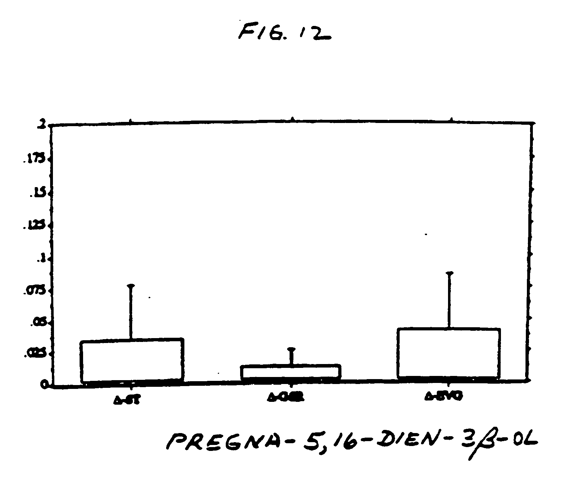

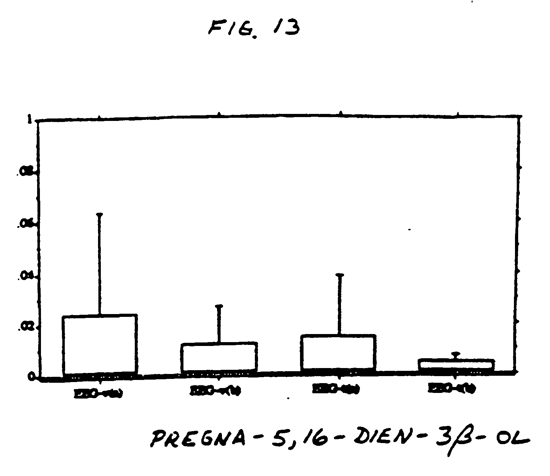

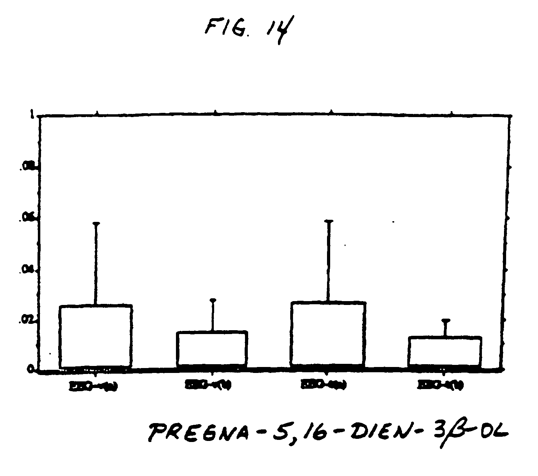

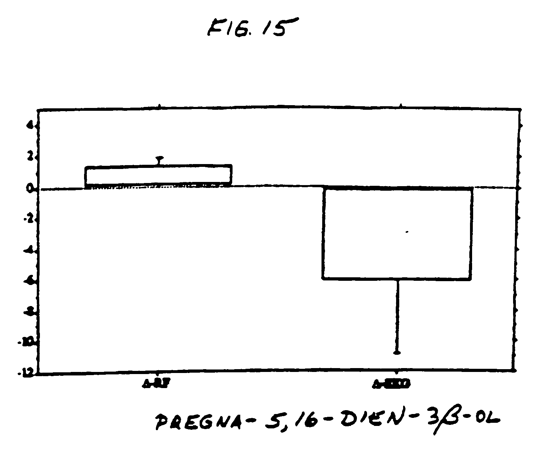

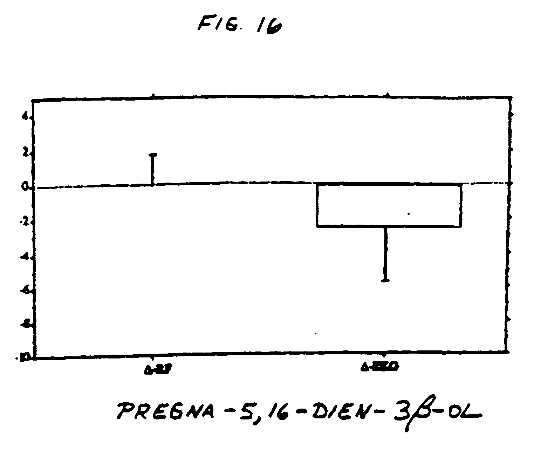

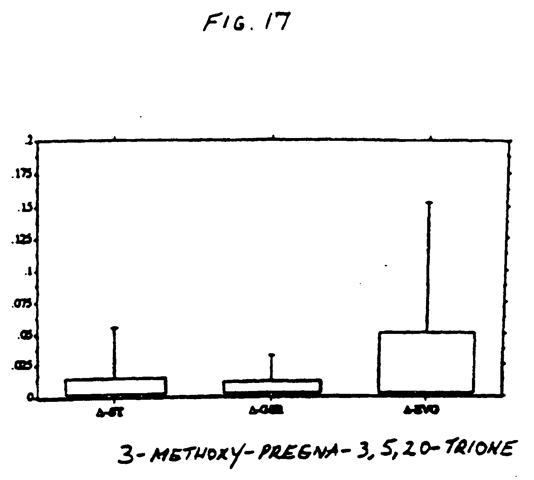

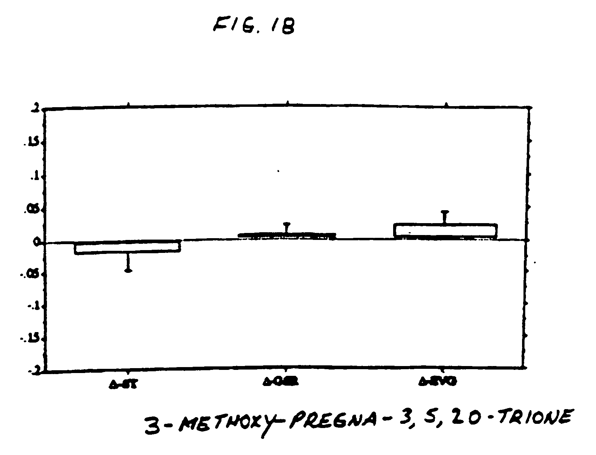

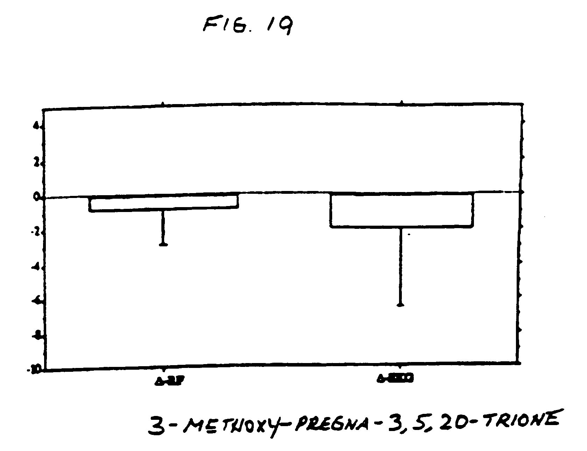

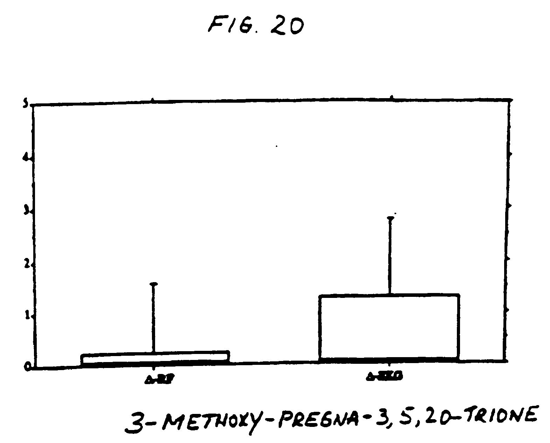

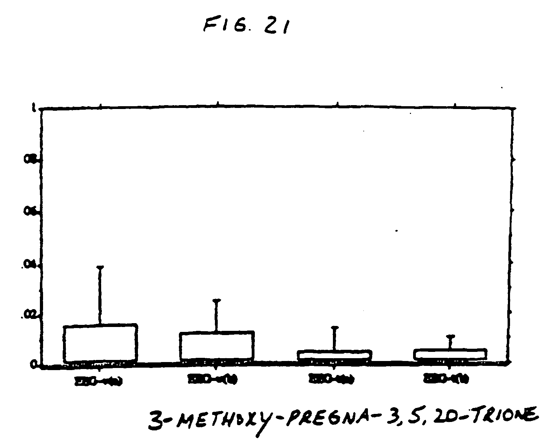

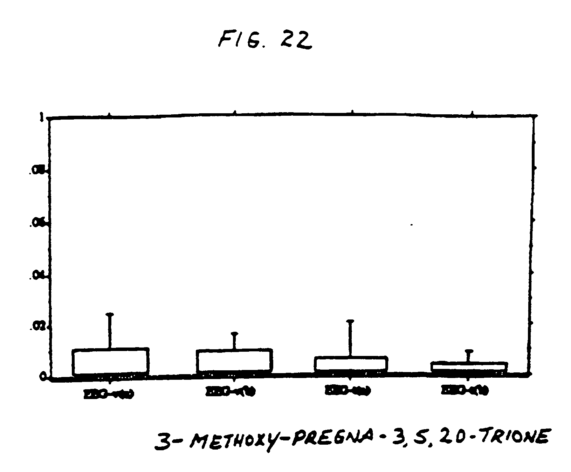

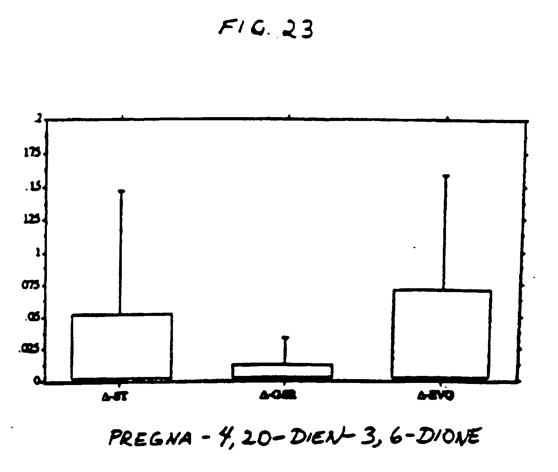

The compounds used in the methods of this invention are pregnane steroids substituted at the 3-, 6-, 19-, 20- and 21- positions. Many of the 3-substituted steroids are known compounds which may be derived from 3-oxo-steroids. As shown in Figure 1, prena-9,20-diene-3-one (1) can be converted to a 3, 5, 20-triene ether (2) or 1, 4, 20-trien-3-one (3), which are respective starting materials for 6- and 3- substituted hydroxy derivatives.

Alkoxy derivatives are prepared from their corresponding hydroxy steroids by reaction with an alkylating agent such as trimethyloxonium fluoroborate, triethyloxonium fluoroborate or methylfluorosulfonate in an inert chlorocarbon solvent such as methylene chloride. Alternatively, alkylating agents such as NaH, KM or KOBut, silver oxide or barium oxide in polar, aphotic solvents as for example, DMF, DMSO and hexamethylphosphoramide.

General procedures for synthetic reactions of steroids are known to those skilled in art. Where time and temperature of reactions must be determined, these can be determined by a routine methodology. After addition of the required reagents, the mixture is stirred under an inert atmosphere and aliquots are removed at hourly intervals. The aliquots are analyzed by chromatography to monitor the disappearance of starting material, at which point the work-up procedure is initiated. If the starting material is not consumed within twenty-four hours, the mixture is heated to reflux and hourly aliquots are analyzed, as before, until the starting material disappears. In this case the mixture is allowed to cool before the work-up procedure is initiated.

Purification of the products is accomplished by means of chromatography and/or crystallization, as known to those skilled in the art.

Alkoxy derivatives are prepared from their corresponding hydroxy steroids by reaction with an alkylating agent such as trimethyloxonium fluoroborate, triethyloxonium fluoroborate or methylfluorosulfonate in an inert chlorocarbon solvent such as methylene chloride. Alternatively, alkylating agents such as alkyl halides, alkyl tosylates, alkyl mesylates and dialkylsulfate may be used with a base such as NaH, KM or KOBut, silver oxide or barium oxide in polar, aphotic solvents as for example, DMF, DMSO and hexamethylphosphoramide.

General procedures for synthetic.reactions of steroids are known to those skilled in art. Where time and temperature of reactions must be determined, these can be determined by a routine methodology. After addition of the required reagents, the mixture is stirred under an inert atmosphere and aliquots are removed at hourly intervals. The aliquots are analyzed by chromatography to monitor the disappearance of starting material, at which point the work-up procedure is initiated. If the starting material is not consumed within twenty-four hours, the mixture is heated to reflux and hourly aliquots are analyzed, as before, until the starting material disappears. In this case the mixture is allowed to cool before the work-up procedure is initiated.

Purification of the products is accomplished by means of chromatography and/or crystallization, as known to those skilled in the art.

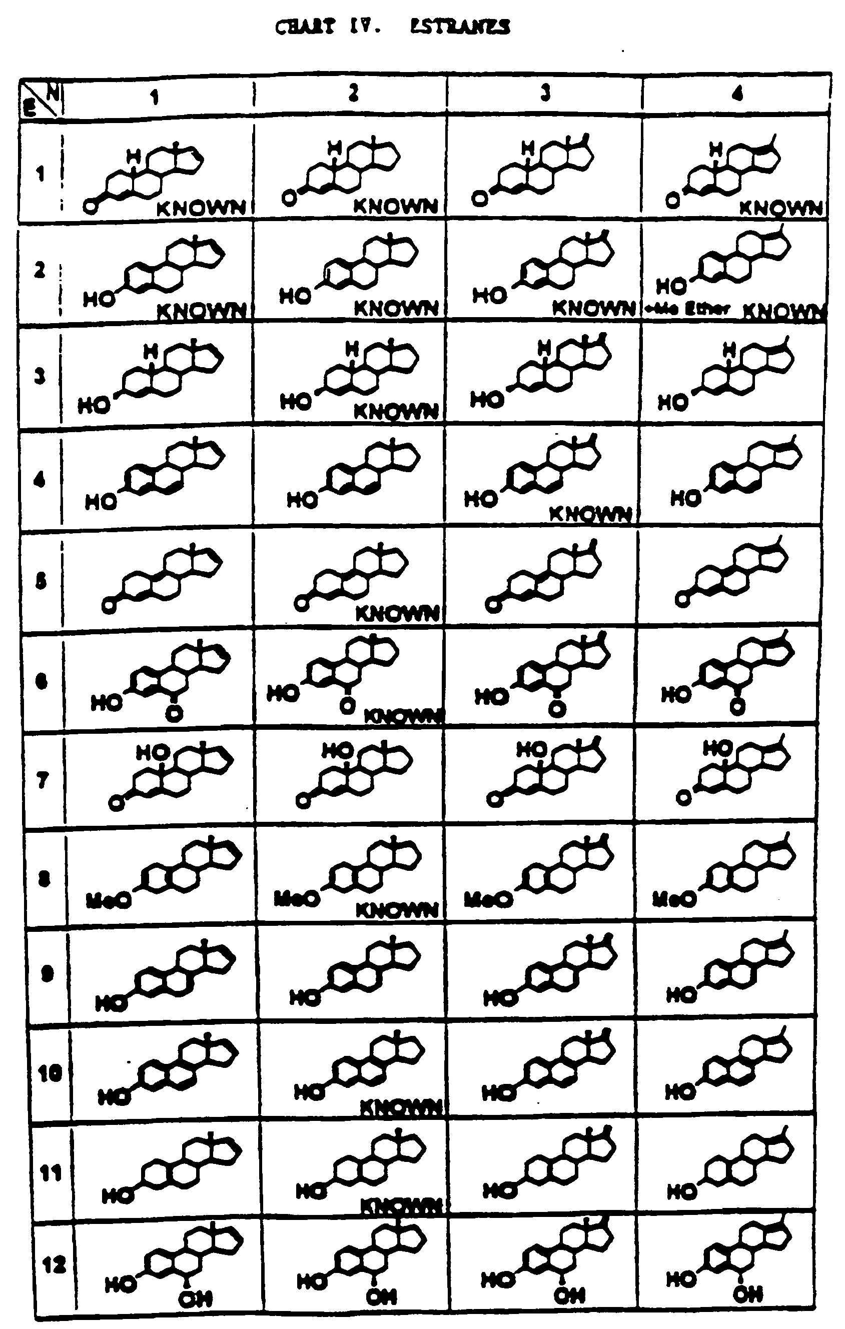

### SUBSTRUCTURE SYNTHESES

Referring to the preceding table, the following are exemplary syntheses for intermediates in a given row (E1 trough E12) or column (N1 through N4).

### Type E

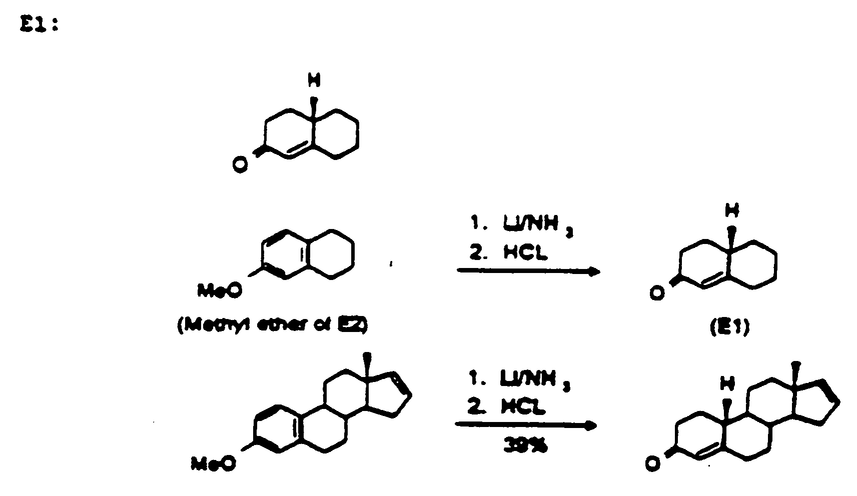

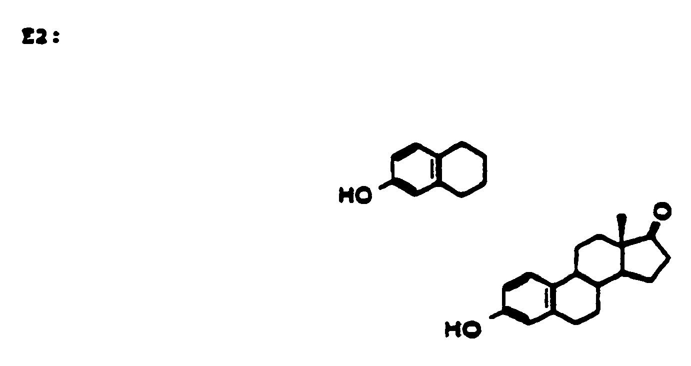

Commercially available substructure, for example, ESTRONE.

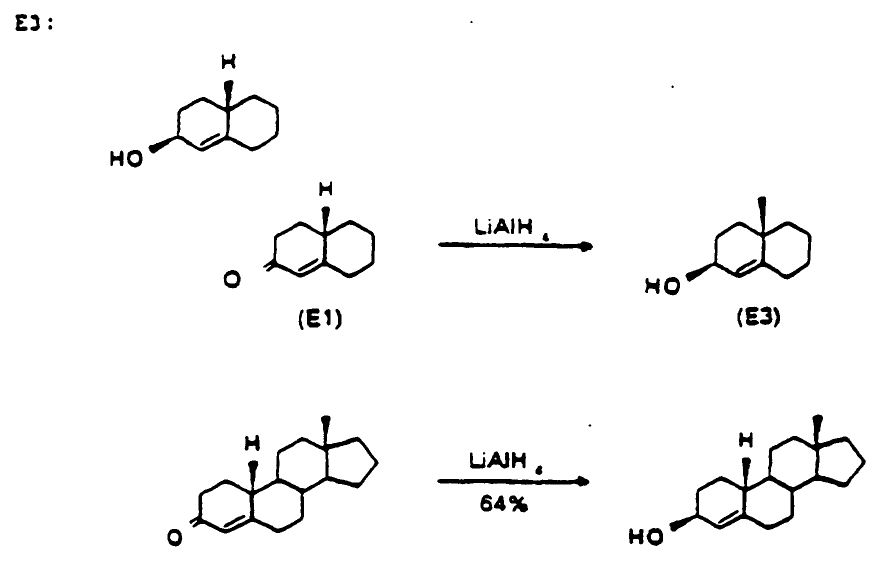

James R. Bull and Jan Floor, J. Chem. See. Perkin I, 1977 (7), 724.

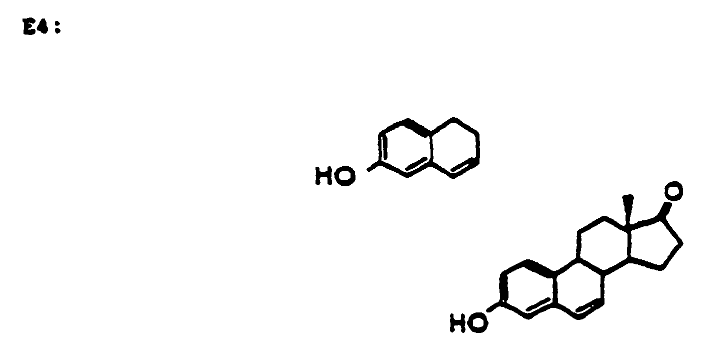

Commerically available substructure, for example, 6-DEHYDROESTRONE.

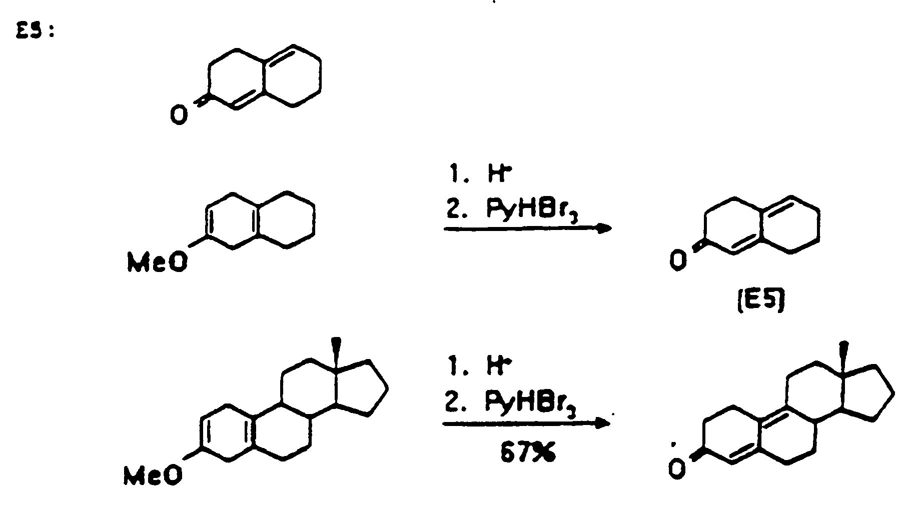

v. I. Mel'nikova and K. K. Pivnitskii, Zhurnal Organickeskoi Khisnii, 1974, Vol. 10, No. 5, pp. 1014-1019).

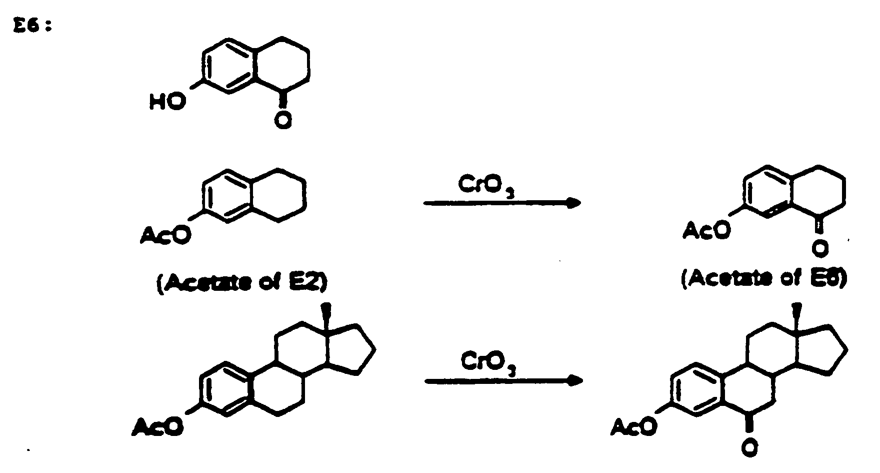

Hidetoshi Takagi, Ken-ichi Komatsu, and Itsuo Yoshisawa, Steroids, 1991, vol. 56, p. 173.

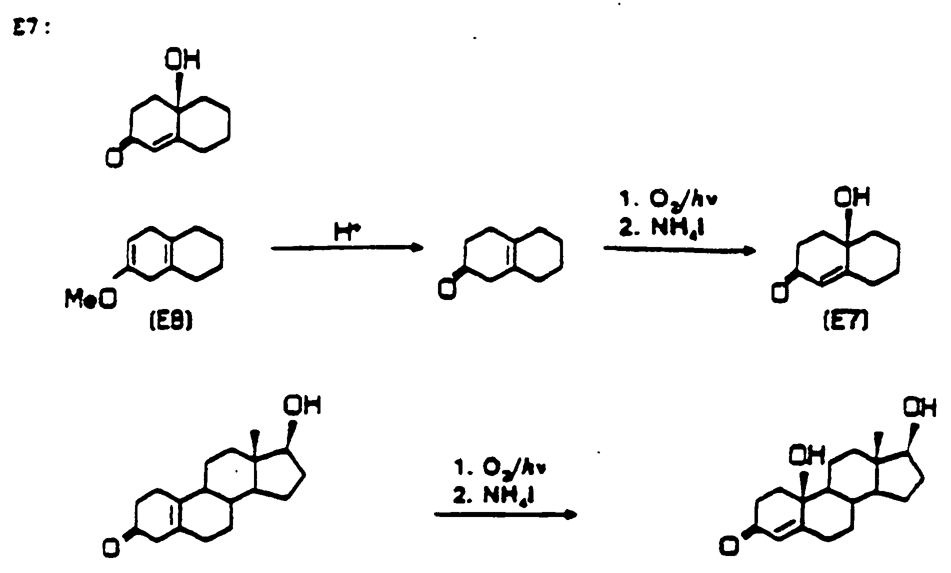

Michel Mauney and Jean Rigaudy, Bull. Soo. Chien, 1976, No. 11-12, 2021.

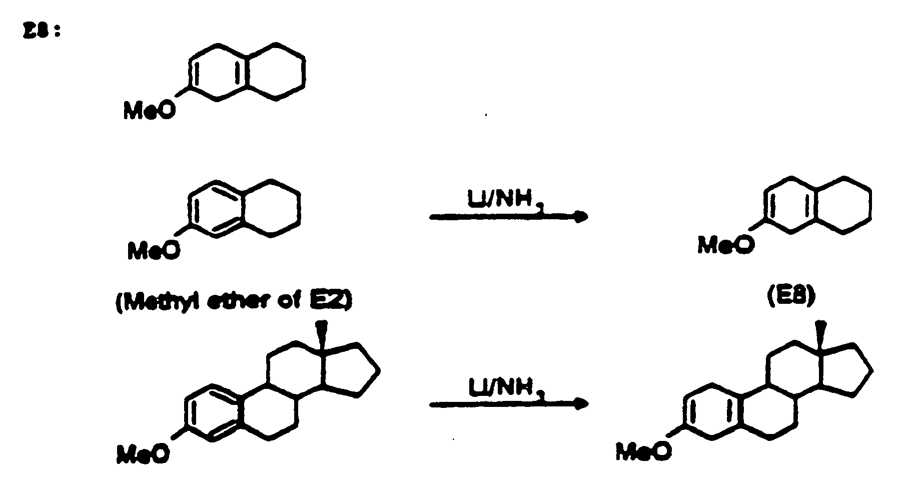

K. J. San, R. H. Blank, R. H. Evans, Jr., L. I. Feldman, and C. E. Holmbund, J. Org. Chem., 1964, 29, 2351.

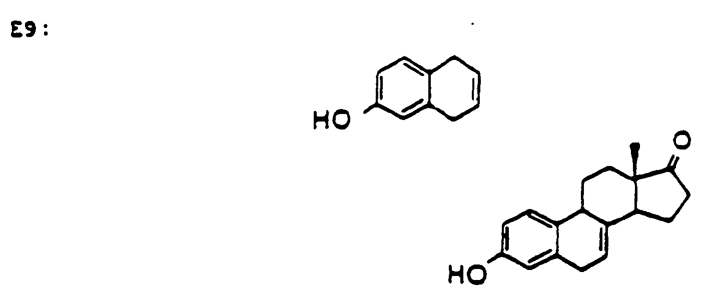

Commercially available substructure, as in EQUILIN.

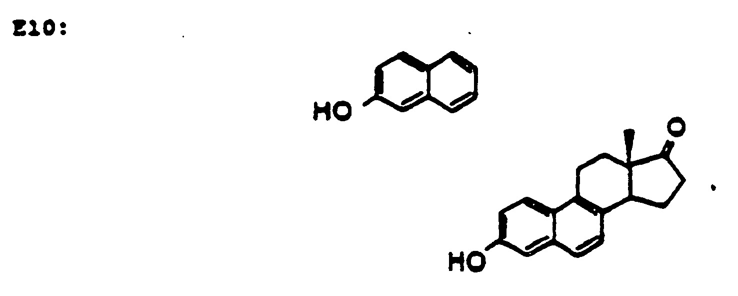

Commercially available substructure, as in EQUILININ.

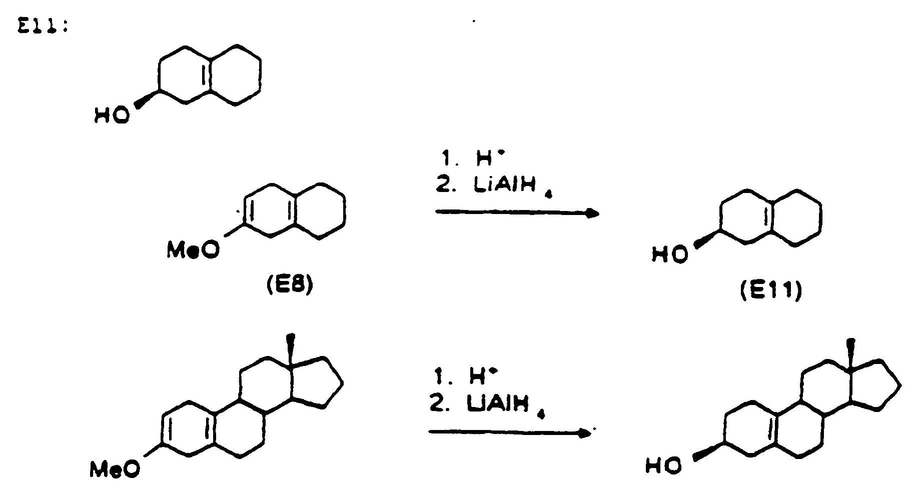

A. N. Cherkasov, A. M. Ponomarav, and K. K. Pivnitskii, Zhurnal Organiskeskoi Khimii, 1971, Vol. 7, No. 5, pp. 940-947.

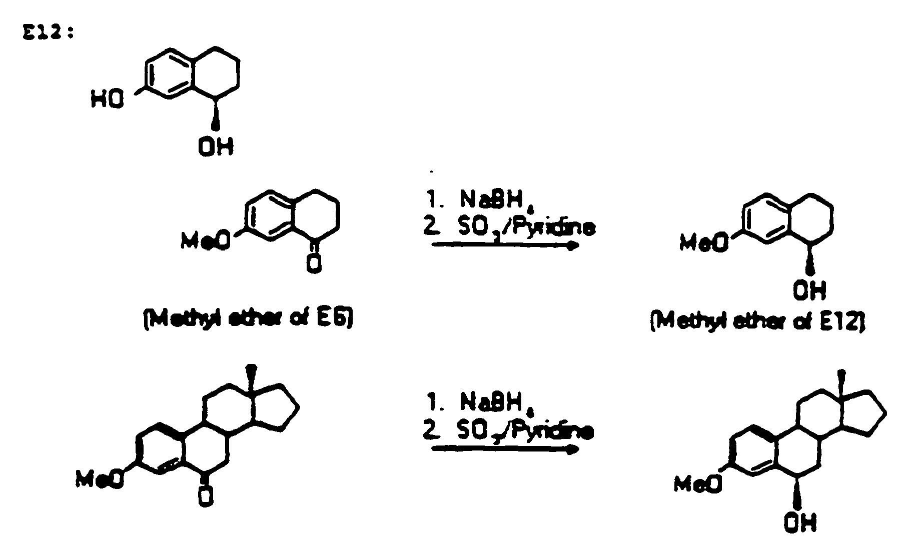

Hidetoshi Takagi, Ken-ichi Komatsu, and Itsuo Yoshisawa, Steroids, 1991, Vol. 56, p. 173.

### Type N

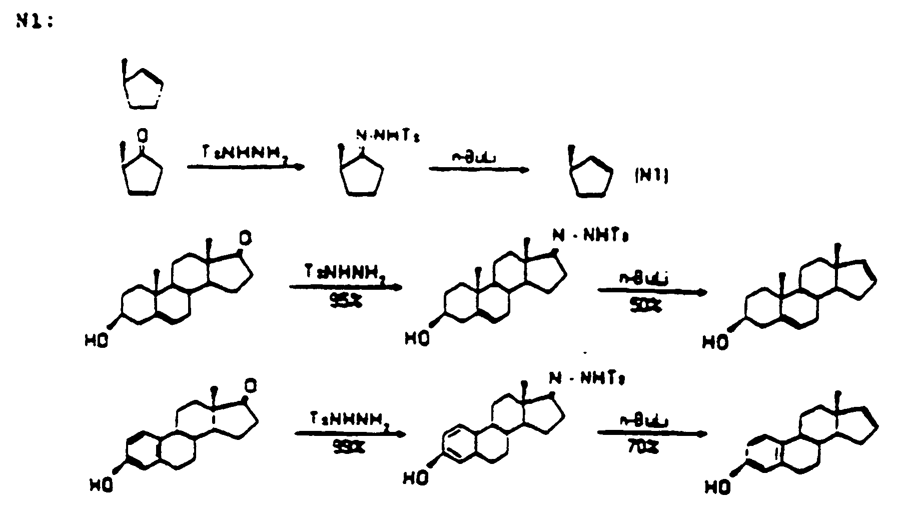

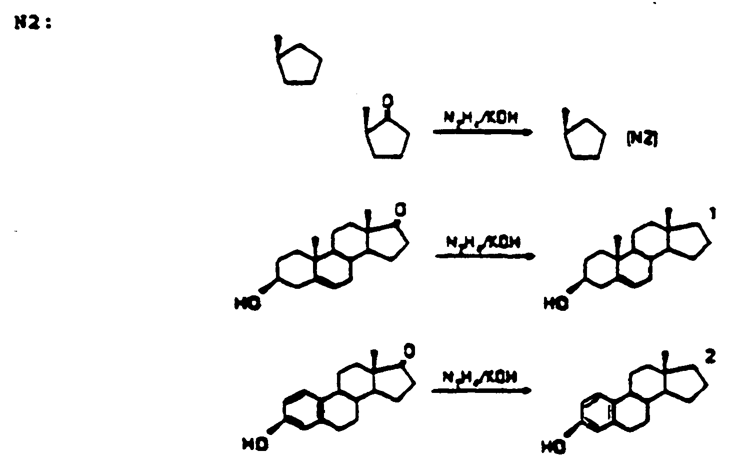

1. Robert H. Shapiro and Carl Djerassi, J. Am. Chem. Soc., 1964, 36, 2825.
2. Pilar Lupon, Frances C. Canals. Arsenio Iglesias, Joan C. Ferrer, Albert Palomar, and Juan-Julio Bonet, J. Org. Chem. 1988, 51, 2193-2198.

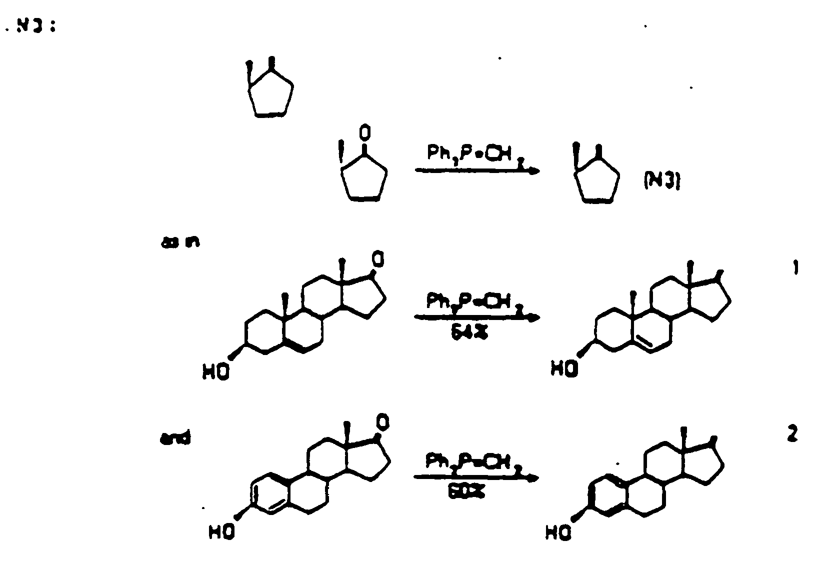

1. Günther Drefahl, Kurt Ponold and Hans Schick, Berichte, 1965, 98, 604.
2. Richard H. Peters, David F. Crows, Mitchell A. Avery, Wesley K. M. Chong, and Masako Tanabe, J. Med. Chem., 1989, 12, 1642.

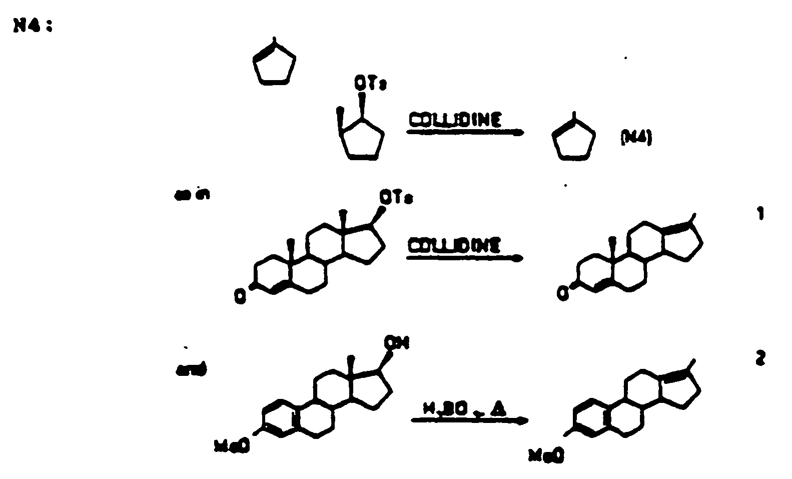

1. Frans Sonheimer, O. Moncara, M. Viquiza & G. Rosenkrans (1955) J. Am. Chem. Soc. 77: 4145.
2. William F. Johns, J. Org. Chem., 1961, 26, 4583.

### Methylestrenes

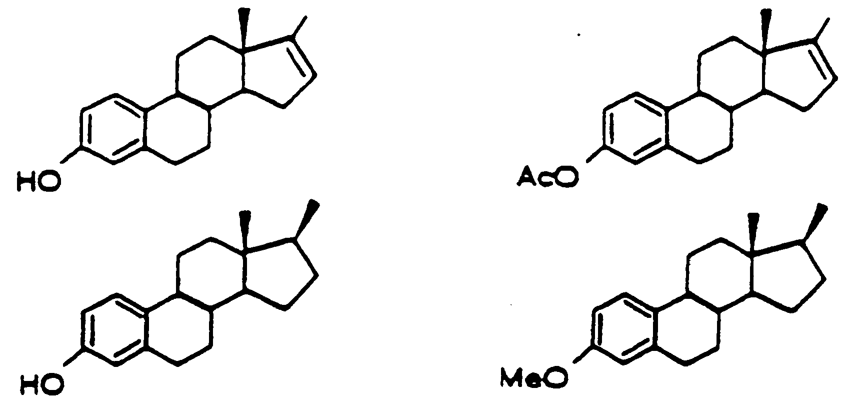

Harold J. Nicholas, J. Org. Chem., 1958, 23, 1747.

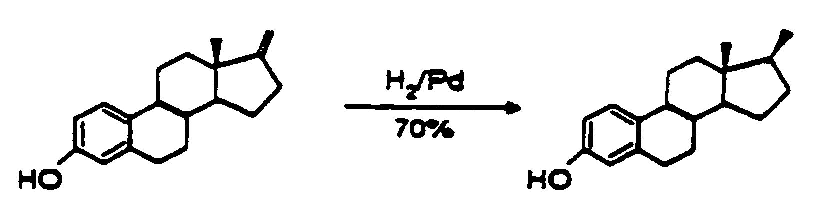

Richard H. Peters, David F. Crows, Mitchell A. Avery, Wesley K. M. Chong, and Masako Tanabe, J. Med. Chem., 1989, 12, 1642.

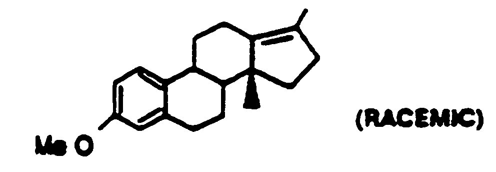

M. D. Green and F. J. Zeelen, Tetrahedron Letters, 1982, Vol. 23, No. 35, pp. 3611-3614.

Synthesisable compounds therefore include these, together with those derived from then; i.e., 17-Methyl-N1, 17β-Methyl-N2, or 14α-Methyl-N4, incombination with E1, E2, E3, E5, E6, E7, E8, E11 or E12.

### Haloestrenes

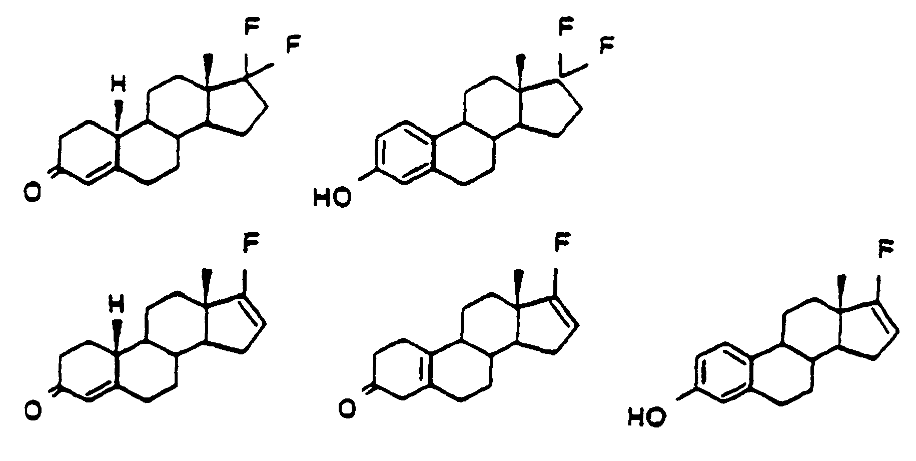

George A. Boswell in patent C.A. 70:58140g, following.

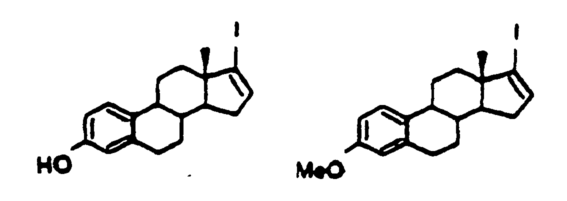

G. Michael Blackburn, Brian F. Taylor, and Andrew F. Worrall, Journal of Labelled Compounds and Radiopharmaceuticals, 1986, Vol. XXIII, No. 2, p. 197.

Synthesisable compounds therefore include these, together with those derived from then; i.e., 17-Fluoro-N1 in combination with E1, E2, E3, E5, E6, E7, E11 or E12. In addition, 17-Iodo-N1 in combination with E2, E6 or E12.

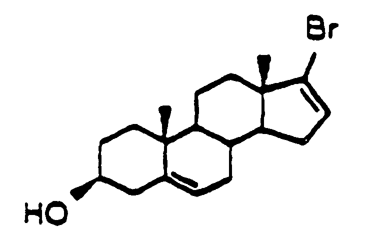

European Patent Application EP 208, 497.

Synthesizable compounds therefore include these, together with those derived from them, i.e., (4-Chloro, 4-Bromo, 6α-Chloro, 6α-Bromo, 6β-Chloro, 6β-Bromo, or 6β-Iodo)-A1 in combination with N1, N2, N3, or N4. In addition, (17-Fluoro 17-Chloro, 17-Bromo, or 17-Iodo)-N1 in combination with A1, A2, A3, A4, A5, A5, A8, A9, A10 or A11.

### D. Methods of Use.

The use of the invention as defined by the appended claims accomplished by means of the non-systemic, nasal administration of certain steroids, combinations of steroids.

This particular mode of administration is distinguished from alternative modes, such as ingestion or injection, in several important ways, these by virtue of the direct contact with the VNO provided by the nasal administration of the steroid ligand. In the methods of this invention, the appropriate ligand is administered directly to the chemoreceptors in the nasal passage and the vomeronasal organ, without pills or needles - i.e., non-invasively. Drug action is mediated through binding of the ligands, described herein, to specific receptors displayed by neuroepithelial cells in the nose, preferably in the VNO. This furthermore, the mode of drug action is through the nervous system and not through the circulatory system - thus brain function can be affected without consideration of the blood-brain barrier. These uses provide a direct means of affecting the hypothalamus through the nervous system because there is only one synaptic junction between pheromone receptors and the hypothalamus. Because sensory nerves are addressed to a specific location in the brain, this method has a highly specific drug effect, thereby greatly reducing the potential of undesirable side-effects.

VNO contact is important because the VNO is associated with chemoreceptive/pheromonal function. The VNO consists of a pair of blind tubular diverticula which are found at the inferior margin of the nasal septum. The VNO contains neuro-epithelia, the axons of which have direct synapses to the amygdala and from there, to the hypothalamus. The existence of the VNO has been well documented in most terrestrial vertebrates including the human fetus; however, in adult humans it is generally thought to be rudimentary. (See Johnson, et al., supra.)

The ligand substances described herein, or their sulfated, cypionated, benzoated, propionated, or glucuronated derivatives, may be administered directly, but are preferably administered as compositions. They are prepared in a liquid dosage form such as, for example, liquids, suspensions or the like, preferably in unit dosage forms suitable for single administration of precise dosages. Liquid dosages may be administered as nose drops or as an aerosol. Alternatively, the active compound can be prepared as a creme or an ointment composition and applied topically within the nasal cavity. In addition, a vomeropherin may be administered as vapor contained in an air puff delivered to the nasal cavity. As another alternative, delivery may occur by controlled release of these agents by encapsulation either in bulk or at a microscopic level using synthetic polymers, such as silicone, and natural polymers such as gelatin and cellulose. The release rate can be controlled by proper choice of the polymeric system used to control the diffusion rate (Langer, R.S. and Peppas, N.A., Biomaterials 2,201, 1981). Natural polymers, such as gelatin and cellulose slowly dissolve in a matter of minutes to hours while silicone remains intact for a period of months. The compositions will include a conventional pharmaceutical carrier or excipient, one or more of the active compound(s). In addition, the compositions may include other medicinal agents, pharmaceutical agents, carriers, adjuvants, etc.

The most likely means of communication of a semichemical ligand is the inhalation of a naturally occurring pheromone present on the skin of another. Since these compounds are relatively nonvolatile, it is estimated that, even during intimate contact, a human subject would inhale picogram amounts of a naturally occurring steroid from the skin of another. From the amount inhaled it is estimated that only about 1% would reach the receptors of the vomeronasal organ.

The amount of vomeropherin administered will of course, be dependent on the subject being treated, the severity of the affliction, the manner of administration, the frequency of administration, and the judgment of the prescribing physician. However, a single dosage of at least about 10 picograms, delivered directly into the lumen of the vomeronasal organ, is effective in eliciting a transient autonomic response. When administered to the nasal cavity, the dosage is about 100 picograms to about 100 micrograms, preferably about 1 nanogram to about 10 micrograms, more preferably about 10 nanograms to 1 about microgram. The frequency of administration is desirably in the range of an hourly dose to a monthly dose, preferably from 8 times/day to once every other day, more preferably 1 to 3 times per day. Ointments containing one or more active compounds and optional pharmaceutical adjuvants in a carrier, such as, for example, water, saline, aqueous dextrose, glycerol, ethanol, and the like, can be prepared using a base such as, for example, petroleum jelly, lard, or lanolin.

Liquified pharmaceutically administrable compositions can, for example, be prepared by dissolving, dispersing, etc. an active compound as defined above and optional pharmaceutical adjuvants in a carrier, such as, for example, water, saline, aqueous dextrose, glycerol, ethanol, and the like, to thereby form a solution or suspension. If desired, the pharmaceutical composition to be administered may also contain minor amounts of nontoxic auxiliary substances such as wetting or emulsifying agents, pH buffering agents and the like, for example, sodium acetate, sorbitan monolaurate, triethanolamine sodium acetate, triethanolamine oleate, etc. Actual methods of preparing such dosage forms are known, or will be apparent, to those skilled in this art; for example, see Remington's Pharmaceutical Sciences, Mack Publishing Co., Easton, PA, 15th Ed., 1975. The composition or formulation to be administered will, in any event, contain a quantity of one or more of the active compound(s) in an amount effective to alleviate the symptoms of the subject being treated.

For aerosol administration, the active ingredient is preferably supplied in finely divided form along with a surfactant and a propellant. Typical percentages of active ingredients are 0.001 to 2% by weight, preferably 0.004 to 0.10%.

Surfactants must, of course, be nontoxic, and preferably soluble in the propellant. Representative of such agents are the esters or partial esters of fatty acids containing from 6 to 22 carbon atoms, such as caproic, octanoic, lauric, palmitic, stearic, linoleic, eleostearic and oleic acids with an aliphatic polyhydric alcohol or its cyclic anhydride such as, for example, ethylene glycol, glycerol, erythritol, arabitol, mannitol, sorbitol, and hexitol anhydrides derived from sorbitol (the sorbitan esters sold under the trademark "Spans") and the polyoxyethylene and polyoxypropylene derivatives of these esters. Mixed esters, such as mixed or natural glycerides, may be employed. The preferred surface-active agents are the oleates orbita, e.g., those sold under the trademarks "Arlacel C" (sorbitan sesquioleate), "Span 80" (sorbitan monoleate) and "Span 85" (sorbitan trioleate). The surfactant may constitute 0.1=20% by weight of the composition, preferably 0.25-5%.

The balance of the composition is ordinarily propellant. Liquefied propellants are typically gases at ambient conditions, and are condensed under pressure. Among suitable liquefied propellants are the lower alkanes containing up to five carbons, such as butane and propane; fluorinated or fluorochlorinated alkanes, such as are sold under the trademark "Freon". Mixtures of the above may also be employed.

In producing the aerosol, a container equipped with a suitable valve is filled with the appropriate propellant, containing the finely divided active ingredient and surfactant. The ingredients are thus maintained at an elevated pressure until released by action of the valve.

Yet another means of administration is topical application of a volatile liquid composition to the skin, preferably facial skin, of an individual. The composition will usually contain an alcohol such as ethanol or isopropanol. A pleasant odorant may also be included in the composition.

### E. Antifertility Activity. (Does not fall within the scope of the claims)

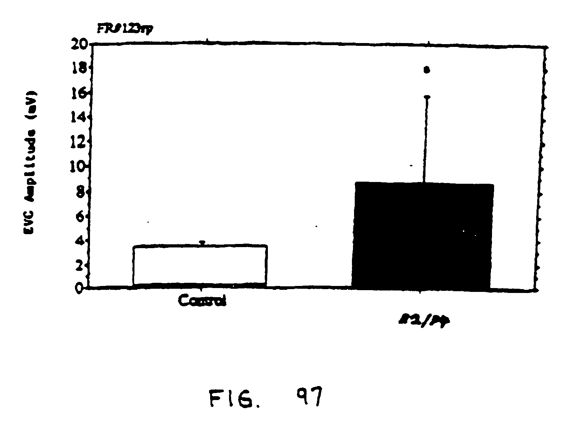

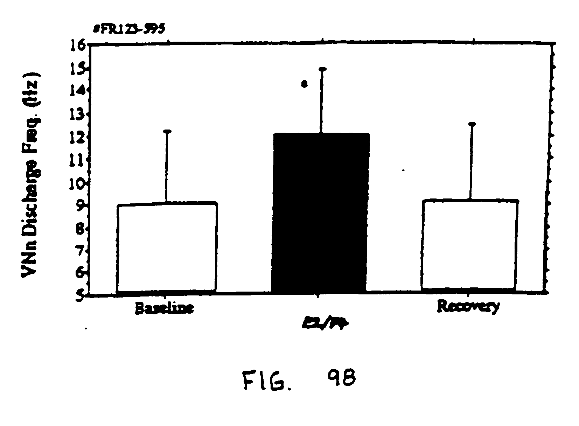

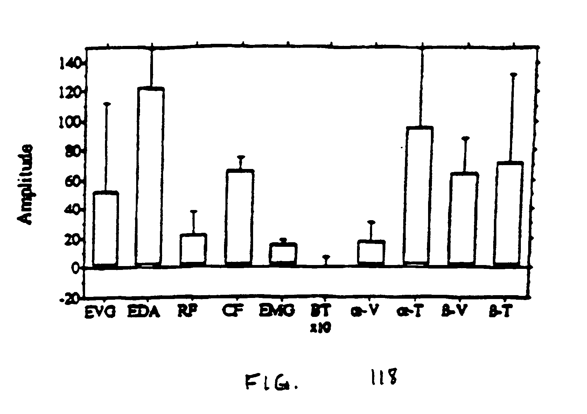

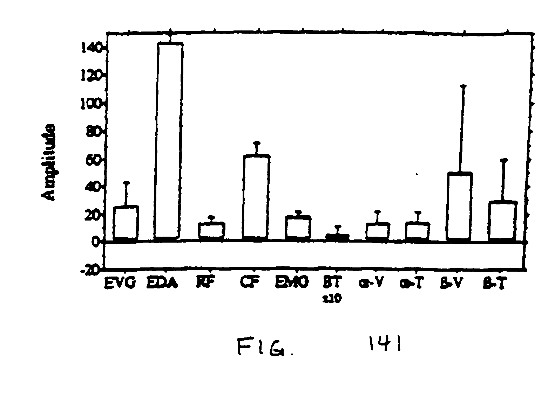

The steroid 19-norpregna-1,3,5(10)-trien-3-ol was tested in the VNO of female rats. The EVG and vomeronasal nerve (VNn) discharge frequency are shown in FIGS. 97 and 98, respectively. This data shows stimulation of the VNO. The steroid E2/P4 was shown to have postcoital antifertility activity when administered orally to female rats while having low hormonal activity (measured by estrogen-receptor binding). (Peters, et al., J. Med. Chem., 1989, 32, 1642-52.) The data in FIGS. 97 and 98 suggest that this antifertility activity is explainable because E2/P4 is not a hormone, but acts as a vomeropherin through stimulation of the VNO, which in turn affects the hypothalamus. Consistent with the rat model data, the compound E2/P4 also shows VNO stimulation in women (see FIG. 118), and to a lesser extent, in men (see FIG. 140), and thus it is expected that the vomeropherins have antifertility activity in humans.

Stimulation of the hypothalamus via the VNO allows one to suppress release of LH and FSH. This can provide a clinical method for treatment of prostatic cancer, precocious puberty (in males and females), endometriosis, uterine leiomyoma, breast cancer, premenstrual syndrome and disfunctional uterine bleeding.

### F. Measuring Affect, Mood and Character Trait.

Feeling states associated with affects, moods and character traits are generally measured by use of a questionnaire. For example questionnaires comprising a number of adjectives which refer to feeling states may be administered to an individual. The individual evaluates his or her feeling state described by the adjective and rates the intensity of the feeling on a numerical scale. Clustering of related adjectives and statistical analysis of a subject's evaluation of each adjective provides a basis for the measurement of various feeling states.

Alternatively, feeling states may be measured by autonomic changes, such as those used in polygraphic evaluations (galvanic skin response, pulse rate and the like). Cabanac, M. Annual Review of Physiology (1975) 37:415; Hardy, J.D., "Body Temperature Regulation", Chapter 59, pp. 1417. In: Medical Physiology. Vol. II Ed.: VB Mountcastle (1980); Wolfram Bouscein. Electrodermal Activity (Plenum Press 1992). In addition, non-verbal cues such as facial expression and body posture may be evaluated.

### Treatment of Premenstrual Syndrome

Patients entered in the study (women 20-45 years old) participated in a recording session that takes place the day when PMDD symptoms are at the highest level (day 24 to 28 of the menstrual calendar). The entire procedure lasted approximately one hour in a quiet room with the subject laying supine.

The active ingredient used in the study was 16α,17α-epoxyestra-1,3,5(10)-trien-3-ol. The opening of the vomeronasal organ to the nose is identified at both sides of the nasal septum and a nasal probe (vomeronasal applicator) is positioned in one nostnl with its output orifice pointing toward the VNO opening. One pulse of active substance, or placebo was delivered to the VNO. Following, the same procedure was repeated in the other nostril. The bilateral puffing of the vomeronasal organ was repeated 30 minutes after the first application.

The activation of the device delivers pulses of 200 µl, lasting 1 second. The quantity of active substance delivered in a pulse is 100 pg. The total quantity of active substance delivered to a patient's VNOs during the entire session is 400 pg (Table II).

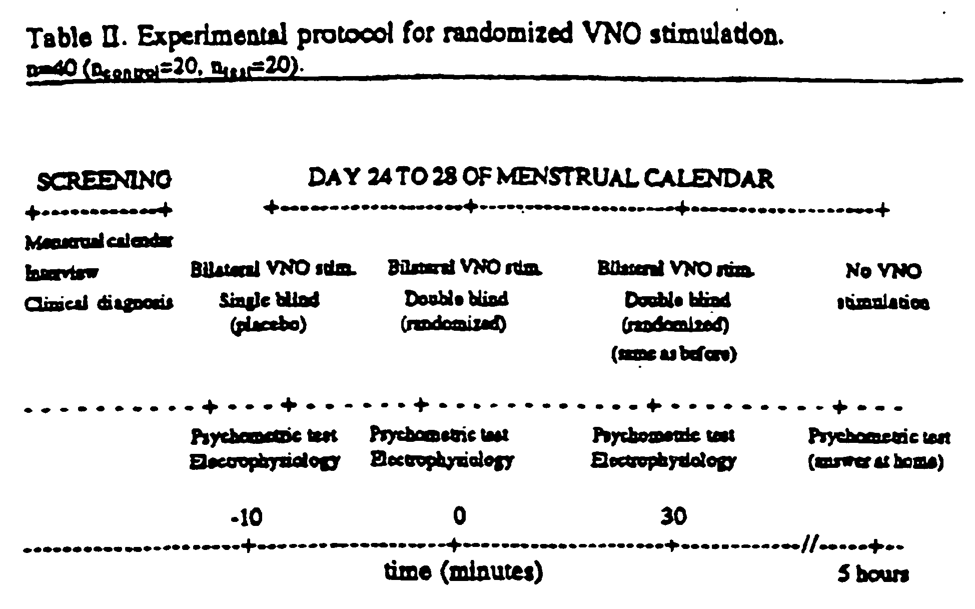

Candidates were tested while they had clear PMDD symptomatology. This usually happens during day 24 to 28 of their menstrual calendar. The psychometric test (Table III) was presented ten minutes before, and thirty minutes after bilateral VNO stimulation with either the test substance or placebo (also, see Table II). After completion of the 30-minutes questionnaire, both VNOs were again stimulated with the same substance (vomeropherin, or placebo), and the patient was given again a similar psychometric test, to answer at home 5 hours after leaving the recording laboratory. Interviews of the patients the day after the study session indicated mood changes, feelings, and how she was perceived by other people, after the second puffing to the VNO.

### Autonomic Reflexes and EEG

Several peripheral electrode leads attached to the patient's skin were used to study autonomic function and electroencephalogram (EEG). Recordings are produced 15 minutes before, and 30 minutes after stimulation of the VNO. Electrodermal activity (EDA), was recorded using two silver dectrode disks placed in the palmar skin of the major and annular finger (right hand). Electrocardiogram (ECG) was monitored from lead I (standard frontal-plane I). Respiratory frequency (RF) was recorded using a strain gauge placed around the lower thorax. The sinus respiratory physiological arrythmia was obtained from the correlation of RF and the ECG frequency. To assess changes in parasympathetic tone. Body temperature (BT) was taken from the external ear canal using a mini thermistor probe. A bipolar elcctromyographic recording (EMG) was accomplished with two electrodes placed in the chin.
Electroencephalogram is recorded from CzA1 and T3A1 of the standard 10/20 system. All signals were amplified and digitized (Biopac Systems), and continuously monitored and stored using a computer (Macintosh LC-III). The autonomic function and EEG are recorded during the screening session and again in both double blind sessions, after VNO stimulation (see Table II).

Mood changes are evaluated in a scale from zero to four, using questionnaires. Autonomic function recorded before, and 30 minutes after the administration of placebo or test substance to the VNO, was processed using "aqknowledge" software (Biopac Systems). The significance of the results was assessed using t-tests and analysis of variance.

Fourteen patients diagnosed with PMDD were screened. Two patients responded to the placebo and were dropped from the study. Six of the patients received the steroid in their VNOs, and the other six received placebo. A summary of the results is shown in Table III.

The patient were asked to score a list of eleven PMDD symptoms before, and 30 minutes after stimulation of the VNO with the steroid (total dose=200 pg), or placebo. Bilateral stimulation of the VNO with placebo did not change significantly the magnitude of the symptoms, from control level (FIG. 210, shaded bars). However, 30 minutes after bilateral stimulation of the VNO with the steroid (total dose=200 pg), there was significant reduction of most PMDD symptoms (p=or<0.03). For some symptoms like "I feel like throwing things", and "I am angry", the improvement was significant at p=0.01. The score for other symptoms like "My mind feels cloudy", and "I feel the world would be better without me", was not different from control after administration of the steroid (p>0.05).

The analysis of autonomic function 30 minutes after stimulation of the VNO with the steroid shows changes of some reflexes, that correlatate with the mood improvement of the patient. The activity of skeletal muscles measured through the electromyograrn (EMG), decreases thirty minutes after delivery of the steroid to the VNO, but not after administration of placebo (FIG. 211). Also, the frequency of electrodermal activity events, decreases after administration of the vomeropherin, but not after placebo (FIG. 212). Other autonomic reflexes do not show significant change. The electroencephalographic pattern does not change substantially from baseline, after administration of the steroid to the VNO. However, in four patients treated with the vomeropherin, there was increase in alpha cortical activity.

The score of PMDD symptoms was also studied five hours after the second application of vomeropherin to the VNO. At this time there was no significant difference with control. However, during the telephone interview done during the morning after the recording session, the patients referred feeling better after leaving the recording laboratory, and that this effect lasted from one to two hours.

Small quantities of the steroid (200 pg) delivered in vapor form to the VNO of patients suffering from PMDD, produced significant improvement. As shown in FIG. 210, the majority of the symptoms are decreased after 30 minutes of administration of the substance. This effect is not seen after administration of placebo to the VNO. Symptoms reduction is accompanied by relaxation, measured by decrease of the EMG and decrease of EDA frequency. Finally, the patients improvement is substantiated by verbal reports (telephone interview), obtained the morning after the recording session.

**Table III. Summary of effects of vomeropherin in PMDD patients. n=12**

| Symptom | Improvement |
|---|---|
| I feel like running away | ++ |
| I feel like throwing things | ++++ |
| I am angry | ++++ |
| I feel swolles up | +++ |
| My breast hurt | ++ |
| I feel like crying | ++ |
| I feel overwbelmed | ++ |
| I get emotional easily | +++ |
| I blow up at little things | +++ |
| My mind feel cloudy | 0 |
| I feel the world would be better withiut me | 0 |
| Skeletal muscle relaxation | ++ |
| Electrodermal activity relaxation | ++ |
| Alpha brain wave activity | + |

### Anxiety Tests (do not fall within the scope of the claims)

Eight patients were treated in the VNO with placebo and eleven were treated with androsta-4,16-dien-3β-ol. Scores on anxiety symptoms were taken using the convention Hamilton-A anxiety tests which measure the following:

| TOTAL HAMILTON-A | INSTANT HAMILTON-A | STATIC HAMILTON-A |
|---|---|---|
| anxious mood | anxious mood | fears |
| tension | tension | insomnia |
| fears | intellectual | depressed mood |
| insomnia | somatic (muscular) | gastrointestinal |
| intellectual | somatic (sensory) | symptoms |
| depressed mood | cardiovascular symptoms | genitourinary symptoms |
| somatic (muscular) | respiratory symptoms | |
| somatic (sensory) | autonomic symptoms | |
| cardiovascular symptoms | behavior at interview | |
| respiratory symptoms | | |
| gastrointestinal symptoms | | |
| genitourinary symptoms | | |
| autonomic symptoms | | |
| behavior at interview | | |

The results are summarized in FIGs. 213 through 218. In FIG. 213 the treated subjects (T) show an improvement over the control (C) in the instant, static and total Hamilton-A results. Referring to FIG. 214, only 25% of the subjects given placebo showed a Hamilton-A score drop of 45% or more, but 64% of the treated subjects showed a score drop of 45% or more.

FIG. 215 shows a significant decrease in respiratory frequency (RF) and cardiac frequency (CF) in treated anxious subjects compared to control.

FIG. 216 shows improved parasympathetic tone in treated anxious subjects as compared to control. This characteristic was measured through a standard test for physiologic sinus arrhythmia.

FIG. 217 shows an improved (decrease) on electrodermal activity (EDA) in treated anxious subjects as compared to control.

FIG. 218 shows an improved (increase) in body temperature in treated anxious subjects compared to control.

In 10 male and 10 female subjects (Group C) PDD was administered at a concentration of 5 x 10⁻⁹ M, every 10 minutes, during 6 hours. Pulsatility analysis was applied to the LH and FSH serum concentrations obtained at each sampling time. As shown in Table III, the mean area under the peaks (concentration/time) and the increment above basal were significantly reduced during the treatment with PDD for both gonadotropins but only in males without apparent changes in the female subpopulation.

In PDD treated males, FSH values were significantly decreased for both M. Area and I.A. Basal measurements: Treated 12.7 vs. Control 17.6, and Treated 0.63 vs. Control 0.89, respectively. Likewise, for the same group, LH values were significantly decreased for both M. Area and I.A. Basal measurements: Treated 44 vs. control 77 and Treated 1.4 vs. Control 2.0, respectively.

For all measurements as described, no statistically significant changes could be detected in female subjects treated with PDD. (Table III).

The major change was observed in the reduction of the increments above basal values particularly for LH (2.0 vs. 1.4 p<0.009).

Similarly to the findings during the administration of ETA, other pituitary hormones analyzed (PRL and TSH) did not show statistically significant changes either in their absolute concentrations or in the pulsatility characteristics.

Vomeropherins ETA and PDD appear to be gender specific both in their effect on the VNO and on hypothalamic responses. They significantly lower gonadotropin pulsatility in male, but not in female subjects (see Table III). This gender specific effect on the VNO (electrovomerogram) has been reported previously using naturally occurring vomeropherins (pheromones) from the human skin: Estra- 1,3,5(10),16-tetraen-3-ol specifically stimulates the human VNO in males, but not in females. However, Androsta- 4,16-dien- 3-one specifically stimulates the human VNO in females, but not in males.

The data provides support for a functional connection between the VNO and the basal forebrain. Furthermore, stimulation of the human VNO in males with ETA (3x10⁻⁹ M) significantly changes LH pulsatility, but not FSH. PDD (5x10⁻⁹ M) significantly changes LH and FSH pulsatility in males only. These findings open an opportunity to use a specific vomeropherin (chemically synthesized and not found in nature) that could be used in the treatment of certain diseases which are gender specific.

The release of LHRH and gonadotropins through exposure to semiochemicals of conspecifics of the opposite gender has been reported in several mammalian species (Beltramino et al, Neuroendocrinology 36 (1983):53-58, Coquelin, et al. J. Neuroscience 4 (1984):2230-2236. It was also shown in laboratory animals that VNO receptors are essential to trigger this neuroendocrine reflex (Johns 1978, Wysocki 2979, Meredith 1994). The present results here show that this neuroendocrine reflex is functional in humans and that it can be modulated through VNO receptor sites that are sensitive to vomeropherins. See Tables II and III in that there is the decrease in LH and FSH peak height, and frequency and increase in the peak interval. Therefore, ETA and PDD must be affecting vomeronasal afferent signals that modulate the pool of hypothalamic LHRH neurons.

Experimental studies in several animal species have demonstrated a close correlation between LH pulsatility and episodic GnRH release in hypothalamic-pituitary-portal blood (Veldhuis et al., J. Clin. Endocr.Metab., 64 (1987):1275-1282). The pulsatile release of LH in normal men, post-menopausal women and young women studied throughout the menstrual cycle reflects episodic endogenous gonadotrophic releasing hormone (GnRH) action. Although variations in bioactive LH pulse amplitude can result.from changes in either amplitude of the endogenous GnRH pulse signal and/or changes in pituitary gonadotropic-cell responsiveness, significant alterations in LH pulse frequency can be taken to reflect corresponding modulation of the hypothalamic GnRH pulse (Knobil, E. 1980, Recent Prog Horm Res 36:53). The changes observed in the amount and pattern of release of gonadotropins in male subjects during administration of femtomolar quantities of vomeropherins to the VNO result in a neuro-pharmacological effect at the hypothalamic/pituitary level.

Pulsatile release of LHRH from the preoptic hypothalamus drives the release of both gonadotropins (LH and FSH) from the pituitary (Yenand Jaffe, Textbook of Reproductive Endocrinology).

Data on several patients are shown for blood testosterone level (subjects 1, 2, 4 and 8, respectively, shown in FIGS. 204, 205, 206 and 207). Visit "A" shows the levels after administration of pregna-4,20-dien-3,6-dione to the VNO. Visit "B" shows testosterone levels after administration of a placebo. The slopes of the curves for levels after treatment with the pregnadiendione are statistically different from the slopes of the curves of the control. Also, in most instances, the levels of testosterone are lower after treatment of the pregnadiendione compared to the control. This shows the ability to affect blood testosterone level by administration of a vomeropherin to the VNO.

### III. Examples

The following examples are intended to illustrate but not to limit the invention.

Abbreviations used in the examples are as follows: aq.=aqueous; RT.=room temperature; PE=petroleum ether (b.p. 50-70°); DMF=N,N=dimethylformamide; DMSO=dimethyl sulfoxide; THF=tetrahydrofuran.

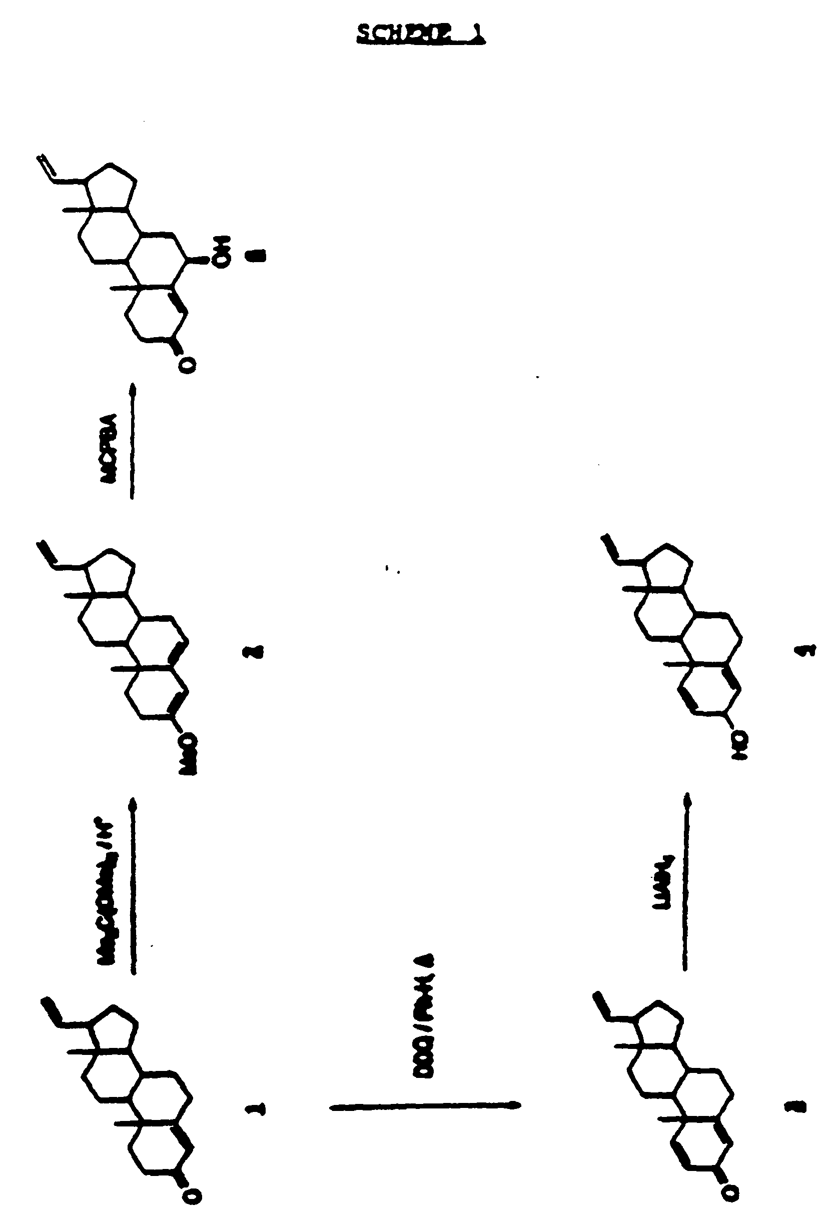

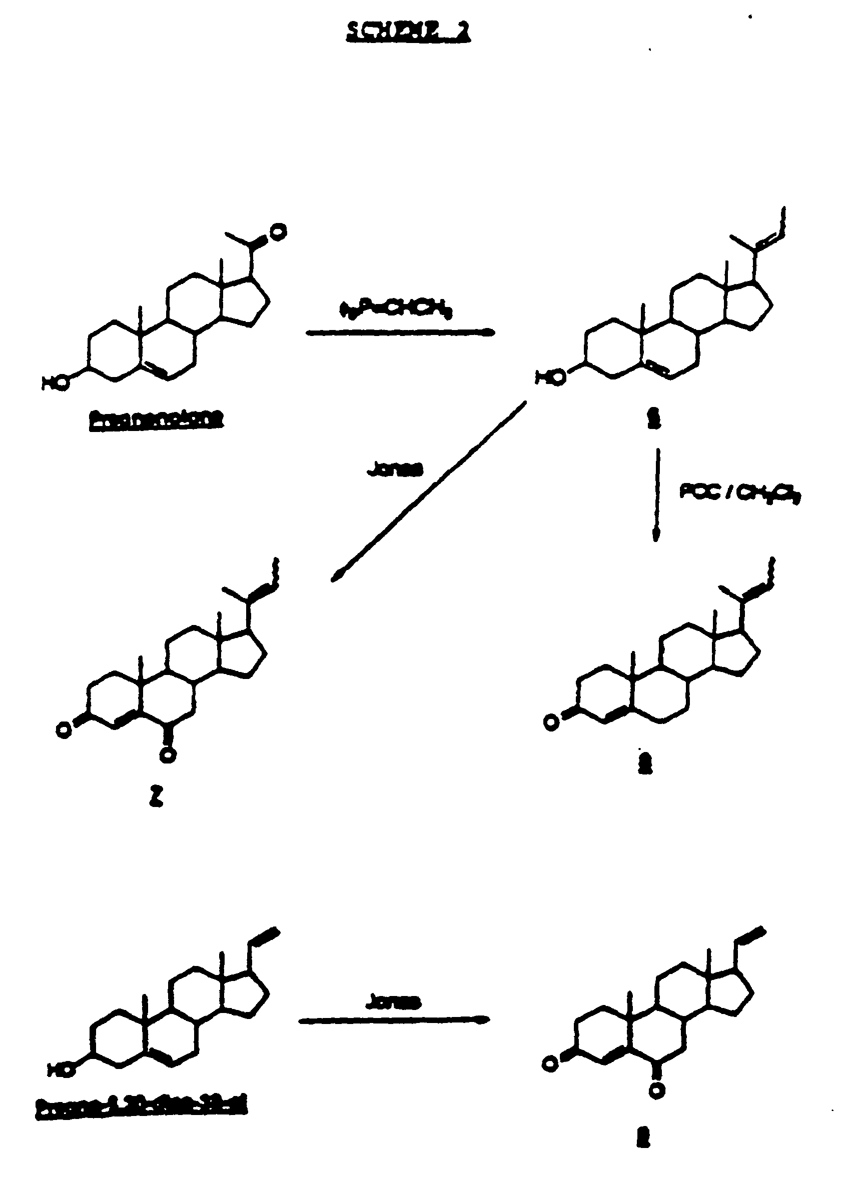

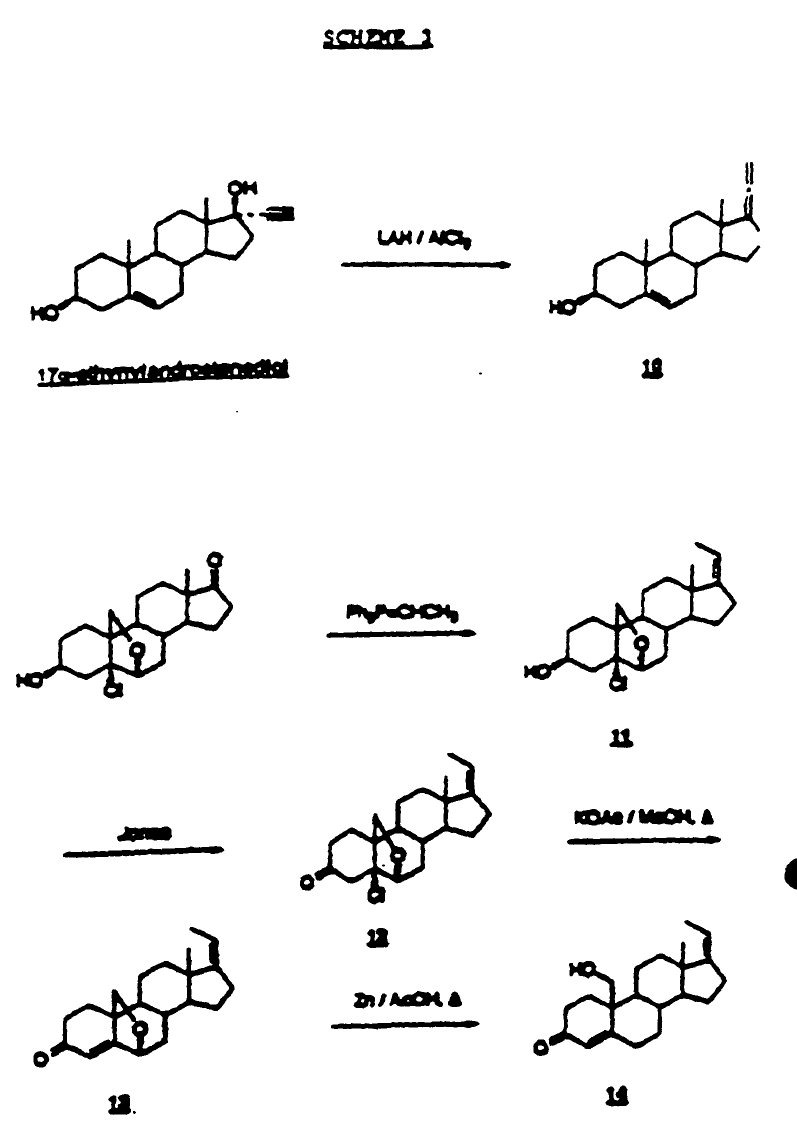

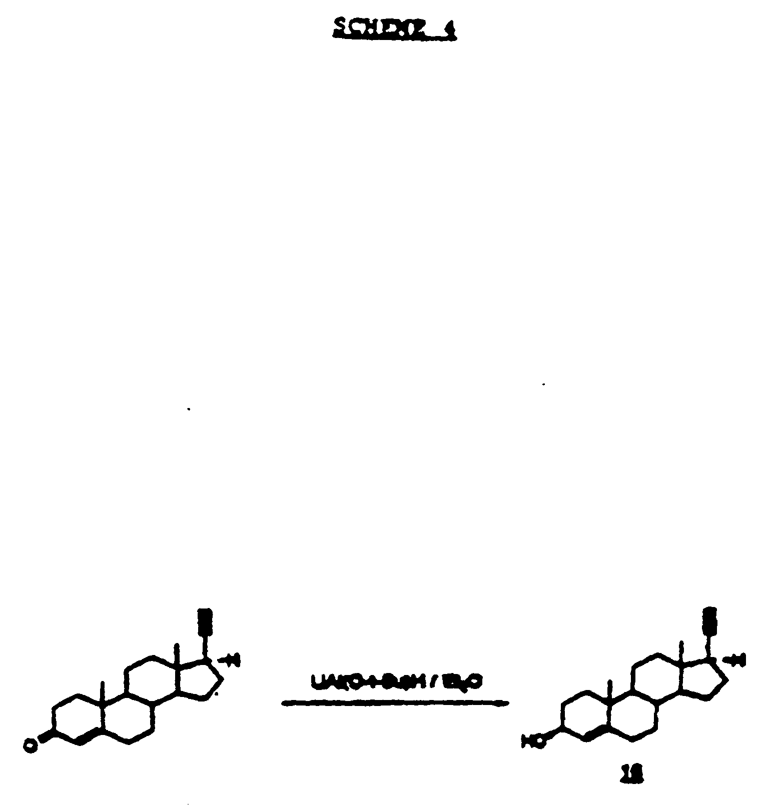

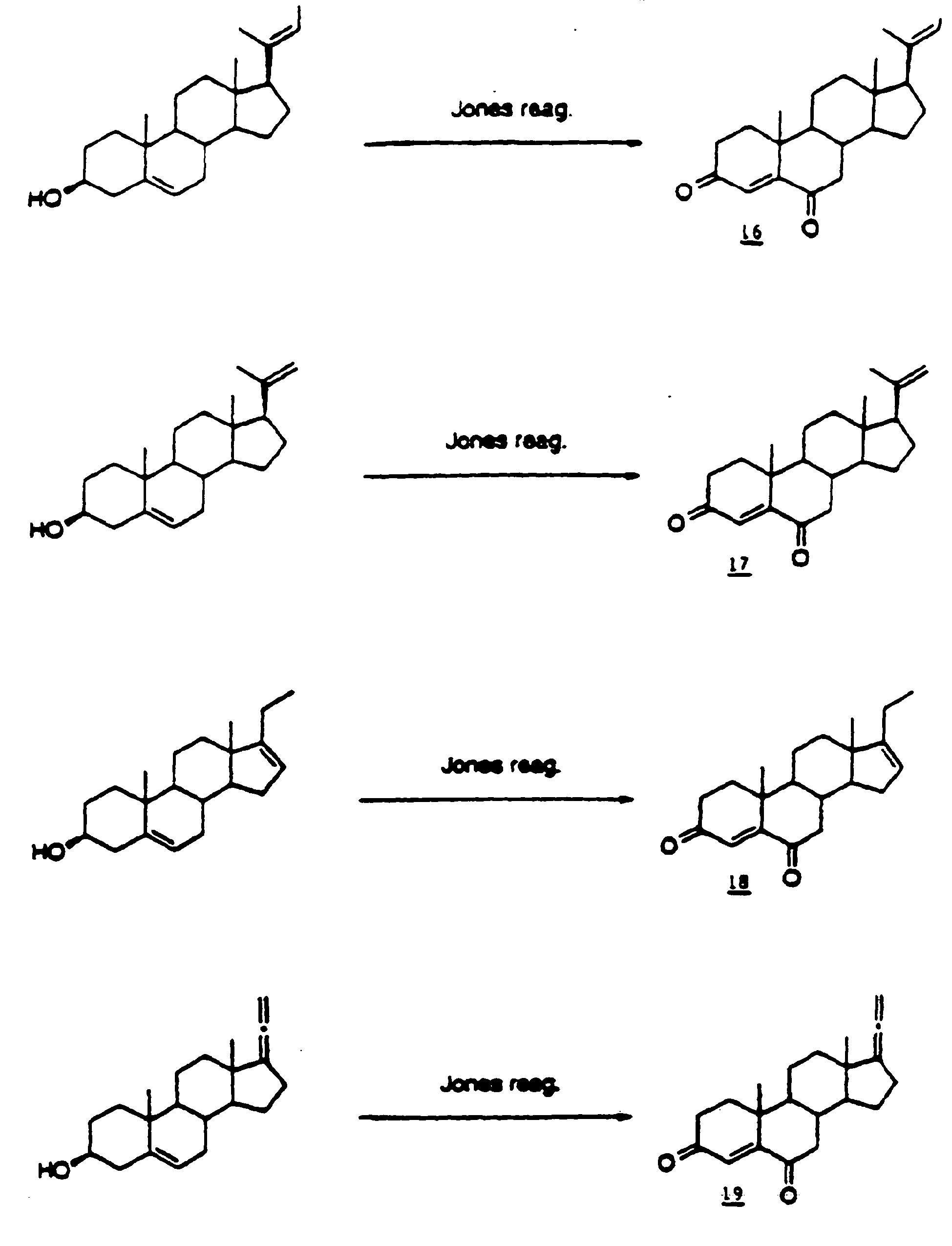

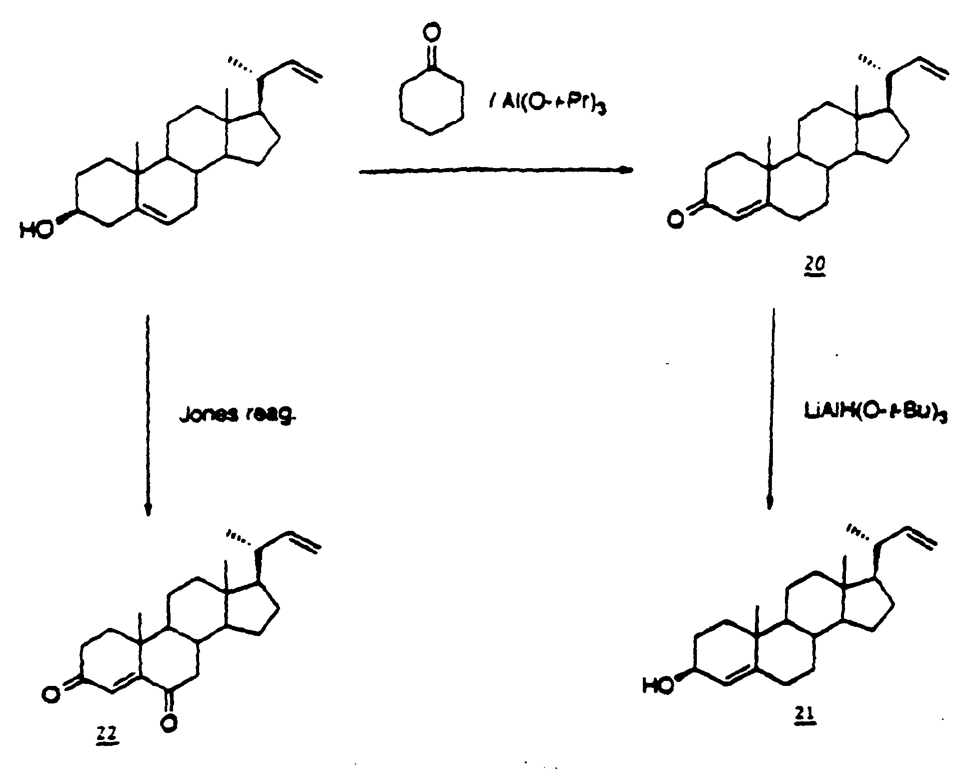

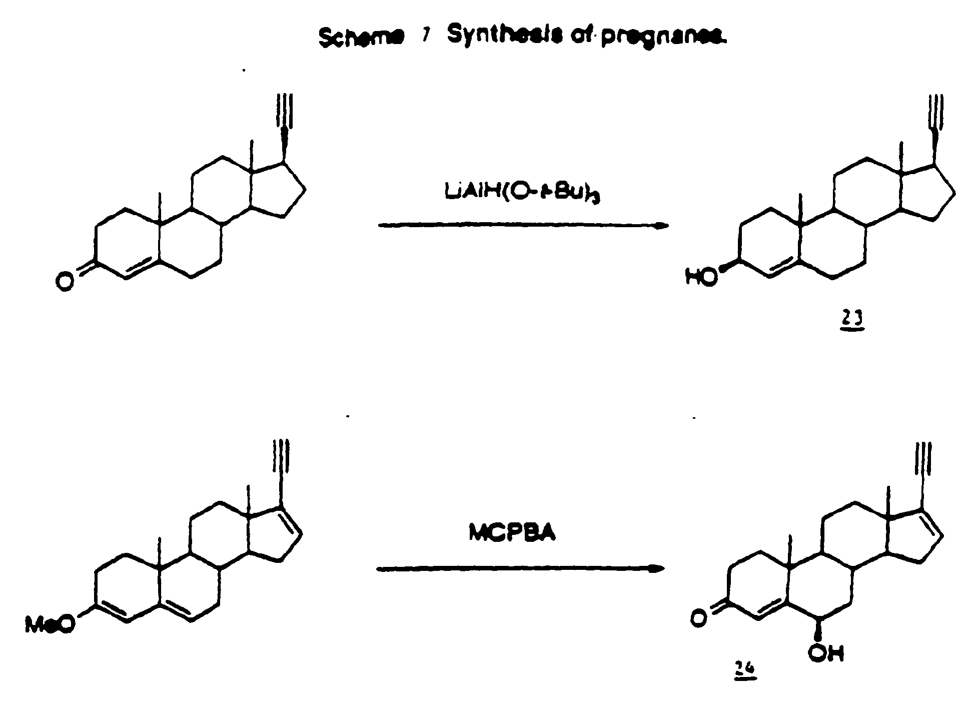

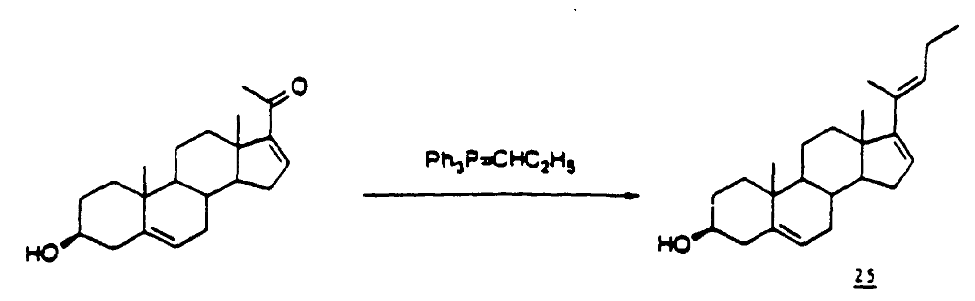

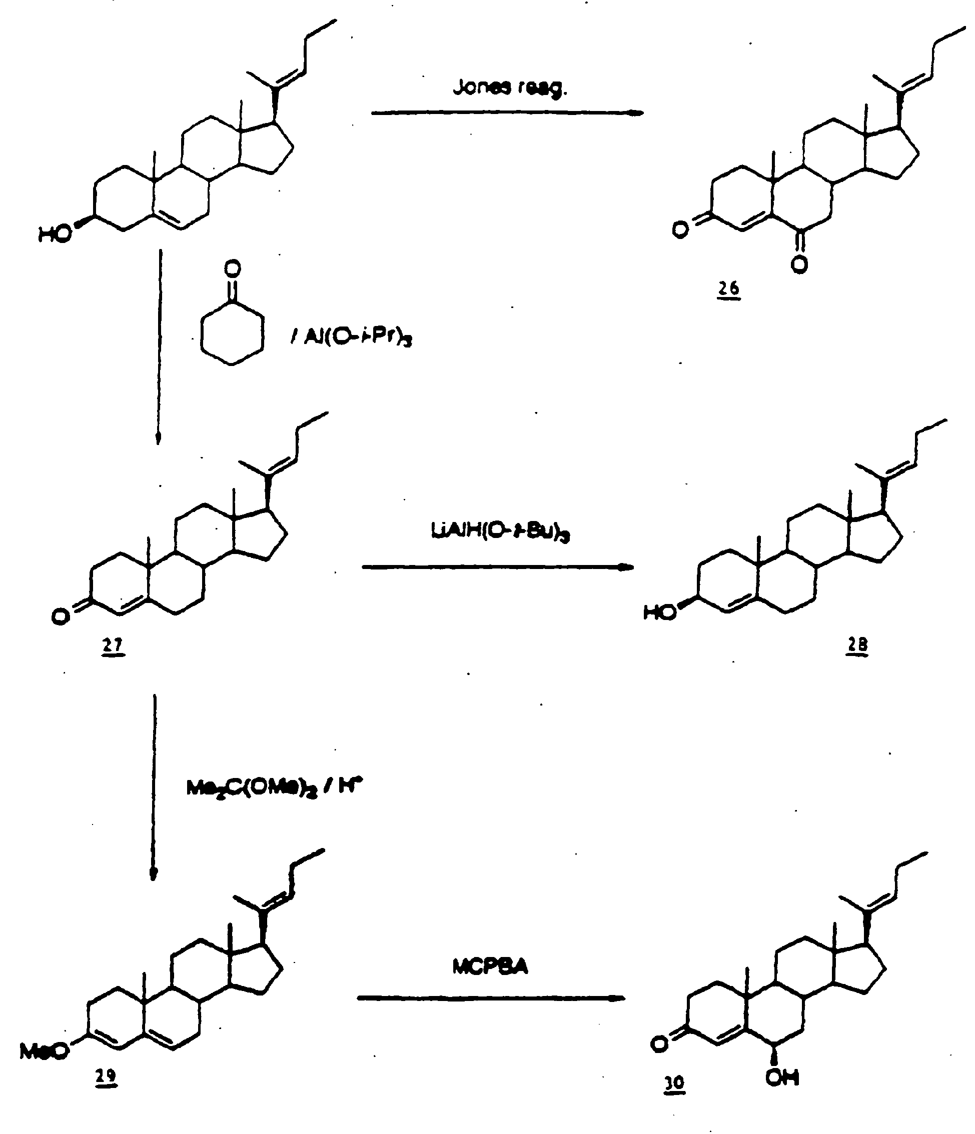

### Examples

Example 133 - 16α,17α-Epoxyestra-1,3,5 (10)-trien-3-ol, 2 (Scheme 15): To a solution of 1,3,5(10),16-estratetraen-3-ol (2, CAS No. [1150-90-9], 636.0 mg, 2.500 mmol) in 15 ml of 1,2-dimethoxyethane (DME) was added m-chloroperbenzoic acid (862.9) mg, 5,000 mmol) in 25 mL of DME over 3 minutes and the reaction was stirred for 6 h. The mixture was poured into 140 g of 5% (w/w) sodium thiosulfate pentahydrate and extracted three times with 100 mL portions of ethyl acetate. The combined organic extracts were washed with 100 mL of saturated sodium bicarbonate + three 100 mL aliquots of saturated sodium chloride, dried over magnesium sulfate, and filtered through Celite® 503. The residue was washed with 50 mL of ethyl acetate and the combined filtrates were concentrated under reduced pressure. Flash chromatography (20% ethyl acetate/hexanes on silica gel) of the resulting residue, followed by recystallization from ethyl acetate gave lustrous white platelets (349.9 mg, 1.294 mmol, 52%), m.p. 217-219°C (lit. [Prelog, V., Ruzicka, L., and Wieland, P., (1945), "Steroids and Sexualhormone", (111.Mitteilung). Uberein neues Stereoisomeres des Oestriols., Helv. Chem. Acta. 28:250-256] m.p. 215°C), homogeneous to TLC (20% ethyl acetate/hexanes on silica gel; R_{f} 0.32; starting material R_{f} 0.50).

### Example 134 - Estra-4,16-dien-10β-ol-3-one, 4 (Scheme 15):

To a solution of estra-5(10), 16-dien-3-one (3, 256.4 mg, 1.000 mmol) in 6 ml of 1,2-dimethoxyethane (DME) was added MCPBA (189.8 MG, 1.100 mmol) in 6 mL of DME + 2.4 mL of water. After stirring 1/2 h, the reaction mixture was poured into 30 g of 5% (w/w) sodium thiosulfate pentahydrate and extracted three times with 30 mL aliquots of ethyl acetate. The combined organic extracts were washed with 30 mL of saturated sodium bicarbonate + three 30 mL portions of brine, dried over magnesium sulfate, and filtered through diatomaceous earth. The residue was washed with 10 mL of ethyl acetate and the combined filtrates were concentrated under reduced pressure. To the resulting crystalline film was added 45 mL of 5% (w/v) potassium hydroxide in methanol and the mixture was refluxed with exclusion of moisture for 1 h, after which it was poured into 100 mL of ice water and extracted three times with 70 mL aliquots of ether. The combined ethereal extracts were washed three times with 70 mL portions of brine, dried over magnesium sulfate, and filtered through diatomaceous earth. The residue was washed with 25 mL of ether and the combined filtrates were concentrated under reduced pressure. Preparative TLC (50% ethyl acetate/hexanes on alumina GF, 1000 µ) of the residual resin, followed by recystallization from aqueous ethanol gave light yellow needles (62.4 mg, 0.229 mmol, 23%), m.p. 156-166°C. TLC (50% ethyl acetate/hexanes on silica gel; estrone R_{f} 0.59) showed a major product (R_{f} 0.44) with minor contaminants at R_{f} 0.62 and 0.73.

### Example 114A - 15α,17α-Epoxyestr-4-en-10β-ol-3-one,3 Scheme 15 :

A solution of estra-5(10), 16-dien-3-one was treated MCPSA as in Example 134, except that 4 equivalents of MCPBA are used instead of a slight excess of 1 equivalent. After refluxing, the resulting product in 5% (w/v) potassium hydroxide in methanol, and after work-up and purification as in Example 134, 16α,17α-epoxyestr-4-en-10β-ol-3-one was obtained.

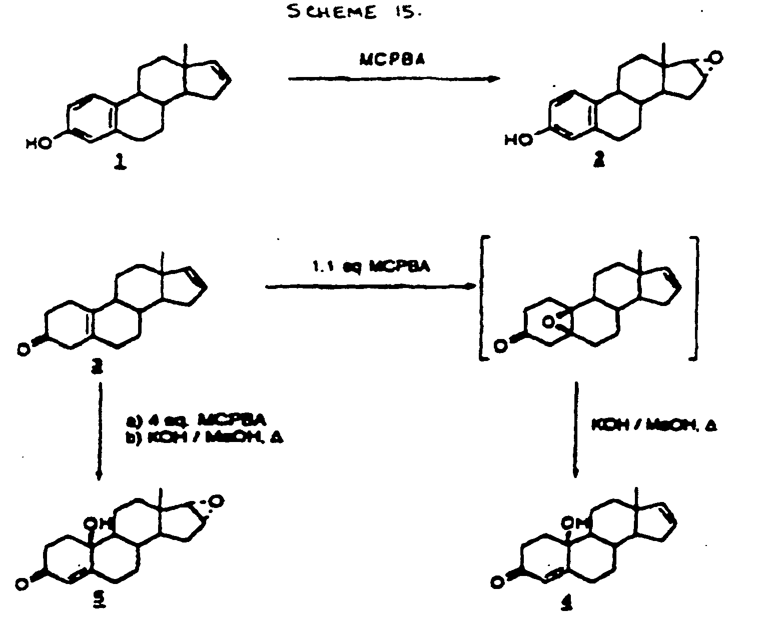

### Example 135A - 16α,17α-Epoxyestr-4-en-10β-ol-3-one,3 (Scheme 16):

Estra-5(10),16-dien-3-one (2, 270.4 mg, 1.055 mmol) in 6.7 mL of chloroform was cooled in an acetone/dry ice bath and 3-chloroperoxybenzoic acid (724.8 mg, 4.200 mmol) in 7.4 mL of ether was added. After stirring 2 h, the mixture was placed in the refrigerator. After 18 h, the mixture was poured into 60 g of 5% (w/w) sodium thiosulfate pentahydrate and was extracted three times with 25 mL aliquots of ethyl acetate. The combined organic extracts were washed with 25 mL of saturated sodium bicarbonate +25 mL of brine, dried over magnesium sulfate, and filtered through diatomaceous earth. The residue was washed with 10 mL of ethyl acetate and the combined filtrates were concentrated under reduced pressure. To the resulting white solid were added 50 mL of 5% (w/v) potassium hydroxide in methanol and the mixture was refluxed 1 h with exclusion of moisture. After cooling, the mixture was poured into 100 mL of ice water and extracted three times with 70 mL aliquots of ether. The combined organic extracts.were washed 3 times with 70 mL portions of brine, dried over magnesium sulfate, and filtered through diatomaceous earth. The residue was washed with 25 mL of ether and the combined filtrates were concentrated under reduced pressure. The resulting yellow resin was chromatographed twice on alumina GF preparative TLC plates (1000 µ) to give a white crystalline solid (55.1 mg, 0.191 mmol, 18%).

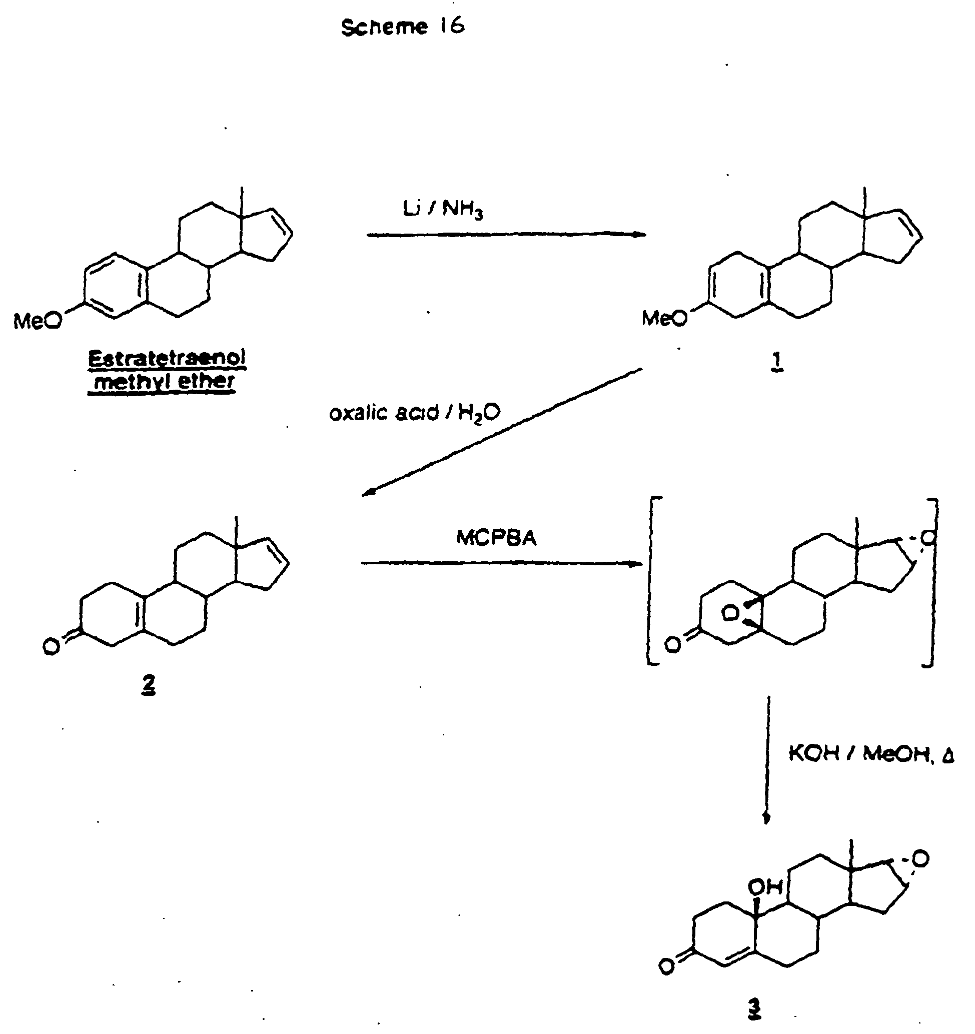

Example 143 - Electrophysiological studies were performed on the compounds of Examples 133, 134 and 134A in clinically normal adult females. The following chart summarizes the results. The data for the EVG and α-CA are shown in FIGS. 219 and 220, respectively.

Summary of effects of estrene vomeropherins on the human female VNO, autonomic activity, electromyogram, and alpha brain waves.

| | **EVG** | **RF** | **CF** | **EDA** | **BT** | **EMG** | **α-CA** |
|---|---|---|---|---|---|---|---|
| Example 133 | +++ | 0 | 0 | 0 | 0 | 0 | +++ |
| Example 134 | +++ | 0 | 0 | 0 | 0 | 0 | + |
| Example 134A | ++++ | 0 | 0 | 0 | 0 | 0 | 0 |

| | | | | | | | |
|---|---|---|---|---|---|---|---|
| Example 133: 16α,17α-Epoxyestra-1,3,5(10)-trien-3-ol Example 134: Estra-4,16-dien-10β-ol-3-one Example 134A: 16α,17α-Epoxyestr-4-en-10β-ol-3-one EVG = Electrovomeronasogram RF = Respiratory Frequency CF = Cardiac Frequency EDA = Electrodermal Activity BT = Body Temperature EMG = Electromyogram α-CA = Alpha Brain Waves 0 Compound showed an effect not significantly different from control + Arbitrary scale of effects ++ better than the control, correlated +++ to the significance of differences between ++++ the data (p-value) | | | | | | | |

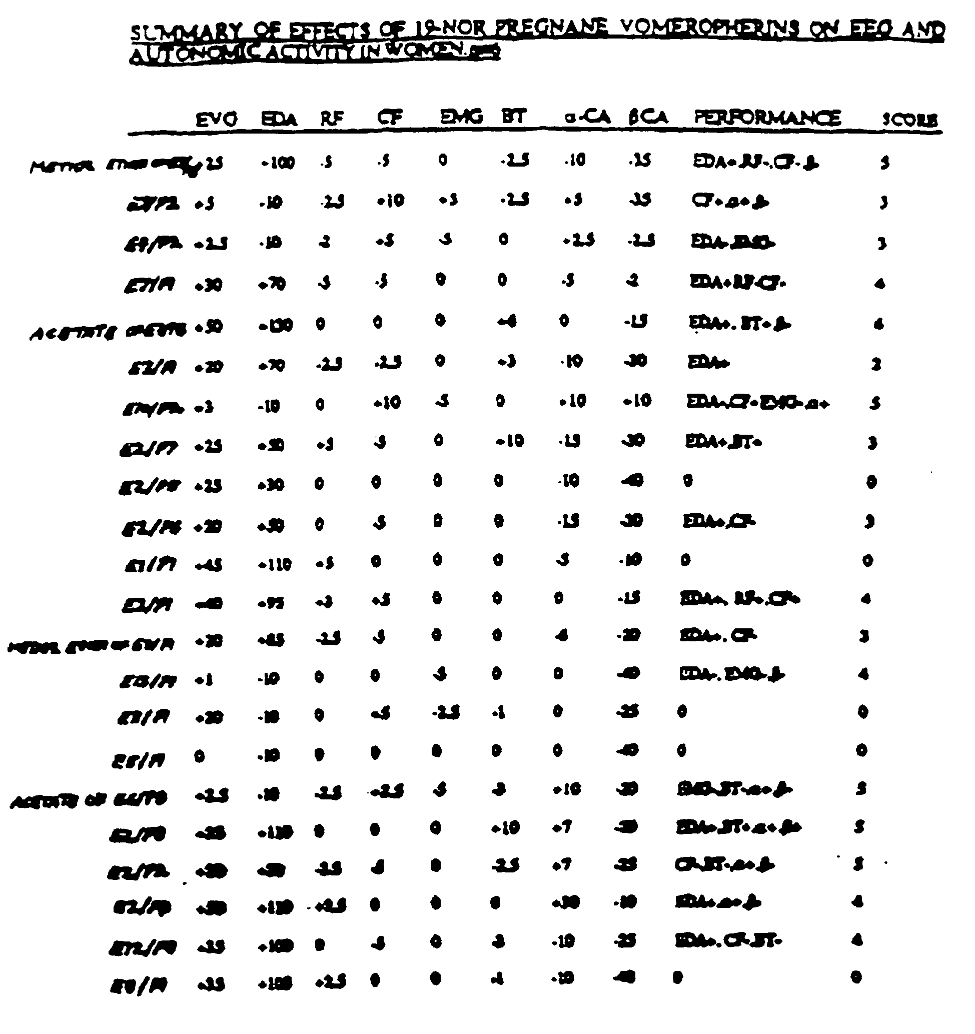

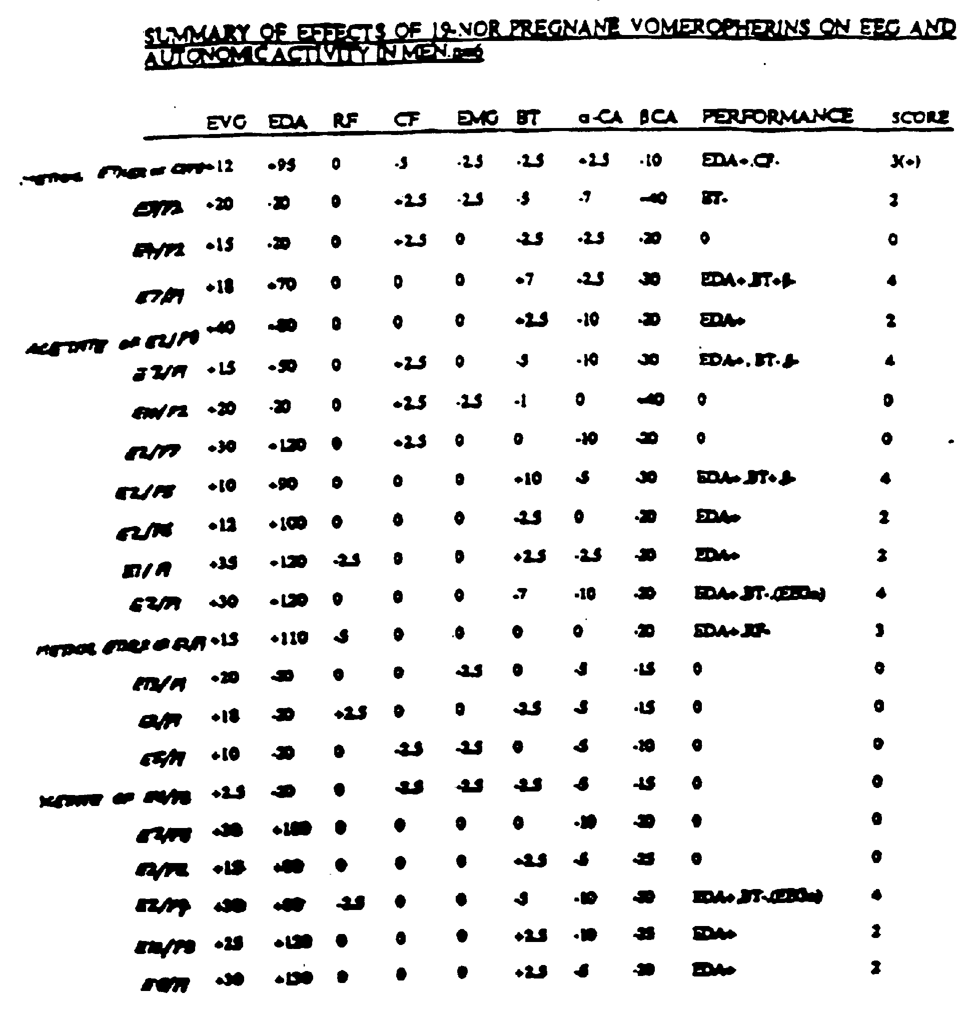

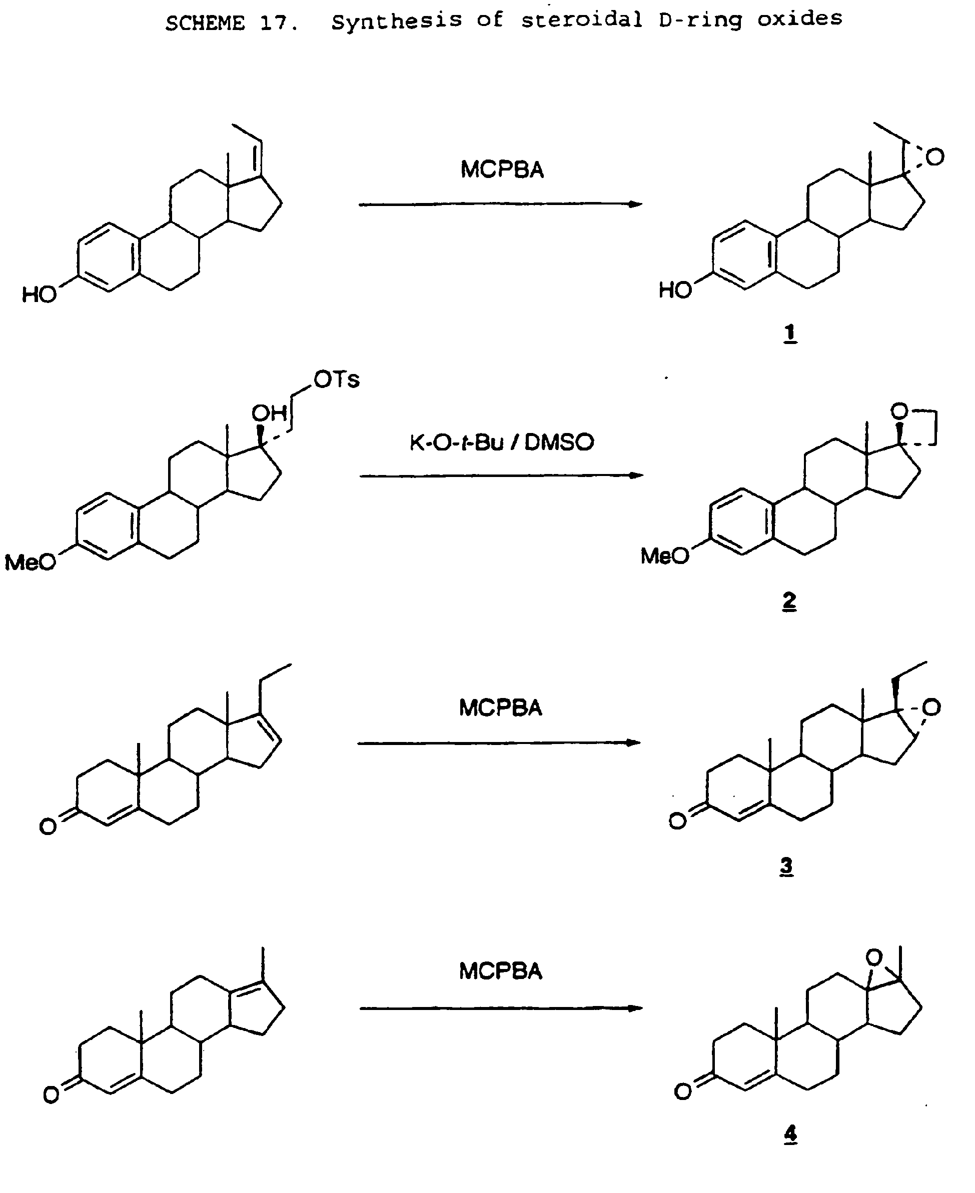

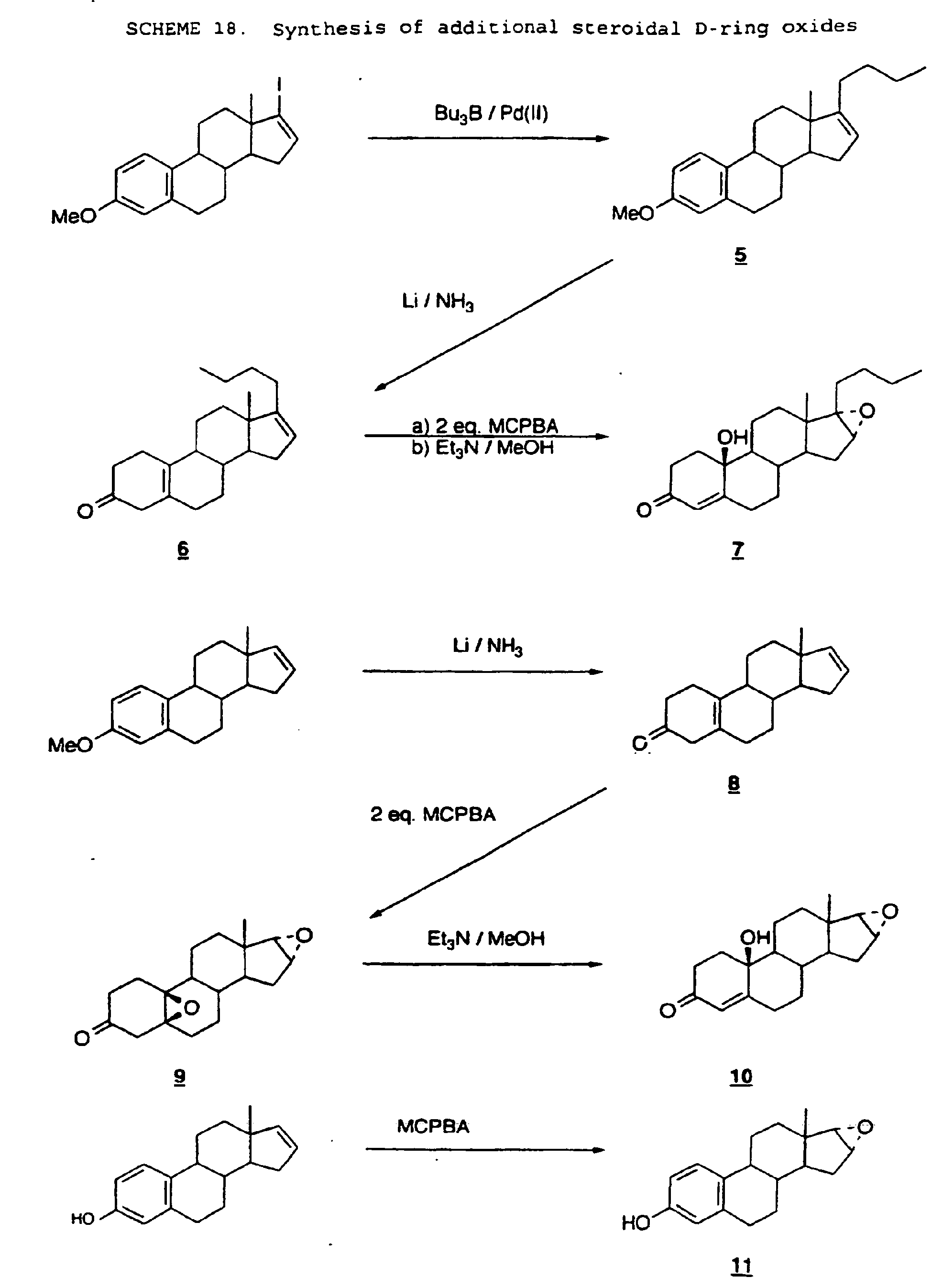

Example 144 - 17α,20α-Epoxy-19-norpregna-1,3,5(10)-trien-3-ol, 1: To a solution of 19-norpregna 1,3,5(10),17Z-tetraen-3-ol (282.4 mg, 0.9999 mmol) in 6 mL of 1,2-dimethoxyethane (DME) was added 3-chloroperoxybenzoic acid (92.2%, 207.1 mg, 1.200 mmol) in 6 mL of DME and the reaction was stirred 5 h. The mixture was poured into 50 g of 5% (w/w) sodium thiosulfate pentahydrate and extracted into three 20 mL portions of ethyl acetate. The combined organic extracts were washed with 20 mL of saturated sodium bicarbonate +20 mL of brine, dried over magnesium sulfate, and filtered through Celite 503. The residue was washed with 5 mL of ethyl acetate and the combined filtrates were concentrated under reduced pressure. Two-fold crystallization of the resulting white solid, first from methyl *t*-butyl ether (MTBE) / hexanes and then from aqueous ethanol, gave fine, lustrous, off-white platelets (160.0 ma, 0.5362 mmol, 54%), m.p. 154-159°C, homogeneous to TLC (25% ethyl acetate/hexanes on silica gel, product R_{f} 0.42, starting material R_{f} 0.57, 0.42 (trace)).

Example 146 - 16α,17α-Epoxypregn-4-en-3-one, 3: To a solution of pregna-4. 16 -dien-3 -one (31.0 ma, 0.104 mmol) in 0.6 mL of DME was added 3-chloroperoxybenzoic acid (92%, 21.5 ma, 0.125 mmol) in 0.6 mL of DME and the reaction was stirred 5 h. The mixture was subjected directly to preparative TLC (30% ethyl acetate/hexanes on silica gel GF, 1000 µ) to give a white crystalline solid (13.1 mg, 41.7 µmol, 40%). TLC (30% ethyl acetate/hexanes on silica gel; starting material R_{f} 0.53, 0.00 (trace)) showed product (R_{f} 0.33) with a trace contaminant (R_{f} 0.38).

### Example 147 - 13,17-Epoxy-10,17-dimethylgona-4-en-3-one, 4:

To a solution of 10,17-dimethylgona-4,13(17)-dien-3-one (342.1 ma, 1.265 mmol) in 7.6 mL of DME was added 3 - chloroperoxybenzoic acid (92%, 262.0 ma, 1.518 mmol) in 7.6 mL of DME. After stirring 5 h, the reaction mixture was poured into 60 g of 5% (w/w) sodium thiosulfate pentahydrate and then extracted three times with 30 mL portions of ethyl acetate. The combined organic extracts were washed with 30 mL of saturated sodium bicarbonate +30 mL of brine, dried over magnesium sulfate, and filtered through Celite 503. The residue was washed with 10 mL of ethyl acetate and combined filtrates were concentrated under reduced pressure. Flash chromatography (30% ethyl acetate/hexanes on silica gel) of the resulting yellow resin, followed by preparative TLC (30% ethyl acetate/hexanes on silica gel GF,1000 µ) gave a light yellow solid (87.2 ma, 0.304 mmol, 24%) showing 3 spots (R_{f} 0.23, 0.29, and 0.32) on TLC (30% ethyl acetate/hexanes on silica gel; pregna-4,16-dien-3-one R_{f} 0.54).

### Example 149 - 19,21-Bisnorchola-5(10), 16-dien-3-one, 6:

Approximately 30 mL of anh. NH, was distilled through KOH into a flame-dried 3-neck flask fitted with an inlet adapter, magnetic stirring bar, dry ice/acetone condenser, and glass stopper. 19,21-Bisnorchola-1,3,5(10),16-tetraen-3-yl methyl ether (5, 300.0 ma, 0.9245 mmol) in 8 mL of anh. THF and 3.09 g (41.7 mmol) of t-butyl alcohol was added, after which lithium wire (high sodium, 0.13 g,19 mg-atom) cut in small pieces was added. The reaction mixture was stirred h and then quenched with 1.0 mL of methanol. After allowing NH3 to boil off overnight, water (25 mL) was added and the reaction mixture was extracted 3 times with 25 mL aliquots of MTBE. The combined organic extracts were washed 3 times with 25 mL portions of brine, dried over magnesium sulfate, and filtered through Celite 503. The residue was washed with 10 mL of MTBE and the combined filtrates were concentrated under reduced pressure. The resulting syrup was taken up in 25 mL of acetone, oxalic acid dihydrate (0.37 g) in 5 mL of water was added, and the suspension was stirred 4 h. The hydrolysis mixture was poured into 10 mL of saturated sodium bicarbonate and extracted three times with 10 mL aliquots of MTBE. The combined organic extracts were washed 3 times with 10 mL portions of brine, dried over magnesium sulfate, and filtered through Celite 503. The residue was washed with 5 mL of MTBE and the combined filtrates were concentrated under reduced pressure. The residual syrup was taken up in 5 mL of hexanes, dried over magnesium sulfate, and filtered through Celite 503. The residue was washed with 2 mL of hexanes and the combined filtrates were boiled down to about 2 mL. The resulting solution was applied to a preparative TLC plate (silica gel GF, 1000 p) and developed with 10% ethyl acetate/hexanes. Extraction and concentration of the appropriate band gave a colorless syrup (226.4 ma, 0.7245 mmol, 78%). TLC (10% ethyl acetate/hexanes on silica gel) showed product (R_{f} 0.34) with a trace contaminant (R_{f} 0.14).

Example 150 - 16α,17α-Epoxy-19,21-bisnorchol-4-en-10β-ol-3-one, 7: To a cooled (dry ice/acetone) solution of 19,21-bisnorchola-5(10),16-dien-3-one (6, 270.2 ma, 0.8647 mmol) in 1.6 mL methylene chloride was added 3-chloroperoxybenzoic acid (97.4%,328.3 mg,1.902 mmol) in 6.3 mL of methylene chloride over 3 min. Remaining peracid was rinsed into the vessel with 1.8 mL of methylene chloride and the reaction was stirred 5 h. The cooling bath was removed and the reaction was stirred a further 1 h. The reaction was then filtered through Celite 503 and the residue was washed with 1 mL of methylene chloride. The combined filtrates were washed with 5 g of 5% (w/w) sodium thiosulfate pentahydrate +5 mL of saturated sodium bicarbonate +5 mL of brine, dried over magnesium sulfate, and filtered through Celite 503. The residue was washed with 1 mL of methylene chloride and the combined filtrates were concentrated under reduced pressure. The resulting light yellow solid was taken up in 22 mL of methanol with warming, 3.6.mL of triethylamine were added, and the reaction was stirred 2 h. The mixture was then concentrated under reduced pressure to give an amber syrup. This was purified by preparative TLC (50% ethyl acetate/hexanes on silica gel GF, 1000 µ) to produce a white crystalline solid (133.0 ma, 0.3861 mmol, 45%). TLC (50% ethyl acetate/hexanes on silica gel; 16α,17α-epoxyestr-en-10β-ol-3-one R_{f} 0.26) showed product (R_{f} 0.38) with a trace contaminant (R, 0.30).

### Example 153 - 16α,17α-Epoxyestr-4-en-10β-ol-3-one, 10:

5β,10β,16α,17α-Bisepoxyestran-3-one (9, 11.30 g, 39.18 mmol) was dissolved in 1 L of methanol with warming and triethylamine (166 mL, 1.19 mmol) was added. After stirring 6 h, the solution was concentrated under reduced pressure and the resulting yellow-tinged solid was taken up in 250 mL of hot 95% ethanol. Charcoal (Darco G-60, 0.58 g) was added and the mixture was heated to boiling. The hot suspension was filtered through Celite 503 and the residue was washed with 50 mL of hot ethanol. The combined filtrates were boiled down to approx. 150 mL and then cooled in tap water with swirling. The solution was seeded with crystals of "byproduct" and was then refrigerated overnight following onset of crystallization. The "byproduct" was filtered off and washed with 10 mL of cold ethanol (after drying the residue weighed 3.2762 g and had m.p. 262-269°C). The combined filtrates were boiled down to approx. 50 mL and then cooled in tap water with swirling. The solution was seeded with authentic product and then refrigerated overnight following onset of crystallization. The suspension was filtered and the residue dried over P2O5 in vacuo to give flat, white crystals (4.6827 g, 16.283 mmol, 41%), m.p. 193-195°C, homogeneous to TLC (50% ethyl acetate/hexanes on silica gel; product R_{f} 0.47; authentic sample R_{f} 0 47)

### Example 154 - 16α,17α-Epoxyestra-1,3,5(10)-trien-3-ol, 11:

To a solution of 1,3,5(10),16-estratetraen-3-ol (CAS No. 11150-90-91, 636.0 ma, 2.500 mmol) in 15 mL of DME was added mchloroperbenzoic acid (862.9 ma, 5.000 mmol) in 25 mL of DME over 3 min., and the reaction was stirred for 6 h. The mixture was poured into 140 g of 5% (w/w) sodium thiosulfate pentahydrate and extracted three times with 100 mL portions of ethyl acetate. The combined organic extracts were washed with 100 mL of saturated sodium bicarbonate + three 100 mL aliquots of saturated sodium chloride, dried over magnesium sulfate, and filtered through Celite 503. The residue was washed with 50 mL of ethyl acetate and the combined filtrates were concentrated under reduced pressure. Flash chromatography (20% ethyl acetate/hexanes on silica gel) of the resulting residue, followed by recrystallization from ethyl acetate gave lustrous white platelets (349.9 ma, 1.294 mmol, 52%), m.p. 217-219°C (lit. (Prelog, V., Ruzicka, L., and Wieland, P. (1945): Steroide und Sexualhormone. (111. Mitteilung). Über ein neues Stereoisomeres des Oestriols. Helv. Chim. Acta 28, 250-256.1 m.p. 215°C), homogeneous to TLC (20% ethyl acetate/hexanes on silica gel; R_{f} 0.32; starting material R_{f} 0.50).

Example 155 - Electrophysical studies were performed on the compounds of Examples 144, 146, 147, 150, 153 and 154, as well as two other gint-D epoxy steroids in clinically normal adult men and women. The results are summarized in FIGS. 221A and 221B.

The preparation of 17,20-EPOXYPREGN-4-EN-3-ONE is described by J. Hader and E. Blanke in British Patent 1,049,988 (1966).

The preparation of 16B,20B-EPOXYPREGN-4-EN-3B-OL is described by M. Matsui and D. Fukushima, *J. Org. Chem.* 35(3), 561 (1970).

The preparation of 18,20-EPOXYPREGN-4-EN-3-ONE is described by G. Cainelli, B. Kamber, J. Keller, M.Lj. Michailovic, D. Arigoni and O. Jeger, *Helv. Chim. Acta,* 62, 518 (1961).

16a,17a-EPOXYANDROST-4-EN-3-ONE (ANDROSTEINOXIDE) was prepared according to the literature procedure: F. Sondheimer, O. Mancera, M. Urquiza and G. Rosenkranz, *J. Chem. Soc.* 77, 4145 (1955).

17B,21-EPOXY-3-METHOXY-19-NOR-17a-PREGNA-1,3,5,(10)-TRIENE was prepared according to the literature procedure: B. Singh and R.G. Christiansen, *J. Pharm. Sci.* 60, 491 (1971).

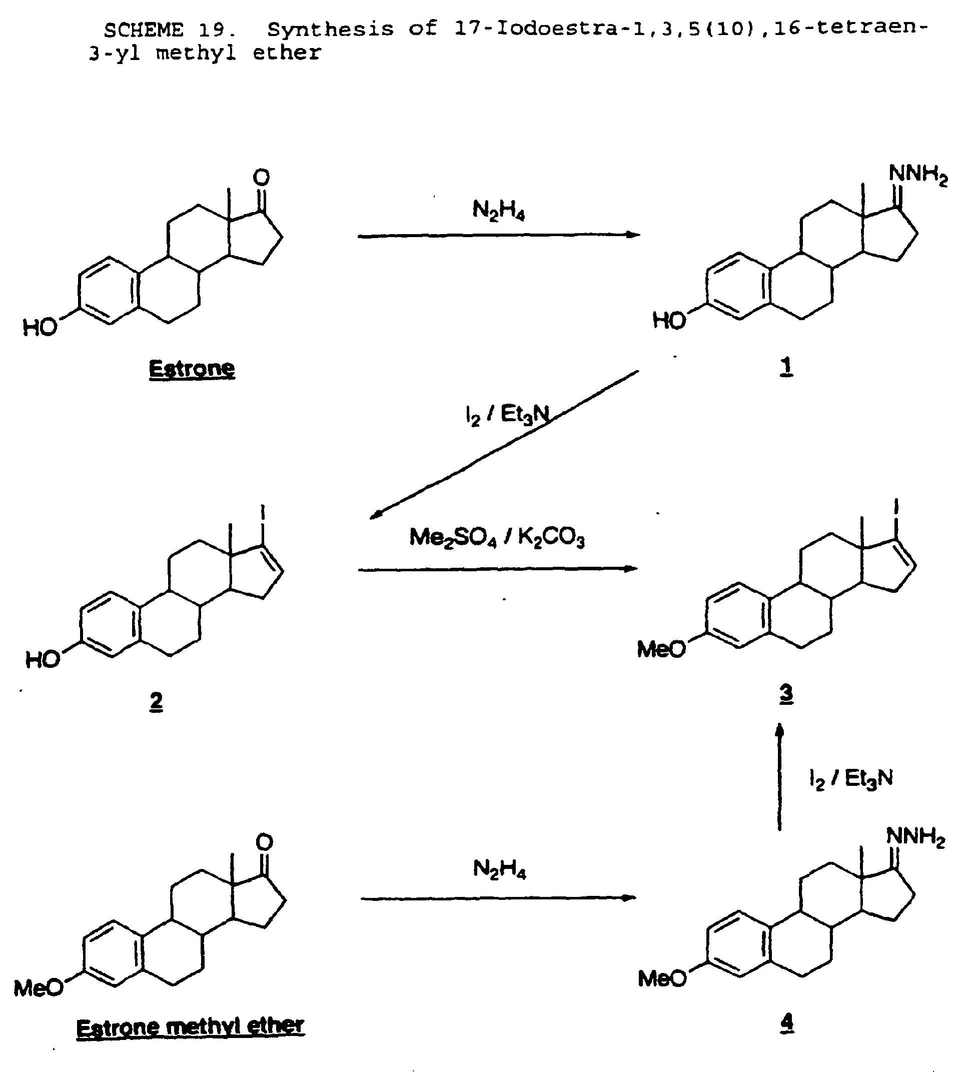

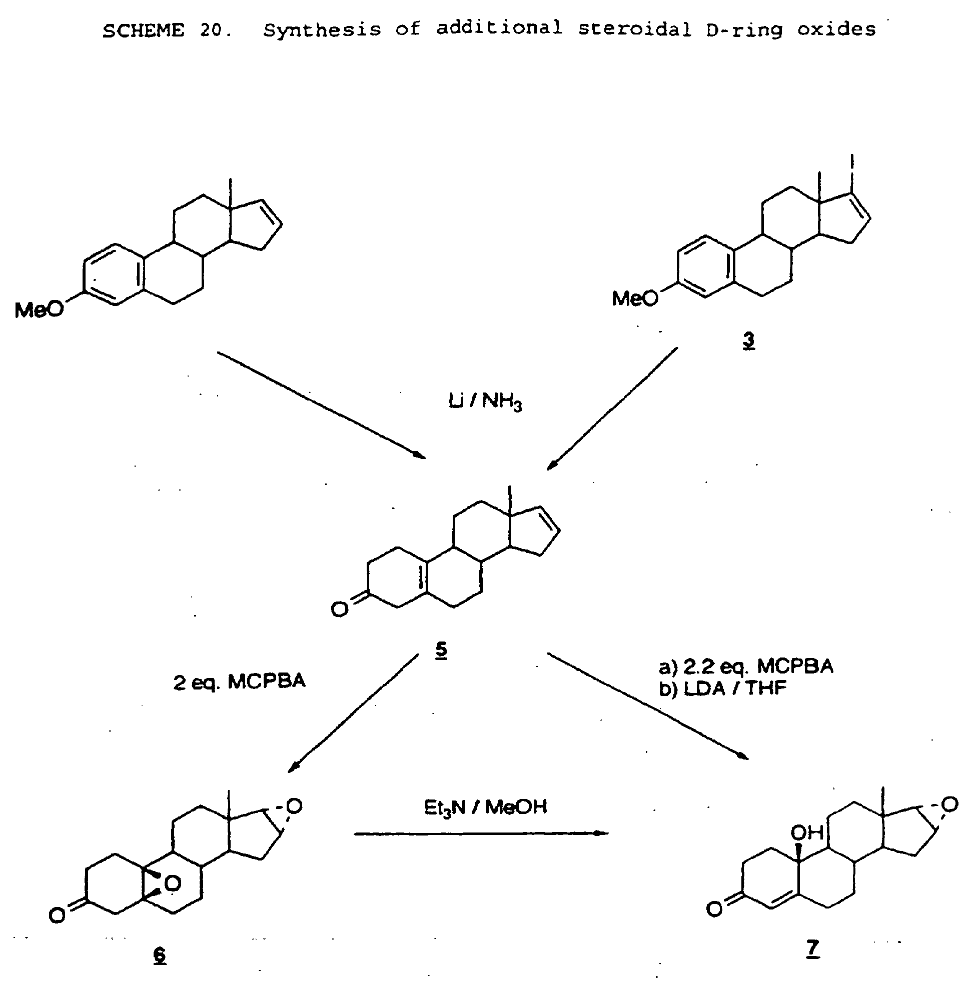

Example 165 - 16α,17α-Epoxyestr-4-en-10β-ol-3-one, 7, from estra-5(10),16-dien-3-one: To a solution of estra-5(10),16-dien-3-one (5, 331.8 ma, 1.294 mmol) in 6 mL of 1,2-dimethoxyethane (DME) was added 3-chloroperoxybenzoic acid (95.6%, 491.3 ma, 2.847 mmol) in 16 mL of DME. After stirring 24 h, the reaction mixture was poured into 15 g of 5% (w/w) sodium thiosulfate pentahydrate and extracted three times into 30 mL aliquots of ethyl acetate. The combined organic extracts were washed with 30 mL of saturated sodium bicarbonate + three 30 mL portions of brine, dried over magnesium sulfate, and filtered through Celite 503. The residue was washed with 15 mL of ethyl acetate and the combined filtrates were concentrated under reduced pressure. The resulting white solid was taken up in 20 mL of anh. THF under argon and cooled in an acetone/dry ice bath. Lithium diisopropylamide (LDA, 1.5 M in cyclohexane, 0.86 mL, 1.3 mmol) was added over 2 rain, and the reaction mixture was stirred a further 25 min. Saturated ammonium chloride (0.5 mL) and ether (30 mL) were added, and the mixture was washed three times with 10 mL portions of brine, dried over magnesium sulfate, and filtered through Celite 503. The residue was washed with 5 mL of ether and the combined filtrates were concentrated under reduced pressure. Flash chromatography (50% ethyl acetate/hexanes on silica gel 150) of the resulting moist yellow solid, followed by preparative TLC (50% ethyl acetate/ hexanes on silica gel OF, 1000 p) and recrystallization from methylene chloride/hexanes gave white crystals (47.0 ma, 0.163 mmol, 13%), m.p. 192-194°C, homogeneous to TLC (50% ethyl acetate/ hexanes on silica gel; product Rf 0.32; estrone R_{f} 0.59). UV: λmass 236 nm, ε = 13,000. IR: 3508 cm⁻¹ (OH str.), 1661 cm⁻¹ (unsat'd. C=O str.), 1620 cm⁻¹ (C=C str.). ¹H-NMR (in d₆acetone) : 5.₆₋δ (1H, s, H-4), 4.1 δ (1H, s, 10β-OH), 3.1-3.3 (2H, AB q, 160-H & 17,0-H), 0.82 δ (3H, s, 18-H). Anal.: Calculated for C₁₈H₂₄O₃: 74.96%, H 8.33%. Found: C 74.94 ± 0.06%, H 8.33 + 0.07%. HR-EIMS: Calculated for C₁₈H₂₄O₃: 288.172544. Found: 288.1726. 180).

### Example 166 - 16α, 17α-Epoxyestran-4-en-10β-ol-3-one, 7:

5β,10β,16α,17α-Bisepoxyestr-3-one (6, 11.30 g, 39.18 mmol) was dissolved in 1 L of methanol with warming, and triethylamine (166 mL, 1.19 mmol) was added. After stirring 6 h, the solution was concentrated under reduced pressure and the resulting yellow-tinged solid was taken up in 250 mL of hot 95% ethanol. Charcoal (Darco G-60, 0.58 g) was added and the mixture was heated to boiling. The hot suspension was filtered through Celite 503 and the residue was washed with 50 mL of hot ethanol. The combined filtrates were boiled down to approx. 150 mL and then cooled in tap water with swirling. The solution was seeded with crystals of byproduct and was then refrigerated overnight following onset of crystallization. The byproduct was filtered off and washed with 10 mL of cold ethanol (after drying, the byproduct residue weighed 3.2762 g and had m.p. 262-269°C). The combined filtrates were boiled down to approx. 50 mL and then cooled in tap water with swirling. The solution was seeded with authentic product and then refrigerated overnight following onset of crystallization. The suspension was filtered and the residue dried over P₂O₅ in vacuo to give flat, white crystals (4.6827 g, 16.283 mmol, 41%), m.p. 193- 195°C, homogeneous to TLC (50% ethyl acetate/hexanes on silica gel; product R_{f} 0.47; authentic sample R_{f} 0.47).

## Claims

1. The use of a steroid compound in the manufacture of a medicament for treating premenstrual dysphoric disorders in an individual, wherein said medicament is adapted for nasal administration and said steroid compound is an estrene having the formula: 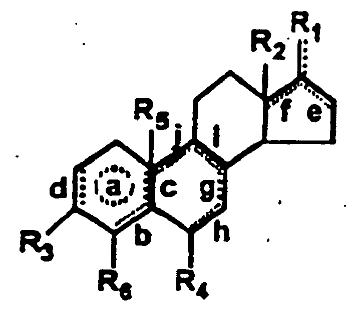 wherein R₁ is selected from the group consisting of one or two hydrogen atoms, methyl, methylene, and one or two halo atoms; R₂ is absent or is selected from the group consisting of hydrogen and methyl; R₃ is selected from the group consisting of oxo, hydroxy, lower alkoxy, lower acyloxy, benzoyl, cypionyl, glucuronide and sulfonyl; R₄ is selected from the group consisting of hydrogen, hydroxy, lower alkoxy, lower acyloxy and halo; R₅ is absent or is selected from the group consisting of hydrogen, hydroxy, lower alkoxy and lower acyloxy; R₆ is a hydrogen or a halo; and "a" represents optional aromatic unsaturation of ring A of said steroid, or "b", "c", and "d" are each optional double bonds; "e", "f", "g", "h", "i" and "j" are each optional double bonds; and "e" forms an epoxy ring with C₁₆ and C₁₇.

2. The use according to Claim 1 wherein said estrene is 16α,17α-epoxy-estra-1,3,5(10)-trien-3-ol.

3. The use according to Claim 1 wherein said estrene is 16α,17α-epoxy-estr-4-en-10β-ol-3-one.

4. A steroid selected from 16α,17α-epoxy-estr-4-en-10β-ol-3-one; 17α,20α-epoxy-19-norpregna-1,3,5(10)-trien-3-ol; 16α,17α-epoxypregn-4-en-3-one; 13,17-epoxy-10,17-dimethylgon-4-en-3-one; and 16α,17α-epoxy-19,21-bisnorchol-4-en-10β-ol-3-one.

## Patentansprüche

1. Verwendung einer Steroid-Verbindung bei der Herstellung eines Medikaments zur Behandlung von prämenstruellen dysphorischen Störungen in einem Individuum, wobei das Medikament für die nasale Verabreichung angepasst ist und die Steroid-Verbindung ein Estren mit der Formel: 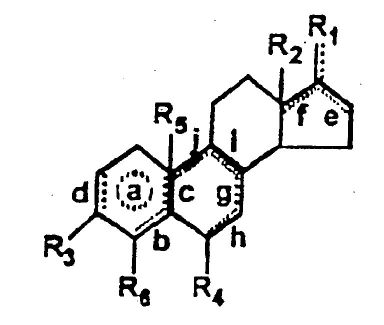 ist, in der R₁ ausgewählt ist aus der Gruppe bestehend aus einem oder zwei Wasserstoffatomen, Methyl, Methylen und einem oder zwei Halogenatomen; R₂ abwesend ist oder ausgewählt ist aus der Gruppe bestehend aus Wasserstoff und Methyl; R₃ ausgewählt ist aus der Gruppe bestehend aus Oxo, Hydroxy, Niederalkoxy, Niederacyloxy, Benzoyl, Cipionyl, Glucuronid und Sulfonyl; R₄ ausgewählt ist aus der Gruppe bestehend aus Wasserstoff, Hydroxy, Niederalkoxy, Niederacyloxy und Halogen; R₅ abwesend ist oder ausgewählt ist aus der Gruppe bestehend aus Wasserstoff, Hydroxy, Niederalkoxy und Niederacyloxy; R₆ ein Wasserstoff oder ein Halogen ist; und "a" eine fakultative aromatische Unsättigung des Rings A des Steroids ist oder "b", "c" und "d" jeweils fakultative Doppelbindungen sind; "e", "f", "g", "h", "i" und "j" jeweils fakultative Doppelbindungen sind; und "e" einen Epoxyring mit C₁₆ und C₁₇ bildet.

2. Verwendung nach Anspruch 1, bei der das Estren 16α,17α-Epoxyestra-1,3,5(10)-trien-3-ol ist.

3. Verwendung nach Anspruch 1, bei der das Estren 16α,17α-Epoxyestr-4-en-10β-ol-3-on ist.

4. Steroid, ausgewählt aus 16α,17α-Epoxyestr-4-en-10β-ol-3-on; 17α,20α-Epoxy-19-norpregna-1,3,5(10)-trien-3-ol; 16α,17α-Epoxypregn-4-en-3-on; 13,17-Epoxy-10,17-dimethylgon-4-en-3-on; und 16α,17α-Epoxy-19,21-bisnorchol-4-en-10β-ol-3-on.

## Revendications

1. Emploi d'un stéroïde dans la fabrication d'un médicament destiné au traitement du syndrome prémenstruel dysphorique chez un individu, médicament adapté pour administration nasale, lequel stéroïde est un oestrène de formule 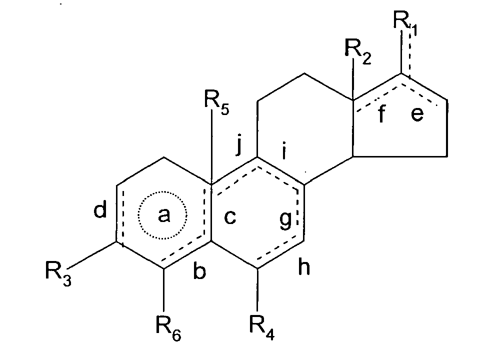 dans laquelle
- R¹ représente une entité choisie parmi un seul atome ou deux atomes d'hydrogène, un groupe méthyle, un groupe méthylène, et un seul atome ou deux atomes d'halogène,
- R² ne représente rien ou représente un atome d'hydrogène ou un groupe méthyle,
- R³ représente une entité choisie parmi les substituants oxo, hydroxy, alcoxy inférieurs, acyloxy inférieurs, benzoyle, cypionyle (cyclopentylpropionyle), glucuronide et sulfonyle,
- R⁴ représente une entité choisie parmi les atomes d'hydrogène et d'halogène et les groupes hydroxyle, alcoxy inférieurs et acyloxy inférieurs,
- R⁵ ne représente rien ou représente une entité choisie parmi un atome d'hydrogène et les groupes hydroxyle, alcoxy inférieurs et acyloxy inférieurs,
- R⁶ représente un atome d'hydrogène ou d'halogène,
- "a" indique l'insaturation aromatique éventuelle du cycle A dudit stéroïde ou "b", "c" et "d" indiquent chacun la présence éventuelle d'une double liaison,
- "e", "f", "g", "h", "i" et "j" indiquent chacun la présence éventuelle d'une double liaison, "e" pouvant aussi indiquer la présence d'un cycle époxy formé avec les atomes de carbone n° 16 et 17.

2. Emploi conforme à la revendication 1, dans lequel ledit oestrène est le 16α,17α-époxy-oestra-1,3,5(10)-trièn-3-ol.

3. Emploi conforme à la revendication 1, dans lequel ledit oestrène est la 16α,17α-époxy-oestr-4-èn-10β-ol-3-one.

4. Stéroïde choisi parmi les suivants :
- 16α,17α-époxy-oestr-4-èn-10β-ol-3-one ;
- 17α,20α-époxy-19-nor-prégna-1,3,5(10)-trièn-3-ol ;
- 16α,17α-époxy-prégn-4-èn-3-one ;
- 13,17-époxy-10,17-diméthyl-gon-4-èn-3-one ;
- et 16α,17α-époxy-19,21-bisnor-chol-4-èn-10β-ol-3-one.
